# EUROPEAN PATENT APPLICATION

(11) **EP 3 480 319 A1**
(43) Date of publication of application: **08.05.2019**
(21) Application number: 17819589.7
(22) Date of filing: 03.04.2017
(51) Int. Cl.: C12Q 1/68, C12Q 1/6806, C12N 15/10, C40B 40/06, C40B 50/06

(54) **METHOD FOR PRODUCING DNA LIBRARY AND METHOD FOR ANALYZING GENOMIC DNA USING DNA LIBRARY**

(30) Priority: 29.06.2016 JP 2016129048; 13.09.2016 JP 2016178528; 31.03.2017 JP 2017071020
(71) Applicant: TOYOTA JIDOSHA KABUSHIKI KAISHA, Toyota-shi, Aichi-ken, 471-8571 (JP)
(72) Inventor: ENOKI, Hiroyuki, Toyota-shi, Aichi-ken, 471-8571 (JP); TAKEUCHI, Yoshie, Toyota-shi, Aichi-ken, 471-8571 (JP)
(74) Representative: Winter, Brandl, Fürniss, Hübner, Röss, Kaiser, Polte - Partnerschaft mbB
(86) International application number: PCT/JP2017/013965
(87) International publication number: WO 2018/003220

(57) **Abstract**

A DNA library with excellent reproducibility is readily produced. A nucleic acid amplification reaction is conducted in a reaction solution containing genomic DNA and a random primer at a high concentration to obtain a DNA fragment by the nucleic acid amplification reaction using the genomic DNA as a template.

SEQUENCE LISTING

<110>  TOYOTA JIDOSHA KABUSHIKI KAISHA

<120>  A method for producing a DNA library and a method for analyzing
       genomic DNA using the DNA library

<130>  PH-6724-PCT

<150> JP 2016-129048
<151> 2016-06-29

<150> JP 2016-178528
<151> 2016-09-13

<150> JP 2017-071020
<151> 2017-03-31

<160>  2059

<170>  PatentIn version 3.5

<210>  1
<211>  10
<212>  DNA
<213>  Artificial

<220>
<223>  synthetic DNA

<400>  1
agacgtcgtt                                                              10


<210>  2
<211>  10
<212>  DNA
<213>  Artificial

<220>
<223>  synthetic DNA

<400>  2
gaggcgatat                                                              10


<210>  3
<211>  10
<212>  DNA
<213>  Artificial

<220>
<223>  synthetic DNA

<400>  3
gtgcgaacgt                                                              10


<210>  4
<211>  10
<212>  DNA
<213>  Artificial

<220>
<223>  synthetic DNA

<400>  4
ttatactgcc                                                              10


<210>  5
<211>  10
<212>  DNA
<213>  Artificial

<220>
<223>  synthetic DNA

<400>  5
caagttcgca                                                              10


<210>  6
<211>  10
<212>  DNA
<213>  Artificial

<220>
<223>  synthetic DNA

<400>  6
acaaggtagt                                                              10


<210>  7
<211>  10
<212>  DNA
<213>  Artificial

<220>
<223>  synthetic DNA

<400>  7
acacagcgac                                                              10


<210>  8
<211>  10
<212>  DNA
<213>  Artificial

<220>
<223>  synthetic DNA

<400>  8
ttaccgatgt                                                              10


<210>  9
<211>  10
<212>  DNA
<213>  Artificial

<220>
<223>  synthetic DNA

<400>  9
cacagagtcg                                                              10


<210>  10
<211>  10
<212>  DNA
<213>  Artificial

<220>
<223>  synthetic DNA

<400>  10
ttcagcgcgt                                                              10


<210>  11
<211>  10
<212>  DNA
<213>  Artificial

<220>
<223>  synthetic DNA

<400>  11
aggaccgtga                                                              10


<210>  12
<211>  10
<212>  DNA
<213>  Artificial

<220>
<223>  synthetic DNA

<400>  12
gtctgttcgc                                                              10


<210>  13
<211>  10
<212>  DNA
<213>  Artificial

<220>
<223>  synthetic DNA

<400>  13
acctgtccac                                                              10


<210>  14
<211>  10
<212>  DNA
<213>  Artificial

<220>
<223>  synthetic DNA

<400>  14
ccgcaatgac                                                              10

<210>  15
<211>  10
<212>  DNA
<213>  Artificial

<220>
<223>  synthetic DNA

<400>  15
ctgccgatca                                                              10


<210>  16
<211>  10
<212>  DNA
<213>  Artificial

<220>
<223>  synthetic DNA

<400>  16
tacacggagc                                                              10


<210>  17
<211>  10
<212>  DNA
<213>  Artificial

<220>
<223>  synthetic DNA

<400>  17
ccgcattcat                                                              10


<210>  18
<211>  10
<212>  DNA
<213>  Artificial

<220>
<223>  synthetic DNA

<400>  18
gactctagac                                                              10


<210>  19
<211>  10
<212>  DNA
<213>  Artificial

<220>
<223>  synthetic DNA

<400>  19
ggagaactta                                                              10


<210>  20
<211>  10

<212>  DNA
<213>  Artificial

<220>
<223>  synthetic DNA

<400>  20
tccggtatgc                                                              10


<210>  21
<211>  10
<212>  DNA
<213>  Artificial

<220>
<223>  synthetic DNA

<400>  21
ggtcaggagt                                                              10


<210>  22
<211>  10
<212>  DNA
<213>  Artificial

<220>
<223>  synthetic DNA

<400>  22
acattggcag                                                              10


<210>  23
<211>  10
<212>  DNA
<213>  Artificial

<220>
<223>  synthetic DNA

<400>  23
cgtagactgc                                                              10


<210>  24
<211>  10
<212>  DNA
<213>  Artificial

<220>
<223>  synthetic DNA

<400>  24
agactgtact                                                              10


<210>  25
<211>  10
<212>  DNA
<213>  Artificial

<220>
<223>  synthetic DNA

<400>  25
tagacgcagt                                                              10


<210>  26
<211>  10
<212>  DNA
<213>  Artificial

<220>
<223>  synthetic DNA

<400>  26
ccgataatct                                                              10


<210>  27
<211>  10
<212>  DNA
<213>  Artificial

<220>
<223>  synthetic DNA

<400>  27
gagagctagt                                                              10


<210>  28
<211>  10
<212>  DNA
<213>  Artificial

<220>
<223>  synthetic DNA

<400>  28
gtaccgcgtt                                                              10


<210>  29
<211>  10
<212>  DNA
<213>  Artificial

<220>
<223>  synthetic DNA

<400>  29
gacttgcgca                                                              10


<210>  30
<211>  10
<212>  DNA
<213>  Artificial

<220>
<223>  synthetic DNA

<400>  30
cgtgattgcg                                                              10


<210>  31
<211>  10
<212>  DNA
<213>  Artificial

<220>
<223>  synthetic DNA

<400>  31
atcgtctctg                                                              10


<210>  32
<211>  10
<212>  DNA
<213>  Artificial

<220>
<223>  synthetic DNA

<400>  32
cgtagctacg                                                              10


<210>  33
<211>  10
<212>  DNA
<213>  Artificial

<220>
<223>  synthetic DNA

<400>  33
gccgaatagt                                                              10


<210>  34
<211>  10
<212>  DNA
<213>  Artificial

<220>
<223>  synthetic DNA

<400>  34
gtacctaggc                                                              10


<210>  35
<211>  10
<212>  DNA
<213>  Artificial

<220>
<223>  synthetic DNA

<400>  35
gcttacatga                                                              10

<210>  36
<211>  10
<212>  DNA
<213>  Artificial

<220>
<223>  synthetic DNA

<400>  36
tccacgtagt                                                              10


<210>  37
<211>  10
<212>  DNA
<213>  Artificial

<220>
<223>  synthetic DNA

<400>  37
agaggccatc                                                              10


<210>  38
<211>  10
<212>  DNA
<213>  Artificial

<220>
<223>  synthetic DNA

<400>  38
cggtgatgct                                                              10


<210>  39
<211>  10
<212>  DNA
<213>  Artificial

<220>
<223>  synthetic DNA

<400>  39
cactgtgctt                                                              10


<210>  40
<211>  10
<212>  DNA
<213>  Artificial

<220>
<223>  synthetic DNA

<400>  40
catgatggct                                                              10


<210>  41
<211>  10

<212>  DNA
<213>  Artificial

<220>
<223>  synthetic DNA

<400>  41
gccacacatg                                                              10


<210>  42
<211>  10
<212>  DNA
<213>  Artificial

<220>
<223>  synthetic DNA

<400>  42
cacacactgt                                                              10


<210>  43
<211>  10
<212>  DNA
<213>  Artificial

<220>
<223>  synthetic DNA

<400>  43
cagaatcata                                                              10


<210>  44
<211>  10
<212>  DNA
<213>  Artificial

<220>
<223>  synthetic DNA

<400>  44
atcgtctacg                                                              10


<210>  45
<211>  10
<212>  DNA
<213>  Artificial

<220>
<223>  synthetic DNA

<400>  45
cgagcaatac                                                              10


<210>  46
<211>  10
<212>  DNA
<213>  Artificial

<220>
<223>  synthetic DNA

<400>  46
acaagcgcac                                                              10


<210>  47
<211>  10
<212>  DNA
<213>  Artificial

<220>
<223>  synthetic DNA

<400>  47
gcttagatgt                                                              10


<210>  48
<211>  10
<212>  DNA
<213>  Artificial

<220>
<223>  synthetic DNA

<400>  48
tgcattctgg                                                              10


<210>  49
<211>  10
<212>  DNA
<213>  Artificial

<220>
<223>  synthetic DNA

<400>  49
tgtcggacca                                                              10


<210>  50
<211>  10
<212>  DNA
<213>  Artificial

<220>
<223>  synthetic DNA

<400>  50
aggcactcgt                                                              10


<210>  51
<211>  10
<212>  DNA
<213>  Artificial

<220>
<223>  synthetic DNA

<400>  51
ctgcatgtga                                                              10


<210>  52
<211>  10
<212>  DNA
<213>  Artificial

<220>
<223>  synthetic DNA

<400>  52
accacgccta                                                              10


<210>  53
<211>  10
<212>  DNA
<213>  Artificial

<220>
<223>  synthetic DNA

<400>  53
gaggtcgtac                                                              10


<210>  54
<211>  10
<212>  DNA
<213>  Artificial

<220>
<223>  synthetic DNA

<400>  54
aatactctgt                                                              10


<210>  55
<211>  10
<212>  DNA
<213>  Artificial

<220>
<223>  synthetic DNA

<400>  55
tgccaactga                                                              10


<210>  56
<211>  10
<212>  DNA
<213>  Artificial

<220>
<223>  synthetic DNA

<400>  56
cctgttcggt                                                              10

<210>  57
<211>  10
<212>  DNA
<213>  Artificial

<220>
<223>  synthetic DNA

<400>  57
gtagagagtt                                                              10


<210>  58
<211>  10
<212>  DNA
<213>  Artificial

<220>
<223>  synthetic DNA

<400>  58
tacagcgtaa                                                              10


<210>  59
<211>  10
<212>  DNA
<213>  Artificial

<220>
<223>  synthetic DNA

<400>  59
tgacgtgatg                                                              10


<210>  60
<211>  10
<212>  DNA
<213>  Artificial

<220>
<223>  synthetic DNA

<400>  60
agacgtcggt                                                              10


<210>  61
<211>  10
<212>  DNA
<213>  Artificial

<220>
<223>  synthetic DNA

<400>  61
cgctaggttc                                                              10


<210>  62
<211>  10

<212>  DNA
<213>  Artificial

<220>
<223>  synthetic DNA

<400>  62
gccttatagc                                                              10


<210>  63
<211>  10
<212>  DNA
<213>  Artificial

<220>
<223>  synthetic DNA

<400>  63
ccttcgatct                                                              10


<210>  64
<211>  10
<212>  DNA
<213>  Artificial

<220>
<223>  synthetic DNA

<400>  64
aggcaacgtg                                                              10


<210>  65
<211>  10
<212>  DNA
<213>  Artificial

<220>
<223>  synthetic DNA

<400>  65
tgagcggtgt                                                              10


<210>  66
<211>  10
<212>  DNA
<213>  Artificial

<220>
<223>  synthetic DNA

<400>  66
gtgtcgaacg                                                              10


<210>  67
<211>  10
<212>  DNA
<213>  Artificial

<220>
<223>  synthetic DNA

<400>  67
cgatgttgcg                                                              10


<210>  68
<211>  10
<212>  DNA
<213>  Artificial

<220>
<223>  synthetic DNA

<400>  68
aacaagacac                                                              10


<210>  69
<211>  10
<212>  DNA
<213>  Artificial

<220>
<223>  synthetic DNA

<400>  69
gatgctggtt                                                              10


<210>  70
<211>  10
<212>  DNA
<213>  Artificial

<220>
<223>  synthetic DNA

<400>  70
accggtagtc                                                              10


<210>  71
<211>  10
<212>  DNA
<213>  Artificial

<220>
<223>  synthetic DNA

<400>  71
gtgactagca                                                              10


<210>  72
<211>  10
<212>  DNA
<213>  Artificial

<220>
<223>  synthetic DNA

<400>  72
agcctatatt                                                              10


<210>  73
<211>  10
<212>  DNA
<213>  Artificial

<220>
<223>  synthetic DNA

<400>  73
tcgtgagctt                                                              10


<210>  74
<211>  10
<212>  DNA
<213>  Artificial

<220>
<223>  synthetic DNA

<400>  74
acactatggc                                                              10


<210>  75
<211>  10
<212>  DNA
<213>  Artificial

<220>
<223>  synthetic DNA

<400>  75
gactctgtcg                                                              10


<210>  76
<211>  10
<212>  DNA
<213>  Artificial

<220>
<223>  synthetic DNA

<400>  76
tcgatgatgc                                                              10


<210>  77
<211>  10
<212>  DNA
<213>  Artificial

<220>
<223>  synthetic DNA

<400>  77
cttggacact                                                              10

<210>  78
<211>  10
<212>  DNA
<213>  Artificial

<220>
<223>  synthetic DNA

<400>  78
ggctgatcgt                                                              10


<210>  79
<211>  10
<212>  DNA
<213>  Artificial

<220>
<223>  synthetic DNA

<400>  79
actcacaggc                                                              10


<210>  80
<211>  10
<212>  DNA
<213>  Artificial

<220>
<223>  synthetic DNA

<400>  80
atgtgcgtac                                                              10


<210>  81
<211>  10
<212>  DNA
<213>  Artificial

<220>
<223>  synthetic DNA

<400>  81
caccatcgat                                                              10


<210>  82
<211>  10
<212>  DNA
<213>  Artificial

<220>
<223>  synthetic DNA

<400>  82
agccattaac                                                              10


<210>  83
<211>  10

<212>  DNA
<213>  Artificial

<220>
<223>  synthetic DNA

<400>  83
aatcgactgt                                                              10


<210>  84
<211>  10
<212>  DNA
<213>  Artificial

<220>
<223>  synthetic DNA

<400>  84
aatactagcg                                                              10


<210>  85
<211>  10
<212>  DNA
<213>  Artificial

<220>
<223>  synthetic DNA

<400>  85
tcgtcactga                                                              10


<210>  86
<211>  10
<212>  DNA
<213>  Artificial

<220>
<223>  synthetic DNA

<400>  86
caggctctta                                                              10


<210>  87
<211>  10
<212>  DNA
<213>  Artificial

<220>
<223>  synthetic DNA

<400>  87
ggtcggtgat                                                              10


<210>  88
<211>  10
<212>  DNA
<213>  Artificial

<220>
<223>  synthetic DNA

<400>  88
cattaggcgt                                                              10


<210>  89
<211>  10
<212>  DNA
<213>  Artificial

<220>
<223>  synthetic DNA

<400>  89
actcgcgagt                                                              10


<210>  90
<211>  10
<212>  DNA
<213>  Artificial

<220>
<223>  synthetic DNA

<400>  90
ttccgaataa                                                              10


<210>  91
<211>  10
<212>  DNA
<213>  Artificial

<220>
<223>  synthetic DNA

<400>  91
tgagcatcgt                                                              10


<210>  92
<211>  10
<212>  DNA
<213>  Artificial

<220>
<223>  synthetic DNA

<400>  92
gccacgtaac                                                              10


<210>  93
<211>  10
<212>  DNA
<213>  Artificial

<220>
<223>  synthetic DNA

<400>  93
gaactacatg                                                              10


<210>  94
<211>  10
<212>  DNA
<213>  Artificial

<220>
<223>  synthetic DNA

<400>  94
tcgtgaggac                                                              10


<210>  95
<211>  10
<212>  DNA
<213>  Artificial

<220>
<223>  synthetic DNA

<400>  95
gcggccttaa                                                              10


<210>  96
<211>  10
<212>  DNA
<213>  Artificial

<220>
<223>  synthetic DNA

<400>  96
gctaaggacc                                                              10


<210>  97
<211>  10
<212>  DNA
<213>  Artificial

<220>
<223>  synthetic DNA

<400>  97
atagccatta                                                              10


<210>  98
<211>  10
<212>  DNA
<213>  Artificial

<220>
<223>  synthetic DNA

<400>  98
cagtaatcat                                                              10

<210>  99
<211>  10
<212>  DNA
<213>  Artificial

<220>
<223>  synthetic DNA

<400>  99
actccttaat                                                              10


<210>  100
<211>  10
<212>  DNA
<213>  Artificial

<220>
<223>  synthetic DNA

<400>  100
tcgaacatta                                                              10


<210>  101
<211>  10
<212>  DNA
<213>  Artificial

<220>
<223>  synthetic DNA

<400>  101
attatgaggt                                                              10


<210>  102
<211>  10
<212>  DNA
<213>  Artificial

<220>
<223>  synthetic DNA

<400>  102
aatcttagag                                                              10


<210>  103
<211>  10
<212>  DNA
<213>  Artificial

<220>
<223>  synthetic DNA

<400>  103
ttagatgatg                                                              10


<210>  104
<211>  10

<212>  DNA
<213>  Artificial

<220>
<223>  synthetic DNA

<400>  104
tacatatctg                                                              10


<210>  105
<211>  10
<212>  DNA
<213>  Artificial

<220>
<223>  synthetic DNA

<400>  105
tccttaatca                                                              10


<210>  106
<211>  10
<212>  DNA
<213>  Artificial

<220>
<223>  synthetic DNA

<400>  106
gttgagatta                                                              10


<210>  107
<211>  10
<212>  DNA
<213>  Artificial

<220>
<223>  synthetic DNA

<400>  107
tgttaacgta                                                              10


<210>  108
<211>  10
<212>  DNA
<213>  Artificial

<220>
<223>  synthetic DNA

<400>  108
catacagtaa                                                              10


<210>  109
<211>  10
<212>  DNA
<213>  Artificial

<220>
<223>  synthetic DNA

<400>  109
cttatacgaa                                                              10


<210>  110
<211>  10
<212>  DNA
<213>  Artificial

<220>
<223>  synthetic DNA

<400>  110
agatctatgt                                                              10


<210>  111
<211>  10
<212>  DNA
<213>  Artificial

<220>
<223>  synthetic DNA

<400>  111
aagacttagt                                                              10


<210>  112
<211>  10
<212>  DNA
<213>  Artificial

<220>
<223>  synthetic DNA

<400>  112
tgcgcaataa                                                              10


<210>  113
<211>  10
<212>  DNA
<213>  Artificial

<220>
<223>  synthetic DNA

<400>  113
ttggccatat                                                              10


<210>  114
<211>  10
<212>  DNA
<213>  Artificial

<220>
<223>  synthetic DNA

<400>  114
tattacgagg                                                              10


<210>  115
<211>  10
<212>  DNA
<213>  Artificial

<220>
<223>  synthetic DNA

<400>  115
ttatgatcgc                                                              10


<210>  116
<211>  10
<212>  DNA
<213>  Artificial

<220>
<223>  synthetic DNA

<400>  116
aacttaggag                                                              10


<210>  117
<211>  10
<212>  DNA
<213>  Artificial

<220>
<223>  synthetic DNA

<400>  117
tcacaatcgt                                                              10


<210>  118
<211>  10
<212>  DNA
<213>  Artificial

<220>
<223>  synthetic DNA

<400>  118
gagtatatgg                                                              10


<210>  119
<211>  10
<212>  DNA
<213>  Artificial

<220>
<223>  synthetic DNA

<400>  119
atcaggacaa                                                              10

<210>  120
<211>  10
<212>  DNA
<213>  Artificial

<220>
<223>  synthetic DNA

<400>  120
gtactgatag                                                              10


<210>  121
<211>  10
<212>  DNA
<213>  Artificial

<220>
<223>  synthetic DNA

<400>  121
cttatactcg                                                              10


<210>  122
<211>  10
<212>  DNA
<213>  Artificial

<220>
<223>  synthetic DNA

<400>  122
taacggacta                                                              10


<210>  123
<211>  10
<212>  DNA
<213>  Artificial

<220>
<223>  synthetic DNA

<400>  123
gcgttgtata                                                              10


<210>  124
<211>  10
<212>  DNA
<213>  Artificial

<220>
<223>  synthetic DNA

<400>  124
cttaagtgct                                                              10


<210>  125
<211>  10

<212>  DNA
<213>  Artificial

<220>
<223>  synthetic DNA

<400>  125
atacgactgt                                                              10


<210>  126
<211>  10
<212>  DNA
<213>  Artificial

<220>
<223>  synthetic DNA

<400>  126
actgttatcg                                                              10


<210>  127
<211>  10
<212>  DNA
<213>  Artificial

<220>
<223>  synthetic DNA

<400>  127
aatcttgacg                                                              10


<210>  128
<211>  10
<212>  DNA
<213>  Artificial

<220>
<223>  synthetic DNA

<400>  128
acatcacctt                                                              10


<210>  129
<211>  10
<212>  DNA
<213>  Artificial

<220>
<223>  synthetic DNA

<400>  129
ggtatagtac                                                              10


<210>  130
<211>  10
<212>  DNA
<213>  Artificial

<220>
<223>  synthetic DNA

<400>  130
ctaatccaca                                                              10


<210>  131
<211>  10
<212>  DNA
<213>  Artificial

<220>
<223>  synthetic DNA

<400>  131
gcaccttatt                                                              10


<210>  132
<211>  10
<212>  DNA
<213>  Artificial

<220>
<223>  synthetic DNA

<400>  132
attgacggta                                                              10


<210>  133
<211>  10
<212>  DNA
<213>  Artificial

<220>
<223>  synthetic DNA

<400>  133
gacatatggt                                                              10


<210>  134
<211>  10
<212>  DNA
<213>  Artificial

<220>
<223>  synthetic DNA

<400>  134
gatagtcgta                                                              10


<210>  135
<211>  10
<212>  DNA
<213>  Artificial

<220>
<223>  synthetic DNA

<400>  135
caattatcgc                                                              10


<210>  136
<211>  10
<212>  DNA
<213>  Artificial

<220>
<223>  synthetic DNA

<400>  136
cttaggtgat                                                              10


<210>  137
<211>  10
<212>  DNA
<213>  Artificial

<220>
<223>  synthetic DNA

<400>  137
catactactg                                                              10


<210>  138
<211>  10
<212>  DNA
<213>  Artificial

<220>
<223>  synthetic DNA

<400>  138
taacgcgaat                                                              10


<210>  139
<211>  10
<212>  DNA
<213>  Artificial

<220>
<223>  synthetic DNA

<400>  139
caagttacga                                                              10


<210>  140
<211>  10
<212>  DNA
<213>  Artificial

<220>
<223>  synthetic DNA

<400>  140
aatctcaagg                                                              10

<210>  141
<211>  10
<212>  DNA
<213>  Artificial

<220>
<223>  synthetic DNA

<400>  141
gcaatcatca                                                              10


<210>  142
<211>  10
<212>  DNA
<213>  Artificial

<220>
<223>  synthetic DNA

<400>  142
tgtaacgttc                                                              10


<210>  143
<211>  10
<212>  DNA
<213>  Artificial

<220>
<223>  synthetic DNA

<400>  143
tatcgttggt                                                              10


<210>  144
<211>  10
<212>  DNA
<213>  Artificial

<220>
<223>  synthetic DNA

<400>  144
cgcttaagat                                                              10


<210>  145
<211>  10
<212>  DNA
<213>  Artificial

<220>
<223>  synthetic DNA

<400>  145
ttagaactgg                                                              10


<210>  146
<211>  10

<212>  DNA
<213>  Artificial

<220>
<223>  synthetic DNA

<400>  146
gtcataacgt                                                              10


<210>  147
<211>  10
<212>  DNA
<213>  Artificial

<220>
<223>  synthetic DNA

<400>  147
agagcagtat                                                              10


<210>  148
<211>  10
<212>  DNA
<213>  Artificial

<220>
<223>  synthetic DNA

<400>  148
caacatcact                                                              10


<210>  149
<211>  10
<212>  DNA
<213>  Artificial

<220>
<223>  synthetic DNA

<400>  149
cagaagctta                                                              10


<210>  150
<211>  10
<212>  DNA
<213>  Artificial

<220>
<223>  synthetic DNA

<400>  150
aactaacgtg                                                              10


<210>  151
<211>  10
<212>  DNA
<213>  Artificial

<220>
<223>  synthetic DNA

<400>  151
ttataccgct                                                              10


<210>  152
<211>  10
<212>  DNA
<213>  Artificial

<220>
<223>  synthetic DNA

<400>  152
gaattcgaga                                                              10


<210>  153
<211>  10
<212>  DNA
<213>  Artificial

<220>
<223>  synthetic DNA

<400>  153
ttacgtaacc                                                              10


<210>  154
<211>  10
<212>  DNA
<213>  Artificial

<220>
<223>  synthetic DNA

<400>  154
gcatggttaa                                                              10


<210>  155
<211>  10
<212>  DNA
<213>  Artificial

<220>
<223>  synthetic DNA

<400>  155
gcacctaatt                                                              10


<210>  156
<211>  10
<212>  DNA
<213>  Artificial

<220>
<223>  synthetic DNA

<400>  156
tgtaggttgt                                                              10


<210>  157
<211>  10
<212>  DNA
<213>  Artificial

<220>
<223>  synthetic DNA

<400>  157
ccatctggaa                                                              10


<210>  158
<211>  10
<212>  DNA
<213>  Artificial

<220>
<223>  synthetic DNA

<400>  158
ttcgcgttga                                                              10


<210>  159
<211>  10
<212>  DNA
<213>  Artificial

<220>
<223>  synthetic DNA

<400>  159
aaccgaggtt                                                              10


<210>  160
<211>  10
<212>  DNA
<213>  Artificial

<220>
<223>  synthetic DNA

<400>  160
gtacgctgtt                                                              10


<210>  161
<211>  10
<212>  DNA
<213>  Artificial

<220>
<223>  synthetic DNA

<400>  161
agtatcctgg                                                              10

<210>  162
<211>  10
<212>  DNA
<213>  Artificial

<220>
<223>  synthetic DNA

<400>  162
ggttgtacag                                                              10


<210>  163
<211>  10
<212>  DNA
<213>  Artificial

<220>
<223>  synthetic DNA

<400>  163
acgtacacca                                                              10


<210>  164
<211>  10
<212>  DNA
<213>  Artificial

<220>
<223>  synthetic DNA

<400>  164
tgtcgagcaa                                                              10


<210>  165
<211>  10
<212>  DNA
<213>  Artificial

<220>
<223>  synthetic DNA

<400>  165
gtcgtgttac                                                              10


<210>  166
<211>  10
<212>  DNA
<213>  Artificial

<220>
<223>  synthetic DNA

<400>  166
gtgcaatagg                                                              10


<210>  167
<211>  10

<212>  DNA
<213>  Artificial

<220>
<223>  synthetic DNA

<400>  167
actcgatgct                                                              10


<210>  168
<211>  10
<212>  DNA
<213>  Artificial

<220>
<223>  synthetic DNA

<400>  168
gaatcgcgta                                                              10


<210>  169
<211>  10
<212>  DNA
<213>  Artificial

<220>
<223>  synthetic DNA

<400>  169
cggtcattgt                                                              10


<210>  170
<211>  10
<212>  DNA
<213>  Artificial

<220>
<223>  synthetic DNA

<400>  170
atcaggcgat                                                              10


<210>  171
<211>  10
<212>  DNA
<213>  Artificial

<220>
<223>  synthetic DNA

<400>  171
gtaagatgcg                                                              10


<210>  172
<211>  10
<212>  DNA
<213>  Artificial

<220>
<223>  synthetic DNA

<400>  172
ggtctcttga                                                              10


<210>  173
<211>  10
<212>  DNA
<213>  Artificial

<220>
<223>  synthetic DNA

<400>  173
tcctcgctaa                                                              10


<210>  174
<211>  10
<212>  DNA
<213>  Artificial

<220>
<223>  synthetic DNA

<400>  174
ctgcgtgata                                                              10


<210>  175
<211>  10
<212>  DNA
<213>  Artificial

<220>
<223>  synthetic DNA

<400>  175
catactcgtc                                                              10


<210>  176
<211>  10
<212>  DNA
<213>  Artificial

<220>
<223>  synthetic DNA

<400>  176
atctgagctc                                                              10


<210>  177
<211>  10
<212>  DNA
<213>  Artificial

<220>
<223>  synthetic DNA

<400>  177
acggatagtg                                                              10


<210>  178
<211>  10
<212>  DNA
<213>  Artificial

<220>
<223>  synthetic DNA

<400>  178
actgcaatgc                                                              10


<210>  179
<211>  10
<212>  DNA
<213>  Artificial

<220>
<223>  synthetic DNA

<400>  179
taacgacgtg                                                              10


<210>  180
<211>  10
<212>  DNA
<213>  Artificial

<220>
<223>  synthetic DNA

<400>  180
tagactgtcg                                                              10


<210>  181
<211>  10
<212>  DNA
<213>  Artificial

<220>
<223>  synthetic DNA

<400>  181
cagcacttca                                                              10


<210>  182
<211>  10
<212>  DNA
<213>  Artificial

<220>
<223>  synthetic DNA

<400>  182
aacattcgcc                                                              10

<210>  183
<211>  10
<212>  DNA
<213>  Artificial

<220>
<223>  synthetic DNA

<400>  183
actagtgcgt                                                              10


<210>  184
<211>  10
<212>  DNA
<213>  Artificial

<220>
<223>  synthetic DNA

<400>  184
acgctgttct                                                              10


<210>  185
<211>  10
<212>  DNA
<213>  Artificial

<220>
<223>  synthetic DNA

<400>  185
cgtcgaatgc                                                              10


<210>  186
<211>  10
<212>  DNA
<213>  Artificial

<220>
<223>  synthetic DNA

<400>  186
ctctgacggt                                                              10


<210>  187
<211>  10
<212>  DNA
<213>  Artificial

<220>
<223>  synthetic DNA

<400>  187
gtcgccatgt                                                              10


<210>  188
<211>  10

<212>  DNA
<213>  Artificial

<220>
<223>  synthetic DNA

<400>  188
ggtccacgtt                                                              10


<210>  189
<211>  10
<212>  DNA
<213>  Artificial

<220>
<223>  synthetic DNA

<400>  189
cgagcgactt                                                              10


<210>  190
<211>  10
<212>  DNA
<213>  Artificial

<220>
<223>  synthetic DNA

<400>  190
ttgacgcgtg                                                              10


<210>  191
<211>  10
<212>  DNA
<213>  Artificial

<220>
<223>  synthetic DNA

<400>  191
ctgagagcct                                                              10


<210>  192
<211>  10
<212>  DNA
<213>  Artificial

<220>
<223>  synthetic DNA

<400>  192
cgcgctaact                                                              10


<210>  193
<211>  10
<212>  DNA
<213>  Artificial

<220>
<223>  synthetic DNA

<400>  193
ggtcgtcaag                                                              10


<210>  194
<211>  10
<212>  DNA
<213>  Artificial

<220>
<223>  synthetic DNA

<400>  194
aggttgacca                                                              10


<210>  195
<211>  10
<212>  DNA
<213>  Artificial

<220>
<223>  synthetic DNA

<400>  195
taacggcaac                                                              10


<210>  196
<211>  10
<212>  DNA
<213>  Artificial

<220>
<223>  synthetic DNA

<400>  196
gaggctggat                                                              10


<210>  197
<211>  10
<212>  DNA
<213>  Artificial

<220>
<223>  synthetic DNA

<400>  197
gtgcacacct                                                              10


<210>  198
<211>  10
<212>  DNA
<213>  Artificial

<220>
<223>  synthetic DNA

<400>  198
tgaggaccag                                                              10


<210>  199
<211>  10
<212>  DNA
<213>  Artificial

<220>
<223>  synthetic DNA

<400>  199
tacttgcgag                                                              10


<210>  200
<211>  10
<212>  DNA
<213>  Artificial

<220>
<223>  synthetic DNA

<400>  200
aactgtgaga                                                              10


<210>  201
<211>  10
<212>  DNA
<213>  Artificial

<220>
<223>  synthetic DNA

<400>  201
ctccatcaac                                                              10


<210>  202
<211>  10
<212>  DNA
<213>  Artificial

<220>
<223>  synthetic DNA

<400>  202
cggactgtta                                                              10


<210>  203
<211>  10
<212>  DNA
<213>  Artificial

<220>
<223>  synthetic DNA

<400>  203
taggacagtc                                                              10

<210>  204
<211>  10
<212>  DNA
<213>  Artificial

<220>
<223>  synthetic DNA

<400>  204
agaggacaca                                                              10


<210>  205
<211>  10
<212>  DNA
<213>  Artificial

<220>
<223>  synthetic DNA

<400>  205
acattcgcgg                                                              10


<210>  206
<211>  10
<212>  DNA
<213>  Artificial

<220>
<223>  synthetic DNA

<400>  206
gcttactgca                                                              10


<210>  207
<211>  10
<212>  DNA
<213>  Artificial

<220>
<223>  synthetic DNA

<400>  207
caatacgtaa                                                              10


<210>  208
<211>  10
<212>  DNA
<213>  Artificial

<220>
<223>  synthetic DNA

<400>  208
agacttgcgc                                                              10


<210>  209
<211>  10

<212>  DNA
<213>  Artificial

<220>
<223>  synthetic DNA

<400>  209
gagcggtgtt                                                              10


<210>  210
<211>  10
<212>  DNA
<213>  Artificial

<220>
<223>  synthetic DNA

<400>  210
cgtgagaggt                                                              10


<210>  211
<211>  10
<212>  DNA
<213>  Artificial

<220>
<223>  synthetic DNA

<400>  211
aatccgtcag                                                              10


<210>  212
<211>  10
<212>  DNA
<213>  Artificial

<220>
<223>  synthetic DNA

<400>  212
atacgtaccg                                                              10


<210>  213
<211>  10
<212>  DNA
<213>  Artificial

<220>
<223>  synthetic DNA

<400>  213
aactgattcc                                                              10


<210>  214
<211>  10
<212>  DNA
<213>  Artificial

<220>
<223>  synthetic DNA

<400>  214
ctgagcgtac                                                              10


<210>  215
<211>  10
<212>  DNA
<213>  Artificial

<220>
<223>  synthetic DNA

<400>  215
gtcggattcg                                                              10


<210>  216
<211>  10
<212>  DNA
<213>  Artificial

<220>
<223>  synthetic DNA

<400>  216
gccgaccata                                                              10


<210>  217
<211>  10
<212>  DNA
<213>  Artificial

<220>
<223>  synthetic DNA

<400>  217
gcagaactaa                                                              10


<210>  218
<211>  10
<212>  DNA
<213>  Artificial

<220>
<223>  synthetic DNA

<400>  218
ctaacgaccg                                                              10


<210>  219
<211>  10
<212>  DNA
<213>  Artificial

<220>
<223>  synthetic DNA

<400>  219
gctggaccat                                                              10


<210>  220
<211>  10
<212>  DNA
<213>  Artificial

<220>
<223>  synthetic DNA

<400>  220
gacgcggtta                                                              10


<210>  221
<211>  10
<212>  DNA
<213>  Artificial

<220>
<223>  synthetic DNA

<400>  221
agtggtgagc                                                              10


<210>  222
<211>  10
<212>  DNA
<213>  Artificial

<220>
<223>  synthetic DNA

<400>  222
caggcagtca                                                              10


<210>  223
<211>  10
<212>  DNA
<213>  Artificial

<220>
<223>  synthetic DNA

<400>  223
tctgacgtca                                                              10


<210>  224
<211>  10
<212>  DNA
<213>  Artificial

<220>
<223>  synthetic DNA

<400>  224
tacatgacgt                                                              10

<210>  225
<211>  10
<212>  DNA
<213>  Artificial

<220>
<223>  synthetic DNA

<400>  225
tgaggcaacc                                                              10


<210>  226
<211>  10
<212>  DNA
<213>  Artificial

<220>
<223>  synthetic DNA

<400>  226
caactgcagt                                                              10


<210>  227
<211>  10
<212>  DNA
<213>  Artificial

<220>
<223>  synthetic DNA

<400>  227
cggagatacg                                                              10


<210>  228
<211>  10
<212>  DNA
<213>  Artificial

<220>
<223>  synthetic DNA

<400>  228
cttcgcaagt                                                              10


<210>  229
<211>  10
<212>  DNA
<213>  Artificial

<220>
<223>  synthetic DNA

<400>  229
ctggcatacg                                                              10


<210>  230
<211>  10

<212>  DNA
<213>  Artificial

<220>
<223>  synthetic DNA

<400>  230
taacgttcgc                                                              10


<210>  231
<211>  10
<212>  DNA
<213>  Artificial

<220>
<223>  synthetic DNA

<400>  231
ccggcgttaa                                                              10


<210>  232
<211>  10
<212>  DNA
<213>  Artificial

<220>
<223>  synthetic DNA

<400>  232
acaagacgcc                                                              10


<210>  233
<211>  10
<212>  DNA
<213>  Artificial

<220>
<223>  synthetic DNA

<400>  233
ccattagact                                                              10


<210>  234
<211>  10
<212>  DNA
<213>  Artificial

<220>
<223>  synthetic DNA

<400>  234
gtctgtgaca                                                              10


<210>  235
<211>  10
<212>  DNA
<213>  Artificial

<220>
<223>  synthetic DNA

<400>  235
ggcattggac                                                              10


<210>  236
<211>  10
<212>  DNA
<213>  Artificial

<220>
<223>  synthetic DNA

<400>  236
tcttcgcacg                                                              10


<210>  237
<211>  10
<212>  DNA
<213>  Artificial

<220>
<223>  synthetic DNA

<400>  237
tagcctgtgc                                                              10


<210>  238
<211>  10
<212>  DNA
<213>  Artificial

<220>
<223>  synthetic DNA

<400>  238
cactgaccta                                                              10


<210>  239
<211>  10
<212>  DNA
<213>  Artificial

<220>
<223>  synthetic DNA

<400>  239
ccgcacgatt                                                              10


<210>  240
<211>  10
<212>  DNA
<213>  Artificial

<220>
<223>  synthetic DNA

<400>  240
atagcacacg                                                              10


<210>  241
<211>  10
<212>  DNA
<213>  Artificial

<220>
<223>  synthetic DNA

<400>  241
gcacgtcata                                                              10


<210>  242
<211>  10
<212>  DNA
<213>  Artificial

<220>
<223>  synthetic DNA

<400>  242
aagccgttgg                                                              10


<210>  243
<211>  10
<212>  DNA
<213>  Artificial

<220>
<223>  synthetic DNA

<400>  243
cggaccgtta                                                              10


<210>  244
<211>  10
<212>  DNA
<213>  Artificial

<220>
<223>  synthetic DNA

<400>  244
tacacagcgt                                                              10


<210>  245
<211>  10
<212>  DNA
<213>  Artificial

<220>
<223>  synthetic DNA

<400>  245
cggacttcag                                                              10

<210>  246
<211>  10
<212>  DNA
<213>  Artificial

<220>
<223>  synthetic DNA

<400>  246
tagaacgtca                                                              10


<210>  247
<211>  10
<212>  DNA
<213>  Artificial

<220>
<223>  synthetic DNA

<400>  247
ggcattggag                                                              10


<210>  248
<211>  10
<212>  DNA
<213>  Artificial

<220>
<223>  synthetic DNA

<400>  248
ggcactcgtt                                                              10


<210>  249
<211>  10
<212>  DNA
<213>  Artificial

<220>
<223>  synthetic DNA

<400>  249
gtaccgttaa                                                              10


<210>  250
<211>  10
<212>  DNA
<213>  Artificial

<220>
<223>  synthetic DNA

<400>  250
aatacgtgtc                                                              10


<210>  251
<211>  10

<212>  DNA
<213>  Artificial

<220>
<223>  synthetic DNA

<400>  251
ccattgacgt                                                              10


<210>  252
<211>  10
<212>  DNA
<213>  Artificial

<220>
<223>  synthetic DNA

<400>  252
cgtgaatcgc                                                              10


<210>  253
<211>  10
<212>  DNA
<213>  Artificial

<220>
<223>  synthetic DNA

<400>  253
atcaacgcgg                                                              10


<210>  254
<211>  10
<212>  DNA
<213>  Artificial

<220>
<223>  synthetic DNA

<400>  254
cgccaaggta                                                              10


<210>  255
<211>  10
<212>  DNA
<213>  Artificial

<220>
<223>  synthetic DNA

<400>  255
agaagacgcc                                                              10


<210>  256
<211>  10
<212>  DNA
<213>  Artificial

<220>
<223>  synthetic DNA

<400>  256
ccgcatagtc                                                              10


<210>  257
<211>  10
<212>  DNA
<213>  Artificial

<220>
<223>  synthetic DNA

<400>  257
cttatatgtg                                                              10


<210>  258
<211>  10
<212>  DNA
<213>  Artificial

<220>
<223>  synthetic DNA

<400>  258
ggtctcatcg                                                              10


<210>  259
<211>  10
<212>  DNA
<213>  Artificial

<220>
<223>  synthetic DNA

<400>  259
ccaccatgtc                                                              10


<210>  260
<211>  10
<212>  DNA
<213>  Artificial

<220>
<223>  synthetic DNA

<400>  260
acgaatgtgt                                                              10


<210>  261
<211>  10
<212>  DNA
<213>  Artificial

<220>
<223>  synthetic DNA

<400>  261
ggtagtaaca                                                              10


<210>  262
<211>  10
<212>  DNA
<213>  Artificial

<220>
<223>  synthetic DNA

<400>  262
gccacttaat                                                              10


<210>  263
<211>  10
<212>  DNA
<213>  Artificial

<220>
<223>  synthetic DNA

<400>  263
atattgcgcc                                                              10


<210>  264
<211>  10
<212>  DNA
<213>  Artificial

<220>
<223>  synthetic DNA

<400>  264
gaccaatagt                                                              10


<210>  265
<211>  10
<212>  DNA
<213>  Artificial

<220>
<223>  synthetic DNA

<400>  265
aacaacacgg                                                              10


<210>  266
<211>  10
<212>  DNA
<213>  Artificial

<220>
<223>  synthetic DNA

<400>  266
atagccgatg                                                              10

<210>  267
<211>  10
<212>  DNA
<213>  Artificial

<220>
<223>  synthetic DNA

<400>  267
cgagagcata                                                              10


<210>  268
<211>  10
<212>  DNA
<213>  Artificial

<220>
<223>  synthetic DNA

<400>  268
cgagacatga                                                              10


<210>  269
<211>  10
<212>  DNA
<213>  Artificial

<220>
<223>  synthetic DNA

<400>  269
cgccaagtta                                                              10


<210>  270
<211>  10
<212>  DNA
<213>  Artificial

<220>
<223>  synthetic DNA

<400>  270
ttataatcgc                                                              10


<210>  271
<211>  10
<212>  DNA
<213>  Artificial

<220>
<223>  synthetic DNA

<400>  271
tagaagtgca                                                              10


<210>  272
<211>  10

<212>  DNA
<213>  Artificial

<220>
<223>  synthetic DNA

<400>  272
ggaggcatgt                                                              10


<210>  273
<211>  10
<212>  DNA
<213>  Artificial

<220>
<223>  synthetic DNA

<400>  273
gccacttcga                                                              10


<210>  274
<211>  10
<212>  DNA
<213>  Artificial

<220>
<223>  synthetic DNA

<400>  274
tccacggtac                                                              10


<210>  275
<211>  10
<212>  DNA
<213>  Artificial

<220>
<223>  synthetic DNA

<400>  275
caactatgca                                                              10


<210>  276
<211>  10
<212>  DNA
<213>  Artificial

<220>
<223>  synthetic DNA

<400>  276
caaggaggac                                                              10


<210>  277
<211>  10
<212>  DNA
<213>  Artificial

<220>
<223>  synthetic DNA

<400>  277
gaggtaccta                                                              10


<210>  278
<211>  10
<212>  DNA
<213>  Artificial

<220>
<223>  synthetic DNA

<400>  278
gagcgcataa                                                              10


<210>  279
<211>  10
<212>  DNA
<213>  Artificial

<220>
<223>  synthetic DNA

<400>  279
tcgtcacgtg                                                              10


<210>  280
<211>  10
<212>  DNA
<213>  Artificial

<220>
<223>  synthetic DNA

<400>  280
aactgtgaca                                                              10


<210>  281
<211>  10
<212>  DNA
<213>  Artificial

<220>
<223>  synthetic DNA

<400>  281
tccacgtgag                                                              10


<210>  282
<211>  10
<212>  DNA
<213>  Artificial

<220>
<223>  synthetic DNA

<400>  282
acactgctct                                                              10


<210>  283
<211>  10
<212>  DNA
<213>  Artificial

<220>
<223>  synthetic DNA

<400>  283
tacggtgagc                                                              10


<210>  284
<211>  10
<212>  DNA
<213>  Artificial

<220>
<223>  synthetic DNA

<400>  284
cggactaagt                                                              10


<210>  285
<211>  10
<212>  DNA
<213>  Artificial

<220>
<223>  synthetic DNA

<400>  285
aagccacgtt                                                              10


<210>  286
<211>  10
<212>  DNA
<213>  Artificial

<220>
<223>  synthetic DNA

<400>  286
caattactcg                                                              10


<210>  287
<211>  10
<212>  DNA
<213>  Artificial

<220>
<223>  synthetic DNA

<400>  287
tctggccata                                                              10

<210>  288
<211>  10
<212>  DNA
<213>  Artificial

<220>
<223>  synthetic DNA

<400>  288
tcaggctagt                                                              10


<210>  289
<211>  10
<212>  DNA
<213>  Artificial

<220>
<223>  synthetic DNA

<400>  289
ttgacccgga                                                              10


<210>  290
<211>  10
<212>  DNA
<213>  Artificial

<220>
<223>  synthetic DNA

<400>  290
tttttatggt                                                              10


<210>  291
<211>  10
<212>  DNA
<213>  Artificial

<220>
<223>  synthetic DNA

<400>  291
atgtggtgcg                                                              10


<210>  292
<211>  10
<212>  DNA
<213>  Artificial

<220>
<223>  synthetic DNA

<400>  292
aaggcgctag                                                              10


<210>  293
<211>  10

<212>  DNA
<213>  Artificial

<220>
<223>  synthetic DNA

<400>  293
tccaactttg                                                              10


<210>  294
<211>  10
<212>  DNA
<213>  Artificial

<220>
<223>  synthetic DNA

<400>  294
ccatcccatc                                                              10


<210>  295
<211>  10
<212>  DNA
<213>  Artificial

<220>
<223>  synthetic DNA

<400>  295
caatacgagg                                                              10


<210>  296
<211>  10
<212>  DNA
<213>  Artificial

<220>
<223>  synthetic DNA

<400>  296
gagtgttacc                                                              10


<210>  297
<211>  10
<212>  DNA
<213>  Artificial

<220>
<223>  synthetic DNA

<400>  297
gcctcctgta                                                              10


<210>  298
<211>  10
<212>  DNA
<213>  Artificial

<220>
<223>  synthetic DNA

<400>  298
cgaaggttgc                                                              10


<210>  299
<211>  10
<212>  DNA
<213>  Artificial

<220>
<223>  synthetic DNA

<400>  299
gaggtgctat                                                              10


<210>  300
<211>  10
<212>  DNA
<213>  Artificial

<220>
<223>  synthetic DNA

<400>  300
taggataatt                                                              10


<210>  301
<211>  10
<212>  DNA
<213>  Artificial

<220>
<223>  synthetic DNA

<400>  301
cgttgtcctc                                                              10


<210>  302
<211>  10
<212>  DNA
<213>  Artificial

<220>
<223>  synthetic DNA

<400>  302
tgagaccagc                                                              10


<210>  303
<211>  10
<212>  DNA
<213>  Artificial

<220>
<223>  synthetic DNA

<400>  303
tgcccaagct                                                              10


<210>  304
<211>  10
<212>  DNA
<213>  Artificial

<220>
<223>  synthetic DNA

<400>  304
tactgaatcg                                                              10


<210>  305
<211>  10
<212>  DNA
<213>  Artificial

<220>
<223>  synthetic DNA

<400>  305
ttacatagtc                                                              10


<210>  306
<211>  10
<212>  DNA
<213>  Artificial

<220>
<223>  synthetic DNA

<400>  306
acaaaggaaa                                                              10


<210>  307
<211>  10
<212>  DNA
<213>  Artificial

<220>
<223>  synthetic DNA

<400>  307
ctcgcttggg                                                              10


<210>  308
<211>  10
<212>  DNA
<213>  Artificial

<220>
<223>  synthetic DNA

<400>  308
ccttgcgtca                                                              10

<210>  309
<211>  10
<212>  DNA
<213>  Artificial

<220>
<223>  synthetic DNA

<400>  309
taattccgaa                                                              10


<210>  310
<211>  10
<212>  DNA
<213>  Artificial

<220>
<223>  synthetic DNA

<400>  310
gtgagcttga                                                              10


<210>  311
<211>  10
<212>  DNA
<213>  Artificial

<220>
<223>  synthetic DNA

<400>  311
atgccgattc                                                              10


<210>  312
<211>  10
<212>  DNA
<213>  Artificial

<220>
<223>  synthetic DNA

<400>  312
gcttgggctt                                                              10


<210>  313
<211>  10
<212>  DNA
<213>  Artificial

<220>
<223>  synthetic DNA

<400>  313
acaaagcgcc                                                              10


<210>  314
<211>  10

<212>  DNA
<213>  Artificial

<220>
<223>  synthetic DNA

<400>  314
gaaagctcta                                                              10


<210>  315
<211>  10
<212>  DNA
<213>  Artificial

<220>
<223>  synthetic DNA

<400>  315
taccgaccgt                                                              10


<210>  316
<211>  10
<212>  DNA
<213>  Artificial

<220>
<223>  synthetic DNA

<400>  316
tcgaagagac                                                              10


<210>  317
<211>  10
<212>  DNA
<213>  Artificial

<220>
<223>  synthetic DNA

<400>  317
gtcgcttacg                                                              10


<210>  318
<211>  10
<212>  DNA
<213>  Artificial

<220>
<223>  synthetic DNA

<400>  318
gggctctcca                                                              10


<210>  319
<211>  10
<212>  DNA
<213>  Artificial

<220>
<223>  synthetic DNA

<400>  319
gcgcccttgt                                                              10


<210>  320
<211>  10
<212>  DNA
<213>  Artificial

<220>
<223>  synthetic DNA

<400>  320
ggcaataggc                                                              10


<210>  321
<211>  10
<212>  DNA
<213>  Artificial

<220>
<223>  synthetic DNA

<400>  321
caagtcagga                                                              10


<210>  322
<211>  10
<212>  DNA
<213>  Artificial

<220>
<223>  synthetic DNA

<400>  322
gggtcgcaat                                                              10


<210>  323
<211>  10
<212>  DNA
<213>  Artificial

<220>
<223>  synthetic DNA

<400>  323
cagcaaccta                                                              10


<210>  324
<211>  10
<212>  DNA
<213>  Artificial

<220>
<223>  synthetic DNA

<400>  324
ttcccgccac                                                              10


<210>  325
<211>  10
<212>  DNA
<213>  Artificial

<220>
<223>  synthetic DNA

<400>  325
tgtgcatttt                                                              10


<210>  326
<211>  10
<212>  DNA
<213>  Artificial

<220>
<223>  synthetic DNA

<400>  326
atcaacgacg                                                              10


<210>  327
<211>  10
<212>  DNA
<213>  Artificial

<220>
<223>  synthetic DNA

<400>  327
gtgacgtcca                                                              10


<210>  328
<211>  10
<212>  DNA
<213>  Artificial

<220>
<223>  synthetic DNA

<400>  328
cgatctagtc                                                              10


<210>  329
<211>  10
<212>  DNA
<213>  Artificial

<220>
<223>  synthetic DNA

<400>  329
ttacatcctg                                                              10

<210>  330
<211>  10
<212>  DNA
<213>  Artificial

<220>
<223>  synthetic DNA

<400>  330
agccttcaat                                                              10


<210>  331
<211>  10
<212>  DNA
<213>  Artificial

<220>
<223>  synthetic DNA

<400>  331
tccatccgat                                                              10


<210>  332
<211>  10
<212>  DNA
<213>  Artificial

<220>
<223>  synthetic DNA

<400>  332
gactgggtct                                                              10


<210>  333
<211>  10
<212>  DNA
<213>  Artificial

<220>
<223>  synthetic DNA

<400>  333
ttcggtggag                                                              10


<210>  334
<211>  10
<212>  DNA
<213>  Artificial

<220>
<223>  synthetic DNA

<400>  334
gaccagcaca                                                              10


<210>  335
<211>  10

<212>  DNA
<213>  Artificial

<220>
<223>  synthetic DNA

<400>  335
cattaacgga                                                              10


<210>  336
<211>  10
<212>  DNA
<213>  Artificial

<220>
<223>  synthetic DNA

<400>  336
tttttcttga                                                              10


<210>  337
<211>  10
<212>  DNA
<213>  Artificial

<220>
<223>  synthetic DNA

<400>  337
cattgcactg                                                              10


<210>  338
<211>  10
<212>  DNA
<213>  Artificial

<220>
<223>  synthetic DNA

<400>  338
tgcggcgatc                                                              10


<210>  339
<211>  10
<212>  DNA
<213>  Artificial

<220>
<223>  synthetic DNA

<400>  339
atattgcggt                                                              10


<210>  340
<211>  10
<212>  DNA
<213>  Artificial

<220>
<223>  synthetic DNA

<400>  340
gacgtcgctc                                                              10


<210>  341
<211>  10
<212>  DNA
<213>  Artificial

<220>
<223>  synthetic DNA

<400>  341
tcgcttatcg                                                              10


<210>  342
<211>  10
<212>  DNA
<213>  Artificial

<220>
<223>  synthetic DNA

<400>  342
gcgcagacac                                                              10


<210>  343
<211>  10
<212>  DNA
<213>  Artificial

<220>
<223>  synthetic DNA

<400>  343
catgtattgt                                                              10


<210>  344
<211>  10
<212>  DNA
<213>  Artificial

<220>
<223>  synthetic DNA

<400>  344
tctataacct                                                              10


<210>  345
<211>  10
<212>  DNA
<213>  Artificial

<220>
<223>  synthetic DNA

<400>  345
gtggagacaa                                                              10


<210>  346
<211>  10
<212>  DNA
<213>  Artificial

<220>
<223>  synthetic DNA

<400>  346
cgaagattat                                                              10


<210>  347
<211>  10
<212>  DNA
<213>  Artificial

<220>
<223>  synthetic DNA

<400>  347
tagcaactgc                                                              10


<210>  348
<211>  10
<212>  DNA
<213>  Artificial

<220>
<223>  synthetic DNA

<400>  348
ataatcggta                                                              10


<210>  349
<211>  10
<212>  DNA
<213>  Artificial

<220>
<223>  synthetic DNA

<400>  349
caggatgggt                                                              10


<210>  350
<211>  10
<212>  DNA
<213>  Artificial

<220>
<223>  synthetic DNA

<400>  350
gacgattccc                                                              10

<210>  351
<211>  10
<212>  DNA
<213>  Artificial

<220>
<223>  synthetic DNA

<400>  351
cacgccttac                                                              10


<210>  352
<211>  10
<212>  DNA
<213>  Artificial

<220>
<223>  synthetic DNA

<400>  352
agttggttcc                                                              10


<210>  353
<211>  10
<212>  DNA
<213>  Artificial

<220>
<223>  synthetic DNA

<400>  353
tcttatcagg                                                              10


<210>  354
<211>  10
<212>  DNA
<213>  Artificial

<220>
<223>  synthetic DNA

<400>  354
cgagaagttc                                                              10


<210>  355
<211>  10
<212>  DNA
<213>  Artificial

<220>
<223>  synthetic DNA

<400>  355
gtggtagaat                                                              10


<210>  356
<211>  10

<212>  DNA
<213>  Artificial

<220>
<223>  synthetic DNA

<400>  356
taggcttgtg                                                              10


<210>  357
<211>  10
<212>  DNA
<213>  Artificial

<220>
<223>  synthetic DNA

<400>  357
atgcgttacg                                                              10


<210>  358
<211>  10
<212>  DNA
<213>  Artificial

<220>
<223>  synthetic DNA

<400>  358
actaccgagg                                                              10


<210>  359
<211>  10
<212>  DNA
<213>  Artificial

<220>
<223>  synthetic DNA

<400>  359
cgagttggtg                                                              10


<210>  360
<211>  10
<212>  DNA
<213>  Artificial

<220>
<223>  synthetic DNA

<400>  360
ggacgatcaa                                                              10


<210>  361
<211>  10
<212>  DNA
<213>  Artificial

<220>
<223>  synthetic DNA

<400>  361
aacagtatgc                                                              10


<210>  362
<211>  10
<212>  DNA
<213>  Artificial

<220>
<223>  synthetic DNA

<400>  362
ttggctgatc                                                              10


<210>  363
<211>  10
<212>  DNA
<213>  Artificial

<220>
<223>  synthetic DNA

<400>  363
aggattggaa                                                              10


<210>  364
<211>  10
<212>  DNA
<213>  Artificial

<220>
<223>  synthetic DNA

<400>  364
catatggaga                                                              10


<210>  365
<211>  10
<212>  DNA
<213>  Artificial

<220>
<223>  synthetic DNA

<400>  365
ctgcaggttt                                                              10


<210>  366
<211>  10
<212>  DNA
<213>  Artificial

<220>
<223>  synthetic DNA

<400>  366
ctctcttttt                                                              10


<210>  367
<211>  10
<212>  DNA
<213>  Artificial

<220>
<223>  synthetic DNA

<400>  367
agtaggggtc                                                              10


<210>  368
<211>  10
<212>  DNA
<213>  Artificial

<220>
<223>  synthetic DNA

<400>  368
acaccgcaag                                                              10


<210>  369
<211>  10
<212>  DNA
<213>  Artificial

<220>
<223>  synthetic DNA

<400>  369
gaagcgggag                                                              10


<210>  370
<211>  10
<212>  DNA
<213>  Artificial

<220>
<223>  synthetic DNA

<400>  370
gatacggact                                                              10


<210>  371
<211>  10
<212>  DNA
<213>  Artificial

<220>
<223>  synthetic DNA

<400>  371
tacgacgtgt                                                              10

<210>  372
<211>  10
<212>  DNA
<213>  Artificial

<220>
<223>  synthetic DNA

<400>  372
gtgcctcctt                                                              10


<210>  373
<211>  10
<212>  DNA
<213>  Artificial

<220>
<223>  synthetic DNA

<400>  373
ggtgactgat                                                              10


<210>  374
<211>  10
<212>  DNA
<213>  Artificial

<220>
<223>  synthetic DNA

<400>  374
atatcttacg                                                              10


<210>  375
<211>  10
<212>  DNA
<213>  Artificial

<220>
<223>  synthetic DNA

<400>  375
aatcatacgg                                                              10


<210>  376
<211>  10
<212>  DNA
<213>  Artificial

<220>
<223>  synthetic DNA

<400>  376
ctcttgggac                                                              10


<210>  377
<211>  10

<212>  DNA
<213>  Artificial

<220>
<223>  synthetic DNA

<400>  377
gacgacaaat                                                              10


<210>  378
<211>  10
<212>  DNA
<213>  Artificial

<220>
<223>  synthetic DNA

<400>  378
gttgcgaggt                                                              10


<210>  379
<211>  10
<212>  DNA
<213>  Artificial

<220>
<223>  synthetic DNA

<400>  379
aaaccgcacc                                                              10


<210>  380
<211>  10
<212>  DNA
<213>  Artificial

<220>
<223>  synthetic DNA

<400>  380
gctaacacgt                                                              10


<210>  381
<211>  10
<212>  DNA
<213>  Artificial

<220>
<223>  synthetic DNA

<400>  381
atcatgaggg                                                              10


<210>  382
<211>  10
<212>  DNA
<213>  Artificial

<220>
<223>  synthetic DNA

<400>  382
gattcacgta                                                              10


<210>  383
<211>  10
<212>  DNA
<213>  Artificial

<220>
<223>  synthetic DNA

<400>  383
tctcgaaaag                                                              10


<210>  384
<211>  10
<212>  DNA
<213>  Artificial

<220>
<223>  synthetic DNA

<400>  384
ctcgtaacca                                                              10


<210>  385
<211>  10
<212>  DNA
<213>  Artificial

<220>
<223>  synthetic DNA

<400>  385
gttacacacg                                                              10


<210>  386
<211>  10
<212>  DNA
<213>  Artificial

<220>
<223>  synthetic DNA

<400>  386
cgtgaagggt                                                              10


<210>  387
<211>  10
<212>  DNA
<213>  Artificial

<220>
<223>  synthetic DNA

<400>  387
acgagcatct                                                              10


<210>  388
<211>  10
<212>  DNA
<213>  Artificial

<220>
<223>  synthetic DNA

<400>  388
acgagggatt                                                              10


<210>  389
<211>  10
<212>  DNA
<213>  Artificial

<220>
<223>  synthetic DNA

<400>  389
gcaacgtcgg                                                              10


<210>  390
<211>  10
<212>  DNA
<213>  Artificial

<220>
<223>  synthetic DNA

<400>  390
cacggctagg                                                              10


<210>  391
<211>  10
<212>  DNA
<213>  Artificial

<220>
<223>  synthetic DNA

<400>  391
cgtgactctc                                                              10


<210>  392
<211>  10
<212>  DNA
<213>  Artificial

<220>
<223>  synthetic DNA

<400>  392
tctagacgca                                                              10

<210>  393
<211>  10
<212>  DNA
<213>  Artificial

<220>
<223>  synthetic DNA

<400>  393
ctgcgcacat                                                              10


<210>  394
<211>  10
<212>  DNA
<213>  Artificial

<220>
<223>  synthetic DNA

<400>  394
atgcttgaca                                                              10


<210>  395
<211>  10
<212>  DNA
<213>  Artificial

<220>
<223>  synthetic DNA

<400>  395
tttgtcgaca                                                              10


<210>  396
<211>  10
<212>  DNA
<213>  Artificial

<220>
<223>  synthetic DNA

<400>  396
acgtgtcagc                                                              10


<210>  397
<211>  10
<212>  DNA
<213>  Artificial

<220>
<223>  synthetic DNA

<400>  397
gaaaacatta                                                              10


<210>  398
<211>  10

<212>  DNA
<213>  Artificial

<220>
<223>  synthetic DNA

<400>  398
acattaacgg                                                              10


<210>  399
<211>  10
<212>  DNA
<213>  Artificial

<220>
<223>  synthetic DNA

<400>  399
gtacaggtcc                                                              10


<210>  400
<211>  10
<212>  DNA
<213>  Artificial

<220>
<223>  synthetic DNA

<400>  400
ctatgtgtac                                                              10


<210>  401
<211>  10
<212>  DNA
<213>  Artificial

<220>
<223>  synthetic DNA

<400>  401
gcgtacatta                                                              10


<210>  402
<211>  10
<212>  DNA
<213>  Artificial

<220>
<223>  synthetic DNA

<400>  402
gatttgtggc                                                              10


<210>  403
<211>  10
<212>  DNA
<213>  Artificial

<220>
<223>  synthetic DNA

<400>  403
tcgcgcgcta                                                              10


<210>  404
<211>  10
<212>  DNA
<213>  Artificial

<220>
<223>  synthetic DNA

<400>  404
acaagggcga                                                              10


<210>  405
<211>  10
<212>  DNA
<213>  Artificial

<220>
<223>  synthetic DNA

<400>  405
aacgcgcgat                                                              10


<210>  406
<211>  10
<212>  DNA
<213>  Artificial

<220>
<223>  synthetic DNA

<400>  406
cgtaaatgcg                                                              10


<210>  407
<211>  10
<212>  DNA
<213>  Artificial

<220>
<223>  synthetic DNA

<400>  407
taggcactac                                                              10


<210>  408
<211>  10
<212>  DNA
<213>  Artificial

<220>
<223>  synthetic DNA

<400>  408
gcgaggatcg                                                              10


<210>  409
<211>  10
<212>  DNA
<213>  Artificial

<220>
<223>  synthetic DNA

<400>  409
cacgtttact                                                              10


<210>  410
<211>  10
<212>  DNA
<213>  Artificial

<220>
<223>  synthetic DNA

<400>  410
taccaccacg                                                              10


<210>  411
<211>  10
<212>  DNA
<213>  Artificial

<220>
<223>  synthetic DNA

<400>  411
ttaacaggac                                                              10


<210>  412
<211>  10
<212>  DNA
<213>  Artificial

<220>
<223>  synthetic DNA

<400>  412
gctgtataac                                                              10


<210>  413
<211>  10
<212>  DNA
<213>  Artificial

<220>
<223>  synthetic DNA

<400>  413
gttgctggca                                                              10

<210>  414
<211>  10
<212>  DNA
<213>  Artificial

<220>
<223>  synthetic DNA

<400>  414
agtgtggcca                                                              10


<210>  415
<211>  10
<212>  DNA
<213>  Artificial

<220>
<223>  synthetic DNA

<400>  415
ctgcggttgt                                                              10


<210>  416
<211>  10
<212>  DNA
<213>  Artificial

<220>
<223>  synthetic DNA

<400>  416
tagatcagcg                                                              10


<210>  417
<211>  10
<212>  DNA
<213>  Artificial

<220>
<223>  synthetic DNA

<400>  417
ttccggttat                                                              10


<210>  418
<211>  10
<212>  DNA
<213>  Artificial

<220>
<223>  synthetic DNA

<400>  418
gataaactgt                                                              10


<210>  419
<211>  10

<212>  DNA
<213>  Artificial

<220>
<223>  synthetic DNA

<400>  419
tacagttgcc                                                              10


<210>  420
<211>  10
<212>  DNA
<213>  Artificial

<220>
<223>  synthetic DNA

<400>  420
cgatggcgaa                                                              10


<210>  421
<211>  10
<212>  DNA
<213>  Artificial

<220>
<223>  synthetic DNA

<400>  421
ccgacgtcag                                                              10


<210>  422
<211>  10
<212>  DNA
<213>  Artificial

<220>
<223>  synthetic DNA

<400>  422
tatggtgcaa                                                              10


<210>  423
<211>  10
<212>  DNA
<213>  Artificial

<220>
<223>  synthetic DNA

<400>  423
gacgacagtc                                                              10


<210>  424
<211>  10
<212>  DNA
<213>  Artificial

<220>
<223>  synthetic DNA

<400>  424
gtcaccgtcc                                                              10


<210>  425
<211>  10
<212>  DNA
<213>  Artificial

<220>
<223>  synthetic DNA

<400>  425
ggttttaaca                                                              10


<210>  426
<211>  10
<212>  DNA
<213>  Artificial

<220>
<223>  synthetic DNA

<400>  426
gaggacagta                                                              10


<210>  427
<211>  10
<212>  DNA
<213>  Artificial

<220>
<223>  synthetic DNA

<400>  427
gttacctaag                                                              10


<210>  428
<211>  10
<212>  DNA
<213>  Artificial

<220>
<223>  synthetic DNA

<400>  428
atcacgtgtt                                                              10


<210>  429
<211>  10
<212>  DNA
<213>  Artificial

<220>
<223>  synthetic DNA

<400>  429
taaggcctgg                                                              10


<210>  430
<211>  10
<212>  DNA
<213>  Artificial

<220>
<223>  synthetic DNA

<400>  430
tgttcgtagc                                                              10


<210>  431
<211>  10
<212>  DNA
<213>  Artificial

<220>
<223>  synthetic DNA

<400>  431
tgaggacgtg                                                              10


<210>  432
<211>  10
<212>  DNA
<213>  Artificial

<220>
<223>  synthetic DNA

<400>  432
gtgctgtgta                                                              10


<210>  433
<211>  10
<212>  DNA
<213>  Artificial

<220>
<223>  synthetic DNA

<400>  433
gagggtacgc                                                              10


<210>  434
<211>  10
<212>  DNA
<213>  Artificial

<220>
<223>  synthetic DNA

<400>  434
ccgtgattgt                                                              10

<210>  435
<211>  10
<212>  DNA
<213>  Artificial

<220>
<223>  synthetic DNA

<400>  435
aaaatcgcct                                                              10


<210>  436
<211>  10
<212>  DNA
<213>  Artificial

<220>
<223>  synthetic DNA

<400>  436
cgatcgcagt                                                              10


<210>  437
<211>  10
<212>  DNA
<213>  Artificial

<220>
<223>  synthetic DNA

<400>  437
acgcaataag                                                              10


<210>  438
<211>  10
<212>  DNA
<213>  Artificial

<220>
<223>  synthetic DNA

<400>  438
aaggtgcatc                                                              10


<210>  439
<211>  10
<212>  DNA
<213>  Artificial

<220>
<223>  synthetic DNA

<400>  439
cgcgtagata                                                              10


<210>  440
<211>  10

<212>  DNA
<213>  Artificial

<220>
<223>  synthetic DNA

<400>  440
cgagcagtgc                                                              10


<210>  441
<211>  10
<212>  DNA
<213>  Artificial

<220>
<223>  synthetic DNA

<400>  441
atacgtgacg                                                              10


<210>  442
<211>  10
<212>  DNA
<213>  Artificial

<220>
<223>  synthetic DNA

<400>  442
agattgcgcg                                                              10


<210>  443
<211>  10
<212>  DNA
<213>  Artificial

<220>
<223>  synthetic DNA

<400>  443
acgtgatgcc                                                              10


<210>  444
<211>  10
<212>  DNA
<213>  Artificial

<220>
<223>  synthetic DNA

<400>  444
gtacgcatcg                                                              10


<210>  445
<211>  10
<212>  DNA
<213>  Artificial

<220>
<223>  synthetic DNA

<400>  445
tcccgactta                                                              10


<210>  446
<211>  10
<212>  DNA
<213>  Artificial

<220>
<223>  synthetic DNA

<400>  446
gtttttacac                                                              10


<210>  447
<211>  10
<212>  DNA
<213>  Artificial

<220>
<223>  synthetic DNA

<400>  447
cctgagcgtg                                                              10


<210>  448
<211>  10
<212>  DNA
<213>  Artificial

<220>
<223>  synthetic DNA

<400>  448
cggcattgta                                                              10


<210>  449
<211>  10
<212>  DNA
<213>  Artificial

<220>
<223>  synthetic DNA

<400>  449
tagagtgcgt                                                              10


<210>  450
<211>  10
<212>  DNA
<213>  Artificial

<220>
<223>  synthetic DNA

<400>  450
atggccagac                                                              10


<210>  451
<211>  10
<212>  DNA
<213>  Artificial

<220>
<223>  synthetic DNA

<400>  451
cttagcatgc                                                              10


<210>  452
<211>  10
<212>  DNA
<213>  Artificial

<220>
<223>  synthetic DNA

<400>  452
acaacacctg                                                              10


<210>  453
<211>  10
<212>  DNA
<213>  Artificial

<220>
<223>  synthetic DNA

<400>  453
agtgactatc                                                              10


<210>  454
<211>  10
<212>  DNA
<213>  Artificial

<220>
<223>  synthetic DNA

<400>  454
catgctacac                                                              10


<210>  455
<211>  10
<212>  DNA
<213>  Artificial

<220>
<223>  synthetic DNA

<400>  455
aaagcgggcg                                                              10

<210>  456
<211>  10
<212>  DNA
<213>  Artificial

<220>
<223>  synthetic DNA

<400>  456
agatcgccgt                                                              10


<210>  457
<211>  10
<212>  DNA
<213>  Artificial

<220>
<223>  synthetic DNA

<400>  457
cgtagatatt                                                              10


<210>  458
<211>  10
<212>  DNA
<213>  Artificial

<220>
<223>  synthetic DNA

<400>  458
aatggcagac                                                              10


<210>  459
<211>  10
<212>  DNA
<213>  Artificial

<220>
<223>  synthetic DNA

<400>  459
gtataacgtg                                                              10


<210>  460
<211>  10
<212>  DNA
<213>  Artificial

<220>
<223>  synthetic DNA

<400>  460
atgtgcgtca                                                              10


<210>  461
<211>  10

<212>  DNA
<213>  Artificial

<220>
<223>  synthetic DNA

<400>  461
cctgccaact                                                              10


<210>  462
<211>  10
<212>  DNA
<213>  Artificial

<220>
<223>  synthetic DNA

<400>  462
tttataactc                                                              10


<210>  463
<211>  10
<212>  DNA
<213>  Artificial

<220>
<223>  synthetic DNA

<400>  463
acggttacgc                                                              10


<210>  464
<211>  10
<212>  DNA
<213>  Artificial

<220>
<223>  synthetic DNA

<400>  464
tagcctcttg                                                              10


<210>  465
<211>  10
<212>  DNA
<213>  Artificial

<220>
<223>  synthetic DNA

<400>  465
tcgcgaagtt                                                              10


<210>  466
<211>  10
<212>  DNA
<213>  Artificial

<220>
<223>  synthetic DNA

<400>  466
gtctacaacc                                                              10


<210>  467
<211>  10
<212>  DNA
<213>  Artificial

<220>
<223>  synthetic DNA

<400>  467
gtctactgcg                                                              10


<210>  468
<211>  10
<212>  DNA
<213>  Artificial

<220>
<223>  synthetic DNA

<400>  468
gttgcgtctc                                                              10


<210>  469
<211>  10
<212>  DNA
<213>  Artificial

<220>
<223>  synthetic DNA

<400>  469
gggccgctaa                                                              10


<210>  470
<211>  10
<212>  DNA
<213>  Artificial

<220>
<223>  synthetic DNA

<400>  470
gtacgtcgga                                                              10


<210>  471
<211>  10
<212>  DNA
<213>  Artificial

<220>
<223>  synthetic DNA

<400>  471
agcgagagac                                                              10


<210>  472
<211>  10
<212>  DNA
<213>  Artificial

<220>
<223>  synthetic DNA

<400>  472
tggctacggt                                                              10


<210>  473
<211>  10
<212>  DNA
<213>  Artificial

<220>
<223>  synthetic DNA

<400>  473
aggcatcacg                                                              10


<210>  474
<211>  10
<212>  DNA
<213>  Artificial

<220>
<223>  synthetic DNA

<400>  474
tagctcctcg                                                              10


<210>  475
<211>  10
<212>  DNA
<213>  Artificial

<220>
<223>  synthetic DNA

<400>  475
ggctagtcag                                                              10


<210>  476
<211>  10
<212>  DNA
<213>  Artificial

<220>
<223>  synthetic DNA

<400>  476
ctcactttat                                                              10

<210>  477
<211>  10
<212>  DNA
<213>  Artificial

<220>
<223>  synthetic DNA

<400>  477
acggccacgt                                                              10


<210>  478
<211>  10
<212>  DNA
<213>  Artificial

<220>
<223>  synthetic DNA

<400>  478
agcgtatatc                                                              10


<210>  479
<211>  10
<212>  DNA
<213>  Artificial

<220>
<223>  synthetic DNA

<400>  479
gacacgtcta                                                              10


<210>  480
<211>  10
<212>  DNA
<213>  Artificial

<220>
<223>  synthetic DNA

<400>  480
gccagcgtac                                                              10


<210>  481
<211>  10
<212>  DNA
<213>  Artificial

<220>
<223>  synthetic DNA

<400>  481
aacattagcg                                                              10


<210>  482
<211>  10

<212>  DNA
<213>  Artificial

<220>
<223>  synthetic DNA

<400>  482
agtgtgctat                                                              10


<210>  483
<211>  10
<212>  DNA
<213>  Artificial

<220>
<223>  synthetic DNA

<400>  483
cacgagcgtt                                                              10


<210>  484
<211>  10
<212>  DNA
<213>  Artificial

<220>
<223>  synthetic DNA

<400>  484
gtaacgccta                                                              10


<210>  485
<211>  10
<212>  DNA
<213>  Artificial

<220>
<223>  synthetic DNA

<400>  485
cacatagtac                                                              10


<210>  486
<211>  10
<212>  DNA
<213>  Artificial

<220>
<223>  synthetic DNA

<400>  486
cgcgatatcg                                                              10


<210>  487
<211>  10
<212>  DNA
<213>  Artificial

<220>
<223>  synthetic DNA

<400>  487
cgttctgtgc                                                              10


<210>  488
<211>  10
<212>  DNA
<213>  Artificial

<220>
<223>  synthetic DNA

<400>  488
ctgatcgcat                                                              10


<210>  489
<211>  10
<212>  DNA
<213>  Artificial

<220>
<223>  synthetic DNA

<400>  489
tggcgtgaga                                                              10


<210>  490
<211>  10
<212>  DNA
<213>  Artificial

<220>
<223>  synthetic DNA

<400>  490
ttgccaggct                                                              10


<210>  491
<211>  10
<212>  DNA
<213>  Artificial

<220>
<223>  synthetic DNA

<400>  491
gttatacaca                                                              10


<210>  492
<211>  10
<212>  DNA
<213>  Artificial

<220>
<223>  synthetic DNA

<400>  492
agtgccaact                                                              10


<210>  493
<211>  10
<212>  DNA
<213>  Artificial

<220>
<223>  synthetic DNA

<400>  493
tcacgtagca                                                              10


<210>  494
<211>  10
<212>  DNA
<213>  Artificial

<220>
<223>  synthetic DNA

<400>  494
taattcagcg                                                              10


<210>  495
<211>  10
<212>  DNA
<213>  Artificial

<220>
<223>  synthetic DNA

<400>  495
aagtatcgtc                                                              10


<210>  496
<211>  10
<212>  DNA
<213>  Artificial

<220>
<223>  synthetic DNA

<400>  496
cacagttact                                                              10


<210>  497
<211>  10
<212>  DNA
<213>  Artificial

<220>
<223>  synthetic DNA

<400>  497
ccttaccgtg                                                              10

<210>  498
<211>  10
<212>  DNA
<213>  Artificial

<220>
<223>  synthetic DNA

<400>  498
acggtgtcgt                                                              10


<210>  499
<211>  10
<212>  DNA
<213>  Artificial

<220>
<223>  synthetic DNA

<400>  499
cgcgtaagac                                                              10


<210>  500
<211>  10
<212>  DNA
<213>  Artificial

<220>
<223>  synthetic DNA

<400>  500
ttcgcaccag                                                              10


<210>  501
<211>  10
<212>  DNA
<213>  Artificial

<220>
<223>  synthetic DNA

<400>  501
cacgaacaga                                                              10


<210>  502
<211>  10
<212>  DNA
<213>  Artificial

<220>
<223>  synthetic DNA

<400>  502
gttggacatt                                                              10


<210>  503
<211>  10

<212>  DNA
<213>  Artificial

<220>
<223>  synthetic DNA

<400>  503
ggtgcttaag                                                              10


<210>  504
<211>  10
<212>  DNA
<213>  Artificial

<220>
<223>  synthetic DNA

<400>  504
tcggtctcgt                                                              10


<210>  505
<211>  10
<212>  DNA
<213>  Artificial

<220>
<223>  synthetic DNA

<400>  505
tctagtacgc                                                              10


<210>  506
<211>  10
<212>  DNA
<213>  Artificial

<220>
<223>  synthetic DNA

<400>  506
ttaggccgag                                                              10


<210>  507
<211>  10
<212>  DNA
<213>  Artificial

<220>
<223>  synthetic DNA

<400>  507
cgtcaagagc                                                              10


<210>  508
<211>  10
<212>  DNA
<213>  Artificial

<220>
<223>  synthetic DNA

<400>  508
acatgtctac                                                              10


<210>  509
<211>  10
<212>  DNA
<213>  Artificial

<220>
<223>  synthetic DNA

<400>  509
atcgttacgt                                                              10


<210>  510
<211>  10
<212>  DNA
<213>  Artificial

<220>
<223>  synthetic DNA

<400>  510
acggatcgtt                                                              10


<210>  511
<211>  10
<212>  DNA
<213>  Artificial

<220>
<223>  synthetic DNA

<400>  511
aatcttggcg                                                              10


<210>  512
<211>  10
<212>  DNA
<213>  Artificial

<220>
<223>  synthetic DNA

<400>  512
agtatctggt                                                              10


<210>  513
<211>  10
<212>  DNA
<213>  Artificial

<220>
<223>  synthetic DNA

<400>  513
caaccgacgt                                                              10


<210>  514
<211>  10
<212>  DNA
<213>  Artificial

<220>
<223>  synthetic DNA

<400>  514
tggtaacgcg                                                              10


<210>  515
<211>  10
<212>  DNA
<213>  Artificial

<220>
<223>  synthetic DNA

<400>  515
gtgcagacat                                                              10


<210>  516
<211>  10
<212>  DNA
<213>  Artificial

<220>
<223>  synthetic DNA

<400>  516
gtctagttgc                                                              10


<210>  517
<211>  10
<212>  DNA
<213>  Artificial

<220>
<223>  synthetic DNA

<400>  517
caattcgacg                                                              10


<210>  518
<211>  10
<212>  DNA
<213>  Artificial

<220>
<223>  synthetic DNA

<400>  518
cttagcacct                                                              10

<210>  519
<211>  10
<212>  DNA
<213>  Artificial

<220>
<223>  synthetic DNA

<400>  519
taatgtcgca                                                              10


<210>  520
<211>  10
<212>  DNA
<213>  Artificial

<220>
<223>  synthetic DNA

<400>  520
caatcggtac                                                              10


<210>  521
<211>  10
<212>  DNA
<213>  Artificial

<220>
<223>  synthetic DNA

<400>  521
agcacgcatt                                                              10


<210>  522
<211>  10
<212>  DNA
<213>  Artificial

<220>
<223>  synthetic DNA

<400>  522
aggtcctcgt                                                              10


<210>  523
<211>  10
<212>  DNA
<213>  Artificial

<220>
<223>  synthetic DNA

<400>  523
ttgtgcctgc                                                              10


<210>  524
<211>  10

<212>  DNA
<213>  Artificial

<220>
<223>  synthetic DNA

<400>  524
accgcctgta                                                              10


<210>  525
<211>  10
<212>  DNA
<213>  Artificial

<220>
<223>  synthetic DNA

<400>  525
gtacgtcagg                                                              10


<210>  526
<211>  10
<212>  DNA
<213>  Artificial

<220>
<223>  synthetic DNA

<400>  526
gcacacaact                                                              10


<210>  527
<211>  10
<212>  DNA
<213>  Artificial

<220>
<223>  synthetic DNA

<400>  527
tgagcactta                                                              10


<210>  528
<211>  10
<212>  DNA
<213>  Artificial

<220>
<223>  synthetic DNA

<400>  528
gtgccgcata                                                              10


<210>  529
<211>  10
<212>  DNA
<213>  Artificial

<220>
<223>  synthetic DNA

<400>  529
atgttttcgc                                                              10


<210>  530
<211>  10
<212>  DNA
<213>  Artificial

<220>
<223>  synthetic DNA

<400>  530
acacttaggt                                                              10


<210>  531
<211>  10
<212>  DNA
<213>  Artificial

<220>
<223>  synthetic DNA

<400>  531
cgtgccgtga                                                              10


<210>  532
<211>  10
<212>  DNA
<213>  Artificial

<220>
<223>  synthetic DNA

<400>  532
ttactaatca                                                              10


<210>  533
<211>  10
<212>  DNA
<213>  Artificial

<220>
<223>  synthetic DNA

<400>  533
gtggcaggta                                                              10


<210>  534
<211>  10
<212>  DNA
<213>  Artificial

<220>
<223>  synthetic DNA

<400>  534
gcgcgatatg                                                              10


<210>  535
<211>  10
<212>  DNA
<213>  Artificial

<220>
<223>  synthetic DNA

<400>  535
gaacgacgtt                                                              10


<210>  536
<211>  10
<212>  DNA
<213>  Artificial

<220>
<223>  synthetic DNA

<400>  536
atcaggagtg                                                              10


<210>  537
<211>  10
<212>  DNA
<213>  Artificial

<220>
<223>  synthetic DNA

<400>  537
gccagtaagt                                                              10


<210>  538
<211>  10
<212>  DNA
<213>  Artificial

<220>
<223>  synthetic DNA

<400>  538
gcaagaagca                                                              10


<210>  539
<211>  10
<212>  DNA
<213>  Artificial

<220>
<223>  synthetic DNA

<400>  539
aactccgcca                                                              10

<210>  540
<211>  10
<212>  DNA
<213>  Artificial

<220>
<223>  synthetic DNA

<400>  540
acttgagcct                                                              10


<210>  541
<211>  10
<212>  DNA
<213>  Artificial

<220>
<223>  synthetic DNA

<400>  541
cgtgatcgtg                                                              10


<210>  542
<211>  10
<212>  DNA
<213>  Artificial

<220>
<223>  synthetic DNA

<400>  542
aattagcgaa                                                              10


<210>  543
<211>  10
<212>  DNA
<213>  Artificial

<220>
<223>  synthetic DNA

<400>  543
acttccttag                                                              10


<210>  544
<211>  10
<212>  DNA
<213>  Artificial

<220>
<223>  synthetic DNA

<400>  544
tgtgctgata                                                              10


<210>  545
<211>  10

<212>  DNA
<213>  Artificial

<220>
<223>  synthetic DNA

<400>  545
aggcggctga                                                              10


<210>  546
<211>  10
<212>  DNA
<213>  Artificial

<220>
<223>  synthetic DNA

<400>  546
cgtttagagc                                                              10


<210>  547
<211>  10
<212>  DNA
<213>  Artificial

<220>
<223>  synthetic DNA

<400>  547
acgcgtctaa                                                              10


<210>  548
<211>  10
<212>  DNA
<213>  Artificial

<220>
<223>  synthetic DNA

<400>  548
gcgaatgtac                                                              10


<210>  549
<211>  10
<212>  DNA
<213>  Artificial

<220>
<223>  synthetic DNA

<400>  549
cgtgatccaa                                                              10


<210>  550
<211>  10
<212>  DNA
<213>  Artificial

<220>
<223>  synthetic DNA

<400>  550
caaccagatg                                                              10


<210>  551
<211>  10
<212>  DNA
<213>  Artificial

<220>
<223>  synthetic DNA

<400>  551
accattaacc                                                              10


<210>  552
<211>  10
<212>  DNA
<213>  Artificial

<220>
<223>  synthetic DNA

<400>  552
cgattcacgt                                                              10


<210>  553
<211>  10
<212>  DNA
<213>  Artificial

<220>
<223>  synthetic DNA

<400>  553
ctagaacctg                                                              10


<210>  554
<211>  10
<212>  DNA
<213>  Artificial

<220>
<223>  synthetic DNA

<400>  554
cctaacgaca                                                              10


<210>  555
<211>  10
<212>  DNA
<213>  Artificial

<220>
<223>  synthetic DNA

<400>  555
gacgtgcatg                                                              10


<210>  556
<211>  10
<212>  DNA
<213>  Artificial

<220>
<223>  synthetic DNA

<400>  556
atgtaacctt                                                              10


<210>  557
<211>  10
<212>  DNA
<213>  Artificial

<220>
<223>  synthetic DNA

<400>  557
gatacagtcg                                                              10


<210>  558
<211>  10
<212>  DNA
<213>  Artificial

<220>
<223>  synthetic DNA

<400>  558
cgtatgtctc                                                              10


<210>  559
<211>  10
<212>  DNA
<213>  Artificial

<220>
<223>  synthetic DNA

<400>  559
agattatcga                                                              10


<210>  560
<211>  10
<212>  DNA
<213>  Artificial

<220>
<223>  synthetic DNA

<400>  560
atactggtaa                                                              10

<210>  561
<211>  10
<212>  DNA
<213>  Artificial

<220>
<223>  synthetic DNA

<400>  561
gttgagtagc                                                              10


<210>  562
<211>  10
<212>  DNA
<213>  Artificial

<220>
<223>  synthetic DNA

<400>  562
accattatca                                                              10


<210>  563
<211>  10
<212>  DNA
<213>  Artificial

<220>
<223>  synthetic DNA

<400>  563
cacacttcag                                                              10


<210>  564
<211>  10
<212>  DNA
<213>  Artificial

<220>
<223>  synthetic DNA

<400>  564
gactagcggt                                                              10


<210>  565
<211>  10
<212>  DNA
<213>  Artificial

<220>
<223>  synthetic DNA

<400>  565
aattgtcgag                                                              10


<210>  566
<211>  10

<212>  DNA
<213>  Artificial

<220>
<223>  synthetic DNA

<400>  566
ctaaggacgt                                                              10


<210>  567
<211>  10
<212>  DNA
<213>  Artificial

<220>
<223>  synthetic DNA

<400>  567
attacgatga                                                              10


<210>  568
<211>  10
<212>  DNA
<213>  Artificial

<220>
<223>  synthetic DNA

<400>  568
attgaagact                                                              10


<210>  569
<211>  10
<212>  DNA
<213>  Artificial

<220>
<223>  synthetic DNA

<400>  569
gcttgtacgt                                                              10


<210>  570
<211>  10
<212>  DNA
<213>  Artificial

<220>
<223>  synthetic DNA

<400>  570
cctacgtcac                                                              10


<210>  571
<211>  10
<212>  DNA
<213>  Artificial

<220>
<223>  synthetic DNA

<400>  571
cacaacttag                                                              10


<210>  572
<211>  10
<212>  DNA
<213>  Artificial

<220>
<223>  synthetic DNA

<400>  572
gcggttcatc                                                              10


<210>  573
<211>  10
<212>  DNA
<213>  Artificial

<220>
<223>  synthetic DNA

<400>  573
gtactcatct                                                              10


<210>  574
<211>  10
<212>  DNA
<213>  Artificial

<220>
<223>  synthetic DNA

<400>  574
gtgcatcagt                                                              10


<210>  575
<211>  10
<212>  DNA
<213>  Artificial

<220>
<223>  synthetic DNA

<400>  575
tcacatccta                                                              10


<210>  576
<211>  10
<212>  DNA
<213>  Artificial

<220>
<223>  synthetic DNA

<400>  576
cacgcgctat                                                              10


<210>  577
<211>  8
<212>  DNA
<213>  Artificial

<220>
<223>  synthetic DNA

<400>  577
ctatcttg                                                                 8


<210>  578
<211>  8
<212>  DNA
<213>  Artificial

<220>
<223>  synthetic DNA

<400>  578
aagtgcgt                                                                 8


<210>  579
<211>  8
<212>  DNA
<213>  Artificial

<220>
<223>  synthetic DNA

<400>  579
acatgcga                                                                 8


<210>  580
<211>  8
<212>  DNA
<213>  Artificial

<220>
<223>  synthetic DNA

<400>  580
accaatgg                                                                 8


<210>  581
<211>  8
<212>  DNA
<213>  Artificial

<220>
<223>  synthetic DNA

<400>  581
tgcgttga                                                                 8

<210>  582
<211>  8
<212>  DNA
<213>  Artificial

<220>
<223>  synthetic DNA

<400>  582
gacatgtc                                                                 8


<210>  583
<211>  8
<212>  DNA
<213>  Artificial

<220>
<223>  synthetic DNA

<400>  583
ttgtgcgt                                                                 8


<210>  584
<211>  8
<212>  DNA
<213>  Artificial

<220>
<223>  synthetic DNA

<400>  584
acatcgca                                                                 8


<210>  585
<211>  8
<212>  DNA
<213>  Artificial

<220>
<223>  synthetic DNA

<400>  585
gaagacga                                                                 8


<210>  586
<211>  8
<212>  DNA
<213>  Artificial

<220>
<223>  synthetic DNA

<400>  586
tcgataga                                                                 8


<210>  587
<211>  8

<212>  DNA
<213>  Artificial

<220>
<223>  synthetic DNA

<400>  587
tcttgcaa                                                                 8


<210>  588
<211>  8
<212>  DNA
<213>  Artificial

<220>
<223>  synthetic DNA

<400>  588
agcaagtt                                                                 8


<210>  589
<211>  8
<212>  DNA
<213>  Artificial

<220>
<223>  synthetic DNA

<400>  589
ttcatgga                                                                 8


<210>  590
<211>  8
<212>  DNA
<213>  Artificial

<220>
<223>  synthetic DNA

<400>  590
tcaattcg                                                                 8


<210>  591
<211>  8
<212>  DNA
<213>  Artificial

<220>
<223>  synthetic DNA

<400>  591
cggtatgt                                                                 8


<210>  592
<211>  8
<212>  DNA
<213>  Artificial

<220>
<223>  synthetic DNA

<400>  592
accactac                                                                 8


<210>  593
<211>  8
<212>  DNA
<213>  Artificial

<220>
<223>  synthetic DNA

<400>  593
tcgcttat                                                                 8


<210>  594
<211>  8
<212>  DNA
<213>  Artificial

<220>
<223>  synthetic DNA

<400>  594
tctcgact                                                                 8


<210>  595
<211>  8
<212>  DNA
<213>  Artificial

<220>
<223>  synthetic DNA

<400>  595
gaatcggt                                                                 8


<210>  596
<211>  8
<212>  DNA
<213>  Artificial

<220>
<223>  synthetic DNA

<400>  596
gttacaag                                                                 8


<210>  597
<211>  8
<212>  DNA
<213>  Artificial

<220>
<223>  synthetic DNA

<400>  597
ctgtgtag                                                                 8


<210>  598
<211>  8
<212>  DNA
<213>  Artificial

<220>
<223>  synthetic DNA

<400>  598
tggtagaa                                                                 8


<210>  599
<211>  8
<212>  DNA
<213>  Artificial

<220>
<223>  synthetic DNA

<400>  599
atactgcg                                                                 8


<210>  600
<211>  8
<212>  DNA
<213>  Artificial

<220>
<223>  synthetic DNA

<400>  600
aactcgtc                                                                 8


<210>  601
<211>  8
<212>  DNA
<213>  Artificial

<220>
<223>  synthetic DNA

<400>  601
atatgtgc                                                                 8


<210>  602
<211>  8
<212>  DNA
<213>  Artificial

<220>
<223>  synthetic DNA

<400>  602
aagttgcg                                                                 8

<210>  603
<211>  8
<212>  DNA
<213>  Artificial

<220>
<223>  synthetic DNA

<400>  603
gatcatgt                                                                 8


<210>  604
<211>  8
<212>  DNA
<213>  Artificial

<220>
<223>  synthetic DNA

<400>  604
ttgttgct                                                                 8


<210>  605
<211>  8
<212>  DNA
<213>  Artificial

<220>
<223>  synthetic DNA

<400>  605
cctcttag                                                                 8


<210>  606
<211>  8
<212>  DNA
<213>  Artificial

<220>
<223>  synthetic DNA

<400>  606
tcacagct                                                                 8


<210>  607
<211>  8
<212>  DNA
<213>  Artificial

<220>
<223>  synthetic DNA

<400>  607
agattgac                                                                 8


<210>  608
<211>  8

<212>  DNA
<213>  Artificial

<220>
<223>  synthetic DNA

<400>  608
agcctgat                                                                8


<210>  609
<211>  8
<212>  DNA
<213>  Artificial

<220>
<223>  synthetic DNA

<400>  609
cgtcaagt                                                                8


<210>  610
<211>  8
<212>  DNA
<213>  Artificial

<220>
<223>  synthetic DNA

<400>  610
aagtagac                                                                8


<210>  611
<211>  8
<212>  DNA
<213>  Artificial

<220>
<223>  synthetic DNA

<400>  611
tcagacaa                                                                8


<210>  612
<211>  8
<212>  DNA
<213>  Artificial

<220>
<223>  synthetic DNA

<400>  612
tccttgac                                                                8


<210>  613
<211>  8
<212>  DNA
<213>  Artificial

<220>
<223>  synthetic DNA

<400>  613
gtagctgt                                                                 8


<210>  614
<211>  8
<212>  DNA
<213>  Artificial

<220>
<223>  synthetic DNA

<400>  614
cgtcgtaa                                                                 8


<210>  615
<211>  8
<212>  DNA
<213>  Artificial

<220>
<223>  synthetic DNA

<400>  615
ccaatgga                                                                 8


<210>  616
<211>  8
<212>  DNA
<213>  Artificial

<220>
<223>  synthetic DNA

<400>  616
ttgagaga                                                                 8


<210>  617
<211>  8
<212>  DNA
<213>  Artificial

<220>
<223>  synthetic DNA

<400>  617
acaacacc                                                                 8


<210>  618
<211>  8
<212>  DNA
<213>  Artificial

<220>
<223>  synthetic DNA

<400>  618
tctagtac                                                                 8


<210>  619
<211>  8
<212>  DNA
<213>  Artificial

<220>
<223>  synthetic DNA

<400>  619
gaggaagt                                                                 8


<210>  620
<211>  8
<212>  DNA
<213>  Artificial

<220>
<223>  synthetic DNA

<400>  620
gcgtattg                                                                 8


<210>  621
<211>  8
<212>  DNA
<213>  Artificial

<220>
<223>  synthetic DNA

<400>  621
aagtagct                                                                 8


<210>  622
<211>  8
<212>  DNA
<213>  Artificial

<220>
<223>  synthetic DNA

<400>  622
tgaacctt                                                                 8


<210>  623
<211>  8
<212>  DNA
<213>  Artificial

<220>
<223>  synthetic DNA

<400>  623
tgtgttac                                                                 8

<210>  624
<211>  8
<212>  DNA
<213>  Artificial

<220>
<223>  synthetic DNA

<400>  624
taacctga                                                                8


<210>  625
<211>  8
<212>  DNA
<213>  Artificial

<220>
<223>  synthetic DNA

<400>  625
gctattcc                                                                8


<210>  626
<211>  8
<212>  DNA
<213>  Artificial

<220>
<223>  synthetic DNA

<400>  626
gttagatg                                                                8


<210>  627
<211>  8
<212>  DNA
<213>  Artificial

<220>
<223>  synthetic DNA

<400>  627
caggataa                                                                8


<210>  628
<211>  8
<212>  DNA
<213>  Artificial

<220>
<223>  synthetic DNA

<400>  628
accgtagt                                                                8


<210>  629
<211>  8

<212>  DNA
<213>  Artificial

<220>
<223>  synthetic DNA

<400>  629
ccgtgtat                                                                 8


<210>  630
<211>  8
<212>  DNA
<213>  Artificial

<220>
<223>  synthetic DNA

<400>  630
tccactct                                                                 8


<210>  631
<211>  8
<212>  DNA
<213>  Artificial

<220>
<223>  synthetic DNA

<400>  631
tagctcat                                                                 8


<210>  632
<211>  8
<212>  DNA
<213>  Artificial

<220>
<223>  synthetic DNA

<400>  632
cgctaata                                                                 8


<210>  633
<211>  8
<212>  DNA
<213>  Artificial

<220>
<223>  synthetic DNA

<400>  633
tacctctg                                                                 8


<210>  634
<211>  8
<212>  DNA
<213>  Artificial

<220>
<223>  synthetic DNA

<400>  634
tgcactac                                                                 8


<210>  635
<211>  8
<212>  DNA
<213>  Artificial

<220>
<223>  synthetic DNA

<400>  635
cttggaag                                                                 8


<210>  636
<211>  8
<212>  DNA
<213>  Artificial

<220>
<223>  synthetic DNA

<400>  636
aatgcacg                                                                 8


<210>  637
<211>  8
<212>  DNA
<213>  Artificial

<220>
<223>  synthetic DNA

<400>  637
cactgtta                                                                 8


<210>  638
<211>  8
<212>  DNA
<213>  Artificial

<220>
<223>  synthetic DNA

<400>  638
tcgactag                                                                 8


<210>  639
<211>  8
<212>  DNA
<213>  Artificial

<220>
<223>  synthetic DNA

<400>  639
ctaggtta                                                                 8


<210>  640
<211>  8
<212>  DNA
<213>  Artificial

<220>
<223>  synthetic DNA

<400>  640
gcagatgt                                                                 8


<210>  641
<211>  8
<212>  DNA
<213>  Artificial

<220>
<223>  synthetic DNA

<400>  641
agttcaga                                                                 8


<210>  642
<211>  8
<212>  DNA
<213>  Artificial

<220>
<223>  synthetic DNA

<400>  642
ctccatca                                                                 8


<210>  643
<211>  8
<212>  DNA
<213>  Artificial

<220>
<223>  synthetic DNA

<400>  643
tggttacg                                                                 8


<210>  644
<211>  8
<212>  DNA
<213>  Artificial

<220>
<223>  synthetic DNA

<400>  644
acgtagca                                                                 8

<210>  645
<211>  8
<212>  DNA
<213>  Artificial

<220>
<223>  synthetic DNA

<400>  645
ctcttcca                                                                8


<210>  646
<211>  8
<212>  DNA
<213>  Artificial

<220>
<223>  synthetic DNA

<400>  646
cgtcagat                                                                8


<210>  647
<211>  8
<212>  DNA
<213>  Artificial

<220>
<223>  synthetic DNA

<400>  647
tggatcat                                                                8


<210>  648
<211>  8
<212>  DNA
<213>  Artificial

<220>
<223>  synthetic DNA

<400>  648
atatcgac                                                                8


<210>  649
<211>  8
<212>  DNA
<213>  Artificial

<220>
<223>  synthetic DNA

<400>  649
ttgtggag                                                                8


<210>  650
<211>  8

<212>  DNA
<213>  Artificial

<220>
<223>  synthetic DNA

<400>  650
ttagagca                                                                 8


<210>  651
<211>  8
<212>  DNA
<213>  Artificial

<220>
<223>  synthetic DNA

<400>  651
taactacc                                                                 8


<210>  652
<211>  8
<212>  DNA
<213>  Artificial

<220>
<223>  synthetic DNA

<400>  652
ctatgagg                                                                 8


<210>  653
<211>  8
<212>  DNA
<213>  Artificial

<220>
<223>  synthetic DNA

<400>  653
cttctcac                                                                 8


<210>  654
<211>  8
<212>  DNA
<213>  Artificial

<220>
<223>  synthetic DNA

<400>  654
cgttctct                                                                 8


<210>  655
<211>  8
<212>  DNA
<213>  Artificial

<220>
<223>  synthetic DNA

<400>  655
gtcactat                                                                 8


<210>  656
<211>  8
<212>  DNA
<213>  Artificial

<220>
<223>  synthetic DNA

<400>  656
tcgttagc                                                                 8


<210>  657
<211>  8
<212>  DNA
<213>  Artificial

<220>
<223>  synthetic DNA

<400>  657
atcgtgta                                                                 8


<210>  658
<211>  8
<212>  DNA
<213>  Artificial

<220>
<223>  synthetic DNA

<400>  658
gagagcaa                                                                 8


<210>  659
<211>  8
<212>  DNA
<213>  Artificial

<220>
<223>  synthetic DNA

<400>  659
agacgcaa                                                                 8


<210>  660
<211>  8
<212>  DNA
<213>  Artificial

<220>
<223>  synthetic DNA

<400>  660
tccagtta                                                                 8


<210>  661
<211>  8
<212>  DNA
<213>  Artificial

<220>
<223>  synthetic DNA

<400>  661
aatgccac                                                                 8


<210>  662
<211>  8
<212>  DNA
<213>  Artificial

<220>
<223>  synthetic DNA

<400>  662
atcacgtg                                                                 8


<210>  663
<211>  8
<212>  DNA
<213>  Artificial

<220>
<223>  synthetic DNA

<400>  663
actgtgca                                                                 8


<210>  664
<211>  8
<212>  DNA
<213>  Artificial

<220>
<223>  synthetic DNA

<400>  664
tcactgca                                                                 8


<210>  665
<211>  8
<212>  DNA
<213>  Artificial

<220>
<223>  synthetic DNA

<400>  665
gcatccaa                                                                 8

<210>  666
<211>  8
<212>  DNA
<213>  Artificial

<220>
<223>  synthetic DNA

<400>  666
agcactat                                                                 8


<210>  667
<211>  8
<212>  DNA
<213>  Artificial

<220>
<223>  synthetic DNA

<400>  667
cgaaggat                                                                 8


<210>  668
<211>  8
<212>  DNA
<213>  Artificial

<220>
<223>  synthetic DNA

<400>  668
ccttgtgt                                                                 8


<210>  669
<211>  8
<212>  DNA
<213>  Artificial

<220>
<223>  synthetic DNA

<400>  669
tgcggata                                                                 8


<210>  670
<211>  8
<212>  DNA
<213>  Artificial

<220>
<223>  synthetic DNA

<400>  670
aggaatgg                                                                 8


<210>  671
<211>  8

<212>  DNA
<213>  Artificial

<220>
<223>  synthetic DNA

<400>  671
atcgtaac                                                                 8


<210>  672
<211>  8
<212>  DNA
<213>  Artificial

<220>
<223>  synthetic DNA

<400>  672
gaatgtct                                                                 8


<210>  673
<211>  9
<212>  DNA
<213>  Artificial

<220>
<223>  synthetic DNA

<400>  673
ttgctacat                                                                9


<210>  674
<211>  9
<212>  DNA
<213>  Artificial

<220>
<223>  synthetic DNA

<400>  674
taacgtatg                                                                9


<210>  675
<211>  9
<212>  DNA
<213>  Artificial

<220>
<223>  synthetic DNA

<400>  675
cagtatgta                                                                9


<210>  676
<211>  9
<212>  DNA
<213>  Artificial

<220>
<223>  synthetic DNA

<400>  676
tcaataacg                                                                9


<210>  677
<211>  9
<212>  DNA
<213>  Artificial

<220>
<223>  synthetic DNA

<400>  677
cacacttat                                                                9


<210>  678
<211>  9
<212>  DNA
<213>  Artificial

<220>
<223>  synthetic DNA

<400>  678
gactgtaat                                                                9


<210>  679
<211>  9
<212>  DNA
<213>  Artificial

<220>
<223>  synthetic DNA

<400>  679
tatacactg                                                                9


<210>  680
<211>  9
<212>  DNA
<213>  Artificial

<220>
<223>  synthetic DNA

<400>  680
actgcatta                                                                9


<210>  681
<211>  9
<212>  DNA
<213>  Artificial

<220>
<223>  synthetic DNA

<400>  681
acattaagc                                                                 9


<210>  682
<211>  9
<212>  DNA
<213>  Artificial

<220>
<223>  synthetic DNA

<400>  682
catattacg                                                                 9


<210>  683
<211>  9
<212>  DNA
<213>  Artificial

<220>
<223>  synthetic DNA

<400>  683
atatctacg                                                                 9


<210>  684
<211>  9
<212>  DNA
<213>  Artificial

<220>
<223>  synthetic DNA

<400>  684
agtaactgt                                                                 9


<210>  685
<211>  9
<212>  DNA
<213>  Artificial

<220>
<223>  synthetic DNA

<400>  685
atgacgtta                                                                 9


<210>  686
<211>  9
<212>  DNA
<213>  Artificial

<220>
<223>  synthetic DNA

<400>  686
attatgcga                                                                 9

<210>  687
<211>  9
<212>  DNA
<213>  Artificial

<220>
<223>  synthetic DNA

<400>  687
agtatacac                                                                9


<210>  688
<211>  9
<212>  DNA
<213>  Artificial

<220>
<223>  synthetic DNA

<400>  688
ttagcgtta                                                                9


<210>  689
<211>  9
<212>  DNA
<213>  Artificial

<220>
<223>  synthetic DNA

<400>  689
tatgacact                                                                9


<210>  690
<211>  9
<212>  DNA
<213>  Artificial

<220>
<223>  synthetic DNA

<400>  690
attaacgct                                                                9


<210>  691
<211>  9
<212>  DNA
<213>  Artificial

<220>
<223>  synthetic DNA

<400>  691
taggacaat                                                                9


<210>  692
<211>  9

<212>  DNA
<213>  Artificial

<220>
<223>  synthetic DNA

<400>  692
aagacgtta                                                                9


<210>  693
<211>  9
<212>  DNA
<213>  Artificial

<220>
<223>  synthetic DNA

<400>  693
tataagcgt                                                                9


<210>  694
<211>  9
<212>  DNA
<213>  Artificial

<220>
<223>  synthetic DNA

<400>  694
atacctggc                                                                9


<210>  695
<211>  9
<212>  DNA
<213>  Artificial

<220>
<223>  synthetic DNA

<400>  695
ctcgagatc                                                                9


<210>  696
<211>  9
<212>  DNA
<213>  Artificial

<220>
<223>  synthetic DNA

<400>  696
atggtgagg                                                                9


<210>  697
<211>  9
<212>  DNA
<213>  Artificial

<220>
<223>  synthetic DNA

<400>  697
atgtcgacg                                                                9


<210>  698
<211>  9
<212>  DNA
<213>  Artificial

<220>
<223>  synthetic DNA

<400>  698
gacgtctga                                                                9


<210>  699
<211>  9
<212>  DNA
<213>  Artificial

<220>
<223>  synthetic DNA

<400>  699
tacactgcg                                                                9


<210>  700
<211>  9
<212>  DNA
<213>  Artificial

<220>
<223>  synthetic DNA

<400>  700
atcgtcagg                                                                9


<210>  701
<211>  9
<212>  DNA
<213>  Artificial

<220>
<223>  synthetic DNA

<400>  701
tgcacgtac                                                                9


<210>  702
<211>  9
<212>  DNA
<213>  Artificial

<220>
<223>  synthetic DNA

<400>  702
gtcgtgcat                                                                9

<210>  703
<211>  9
<212>  DNA
<213>  Artificial

<220>
<223>  synthetic DNA

<400>  703
gagtgttac                                                                9

<210>  704
<211>  9
<212>  DNA
<213>  Artificial

<220>
<223>  synthetic DNA

<400>  704
agactgtac                                                                9

<210>  705
<211>  9
<212>  DNA
<213>  Artificial

<220>
<223>  synthetic DNA

<400>  705
tgcgactta                                                                9

<210>  706
<211>  9
<212>  DNA
<213>  Artificial

<220>
<223>  synthetic DNA

<400>  706
tgtccgtaa                                                                9

<210>  707
<211>  9
<212>  DNA
<213>  Artificial

<220>
<223>  synthetic DNA

<400>  707
gtaatcgag                                                                9

<210>  708
<211>  9
<212>  DNA
<213>  Artificial

<220>
<223>  synthetic DNA

<400>  708
gtaccttag                                                                9


<210>  709
<211>  9
<212>  DNA
<213>  Artificial

<220>
<223>  synthetic DNA

<400>  709
atcacgtgt                                                                9


<210>  710
<211>  9
<212>  DNA
<213>  Artificial

<220>
<223>  synthetic DNA

<400>  710
acttagcgt                                                                9


<210>  711
<211>  9
<212>  DNA
<213>  Artificial

<220>
<223>  synthetic DNA

<400>  711
gtaatcgtg                                                                9


<210>  712
<211>  9
<212>  DNA
<213>  Artificial

<220>
<223>  synthetic DNA

<400>  712
atgccgtta                                                                9


<210>  713
<211>  9

<212>  DNA
<213>  Artificial

<220>
<223>  synthetic DNA

<400>  713
ataacgtgc                                                                9


<210>  714
<211>  9
<212>  DNA
<213>  Artificial

<220>
<223>  synthetic DNA

<400>  714
ctacgttgt                                                                9


<210>  715
<211>  9
<212>  DNA
<213>  Artificial

<220>
<223>  synthetic DNA

<400>  715
tatgacgca                                                                9


<210>  716
<211>  9
<212>  DNA
<213>  Artificial

<220>
<223>  synthetic DNA

<400>  716
ccgataaca                                                                9


<210>  717
<211>  9
<212>  DNA
<213>  Artificial

<220>
<223>  synthetic DNA

<400>  717
atgcgcata                                                                9


<210>  718
<211>  9
<212>  DNA
<213>  Artificial

<220>
<223>  synthetic DNA

<400>  718
gataagcgt                                                                9


<210>  719
<211>  9
<212>  DNA
<213>  Artificial

<220>
<223>  synthetic DNA

<400>  719
atatctgcg                                                                9


<210>  720
<211>  9
<212>  DNA
<213>  Artificial

<220>
<223>  synthetic DNA

<400>  720
acttagacg                                                                9


<210>  721
<211>  9
<212>  DNA
<213>  Artificial

<220>
<223>  synthetic DNA

<400>  721
atcaccgta                                                                9


<210>  722
<211>  9
<212>  DNA
<213>  Artificial

<220>
<223>  synthetic DNA

<400>  722
taagacacg                                                                9


<210>  723
<211>  9
<212>  DNA
<213>  Artificial

<220>
<223>  synthetic DNA

<400>  723
aatgccgta                                                                9

<210>  724
<211>  9
<212>  DNA
<213>  Artificial

<220>
<223>  synthetic DNA

<400>  724
aatcacgtg                                                                9

<210>  725
<211>  9
<212>  DNA
<213>  Artificial

<220>
<223>  synthetic DNA

<400>  725
tcgttagtc                                                                9

<210>  726
<211>  9
<212>  DNA
<213>  Artificial

<220>
<223>  synthetic DNA

<400>  726
catcatgtc                                                                9

<210>  727
<211>  9
<212>  DNA
<213>  Artificial

<220>
<223>  synthetic DNA

<400>  727
taagacggt                                                                9

<210>  728
<211>  9
<212>  DNA
<213>  Artificial

<220>
<223>  synthetic DNA

<400>  728
tgcatagtg                                                                9

<210>  729
<211>  9
<212>  DNA
<213>  Artificial

<220>
<223>  synthetic DNA

<400>  729
gagcgttat                                                                9


<210>  730
<211>  9
<212>  DNA
<213>  Artificial

<220>
<223>  synthetic DNA

<400>  730
tgccttaca                                                                9


<210>  731
<211>  9
<212>  DNA
<213>  Artificial

<220>
<223>  synthetic DNA

<400>  731
ttcgcgtta                                                                9


<210>  732
<211>  9
<212>  DNA
<213>  Artificial

<220>
<223>  synthetic DNA

<400>  732
gtgttaacg                                                                9


<210>  733
<211>  9
<212>  DNA
<213>  Artificial

<220>
<223>  synthetic DNA

<400>  733
gacactgaa                                                                9


<210>  734
<211>  9

<212>  DNA
<213>  Artificial

<220>
<223>  synthetic DNA

<400>  734
ctgttatcg                                                                9


<210>  735
<211>  9
<212>  DNA
<213>  Artificial

<220>
<223>  synthetic DNA

<400>  735
ggtcgttat                                                                9


<210>  736
<211>  9
<212>  DNA
<213>  Artificial

<220>
<223>  synthetic DNA

<400>  736
cgagagtat                                                                9


<210>  737
<211>  9
<212>  DNA
<213>  Artificial

<220>
<223>  synthetic DNA

<400>  737
atacagtcc                                                                9


<210>  738
<211>  9
<212>  DNA
<213>  Artificial

<220>
<223>  synthetic DNA

<400>  738
aattcacgc                                                                9


<210>  739
<211>  9
<212>  DNA
<213>  Artificial

<220>
<223>  synthetic DNA

<400>  739
tatgtgcac                                                                9


<210>  740
<211>  9
<212>  DNA
<213>  Artificial

<220>
<223>  synthetic DNA

<400>  740
gatgacgta                                                                9


<210>  741
<211>  9
<212>  DNA
<213>  Artificial

<220>
<223>  synthetic DNA

<400>  741
gatgcgata                                                                9


<210>  742
<211>  9
<212>  DNA
<213>  Artificial

<220>
<223>  synthetic DNA

<400>  742
gagcgatta                                                                9


<210>  743
<211>  9
<212>  DNA
<213>  Artificial

<220>
<223>  synthetic DNA

<400>  743
tgtcacaga                                                                9


<210>  744
<211>  9
<212>  DNA
<213>  Artificial

<220>
<223>  synthetic DNA

<400>  744
tactaaccg                                                                9

<210>  745
<211>  9
<212>  DNA
<213>  Artificial

<220>
<223>  synthetic DNA

<400>  745
cataacgag                                                                9

<210>  746
<211>  9
<212>  DNA
<213>  Artificial

<220>
<223>  synthetic DNA

<400>  746
cgtatacct                                                                9

<210>  747
<211>  9
<212>  DNA
<213>  Artificial

<220>
<223>  synthetic DNA

<400>  747
tatcacgtg                                                                9

<210>  748
<211>  9
<212>  DNA
<213>  Artificial

<220>
<223>  synthetic DNA

<400>  748
gaacgttac                                                                9

<210>  749
<211>  9
<212>  DNA
<213>  Artificial

<220>
<223>  synthetic DNA

<400>  749
gtcgtatac                                                                9

<210>  750
<211>  9
<212>  DNA
<213>  Artificial

<220>
<223>  synthetic DNA

<400>  750
atgtcgaca                                                                9


<210>  751
<211>  9
<212>  DNA
<213>  Artificial

<220>
<223>  synthetic DNA

<400>  751
atacagcac                                                                9


<210>  752
<211>  9
<212>  DNA
<213>  Artificial

<220>
<223>  synthetic DNA

<400>  752
tacttacgc                                                                9


<210>  753
<211>  9
<212>  DNA
<213>  Artificial

<220>
<223>  synthetic DNA

<400>  753
aactacggt                                                                9


<210>  754
<211>  9
<212>  DNA
<213>  Artificial

<220>
<223>  synthetic DNA

<400>  754
tagaacggt                                                                9


<210>  755
<211>  9

<212>  DNA
<213>  Artificial

<220>
<223>  synthetic DNA

<400>  755
gaatgtcac                                                                9


<210>  756
<211>  9
<212>  DNA
<213>  Artificial

<220>
<223>  synthetic DNA

<400>  756
tgtacgtct                                                                9


<210>  757
<211>  9
<212>  DNA
<213>  Artificial

<220>
<223>  synthetic DNA

<400>  757
aacattgcg                                                                9


<210>  758
<211>  9
<212>  DNA
<213>  Artificial

<220>
<223>  synthetic DNA

<400>  758
ttgaacgct                                                                9


<210>  759
<211>  9
<212>  DNA
<213>  Artificial

<220>
<223>  synthetic DNA

<400>  759
aatcaggac                                                                9


<210>  760
<211>  9
<212>  DNA
<213>  Artificial

<220>
<223>  synthetic DNA

<400>  760
attcgcaca                                                                9


<210>  761
<211>  9
<212>  DNA
<213>  Artificial

<220>
<223>  synthetic DNA

<400>  761
ccatgtact                                                                9


<210>  762
<211>  9
<212>  DNA
<213>  Artificial

<220>
<223>  synthetic DNA

<400>  762
tgtcctgtt                                                                9


<210>  763
<211>  9
<212>  DNA
<213>  Artificial

<220>
<223>  synthetic DNA

<400>  763
taattgcgc                                                                9


<210>  764
<211>  9
<212>  DNA
<213>  Artificial

<220>
<223>  synthetic DNA

<400>  764
gatagtgtg                                                                9


<210>  765
<211>  9
<212>  DNA
<213>  Artificial

<220>
<223>  synthetic DNA

<400>  765
atagacgca                                                                9

<210>  766
<211>  9
<212>  DNA
<213>  Artificial

<220>
<223>  synthetic DNA

<400>  766
tgtaccgtt                                                                9

<210>  767
<211>  9
<212>  DNA
<213>  Artificial

<220>
<223>  synthetic DNA

<400>  767
attgtcgca                                                                9

<210>  768
<211>  9
<212>  DNA
<213>  Artificial

<220>
<223>  synthetic DNA

<400>  768
gtcacgtaa                                                                9

<210>  769
<211>  11
<212>  DNA
<213>  Artificial

<220>
<223>  synthetic DNA

<400>  769
ttacactatg c                                                            11

<210>  770
<211>  11
<212>  DNA
<213>  Artificial

<220>
<223>  synthetic DNA

<400>  770
gcgatagtcg t                                                            11

<210>  771
<211>  11
<212>  DNA
<213>  Artificial

<220>
<223>  synthetic DNA

<400>  771
ctattcacag t                                                            11


<210>  772
<211>  11
<212>  DNA
<213>  Artificial

<220>
<223>  synthetic DNA

<400>  772
agagtcactg t                                                            11


<210>  773
<211>  11
<212>  DNA
<213>  Artificial

<220>
<223>  synthetic DNA

<400>  773
agagtcgaag c                                                            11


<210>  774
<211>  11
<212>  DNA
<213>  Artificial

<220>
<223>  synthetic DNA

<400>  774
ctgaatatgt g                                                            11


<210>  775
<211>  11
<212>  DNA
<213>  Artificial

<220>
<223>  synthetic DNA

<400>  775
actccacagg a                                                            11


<210>  776
<211>  11

<212>  DNA
<213>  Artificial

<220>
<223>  synthetic DNA

<400>  776
atcctcgtaa g                                                            11


<210>  777
<211>  11
<212>  DNA
<213>  Artificial

<220>
<223>  synthetic DNA

<400>  777
taccatcgcc t                                                            11


<210>  778
<211>  11
<212>  DNA
<213>  Artificial

<220>
<223>  synthetic DNA

<400>  778
aacgcctata a                                                            11


<210>  779
<211>  11
<212>  DNA
<213>  Artificial

<220>
<223>  synthetic DNA

<400>  779
ctgtcgaact t                                                            11


<210>  780
<211>  11
<212>  DNA
<213>  Artificial

<220>
<223>  synthetic DNA

<400>  780
tcagatgtcc g                                                            11


<210>  781
<211>  11
<212>  DNA
<213>  Artificial

<220>
<223>  synthetic DNA

<400>  781
ctgcttatcg t                                                            11


<210>  782
<211>  11
<212>  DNA
<213>  Artificial

<220>
<223>  synthetic DNA

<400>  782
acattcgcac a                                                            11


<210>  783
<211>  11
<212>  DNA
<213>  Artificial

<220>
<223>  synthetic DNA

<400>  783
ccttaatgca t                                                            11


<210>  784
<211>  11
<212>  DNA
<213>  Artificial

<220>
<223>  synthetic DNA

<400>  784
ggctagctac t                                                            11


<210>  785
<211>  11
<212>  DNA
<213>  Artificial

<220>
<223>  synthetic DNA

<400>  785
ttccagttgg c                                                            11


<210>  786
<211>  11
<212>  DNA
<213>  Artificial

<220>
<223>  synthetic DNA

<400>  786
gagtcacaag g                                                            11


<210>  787
<211>  11
<212>  DNA
<213>  Artificial

<220>
<223>  synthetic DNA

<400>  787
cagaaggttc a                                                            11


<210>  788
<211>  11
<212>  DNA
<213>  Artificial

<220>
<223>  synthetic DNA

<400>  788
tcaacgtgca g                                                            11


<210>  789
<211>  11
<212>  DNA
<213>  Artificial

<220>
<223>  synthetic DNA

<400>  789
caagcttact a                                                            11


<210>  790
<211>  11
<212>  DNA
<213>  Artificial

<220>
<223>  synthetic DNA

<400>  790
agaactcgtt g                                                            11


<210>  791
<211>  11
<212>  DNA
<213>  Artificial

<220>
<223>  synthetic DNA

<400>  791
ccgatacaga g                                                            11

<210>  792
<211>  11
<212>  DNA
<213>  Artificial

<220>
<223>  synthetic DNA

<400>  792
gtacgctgat c                                                            11


<210>  793
<211>  11
<212>  DNA
<213>  Artificial

<220>
<223>  synthetic DNA

<400>  793
tcctcagtga a                                                            11


<210>  794
<211>  11
<212>  DNA
<213>  Artificial

<220>
<223>  synthetic DNA

<400>  794
gagccaacat t                                                            11


<210>  795
<211>  11
<212>  DNA
<213>  Artificial

<220>
<223>  synthetic DNA

<400>  795
gagatcgatg g                                                            11


<210>  796
<211>  11
<212>  DNA
<213>  Artificial

<220>
<223>  synthetic DNA

<400>  796
atcgtcagct g                                                            11


<210>  797
<211>  11

<212>  DNA
<213>  Artificial

<220>
<223>  synthetic DNA

<400>  797
gaagcacacg t                                                            11


<210>  798
<211>  11
<212>  DNA
<213>  Artificial

<220>
<223>  synthetic DNA

<400>  798
atcacgcaac c                                                            11


<210>  799
<211>  11
<212>  DNA
<213>  Artificial

<220>
<223>  synthetic DNA

<400>  799
tcgaatagtc g                                                            11


<210>  800
<211>  11
<212>  DNA
<213>  Artificial

<220>
<223>  synthetic DNA

<400>  800
tattaccgtc t                                                            11


<210>  801
<211>  11
<212>  DNA
<213>  Artificial

<220>
<223>  synthetic DNA

<400>  801
cagtcacgac a                                                            11


<210>  802
<211>  11
<212>  DNA
<213>  Artificial

<220>
<223>  synthetic DNA

<400>  802
ttactcgacg t                                                            11


<210>  803
<211>  11
<212>  DNA
<213>  Artificial

<220>
<223>  synthetic DNA

<400>  803
gcaatgttga a                                                            11


<210>  804
<211>  11
<212>  DNA
<213>  Artificial

<220>
<223>  synthetic DNA

<400>  804
gacacgagca a                                                            11


<210>  805
<211>  11
<212>  DNA
<213>  Artificial

<220>
<223>  synthetic DNA

<400>  805
cgagattaca a                                                            11


<210>  806
<211>  11
<212>  DNA
<213>  Artificial

<220>
<223>  synthetic DNA

<400>  806
taccgactac a                                                            11


<210>  807
<211>  11
<212>  DNA
<213>  Artificial

<220>
<223>  synthetic DNA

<400>  807
accgttgcca t                                                            11


<210>  808
<211>  11
<212>  DNA
<213>  Artificial

<220>
<223>  synthetic DNA

<400>  808
atgtaatcgc c                                                            11


<210>  809
<211>  11
<212>  DNA
<213>  Artificial

<220>
<223>  synthetic DNA

<400>  809
aagcctgatg t                                                            11


<210>  810
<211>  11
<212>  DNA
<213>  Artificial

<220>
<223>  synthetic DNA

<400>  810
aagtaacgtg g                                                            11


<210>  811
<211>  11
<212>  DNA
<213>  Artificial

<220>
<223>  synthetic DNA

<400>  811
gtagaggttg g                                                            11


<210>  812
<211>  11
<212>  DNA
<213>  Artificial

<220>
<223>  synthetic DNA

<400>  812
ctcttgcctc a                                                            11

<210>  813
<211>  11
<212>  DNA
<213>  Artificial

<220>
<223>  synthetic DNA

<400>  813
atcgtgaagt g                                                            11


<210>  814
<211>  11
<212>  DNA
<213>  Artificial

<220>
<223>  synthetic DNA

<400>  814
accagcacta t                                                            11


<210>  815
<211>  11
<212>  DNA
<213>  Artificial

<220>
<223>  synthetic DNA

<400>  815
caccagaatg t                                                            11


<210>  816
<211>  11
<212>  DNA
<213>  Artificial

<220>
<223>  synthetic DNA

<400>  816
gagtgaacaa c                                                            11


<210>  817
<211>  11
<212>  DNA
<213>  Artificial

<220>
<223>  synthetic DNA

<400>  817
taacgttacg c                                                            11


<210>  818
<211>  11

<212>  DNA
<213>  Artificial

<220>
<223>  synthetic DNA

<400>  818
cttggatctt g                                                            11


<210>  819
<211>  11
<212>  DNA
<213>  Artificial

<220>
<223>  synthetic DNA

<400>  819
gttccaacgt t                                                            11


<210>  820
<211>  11
<212>  DNA
<213>  Artificial

<220>
<223>  synthetic DNA

<400>  820
caaggaccgt a                                                            11


<210>  821
<211>  11
<212>  DNA
<213>  Artificial

<220>
<223>  synthetic DNA

<400>  821
gacttcacgc a                                                            11


<210>  822
<211>  11
<212>  DNA
<213>  Artificial

<220>
<223>  synthetic DNA

<400>  822
cacactactg g                                                            11


<210>  823
<211>  11
<212>  DNA
<213>  Artificial

<220>
<223>  synthetic DNA

<400>  823
tcagatgaat c                                                            11


<210>  824
<211>  11
<212>  DNA
<213>  Artificial

<220>
<223>  synthetic DNA

<400>  824
tatggatctg g                                                            11


<210>  825
<211>  11
<212>  DNA
<213>  Artificial

<220>
<223>  synthetic DNA

<400>  825
tcttaggtgt g                                                            11


<210>  826
<211>  11
<212>  DNA
<213>  Artificial

<220>
<223>  synthetic DNA

<400>  826
tgtcagcgtc a                                                            11


<210>  827
<211>  11
<212>  DNA
<213>  Artificial

<220>
<223>  synthetic DNA

<400>  827
gtctaggaca g                                                            11


<210>  828
<211>  11
<212>  DNA
<213>  Artificial

<220>
<223>  synthetic DNA

<400>  828
gcctcttcat a                                                              11


<210>  829
<211>  11
<212>  DNA
<213>  Artificial

<220>
<223>  synthetic DNA

<400>  829
agaagtgtta c                                                              11


<210>  830
<211>  11
<212>  DNA
<213>  Artificial

<220>
<223>  synthetic DNA

<400>  830
catgaggctt g                                                              11


<210>  831
<211>  11
<212>  DNA
<213>  Artificial

<220>
<223>  synthetic DNA

<400>  831
tggattgctc a                                                              11


<210>  832
<211>  11
<212>  DNA
<213>  Artificial

<220>
<223>  synthetic DNA

<400>  832
atctacctaa g                                                              11


<210>  833
<211>  11
<212>  DNA
<213>  Artificial

<220>
<223>  synthetic DNA

<400>  833
atgagcagtg a                                                              11

<210>  834
<211>  11
<212>  DNA
<213>  Artificial

<220>
<223>  synthetic DNA

<400>  834
ccaggagata c                                                            11


<210>  835
<211>  11
<212>  DNA
<213>  Artificial

<220>
<223>  synthetic DNA

<400>  835
ccgttatact t                                                            11


<210>  836
<211>  11
<212>  DNA
<213>  Artificial

<220>
<223>  synthetic DNA

<400>  836
ctcagtacaa g                                                            11


<210>  837
<211>  11
<212>  DNA
<213>  Artificial

<220>
<223>  synthetic DNA

<400>  837
ggtgatcgta g                                                            11


<210>  838
<211>  11
<212>  DNA
<213>  Artificial

<220>
<223>  synthetic DNA

<400>  838
cgaacgagac a                                                            11


<210>  839
<211>  11

<212>  DNA
<213>  Artificial

<220>
<223>  synthetic DNA

<400>  839
actacgagct t                                                            11


<210>  840
<211>  11
<212>  DNA
<213>  Artificial

<220>
<223>  synthetic DNA

<400>  840
ttgccacagc a                                                            11


<210>  841
<211>  11
<212>  DNA
<213>  Artificial

<220>
<223>  synthetic DNA

<400>  841
gtcaactcta c                                                            11


<210>  842
<211>  11
<212>  DNA
<213>  Artificial

<220>
<223>  synthetic DNA

<400>  842
tggactgtgt c                                                            11


<210>  843
<211>  11
<212>  DNA
<213>  Artificial

<220>
<223>  synthetic DNA

<400>  843
ggaatggact t                                                            11


<210>  844
<211>  11
<212>  DNA
<213>  Artificial

<220>
<223>  synthetic DNA

<400>  844
cgagaacata a                                                            11


<210>  845
<211>  11
<212>  DNA
<213>  Artificial

<220>
<223>  synthetic DNA

<400>  845
acctggtcag t                                                            11


<210>  846
<211>  11
<212>  DNA
<213>  Artificial

<220>
<223>  synthetic DNA

<400>  846
cgaacgacac a                                                            11


<210>  847
<211>  11
<212>  DNA
<213>  Artificial

<220>
<223>  synthetic DNA

<400>  847
agtctagcca t                                                            11


<210>  848
<211>  11
<212>  DNA
<213>  Artificial

<220>
<223>  synthetic DNA

<400>  848
aggcctagat g                                                            11


<210>  849
<211>  11
<212>  DNA
<213>  Artificial

<220>
<223>  synthetic DNA

<400>  849
ggtgcgttag t                                                            11


<210>  850
<211>  11
<212>  DNA
<213>  Artificial

<220>
<223>  synthetic DNA

<400>  850
attgtgtccg a                                                            11


<210>  851
<211>  11
<212>  DNA
<213>  Artificial

<220>
<223>  synthetic DNA

<400>  851
gcagacatta a                                                            11


<210>  852
<211>  11
<212>  DNA
<213>  Artificial

<220>
<223>  synthetic DNA

<400>  852
attggctcat g                                                            11


<210>  853
<211>  11
<212>  DNA
<213>  Artificial

<220>
<223>  synthetic DNA

<400>  853
gaggttacat g                                                            11


<210>  854
<211>  11
<212>  DNA
<213>  Artificial

<220>
<223>  synthetic DNA

<400>  854
cctataggac c                                                            11

<210>  855
<211>  11
<212>  DNA
<213>  Artificial

<220>
<223>  synthetic DNA

<400>  855
ttagacggtc t                                                            11


<210>  856
<211>  11
<212>  DNA
<213>  Artificial

<220>
<223>  synthetic DNA

<400>  856
gattgacgca c                                                            11


<210>  857
<211>  11
<212>  DNA
<213>  Artificial

<220>
<223>  synthetic DNA

<400>  857
aagacacctc g                                                            11


<210>  858
<211>  11
<212>  DNA
<213>  Artificial

<220>
<223>  synthetic DNA

<400>  858
tcgaataatc g                                                            11


<210>  859
<211>  11
<212>  DNA
<213>  Artificial

<220>
<223>  synthetic DNA

<400>  859
tctatgtcgg a                                                            11


<210>  860
<211>  11

<212>  DNA
<213>  Artificial

<220>
<223>  synthetic DNA

<400>  860
tcgcatgaac c                                                            11


<210>  861
<211>  11
<212>  DNA
<213>  Artificial

<220>
<223>  synthetic DNA

<400>  861
tgttatgtct c                                                            11


<210>  862
<211>  11
<212>  DNA
<213>  Artificial

<220>
<223>  synthetic DNA

<400>  862
tggatcctac a                                                            11


<210>  863
<211>  11
<212>  DNA
<213>  Artificial

<220>
<223>  synthetic DNA

<400>  863
atcgttcagc c                                                            11


<210>  864
<211>  11
<212>  DNA
<213>  Artificial

<220>
<223>  synthetic DNA

<400>  864
taccgcaagc a                                                            11


<210>  865
<211>  12
<212>  DNA
<213>  Artificial

<220>
<223>  synthetic DNA

<400>  865
gctgttgaac cg                                                          12


<210>  866
<211>  12
<212>  DNA
<213>  Artificial

<220>
<223>  synthetic DNA

<400>  866
atactccgag at                                                          12


<210>  867
<211>  12
<212>  DNA
<213>  Artificial

<220>
<223>  synthetic DNA

<400>  867
cttaaggagc gc                                                          12


<210>  868
<211>  12
<212>  DNA
<213>  Artificial

<220>
<223>  synthetic DNA

<400>  868
tatactacaa gc                                                          12


<210>  869
<211>  12
<212>  DNA
<213>  Artificial

<220>
<223>  synthetic DNA

<400>  869
tagtggtcgt ca                                                          12


<210>  870
<211>  12
<212>  DNA
<213>  Artificial

<220>
<223>  synthetic DNA

<400>  870
gtgcttcagg ag                                                          12


<210>  871
<211>  12
<212>  DNA
<213>  Artificial

<220>
<223>  synthetic DNA

<400>  871
gacgcatacc tc                                                          12


<210>  872
<211>  12
<212>  DNA
<213>  Artificial

<220>
<223>  synthetic DNA

<400>  872
cctacctgtg ga                                                          12


<210>  873
<211>  12
<212>  DNA
<213>  Artificial

<220>
<223>  synthetic DNA

<400>  873
gcggtcacat at                                                          12


<210>  874
<211>  12
<212>  DNA
<213>  Artificial

<220>
<223>  synthetic DNA

<400>  874
ctgcattcac ga                                                          12


<210>  875
<211>  12
<212>  DNA
<213>  Artificial

<220>
<223>  synthetic DNA

<400>  875
tggatccttc at                                                          12

<210>  876
<211>  12
<212>  DNA
<213>  Artificial

<220>
<223>  synthetic DNA

<400>  876
ttgtgctgga ct                                                          12


<210>  877
<211>  12
<212>  DNA
<213>  Artificial

<220>
<223>  synthetic DNA

<400>  877
attgagagct at                                                          12


<210>  878
<211>  12
<212>  DNA
<213>  Artificial

<220>
<223>  synthetic DNA

<400>  878
tcgctaatgt ag                                                          12


<210>  879
<211>  12
<212>  DNA
<213>  Artificial

<220>
<223>  synthetic DNA

<400>  879
ctactggcac aa                                                          12


<210>  880
<211>  12
<212>  DNA
<213>  Artificial

<220>
<223>  synthetic DNA

<400>  880
agagccagtc gt                                                          12


<210>  881
<211>  12

<212>  DNA
<213>  Artificial

<220>
<223>  synthetic DNA

<400>  881
aatactggct aa                                                          12


<210>  882
<211>  12
<212>  DNA
<213>  Artificial

<220>
<223>  synthetic DNA

<400>  882
ctgcatgcat aa                                                          12


<210>  883
<211>  12
<212>  DNA
<213>  Artificial

<220>
<223>  synthetic DNA

<400>  883
ttgtcacaac tc                                                          12


<210>  884
<211>  12
<212>  DNA
<213>  Artificial

<220>
<223>  synthetic DNA

<400>  884
tgctaactct cc                                                          12


<210>  885
<211>  12
<212>  DNA
<213>  Artificial

<220>
<223>  synthetic DNA

<400>  885
tctctagttc gg                                                          12


<210>  886
<211>  12
<212>  DNA
<213>  Artificial

<220>
<223>  synthetic DNA

<400>  886
ttacgtccgc aa                                                          12


<210>  887
<211>  12
<212>  DNA
<213>  Artificial

<220>
<223>  synthetic DNA

<400>  887
gtgttgctac ca                                                          12


<210>  888
<211>  12
<212>  DNA
<213>  Artificial

<220>
<223>  synthetic DNA

<400>  888
cgcatgtatg cc                                                          12


<210>  889
<211>  12
<212>  DNA
<213>  Artificial

<220>
<223>  synthetic DNA

<400>  889
cctgttctga tt                                                          12


<210>  890
<211>  12
<212>  DNA
<213>  Artificial

<220>
<223>  synthetic DNA

<400>  890
taagatgctt ga                                                          12


<210>  891
<211>  12
<212>  DNA
<213>  Artificial

<220>
<223>  synthetic DNA

<400>  891
atatatctca gc                                                          12


<210>  892
<211>  12
<212>  DNA
<213>  Artificial

<220>
<223>  synthetic DNA

<400>  892
ttcctcgtgg tt                                                          12


<210>  893
<211>  12
<212>  DNA
<213>  Artificial

<220>
<223>  synthetic DNA

<400>  893
atgtcgatct ag                                                          12


<210>  894
<211>  12
<212>  DNA
<213>  Artificial

<220>
<223>  synthetic DNA

<400>  894
catccactaa tc                                                          12


<210>  895
<211>  12
<212>  DNA
<213>  Artificial

<220>
<223>  synthetic DNA

<400>  895
gcctctggta ac                                                          12


<210>  896
<211>  12
<212>  DNA
<213>  Artificial

<220>
<223>  synthetic DNA

<400>  896
agtcaagaga tt                                                          12

<210>  897
<211>  12
<212>  DNA
<213>  Artificial

<220>
<223>  synthetic DNA

<400>  897
actgaggcgt tc                                                          12


<210>  898
<211>  12
<212>  DNA
<213>  Artificial

<220>
<223>  synthetic DNA

<400>  898
taaggctgac at                                                          12


<210>  899
<211>  12
<212>  DNA
<213>  Artificial

<220>
<223>  synthetic DNA

<400>  899
agttcgcata ca                                                          12


<210>  900
<211>  12
<212>  DNA
<213>  Artificial

<220>
<223>  synthetic DNA

<400>  900
gcagaattgc ga                                                          12


<210>  901
<211>  12
<212>  DNA
<213>  Artificial

<220>
<223>  synthetic DNA

<400>  901
ggttatgaag aa                                                          12


<210>  902
<211>  12

<212>  DNA
<213>  Artificial

<220>
<223>  synthetic DNA

<400>  902
agaagtcgcc tc                                                          12


<210>  903
<211>  12
<212>  DNA
<213>  Artificial

<220>
<223>  synthetic DNA

<400>  903
ttcgcgttat tg                                                          12


<210>  904
<211>  12
<212>  DNA
<213>  Artificial

<220>
<223>  synthetic DNA

<400>  904
tacctggtcg gt                                                          12


<210>  905
<211>  12
<212>  DNA
<213>  Artificial

<220>
<223>  synthetic DNA

<400>  905
ggttaccgag ga                                                          12


<210>  906
<211>  12
<212>  DNA
<213>  Artificial

<220>
<223>  synthetic DNA

<400>  906
acacacttct ag                                                          12


<210>  907
<211>  12
<212>  DNA
<213>  Artificial

<220>
<223>  synthetic DNA

<400>  907
ggaagtgatt aa                                                          12


<210>  908
<211>  12
<212>  DNA
<213>  Artificial

<220>
<223>  synthetic DNA

<400>  908
tccatcagat aa                                                          12


<210>  909
<211>  12
<212>  DNA
<213>  Artificial

<220>
<223>  synthetic DNA

<400>  909
tgtctgtatc at                                                          12


<210>  910
<211>  12
<212>  DNA
<213>  Artificial

<220>
<223>  synthetic DNA

<400>  910
aattggctat ag                                                          12


<210>  911
<211>  12
<212>  DNA
<213>  Artificial

<220>
<223>  synthetic DNA

<400>  911
acgtcggaag gt                                                          12


<210>  912
<211>  12
<212>  DNA
<213>  Artificial

<220>
<223>  synthetic DNA

<400>  912
aggcatccgt tg                                                            12


<210>  913
<211>  12
<212>  DNA
<213>  Artificial

<220>
<223>  synthetic DNA

<400>  913
accgtcgctt ga                                                            12


<210>  914
<211>  12
<212>  DNA
<213>  Artificial

<220>
<223>  synthetic DNA

<400>  914
taccgtcaag tg                                                            12


<210>  915
<211>  12
<212>  DNA
<213>  Artificial

<220>
<223>  synthetic DNA

<400>  915
ctcgatatag tt                                                            12


<210>  916
<211>  12
<212>  DNA
<213>  Artificial

<220>
<223>  synthetic DNA

<400>  916
cgtcaacgtg gt                                                            12


<210>  917
<211>  12
<212>  DNA
<213>  Artificial

<220>
<223>  synthetic DNA

<400>  917
tagtcaacgt ag                                                            12

<210>  918
<211>  12
<212>  DNA
<213>  Artificial

<220>
<223>  synthetic DNA

<400>  918
tgagtaggtc ag                                                          12


<210>  919
<211>  12
<212>  DNA
<213>  Artificial

<220>
<223>  synthetic DNA

<400>  919
cttggcatgt ac                                                          12


<210>  920
<211>  12
<212>  DNA
<213>  Artificial

<220>
<223>  synthetic DNA

<400>  920
tgccgagact tc                                                          12


<210>  921
<211>  12
<212>  DNA
<213>  Artificial

<220>
<223>  synthetic DNA

<400>  921
ctaagactta ag                                                          12


<210>  922
<211>  12
<212>  DNA
<213>  Artificial

<220>
<223>  synthetic DNA

<400>  922
ttctcgtgtg cg                                                          12


<210>  923
<211>  12

<212>  DNA
<213>  Artificial

<220>
<223>  synthetic DNA

<400>  923
cacctgcacg at                                                          12


<210>  924
<211>  12
<212>  DNA
<213>  Artificial

<220>
<223>  synthetic DNA

<400>  924
attaagccta ag                                                          12


<210>  925
<211>  12
<212>  DNA
<213>  Artificial

<220>
<223>  synthetic DNA

<400>  925
ggtggaacca tg                                                          12


<210>  926
<211>  12
<212>  DNA
<213>  Artificial

<220>
<223>  synthetic DNA

<400>  926
actaacgcga ct                                                          12


<210>  927
<211>  12
<212>  DNA
<213>  Artificial

<220>
<223>  synthetic DNA

<400>  927
cagttgtgct at                                                          12


<210>  928
<211>  12
<212>  DNA
<213>  Artificial

<220>
<223>  synthetic DNA

<400>  928
acgctgttag ca                                                          12


<210>  929
<211>  12
<212>  DNA
<213>  Artificial

<220>
<223>  synthetic DNA

<400>  929
gtcaacgcta ag                                                          12


<210>  930
<211>  12
<212>  DNA
<213>  Artificial

<220>
<223>  synthetic DNA

<400>  930
agcttaggta tg                                                          12


<210>  931
<211>  12
<212>  DNA
<213>  Artificial

<220>
<223>  synthetic DNA

<400>  931
cgcaggacga tt                                                          12


<210>  932
<211>  12
<212>  DNA
<213>  Artificial

<220>
<223>  synthetic DNA

<400>  932
aaccggctgt ct                                                          12


<210>  933
<211>  12
<212>  DNA
<213>  Artificial

<220>
<223>  synthetic DNA

<400>  933
gttgctcacg tg                                                          12


<210>  934
<211>  12
<212>  DNA
<213>  Artificial

<220>
<223>  synthetic DNA

<400>  934
gaatcttccg cg                                                          12


<210>  935
<211>  12
<212>  DNA
<213>  Artificial

<220>
<223>  synthetic DNA

<400>  935
agagcgtaca cg                                                          12


<210>  936
<211>  12
<212>  DNA
<213>  Artificial

<220>
<223>  synthetic DNA

<400>  936
aaggctaatg tc                                                          12


<210>  937
<211>  12
<212>  DNA
<213>  Artificial

<220>
<223>  synthetic DNA

<400>  937
tctatgtaga cg                                                          12


<210>  938
<211>  12
<212>  DNA
<213>  Artificial

<220>
<223>  synthetic DNA

<400>  938
agacggtcta gt                                                          12

<210>  939
<211>  12
<212>  DNA
<213>  Artificial

<220>
<223>  synthetic DNA

<400>  939
ttggtcacac gc                                                          12


<210>  940
<211>  12
<212>  DNA
<213>  Artificial

<220>
<223>  synthetic DNA

<400>  940
gtcgatatat gg                                                          12


<210>  941
<211>  12
<212>  DNA
<213>  Artificial

<220>
<223>  synthetic DNA

<400>  941
aacatggata cg                                                          12


<210>  942
<211>  12
<212>  DNA
<213>  Artificial

<220>
<223>  synthetic DNA

<400>  942
ttcgcagttc ct                                                          12


<210>  943
<211>  12
<212>  DNA
<213>  Artificial

<220>
<223>  synthetic DNA

<400>  943
cgcatgttgt gc                                                          12


<210>  944
<211>  12

<212>  DNA
<213>  Artificial

<220>
<223>  synthetic DNA

<400>  944
tgttaagttg ga                                                          12


<210>  945
<211>  12
<212>  DNA
<213>  Artificial

<220>
<223>  synthetic DNA

<400>  945
caagtgtgat ga                                                          12


<210>  946
<211>  12
<212>  DNA
<213>  Artificial

<220>
<223>  synthetic DNA

<400>  946
ctggtaccac gt                                                          12


<210>  947
<211>  12
<212>  DNA
<213>  Artificial

<220>
<223>  synthetic DNA

<400>  947
cgctaggatc ac                                                          12


<210>  948
<211>  12
<212>  DNA
<213>  Artificial

<220>
<223>  synthetic DNA

<400>  948
tgctcattac gg                                                          12


<210>  949
<211>  12
<212>  DNA
<213>  Artificial

<220>
<223>  synthetic DNA

<400>  949
tgctcagtaa ca                                                          12


<210>  950
<211>  12
<212>  DNA
<213>  Artificial

<220>
<223>  synthetic DNA

<400>  950
acgatcatag cc                                                          12


<210>  951
<211>  12
<212>  DNA
<213>  Artificial

<220>
<223>  synthetic DNA

<400>  951
acgatacgtg ga                                                          12


<210>  952
<211>  12
<212>  DNA
<213>  Artificial

<220>
<223>  synthetic DNA

<400>  952
gttcgatgat gg                                                          12


<210>  953
<211>  12
<212>  DNA
<213>  Artificial

<220>
<223>  synthetic DNA

<400>  953
aagagctgtg cc                                                          12


<210>  954
<211>  12
<212>  DNA
<213>  Artificial

<220>
<223>  synthetic DNA

<400>  954
ggttggatca ac                                                            12


<210>  955
<211>  12
<212>  DNA
<213>  Artificial

<220>
<223>  synthetic DNA

<400>  955
gcgcgcttat ga                                                            12


<210>  956
<211>  12
<212>  DNA
<213>  Artificial

<220>
<223>  synthetic DNA

<400>  956
cgtcgatcat ca                                                            12


<210>  957
<211>  12
<212>  DNA
<213>  Artificial

<220>
<223>  synthetic DNA

<400>  957
gagactgcac tc                                                            12


<210>  958
<211>  12
<212>  DNA
<213>  Artificial

<220>
<223>  synthetic DNA

<400>  958
gatagatcgc at                                                            12


<210>  959
<211>  12
<212>  DNA
<213>  Artificial

<220>
<223>  synthetic DNA

<400>  959
ggccatcatc ag                                                            12

<210>  960
<211>  12
<212>  DNA
<213>  Artificial

<220>
<223>  synthetic DNA

<400>  960
ggtgttccac tg                                                          12


<210>  961
<211>  14
<212>  DNA
<213>  Artificial

<220>
<223>  synthetic DNA

<400>  961
agctatacag aggt                                                        14


<210>  962
<211>  14
<212>  DNA
<213>  Artificial

<220>
<223>  synthetic DNA

<400>  962
aggccgttct gtct                                                        14


<210>  963
<211>  14
<212>  DNA
<213>  Artificial

<220>
<223>  synthetic DNA

<400>  963
cattggtctg ctat                                                        14


<210>  964
<211>  14
<212>  DNA
<213>  Artificial

<220>
<223>  synthetic DNA

<400>  964
ctacatacgc gcca                                                        14


<210>  965
<211>  14

<212>  DNA
<213>  Artificial

<220>
<223>  synthetic DNA

<400>  965
gcttaacggc gctt                                                          14


<210>  966
<211>  14
<212>  DNA
<213>  Artificial

<220>
<223>  synthetic DNA

<400>  966
tacgatactc cacc                                                          14


<210>  967
<211>  14
<212>  DNA
<213>  Artificial

<220>
<223>  synthetic DNA

<400>  967
accggcataa gaag                                                          14


<210>  968
<211>  14
<212>  DNA
<213>  Artificial

<220>
<223>  synthetic DNA

<400>  968
ggatgcttcg ataa                                                          14


<210>  969
<211>  14
<212>  DNA
<213>  Artificial

<220>
<223>  synthetic DNA

<400>  969
gtgtacctga atgt                                                          14


<210>  970
<211>  14
<212>  DNA
<213>  Artificial

<220>
<223>  synthetic DNA

<400>  970
cgcggataca caga                                                         14


<210>  971
<211>  14
<212>  DNA
<213>  Artificial

<220>
<223>  synthetic DNA

<400>  971
ttccacggca ctgt                                                         14


<210>  972
<211>  14
<212>  DNA
<213>  Artificial

<220>
<223>  synthetic DNA

<400>  972
tagccaggca acaa                                                         14


<210>  973
<211>  14
<212>  DNA
<213>  Artificial

<220>
<223>  synthetic DNA

<400>  973
agcgtcaaca cgta                                                         14


<210>  974
<211>  14
<212>  DNA
<213>  Artificial

<220>
<223>  synthetic DNA

<400>  974
taacgctact cgcg                                                         14


<210>  975
<211>  14
<212>  DNA
<213>  Artificial

<220>
<223>  synthetic DNA

<400>  975
tagatagacg atct                                                         14


<210>  976
<211>  14
<212>  DNA
<213>  Artificial

<220>
<223>  synthetic DNA

<400>  976
actcttgcaa tgct                                                         14


<210>  977
<211>  14
<212>  DNA
<213>  Artificial

<220>
<223>  synthetic DNA

<400>  977
actcggttag gtcg                                                         14


<210>  978
<211>  14
<212>  DNA
<213>  Artificial

<220>
<223>  synthetic DNA

<400>  978
cattatctac gcat                                                         14


<210>  979
<211>  14
<212>  DNA
<213>  Artificial

<220>
<223>  synthetic DNA

<400>  979
cacaccggcg atta                                                         14


<210>  980
<211>  14
<212>  DNA
<213>  Artificial

<220>
<223>  synthetic DNA

<400>  980
tacgcagtac tgtg                                                         14

<210>  981
<211>  14
<212>  DNA
<213>  Artificial

<220>
<223>  synthetic DNA

<400>  981
caagcgcgtg aatg                                                          14


<210>  982
<211>  14
<212>  DNA
<213>  Artificial

<220>
<223>  synthetic DNA

<400>  982
gaatggactg acga                                                          14


<210>  983
<211>  14
<212>  DNA
<213>  Artificial

<220>
<223>  synthetic DNA

<400>  983
ctagcgctga agtt                                                          14


<210>  984
<211>  14
<212>  DNA
<213>  Artificial

<220>
<223>  synthetic DNA

<400>  984
tgcggcagac caat                                                          14


<210>  985
<211>  14
<212>  DNA
<213>  Artificial

<220>
<223>  synthetic DNA

<400>  985
aaggcataga gatt                                                          14


<210>  986
<211>  14

<212>  DNA
<213>  Artificial

<220>
<223>  synthetic DNA

<400>  986
ttctcctcgc catg                                                          14


<210>  987
<211>  14
<212>  DNA
<213>  Artificial

<220>
<223>  synthetic DNA

<400>  987
tcattggtcg tgaa                                                          14


<210>  988
<211>  14
<212>  DNA
<213>  Artificial

<220>
<223>  synthetic DNA

<400>  988
attacgctat acga                                                          14


<210>  989
<211>  14
<212>  DNA
<213>  Artificial

<220>
<223>  synthetic DNA

<400>  989
atgatcctcc acgg                                                          14


<210>  990
<211>  14
<212>  DNA
<213>  Artificial

<220>
<223>  synthetic DNA

<400>  990
cgtcgttagt aatc                                                          14


<210>  991
<211>  14
<212>  DNA
<213>  Artificial

<220>
<223>  synthetic DNA

<400>  991
tgcacatagt ctca                                                          14


<210>  992
<211>  14
<212>  DNA
<213>  Artificial

<220>
<223>  synthetic DNA

<400>  992
gtcaaggagt cacg                                                          14


<210>  993
<211>  14
<212>  DNA
<213>  Artificial

<220>
<223>  synthetic DNA

<400>  993
ggttggaatc ttgc                                                          14


<210>  994
<211>  14
<212>  DNA
<213>  Artificial

<220>
<223>  synthetic DNA

<400>  994
catcggtgca ctca                                                          14


<210>  995
<211>  14
<212>  DNA
<213>  Artificial

<220>
<223>  synthetic DNA

<400>  995
aatgcactag acgt                                                          14


<210>  996
<211>  14
<212>  DNA
<213>  Artificial

<220>
<223>  synthetic DNA

<400>  996
tacagtcagg ctcg                                                         14


<210>  997
<211>  14
<212>  DNA
<213>  Artificial

<220>
<223>  synthetic DNA

<400>  997
agagaagctt agcc                                                         14


<210>  998
<211>  14
<212>  DNA
<213>  Artificial

<220>
<223>  synthetic DNA

<400>  998
ccataggatc gtat                                                         14


<210>  999
<211>  14
<212>  DNA
<213>  Artificial

<220>
<223>  synthetic DNA

<400>  999
ttgtgctaca cctg                                                         14


<210>  1000
<211>  14
<212>  DNA
<213>  Artificial

<220>
<223>  synthetic DNA

<400>  1000
ctccagtaat acta                                                         14


<210>  1001
<211>  14
<212>  DNA
<213>  Artificial

<220>
<223>  synthetic DNA

<400>  1001
tgatgccgat gtgg                                                         14

<210>  1002
<211>  14
<212>  DNA
<213>  Artificial

<220>
<223>  synthetic DNA

<400>  1002
gtcataccgc ttaa                                                          14


<210>  1003
<211>  14
<212>  DNA
<213>  Artificial

<220>
<223>  synthetic DNA

<400>  1003
acgttctctt gaga                                                          14


<210>  1004
<211>  14
<212>  DNA
<213>  Artificial

<220>
<223>  synthetic DNA

<400>  1004
cagccatatc gtgt                                                          14


<210>  1005
<211>  14
<212>  DNA
<213>  Artificial

<220>
<223>  synthetic DNA

<400>  1005
ttgaacgtag caat                                                          14


<210>  1006
<211>  14
<212>  DNA
<213>  Artificial

<220>
<223>  synthetic DNA

<400>  1006
acaatcgcgg taat                                                          14


<210>  1007
<211>  14

<212>  DNA
<213>  Artificial

<220>
<223>  synthetic DNA

<400>  1007
gttcctgtag atcc                                                          14


<210>  1008
<211>  14
<212>  DNA
<213>  Artificial

<220>
<223>  synthetic DNA

<400>  1008
agagccttac ggca                                                          14


<210>  1009
<211>  14
<212>  DNA
<213>  Artificial

<220>
<223>  synthetic DNA

<400>  1009
aatatggcgc cacc                                                          14


<210>  1010
<211>  14
<212>  DNA
<213>  Artificial

<220>
<223>  synthetic DNA

<400>  1010
accatatagg ttcg                                                          14


<210>  1011
<211>  14
<212>  DNA
<213>  Artificial

<220>
<223>  synthetic DNA

<400>  1011
atgcaccaca gctg                                                          14


<210>  1012
<211>  14
<212>  DNA
<213>  Artificial

<220>
<223>  synthetic DNA

<400>  1012
ctactattga acag                                                         14


<210>  1013
<211>  14
<212>  DNA
<213>  Artificial

<220>
<223>  synthetic DNA

<400>  1013
tgccatcact ctag                                                         14


<210>  1014
<211>  14
<212>  DNA
<213>  Artificial

<220>
<223>  synthetic DNA

<400>  1014
gcgaacgaga atcg                                                         14


<210>  1015
<211>  14
<212>  DNA
<213>  Artificial

<220>
<223>  synthetic DNA

<400>  1015
gaatcaagga gacc                                                         14


<210>  1016
<211>  14
<212>  DNA
<213>  Artificial

<220>
<223>  synthetic DNA

<400>  1016
caacatctat gcag                                                         14


<210>  1017
<211>  14
<212>  DNA
<213>  Artificial

<220>
<223>  synthetic DNA

<400>  1017
caatccgtca tgga                                                         14


<210>  1018
<211>  14
<212>  DNA
<213>  Artificial

<220>
<223>  synthetic DNA

<400>  1018
agctcttagc cata                                                         14


<210>  1019
<211>  14
<212>  DNA
<213>  Artificial

<220>
<223>  synthetic DNA

<400>  1019
aacaaggcaa ctgg                                                         14


<210>  1020
<211>  14
<212>  DNA
<213>  Artificial

<220>
<223>  synthetic DNA

<400>  1020
gtcgtcgctc ctat                                                         14


<210>  1021
<211>  14
<212>  DNA
<213>  Artificial

<220>
<223>  synthetic DNA

<400>  1021
gtcatcatta gatg                                                         14


<210>  1022
<211>  14
<212>  DNA
<213>  Artificial

<220>
<223>  synthetic DNA

<400>  1022
gcactaagta gcag                                                         14

<210>  1023
<211>  14
<212>  DNA
<213>  Artificial

<220>
<223>  synthetic DNA

<400>  1023
accttaccgg acct                                                         14


<210>  1024
<211>  14
<212>  DNA
<213>  Artificial

<220>
<223>  synthetic DNA

<400>  1024
gctcaggtat gtca                                                         14


<210>  1025
<211>  14
<212>  DNA
<213>  Artificial

<220>
<223>  synthetic DNA

<400>  1025
tgtcacgagt tagt                                                         14


<210>  1026
<211>  14
<212>  DNA
<213>  Artificial

<220>
<223>  synthetic DNA

<400>  1026
cagatgactt acgt                                                         14


<210>  1027
<211>  14
<212>  DNA
<213>  Artificial

<220>
<223>  synthetic DNA

<400>  1027
gaagtagcga ttga                                                         14


<210>  1028
<211>  14

<212>  DNA
<213>  Artificial

<220>
<223>  synthetic DNA

<400>  1028
gcaggcaatc tgta                                                          14


<210>  1029
<211>  14
<212>  DNA
<213>  Artificial

<220>
<223>  synthetic DNA

<400>  1029
ccttatacaa caag                                                          14


<210>  1030
<211>  14
<212>  DNA
<213>  Artificial

<220>
<223>  synthetic DNA

<400>  1030
ccttagattg attg                                                          14


<210>  1031
<211>  14
<212>  DNA
<213>  Artificial

<220>
<223>  synthetic DNA

<400>  1031
agccacgagt gata                                                          14


<210>  1032
<211>  14
<212>  DNA
<213>  Artificial

<220>
<223>  synthetic DNA

<400>  1032
ggatgactcg tgac                                                          14


<210>  1033
<211>  14
<212>  DNA
<213>  Artificial

<220>
<223>  synthetic DNA

<400>  1033
cttcgttcgc catt                                                         14


<210>  1034
<211>  14
<212>  DNA
<213>  Artificial

<220>
<223>  synthetic DNA

<400>  1034
tcttgcgtat tgat                                                         14


<210>  1035
<211>  14
<212>  DNA
<213>  Artificial

<220>
<223>  synthetic DNA

<400>  1035
cttaacgtgg tggc                                                         14


<210>  1036
<211>  14
<212>  DNA
<213>  Artificial

<220>
<223>  synthetic DNA

<400>  1036
tgctgttacg gaag                                                         14


<210>  1037
<211>  14
<212>  DNA
<213>  Artificial

<220>
<223>  synthetic DNA

<400>  1037
ctgaattagt tctc                                                         14


<210>  1038
<211>  14
<212>  DNA
<213>  Artificial

<220>
<223>  synthetic DNA

<400>  1038
cctccaagta caga                                                         14


<210>  1039
<211>  14
<212>  DNA
<213>  Artificial

<220>
<223>  synthetic DNA

<400>  1039
ctggtaattc gcgg                                                         14


<210>  1040
<211>  14
<212>  DNA
<213>  Artificial

<220>
<223>  synthetic DNA

<400>  1040
cgactgcaat ctgg                                                         14


<210>  1041
<211>  14
<212>  DNA
<213>  Artificial

<220>
<223>  synthetic DNA

<400>  1041
tggatcgcga ttgg                                                         14


<210>  1042
<211>  14
<212>  DNA
<213>  Artificial

<220>
<223>  synthetic DNA

<400>  1042
cgactattcc tgcg                                                         14


<210>  1043
<211>  14
<212>  DNA
<213>  Artificial

<220>
<223>  synthetic DNA

<400>  1043
caagtaggtc cgtc                                                         14

<210>  1044
<211>  14
<212>  DNA
<213>  Artificial

<220>
<223>  synthetic DNA

<400>  1044
agtaatcagt gttc                                                          14


<210>  1045
<211>  14
<212>  DNA
<213>  Artificial

<220>
<223>  synthetic DNA

<400>  1045
ttattctcac tacg                                                          14


<210>  1046
<211>  14
<212>  DNA
<213>  Artificial

<220>
<223>  synthetic DNA

<400>  1046
catgtcttct tcgt                                                          14


<210>  1047
<211>  14
<212>  DNA
<213>  Artificial

<220>
<223>  synthetic DNA

<400>  1047
aggcacatac catc                                                          14


<210>  1048
<211>  14
<212>  DNA
<213>  Artificial

<220>
<223>  synthetic DNA

<400>  1048
aggttagagg atgt                                                          14


<210>  1049
<211>  14

<212>  DNA
<213>  Artificial

<220>
<223>  synthetic DNA

<400>  1049
caactggcaa gtgc                                                          14


<210>  1050
<211>  14
<212>  DNA
<213>  Artificial

<220>
<223>  synthetic DNA

<400>  1050
cgctcacata gagg                                                          14


<210>  1051
<211>  14
<212>  DNA
<213>  Artificial

<220>
<223>  synthetic DNA

<400>  1051
gcaatgtcga gatc                                                          14


<210>  1052
<211>  14
<212>  DNA
<213>  Artificial

<220>
<223>  synthetic DNA

<400>  1052
gttctgtggt gctc                                                          14


<210>  1053
<211>  14
<212>  DNA
<213>  Artificial

<220>
<223>  synthetic DNA

<400>  1053
aagtgatcag acta                                                          14


<210>  1054
<211>  14
<212>  DNA
<213>  Artificial

<220>
<223>  synthetic DNA

<400>  1054
attgaaggat tcca                                                          14


<210>  1055
<211>  14
<212>  DNA
<213>  Artificial

<220>
<223>  synthetic DNA

<400>  1055
acgccatgct acta                                                          14


<210>  1056
<211>  14
<212>  DNA
<213>  Artificial

<220>
<223>  synthetic DNA

<400>  1056
ctgaagatgt ctgc                                                          14


<210>  1057
<211>  16
<212>  DNA
<213>  Artificial

<220>
<223>  synthetic DNA

<400>  1057
gacaatctct gccgat                                                        16


<210>  1058
<211>  16
<212>  DNA
<213>  Artificial

<220>
<223>  synthetic DNA

<400>  1058
ggtccgccta atgtaa                                                        16


<210>  1059
<211>  16
<212>  DNA
<213>  Artificial

<220>
<223>  synthetic DNA

<400>  1059
agccacaggc aattcc                                                        16


<210>  1060
<211>  16
<212>  DNA
<213>  Artificial

<220>
<223>  synthetic DNA

<400>  1060
atctcaagtt ctcaac                                                        16


<210>  1061
<211>  16
<212>  DNA
<213>  Artificial

<220>
<223>  synthetic DNA

<400>  1061
tgtaacgcat acgacg                                                        16


<210>  1062
<211>  16
<212>  DNA
<213>  Artificial

<220>
<223>  synthetic DNA

<400>  1062
tatctcgaat accagc                                                        16


<210>  1063
<211>  16
<212>  DNA
<213>  Artificial

<220>
<223>  synthetic DNA

<400>  1063
accgcaacac aggcaa                                                        16


<210>  1064
<211>  16
<212>  DNA
<213>  Artificial

<220>
<223>  synthetic DNA

<400>  1064
ggccagtaac atgact                                                        16

<210>  1065
<211>  16
<212>  DNA
<213>  Artificial

<220>
<223>  synthetic DNA

<400>  1065
gtgaacagtt aaggtg                                                        16


<210>  1066
<211>  16
<212>  DNA
<213>  Artificial

<220>
<223>  synthetic DNA

<400>  1066
ccaggatccg tattgc                                                        16


<210>  1067
<211>  16
<212>  DNA
<213>  Artificial

<220>
<223>  synthetic DNA

<400>  1067
gacctagcac tagacc                                                        16


<210>  1068
<211>  16
<212>  DNA
<213>  Artificial

<220>
<223>  synthetic DNA

<400>  1068
cgccatccta ttcacg                                                        16


<210>  1069
<211>  16
<212>  DNA
<213>  Artificial

<220>
<223>  synthetic DNA

<400>  1069
aagtgcagta atggaa                                                        16


<210>  1070
<211>  16

<212>  DNA
<213>  Artificial

<220>
<223>  synthetic DNA

<400>  1070
tcaacgcgtt cgtcta                                                        16


<210>  1071
<211>  16
<212>  DNA
<213>  Artificial

<220>
<223>  synthetic DNA

<400>  1071
agcggccact atctaa                                                        16


<210>  1072
<211>  16
<212>  DNA
<213>  Artificial

<220>
<223>  synthetic DNA

<400>  1072
ctcggcgcca tataga                                                        16


<210>  1073
<211>  16
<212>  DNA
<213>  Artificial

<220>
<223>  synthetic DNA

<400>  1073
cgataactta gaagaa                                                        16


<210>  1074
<211>  16
<212>  DNA
<213>  Artificial

<220>
<223>  synthetic DNA

<400>  1074
cataggatgt gacgcc                                                        16


<210>  1075
<211>  16
<212>  DNA
<213>  Artificial

<220>
<223>  synthetic DNA

<400>  1075
ggcttgtcgt cgtatc                                                       16


<210>  1076
<211>  16
<212>  DNA
<213>  Artificial

<220>
<223>  synthetic DNA

<400>  1076
cttgtctgaa tattag                                                       16


<210>  1077
<211>  16
<212>  DNA
<213>  Artificial

<220>
<223>  synthetic DNA

<400>  1077
acagttcgag tgtcgg                                                       16


<210>  1078
<211>  16
<212>  DNA
<213>  Artificial

<220>
<223>  synthetic DNA

<400>  1078
ctctaacctg tgacgt                                                       16


<210>  1079
<211>  16
<212>  DNA
<213>  Artificial

<220>
<223>  synthetic DNA

<400>  1079
cgcgctaatt caacaa                                                       16


<210>  1080
<211>  16
<212>  DNA
<213>  Artificial

<220>
<223>  synthetic DNA

<400>  1080
actcacgaat gcggca                                                        16


<210>  1081
<211>  16
<212>  DNA
<213>  Artificial

<220>
<223>  synthetic DNA

<400>  1081
aatcttcggc attcat                                                        16


<210>  1082
<211>  16
<212>  DNA
<213>  Artificial

<220>
<223>  synthetic DNA

<400>  1082
aagtatcagg atcgcg                                                        16


<210>  1083
<211>  16
<212>  DNA
<213>  Artificial

<220>
<223>  synthetic DNA

<400>  1083
agtaactctg cagaca                                                        16


<210>  1084
<211>  16
<212>  DNA
<213>  Artificial

<220>
<223>  synthetic DNA

<400>  1084
ggattgaaca ttgtgc                                                        16


<210>  1085
<211>  16
<212>  DNA
<213>  Artificial

<220>
<223>  synthetic DNA

<400>  1085
gtgatgctca cgcatc                                                        16

<210>  1086
<211>  16
<212>  DNA
<213>  Artificial

<220>
<223>  synthetic DNA

<400>  1086
cgtagcgtaa cggata                                                        16


<210>  1087
<211>  16
<212>  DNA
<213>  Artificial

<220>
<223>  synthetic DNA

<400>  1087
tgcgatgcac cgttag                                                        16


<210>  1088
<211>  16
<212>  DNA
<213>  Artificial

<220>
<223>  synthetic DNA

<400>  1088
ccagtatgct ctcagg                                                        16


<210>  1089
<211>  16
<212>  DNA
<213>  Artificial

<220>
<223>  synthetic DNA

<400>  1089
aatgacgttg aagcct                                                        16


<210>  1090
<211>  16
<212>  DNA
<213>  Artificial

<220>
<223>  synthetic DNA

<400>  1090
tcgattctat aggagt                                                        16


<210>  1091
<211>  16

<212>  DNA
<213>  Artificial

<220>
<223>  synthetic DNA

<400>  1091
cgataggttc agctat                                                        16


<210>  1092
<211>  16
<212>  DNA
<213>  Artificial

<220>
<223>  synthetic DNA

<400>  1092
ccatgttgat agaata                                                        16


<210>  1093
<211>  16
<212>  DNA
<213>  Artificial

<220>
<223>  synthetic DNA

<400>  1093
gagccacttc tacagg                                                        16


<210>  1094
<211>  16
<212>  DNA
<213>  Artificial

<220>
<223>  synthetic DNA

<400>  1094
gcgaactctc ggtaat                                                        16


<210>  1095
<211>  16
<212>  DNA
<213>  Artificial

<220>
<223>  synthetic DNA

<400>  1095
gacctgagta gctggt                                                        16


<210>  1096
<211>  16
<212>  DNA
<213>  Artificial

<220>
<223>  synthetic DNA

<400>  1096
cgagtctatt agcctg                                                        16


<210>  1097
<211>  16
<212>  DNA
<213>  Artificial

<220>
<223>  synthetic DNA

<400>  1097
gtagtgccat acacct                                                        16


<210>  1098
<211>  16
<212>  DNA
<213>  Artificial

<220>
<223>  synthetic DNA

<400>  1098
ccagtggtct atagca                                                        16


<210>  1099
<211>  16
<212>  DNA
<213>  Artificial

<220>
<223>  synthetic DNA

<400>  1099
gtcagtgcgt tattgc                                                        16


<210>  1100
<211>  16
<212>  DNA
<213>  Artificial

<220>
<223>  synthetic DNA

<400>  1100
agtgtcggag tgacga                                                        16


<210>  1101
<211>  16
<212>  DNA
<213>  Artificial

<220>
<223>  synthetic DNA

<400>  1101
aatctccgct atagtt                                                        16


<210>  1102
<211>  16
<212>  DNA
<213>  Artificial

<220>
<223>  synthetic DNA

<400>  1102
cgagtaggtc tgactt                                                        16


<210>  1103
<211>  16
<212>  DNA
<213>  Artificial

<220>
<223>  synthetic DNA

<400>  1103
ctgtcgctct aataac                                                        16


<210>  1104
<211>  16
<212>  DNA
<213>  Artificial

<220>
<223>  synthetic DNA

<400>  1104
gctgtcaata taactg                                                        16


<210>  1105
<211>  16
<212>  DNA
<213>  Artificial

<220>
<223>  synthetic DNA

<400>  1105
agctcaagtt gaatcc                                                        16


<210>  1106
<211>  16
<212>  DNA
<213>  Artificial

<220>
<223>  synthetic DNA

<400>  1106
aattcatgct cctaac                                                        16

<210>  1107
<211>  16
<212>  DNA
<213>  Artificial

<220>
<223>  synthetic DNA

<400>  1107
ccaaggtctg gtgata                                                        16


<210>  1108
<211>  16
<212>  DNA
<213>  Artificial

<220>
<223>  synthetic DNA

<400>  1108
ctccacgtat cttgaa                                                        16


<210>  1109
<211>  16
<212>  DNA
<213>  Artificial

<220>
<223>  synthetic DNA

<400>  1109
tagccgaaca acactt                                                        16


<210>  1110
<211>  16
<212>  DNA
<213>  Artificial

<220>
<223>  synthetic DNA

<400>  1110
agtacacgac atatgc                                                        16


<210>  1111
<211>  16
<212>  DNA
<213>  Artificial

<220>
<223>  synthetic DNA

<400>  1111
acgttctaga ctcctg                                                        16


<210>  1112
<211>  16

<212>  DNA
<213>  Artificial

<220>
<223>  synthetic DNA

<400>  1112
cgactcaagc actgct                                                        16


<210>  1113
<211>  16
<212>  DNA
<213>  Artificial

<220>
<223>  synthetic DNA

<400>  1113
tgaagctcac gattaa                                                        16


<210>  1114
<211>  16
<212>  DNA
<213>  Artificial

<220>
<223>  synthetic DNA

<400>  1114
tatctaacgt atggta                                                        16


<210>  1115
<211>  16
<212>  DNA
<213>  Artificial

<220>
<223>  synthetic DNA

<400>  1115
tataccatgt tccttg                                                        16


<210>  1116
<211>  16
<212>  DNA
<213>  Artificial

<220>
<223>  synthetic DNA

<400>  1116
ttcctacgat gacttc                                                        16


<210>  1117
<211>  16
<212>  DNA
<213>  Artificial

<220>
<223>  synthetic DNA

<400>  1117
ctctccaata tgtgcc                                                       16


<210>  1118
<211>  16
<212>  DNA
<213>  Artificial

<220>
<223>  synthetic DNA

<400>  1118
gagtagagtc ttgcca                                                       16


<210>  1119
<211>  16
<212>  DNA
<213>  Artificial

<220>
<223>  synthetic DNA

<400>  1119
gcgagatgtg gtccta                                                       16


<210>  1120
<211>  16
<212>  DNA
<213>  Artificial

<220>
<223>  synthetic DNA

<400>  1120
aagctacacg gaccac                                                       16


<210>  1121
<211>  16
<212>  DNA
<213>  Artificial

<220>
<223>  synthetic DNA

<400>  1121
atacaactgg caaccg                                                       16


<210>  1122
<211>  16
<212>  DNA
<213>  Artificial

<220>
<223>  synthetic DNA

<400>  1122
cggtagatgc tatgct                                                        16


<210>  1123
<211>  16
<212>  DNA
<213>  Artificial

<220>
<223>  synthetic DNA

<400>  1123
tcttgaccgg tcatca                                                        16


<210>  1124
<211>  16
<212>  DNA
<213>  Artificial

<220>
<223>  synthetic DNA

<400>  1124
agatcgtgca tgcgat                                                        16


<210>  1125
<211>  16
<212>  DNA
<213>  Artificial

<220>
<223>  synthetic DNA

<400>  1125
tcctcgagac agcctt                                                        16


<210>  1126
<211>  16
<212>  DNA
<213>  Artificial

<220>
<223>  synthetic DNA

<400>  1126
tagccggtac cactta                                                        16


<210>  1127
<211>  16
<212>  DNA
<213>  Artificial

<220>
<223>  synthetic DNA

<400>  1127
gtaaggcagc gtgcaa                                                        16

<210>  1128
<211>  16
<212>  DNA
<213>  Artificial

<220>
<223>  synthetic DNA

<400>  1128
tagtctgctc ctggtc                                                       16


<210>  1129
<211>  16
<212>  DNA
<213>  Artificial

<220>
<223>  synthetic DNA

<400>  1129
tggattatag cagcag                                                       16


<210>  1130
<211>  16
<212>  DNA
<213>  Artificial

<220>
<223>  synthetic DNA

<400>  1130
aagaatgatc agacat                                                       16


<210>  1131
<211>  16
<212>  DNA
<213>  Artificial

<220>
<223>  synthetic DNA

<400>  1131
cagcgctata tacctc                                                       16


<210>  1132
<211>  16
<212>  DNA
<213>  Artificial

<220>
<223>  synthetic DNA

<400>  1132
gagtagtacc tccacc                                                       16


<210>  1133
<211>  16

<212>  DNA
<213>  Artificial

<220>
<223>  synthetic DNA

<400>  1133
gacgtgatcc tctaga                                                        16


<210>  1134
<211>  16
<212>  DNA
<213>  Artificial

<220>
<223>  synthetic DNA

<400>  1134
gttccgttca ctacga                                                        16


<210>  1135
<211>  16
<212>  DNA
<213>  Artificial

<220>
<223>  synthetic DNA

<400>  1135
tgcaagcacc aggatg                                                        16


<210>  1136
<211>  16
<212>  DNA
<213>  Artificial

<220>
<223>  synthetic DNA

<400>  1136
ttagttggcg gctgag                                                        16


<210>  1137
<211>  16
<212>  DNA
<213>  Artificial

<220>
<223>  synthetic DNA

<400>  1137
cagatgcaga catacg                                                        16


<210>  1138
<211>  16
<212>  DNA
<213>  Artificial

<220>
<223>  synthetic DNA

<400>  1138
gacgcttgat gattat                                                        16


<210>  1139
<211>  16
<212>  DNA
<213>  Artificial

<220>
<223>  synthetic DNA

<400>  1139
tggatcacga ctagga                                                        16


<210>  1140
<211>  16
<212>  DNA
<213>  Artificial

<220>
<223>  synthetic DNA

<400>  1140
ctcgtcggta taacgc                                                        16


<210>  1141
<211>  16
<212>  DNA
<213>  Artificial

<220>
<223>  synthetic DNA

<400>  1141
aagcacggat gcgatt                                                        16


<210>  1142
<211>  16
<212>  DNA
<213>  Artificial

<220>
<223>  synthetic DNA

<400>  1142
agatcttccg gtgaac                                                        16


<210>  1143
<211>  16
<212>  DNA
<213>  Artificial

<220>
<223>  synthetic DNA

<400>  1143
ggacaatagc aacctg                                                        16


<210>  1144
<211>  16
<212>  DNA
<213>  Artificial

<220>
<223>  synthetic DNA

<400>  1144
gataatcggt tccaat                                                        16


<210>  1145
<211>  16
<212>  DNA
<213>  Artificial

<220>
<223>  synthetic DNA

<400>  1145
ctcaagctac agttgt                                                        16


<210>  1146
<211>  16
<212>  DNA
<213>  Artificial

<220>
<223>  synthetic DNA

<400>  1146
gttggcatga tgtaga                                                        16


<210>  1147
<211>  16
<212>  DNA
<213>  Artificial

<220>
<223>  synthetic DNA

<400>  1147
cagcatgagg taagtg                                                        16


<210>  1148
<211>  16
<212>  DNA
<213>  Artificial

<220>
<223>  synthetic DNA

<400>  1148
gcctcatcac acgtca                                                        16

<210>  1149
<211>  16
<212>  DNA
<213>  Artificial

<220>
<223>  synthetic DNA

<400>  1149
tcgatactac acatcg                                                        16


<210>  1150
<211>  16
<212>  DNA
<213>  Artificial

<220>
<223>  synthetic DNA

<400>  1150
tacacgaggc ttgatc                                                        16


<210>  1151
<211>  16
<212>  DNA
<213>  Artificial

<220>
<223>  synthetic DNA

<400>  1151
ttctcgtgtc cgcatt                                                        16


<210>  1152
<211>  16
<212>  DNA
<213>  Artificial

<220>
<223>  synthetic DNA

<400>  1152
ggtgaagcaa cagcat                                                        16


<210>  1153
<211>  18
<212>  DNA
<213>  Artificial

<220>
<223>  synthetic DNA

<400>  1153
cgaaccgact gtacagtt                                                      18


<210>  1154
<211>  18

<212>  DNA
<213>  Artificial

<220>
<223>  synthetic DNA

<400>  1154
ccgactgcgg ataagtta                                                    18


<210>  1155
<211>  18
<212>  DNA
<213>  Artificial

<220>
<223>  synthetic DNA

<400>  1155
cgacaggtag gtaagcag                                                    18


<210>  1156
<211>  18
<212>  DNA
<213>  Artificial

<220>
<223>  synthetic DNA

<400>  1156
tgatacgttg gtatacag                                                    18


<210>  1157
<211>  18
<212>  DNA
<213>  Artificial

<220>
<223>  synthetic DNA

<400>  1157
ctactataga atacgtag                                                    18


<210>  1158
<211>  18
<212>  DNA
<213>  Artificial

<220>
<223>  synthetic DNA

<400>  1158
agactgtggc aatggcat                                                    18


<210>  1159
<211>  18
<212>  DNA
<213>  Artificial

<220>
<223>  synthetic DNA

<400>  1159
ggaagactga tacaacga                                                    18


<210>  1160
<211>  18
<212>  DNA
<213>  Artificial

<220>
<223>  synthetic DNA

<400>  1160
tatgcacata tagcgctt                                                    18


<210>  1161
<211>  18
<212>  DNA
<213>  Artificial

<220>
<223>  synthetic DNA

<400>  1161
catggtaatc gaccgagg                                                    18


<210>  1162
<211>  18
<212>  DNA
<213>  Artificial

<220>
<223>  synthetic DNA

<400>  1162
gtcattgccg tcattgcc                                                    18


<210>  1163
<211>  18
<212>  DNA
<213>  Artificial

<220>
<223>  synthetic DNA

<400>  1163
cctaagaact ccgaagct                                                    18


<210>  1164
<211>  18
<212>  DNA
<213>  Artificial

<220>
<223>  synthetic DNA

<400>  1164
tcgctcaccg tactagga                                                     18


<210>  1165
<211>  18
<212>  DNA
<213>  Artificial

<220>
<223>  synthetic DNA

<400>  1165
tattactgtc acagcagg                                                     18


<210>  1166
<211>  18
<212>  DNA
<213>  Artificial

<220>
<223>  synthetic DNA

<400>  1166
tgagacaggc tacgagtc                                                     18


<210>  1167
<211>  18
<212>  DNA
<213>  Artificial

<220>
<223>  synthetic DNA

<400>  1167
aagctatgcg aacacgtt                                                     18


<210>  1168
<211>  18
<212>  DNA
<213>  Artificial

<220>
<223>  synthetic DNA

<400>  1168
aacggaggag tgagccaa                                                     18


<210>  1169
<211>  18
<212>  DNA
<213>  Artificial

<220>
<223>  synthetic DNA

<400>  1169
ccactatgga catcatgg                                                     18

<210>  1170
<211>  18
<212>  DNA
<213>  Artificial

<220>
<223>  synthetic DNA

<400>  1170
atggtggtgg atagctcg                                                    18


<210>  1171
<211>  18
<212>  DNA
<213>  Artificial

<220>
<223>  synthetic DNA

<400>  1171
tcaccggtta cacatcgc                                                    18


<210>  1172
<211>  18
<212>  DNA
<213>  Artificial

<220>
<223>  synthetic DNA

<400>  1172
aagatactga gatatgga                                                    18


<210>  1173
<211>  18
<212>  DNA
<213>  Artificial

<220>
<223>  synthetic DNA

<400>  1173
gacctgttct tgaactag                                                    18


<210>  1174
<211>  18
<212>  DNA
<213>  Artificial

<220>
<223>  synthetic DNA

<400>  1174
aagtagagct ctcggtta                                                    18


<210>  1175
<211>  18

<212>  DNA
<213>  Artificial

<220>
<223>  synthetic DNA

<400>  1175
ctatgttctt actctctt                                                    18


<210>  1176
<211>  18
<212>  DNA
<213>  Artificial

<220>
<223>  synthetic DNA

<400>  1176
caaggctata agcggtta                                                    18


<210>  1177
<211>  18
<212>  DNA
<213>  Artificial

<220>
<223>  synthetic DNA

<400>  1177
gaagctaatt aaccgata                                                    18


<210>  1178
<211>  18
<212>  DNA
<213>  Artificial

<220>
<223>  synthetic DNA

<400>  1178
ttcacgtctg ccaagcac                                                    18


<210>  1179
<211>  18
<212>  DNA
<213>  Artificial

<220>
<223>  synthetic DNA

<400>  1179
atcgtataga tcgagaca                                                    18


<210>  1180
<211>  18
<212>  DNA
<213>  Artificial

<220>
<223>  synthetic DNA

<400>  1180
gtcacagatt cacatcat                                                    18


<210>  1181
<211>  18
<212>  DNA
<213>  Artificial

<220>
<223>  synthetic DNA

<400>  1181
gtgcctgtga actatcag                                                    18


<210>  1182
<211>  18
<212>  DNA
<213>  Artificial

<220>
<223>  synthetic DNA

<400>  1182
cagcgtacaa gatagtcg                                                    18


<210>  1183
<211>  18
<212>  DNA
<213>  Artificial

<220>
<223>  synthetic DNA

<400>  1183
gcatggcatg gtagacct                                                    18


<210>  1184
<211>  18
<212>  DNA
<213>  Artificial

<220>
<223>  synthetic DNA

<400>  1184
ggtatgctac tcttcgca                                                    18


<210>  1185
<211>  18
<212>  DNA
<213>  Artificial

<220>
<223>  synthetic DNA

<400>  1185
atgttcagtc acaagcga                                                    18


<210>  1186
<211>  18
<212>  DNA
<213>  Artificial

<220>
<223>  synthetic DNA

<400>  1186
taggaagtgt gtaatagc                                                    18


<210>  1187
<211>  18
<212>  DNA
<213>  Artificial

<220>
<223>  synthetic DNA

<400>  1187
aatccatgta gctgtacg                                                    18


<210>  1188
<211>  18
<212>  DNA
<213>  Artificial

<220>
<223>  synthetic DNA

<400>  1188
ccagattcac tggcatag                                                    18


<210>  1189
<211>  18
<212>  DNA
<213>  Artificial

<220>
<223>  synthetic DNA

<400>  1189
ttgtctctac gtaatatc                                                    18


<210>  1190
<211>  18
<212>  DNA
<213>  Artificial

<220>
<223>  synthetic DNA

<400>  1190
gtggtgcttg tgacaatt                                                    18

<210>  1191
<211>  18
<212>  DNA
<213>  Artificial

<220>
<223>  synthetic DNA

<400>  1191
cagcctactt ggctgaga                                                    18


<210>  1192
<211>  18
<212>  DNA
<213>  Artificial

<220>
<223>  synthetic DNA

<400>  1192
tactcaatgc atctgtgt                                                    18


<210>  1193
<211>  18
<212>  DNA
<213>  Artificial

<220>
<223>  synthetic DNA

<400>  1193
tgtagagaga cgaatata                                                    18


<210>  1194
<211>  18
<212>  DNA
<213>  Artificial

<220>
<223>  synthetic DNA

<400>  1194
gcctacaacc atcctact                                                    18


<210>  1195
<211>  18
<212>  DNA
<213>  Artificial

<220>
<223>  synthetic DNA

<400>  1195
gcgtggcatt gagattca                                                    18


<210>  1196
<211>  18

<212>  DNA
<213>  Artificial

<220>
<223>  synthetic DNA

<400>  1196
gcatgccagc taactgag                                                    18


<210>  1197
<211>  18
<212>  DNA
<213>  Artificial

<220>
<223>  synthetic DNA

<400>  1197
gcgagtaatc cggttgga                                                    18


<210>  1198
<211>  18
<212>  DNA
<213>  Artificial

<220>
<223>  synthetic DNA

<400>  1198
gcctctacca gaacgtca                                                    18


<210>  1199
<211>  18
<212>  DNA
<213>  Artificial

<220>
<223>  synthetic DNA

<400>  1199
gtcagcagaa gactgacc                                                    18


<210>  1200
<211>  18
<212>  DNA
<213>  Artificial

<220>
<223>  synthetic DNA

<400>  1200
gataacagac gtagcagg                                                    18


<210>  1201
<211>  18
<212>  DNA
<213>  Artificial

<220>
<223>  synthetic DNA

<400>  1201
caggagatcg catgtcgt                                                     18


<210>  1202
<211>  18
<212>  DNA
<213>  Artificial

<220>
<223>  synthetic DNA

<400>  1202
ctggaaggaa tggagcca                                                     18


<210>  1203
<211>  18
<212>  DNA
<213>  Artificial

<220>
<223>  synthetic DNA

<400>  1203
attggttctc taccacaa                                                     18


<210>  1204
<211>  18
<212>  DNA
<213>  Artificial

<220>
<223>  synthetic DNA

<400>  1204
ctcattgttg acggctca                                                     18


<210>  1205
<211>  18
<212>  DNA
<213>  Artificial

<220>
<223>  synthetic DNA

<400>  1205
ttcaggactg tagttcat                                                     18


<210>  1206
<211>  18
<212>  DNA
<213>  Artificial

<220>
<223>  synthetic DNA

<400>  1206
agaccgcact aactcaag                                                     18


<210>  1207
<211>  18
<212>  DNA
<213>  Artificial

<220>
<223>  synthetic DNA

<400>  1207
ggaatattgt gcagaccg                                                     18


<210>  1208
<211>  18
<212>  DNA
<213>  Artificial

<220>
<223>  synthetic DNA

<400>  1208
cctattacta atagctca                                                     18


<210>  1209
<211>  18
<212>  DNA
<213>  Artificial

<220>
<223>  synthetic DNA

<400>  1209
atggcatgag tacttcgg                                                     18


<210>  1210
<211>  18
<212>  DNA
<213>  Artificial

<220>
<223>  synthetic DNA

<400>  1210
gacacgtatg cgtctagc                                                     18


<210>  1211
<211>  18
<212>  DNA
<213>  Artificial

<220>
<223>  synthetic DNA

<400>  1211
gaaggtacgg aatctgtt                                                     18

<210>  1212
<211>  18
<212>  DNA
<213>  Artificial

<220>
<223>  synthetic DNA

<400>  1212
tataacgtcc gacactgt                                                    18


<210>  1213
<211>  18
<212>  DNA
<213>  Artificial

<220>
<223>  synthetic DNA

<400>  1213
gctaatacat taccgccg                                                    18


<210>  1214
<211>  18
<212>  DNA
<213>  Artificial

<220>
<223>  synthetic DNA

<400>  1214
gaagccaaca ctcctgac                                                    18


<210>  1215
<211>  18
<212>  DNA
<213>  Artificial

<220>
<223>  synthetic DNA

<400>  1215
cgaataacga gctgtgat                                                    18


<210>  1216
<211>  18
<212>  DNA
<213>  Artificial

<220>
<223>  synthetic DNA

<400>  1216
gcctaccgat cgcactta                                                    18


<210>  1217
<211>  18

<212>  DNA
<213>  Artificial

<220>
<223>  synthetic DNA

<400>  1217
ctgaggagaa tagcctgc                                                    18


<210>  1218
<211>  18
<212>  DNA
<213>  Artificial

<220>
<223>  synthetic DNA

<400>  1218
cagcatggac agtacttc                                                    18


<210>  1219
<211>  18
<212>  DNA
<213>  Artificial

<220>
<223>  synthetic DNA

<400>  1219
ggtatagagc cttcctta                                                    18


<210>  1220
<211>  18
<212>  DNA
<213>  Artificial

<220>
<223>  synthetic DNA

<400>  1220
cgctctgcat atatagca                                                    18


<210>  1221
<211>  18
<212>  DNA
<213>  Artificial

<220>
<223>  synthetic DNA

<400>  1221
cggctctact atgctcgt                                                    18


<210>  1222
<211>  18
<212>  DNA
<213>  Artificial

<220>
<223>  synthetic DNA

<400>  1222
cctaatgcga agctcacc                                                    18


<210>  1223
<211>  18
<212>  DNA
<213>  Artificial

<220>
<223>  synthetic DNA

<400>  1223
acaaccggtg aggcagta                                                    18


<210>  1224
<211>  18
<212>  DNA
<213>  Artificial

<220>
<223>  synthetic DNA

<400>  1224
ttggttcgaa ccaaccgc                                                    18


<210>  1225
<211>  18
<212>  DNA
<213>  Artificial

<220>
<223>  synthetic DNA

<400>  1225
atactaggtt gaactaag                                                    18


<210>  1226
<211>  18
<212>  DNA
<213>  Artificial

<220>
<223>  synthetic DNA

<400>  1226
gcgttgagag taacatat                                                    18


<210>  1227
<211>  18
<212>  DNA
<213>  Artificial

<220>
<223>  synthetic DNA

<400>  1227
agttgtataa taagcgtc                                                    18


<210>  1228
<211>  18
<212>  DNA
<213>  Artificial

<220>
<223>  synthetic DNA

<400>  1228
gtatgatgcc gtccaatt                                                    18


<210>  1229
<211>  18
<212>  DNA
<213>  Artificial

<220>
<223>  synthetic DNA

<400>  1229
ggactctctg aagagtct                                                    18


<210>  1230
<211>  18
<212>  DNA
<213>  Artificial

<220>
<223>  synthetic DNA

<400>  1230
ggactctctt gacttgaa                                                    18


<210>  1231
<211>  18
<212>  DNA
<213>  Artificial

<220>
<223>  synthetic DNA

<400>  1231
gataacagtg cttcgtcc                                                    18


<210>  1232
<211>  18
<212>  DNA
<213>  Artificial

<220>
<223>  synthetic DNA

<400>  1232
ggccattata gatgaact                                                    18

<210>  1233
<211>  18
<212>  DNA
<213>  Artificial

<220>
<223>  synthetic DNA

<400>  1233
atagagagca cagagcag                                                    18


<210>  1234
<211>  18
<212>  DNA
<213>  Artificial

<220>
<223>  synthetic DNA

<400>  1234
gtgtgagtgt atcataac                                                    18


<210>  1235
<211>  18
<212>  DNA
<213>  Artificial

<220>
<223>  synthetic DNA

<400>  1235
ataaccttag tgcgcgtc                                                    18


<210>  1236
<211>  18
<212>  DNA
<213>  Artificial

<220>
<223>  synthetic DNA

<400>  1236
ccgactgata tgcatgga                                                    18


<210>  1237
<211>  18
<212>  DNA
<213>  Artificial

<220>
<223>  synthetic DNA

<400>  1237
ggatatctga tcgcatca                                                    18


<210>  1238
<211>  18

<212>  DNA
<213>  Artificial

<220>
<223>  synthetic DNA

<400>  1238
cagcattaac gaggcgaa                                                    18


<210>  1239
<211>  18
<212>  DNA
<213>  Artificial

<220>
<223>  synthetic DNA

<400>  1239
gcgaggccta catattcg                                                    18


<210>  1240
<211>  18
<212>  DNA
<213>  Artificial

<220>
<223>  synthetic DNA

<400>  1240
cgataagtgg taaggtct                                                    18


<210>  1241
<211>  18
<212>  DNA
<213>  Artificial

<220>
<223>  synthetic DNA

<400>  1241
agatcctgag tcgagcaa                                                    18


<210>  1242
<211>  18
<212>  DNA
<213>  Artificial

<220>
<223>  synthetic DNA

<400>  1242
aagatataac gagaccga                                                    18


<210>  1243
<211>  18
<212>  DNA
<213>  Artificial

<220>
<223>  synthetic DNA

<400>  1243
ccgactgatt gagaacgt                                                    18


<210>  1244
<211>  18
<212>  DNA
<213>  Artificial

<220>
<223>  synthetic DNA

<400>  1244
tcggcttata tgacacgt                                                    18


<210>  1245
<211>  18
<212>  DNA
<213>  Artificial

<220>
<223>  synthetic DNA

<400>  1245
aataacgtac gccggagg                                                    18


<210>  1246
<211>  18
<212>  DNA
<213>  Artificial

<220>
<223>  synthetic DNA

<400>  1246
aacacagcat tgcgcacg                                                    18


<210>  1247
<211>  18
<212>  DNA
<213>  Artificial

<220>
<223>  synthetic DNA

<400>  1247
gtagtctgac agcaacaa                                                    18


<210>  1248
<211>  18
<212>  DNA
<213>  Artificial

<220>
<223>  synthetic DNA

<400>  1248
agaatgactt gagctgct                                                    18


<210>  1249
<211>  20
<212>  DNA
<213>  Artificial

<220>
<223>  synthetic DNA

<400>  1249
actggtagta acgtccacct                                                  20


<210>  1250
<211>  20
<212>  DNA
<213>  Artificial

<220>
<223>  synthetic DNA

<400>  1250
agactggttg ttattcgcct                                                  20


<210>  1251
<211>  20
<212>  DNA
<213>  Artificial

<220>
<223>  synthetic DNA

<400>  1251
tatcattgac agcgagctca                                                  20


<210>  1252
<211>  20
<212>  DNA
<213>  Artificial

<220>
<223>  synthetic DNA

<400>  1252
tggagtctga agaaggactc                                                  20


<210>  1253
<211>  20
<212>  DNA
<213>  Artificial

<220>
<223>  synthetic DNA

<400>  1253
catctggact acggcaacga                                                  20

<210>  1254
<211>  20
<212>  DNA
<213>  Artificial

<220>
<223>  synthetic DNA

<400>  1254
aactgtcata agacagacaa                                                    20


<210>  1255
<211>  20
<212>  DNA
<213>  Artificial

<220>
<223>  synthetic DNA

<400>  1255
cctcaacatg acatacaccg                                                    20


<210>  1256
<211>  20
<212>  DNA
<213>  Artificial

<220>
<223>  synthetic DNA

<400>  1256
caataccgtt cgcgattcta                                                    20


<210>  1257
<211>  20
<212>  DNA
<213>  Artificial

<220>
<223>  synthetic DNA

<400>  1257
gcgtctacgt tgattcggcc                                                    20


<210>  1258
<211>  20
<212>  DNA
<213>  Artificial

<220>
<223>  synthetic DNA

<400>  1258
tgaacagagg cacttgcagg                                                    20


<210>  1259
<211>  20

<212>  DNA
<213>  Artificial

<220>
<223>  synthetic DNA

<400>  1259
cgactagaac ctactactgc                                                   20


<210>  1260
<211>  20
<212>  DNA
<213>  Artificial

<220>
<223>  synthetic DNA

<400>  1260
gcaccgcacg tggagagata                                                   20


<210>  1261
<211>  20
<212>  DNA
<213>  Artificial

<220>
<223>  synthetic DNA

<400>  1261
ctgagagacc gactgatgcg                                                   20


<210>  1262
<211>  20
<212>  DNA
<213>  Artificial

<220>
<223>  synthetic DNA

<400>  1262
tcgtccttct acttaatgat                                                   20


<210>  1263
<211>  20
<212>  DNA
<213>  Artificial

<220>
<223>  synthetic DNA

<400>  1263
caagctatac catccgaatt                                                   20


<210>  1264
<211>  20
<212>  DNA
<213>  Artificial

<220>
<223>  synthetic DNA

<400>  1264
caatacgtat agtcttagat                                                    20


<210>  1265
<211>  20
<212>  DNA
<213>  Artificial

<220>
<223>  synthetic DNA

<400>  1265
ccatccacag tgacctatgt                                                    20


<210>  1266
<211>  20
<212>  DNA
<213>  Artificial

<220>
<223>  synthetic DNA

<400>  1266
tatccgttgg agaaggttca                                                    20


<210>  1267
<211>  20
<212>  DNA
<213>  Artificial

<220>
<223>  synthetic DNA

<400>  1267
cgcctaggta cctgagtacg                                                    20


<210>  1268
<211>  20
<212>  DNA
<213>  Artificial

<220>
<223>  synthetic DNA

<400>  1268
cagagtgctc gtgttcgcga                                                    20


<210>  1269
<211>  20
<212>  DNA
<213>  Artificial

<220>
<223>  synthetic DNA

<400>  1269
cgcttggaca tccttaagaa                                                   20


<210>  1270
<211>  20
<212>  DNA
<213>  Artificial

<220>
<223>  synthetic DNA

<400>  1270
gaccgcatga ttagtcttac                                                   20


<210>  1271
<211>  20
<212>  DNA
<213>  Artificial

<220>
<223>  synthetic DNA

<400>  1271
cttggccgta gtcactcagt                                                   20


<210>  1272
<211>  20
<212>  DNA
<213>  Artificial

<220>
<223>  synthetic DNA

<400>  1272
gatagcgata ttcagttcgc                                                   20


<210>  1273
<211>  20
<212>  DNA
<213>  Artificial

<220>
<223>  synthetic DNA

<400>  1273
atccaacact aagacaacca                                                   20


<210>  1274
<211>  20
<212>  DNA
<213>  Artificial

<220>
<223>  synthetic DNA

<400>  1274
ccattctgtt gcgtgtcctc                                                   20

<210>  1275
<211>  20
<212>  DNA
<213>  Artificial

<220>
<223>  synthetic DNA

<400>  1275
acattctgta cgcttgcagc                                                    20


<210>  1276
<211>  20
<212>  DNA
<213>  Artificial

<220>
<223>  synthetic DNA

<400>  1276
tgctgaacgc caatcgctta                                                    20


<210>  1277
<211>  20
<212>  DNA
<213>  Artificial

<220>
<223>  synthetic DNA

<400>  1277
tcctctacaa gaatattgcg                                                    20


<210>  1278
<211>  20
<212>  DNA
<213>  Artificial

<220>
<223>  synthetic DNA

<400>  1278
cgaccaacgc agcctgattc                                                    20


<210>  1279
<211>  20
<212>  DNA
<213>  Artificial

<220>
<223>  synthetic DNA

<400>  1279
attgcgagct tgagtagcgc                                                    20


<210>  1280
<211>  20

<212>  DNA
<213>  Artificial

<220>
<223>  synthetic DNA

<400>  1280
aaggtgcgag cataggaatc                                                    20


<210>  1281
<211>  20
<212>  DNA
<213>  Artificial

<220>
<223>  synthetic DNA

<400>  1281
cacttaagtg tgatatagat                                                    20


<210>  1282
<211>  20
<212>  DNA
<213>  Artificial

<220>
<223>  synthetic DNA

<400>  1282
atcggtatgc tgacctagac                                                    20


<210>  1283
<211>  20
<212>  DNA
<213>  Artificial

<220>
<223>  synthetic DNA

<400>  1283
tacaatctcg aatgcaggat                                                    20


<210>  1284
<211>  20
<212>  DNA
<213>  Artificial

<220>
<223>  synthetic DNA

<400>  1284
ccatatgaag cgcagccgtc                                                    20


<210>  1285
<211>  20
<212>  DNA
<213>  Artificial

<220>
<223>  synthetic DNA

<400>  1285
cgtctcgtgg acattcgagg                                                   20


<210>  1286
<211>  20
<212>  DNA
<213>  Artificial

<220>
<223>  synthetic DNA

<400>  1286
ccgagtacag aagcgtggaa                                                   20


<210>  1287
<211>  20
<212>  DNA
<213>  Artificial

<220>
<223>  synthetic DNA

<400>  1287
ttacgtggtc gacaggcagt                                                   20


<210>  1288
<211>  20
<212>  DNA
<213>  Artificial

<220>
<223>  synthetic DNA

<400>  1288
agctgcaatc tgcatgatta                                                   20


<210>  1289
<211>  20
<212>  DNA
<213>  Artificial

<220>
<223>  synthetic DNA

<400>  1289
acctgccgaa gcagcctaca                                                   20


<210>  1290
<211>  20
<212>  DNA
<213>  Artificial

<220>
<223>  synthetic DNA

<400>  1290
aacatgataa ccacatggtt                                                   20


<210>  1291
<211>  20
<212>  DNA
<213>  Artificial

<220>
<223>  synthetic DNA

<400>  1291
atccgactga ttgaattacc                                                   20


<210>  1292
<211>  20
<212>  DNA
<213>  Artificial

<220>
<223>  synthetic DNA

<400>  1292
tcacgctgac tcttatcagg                                                   20


<210>  1293
<211>  20
<212>  DNA
<213>  Artificial

<220>
<223>  synthetic DNA

<400>  1293
gcgcgctcga agtacaacat                                                   20


<210>  1294
<211>  20
<212>  DNA
<213>  Artificial

<220>
<223>  synthetic DNA

<400>  1294
acagccagat gcgttgttcc                                                   20


<210>  1295
<211>  20
<212>  DNA
<213>  Artificial

<220>
<223>  synthetic DNA

<400>  1295
ggagctctga cctgcaagaa                                                   20

<210>  1296
<211>  20
<212>  DNA
<213>  Artificial

<220>
<223>  synthetic DNA

<400>  1296
aacattagcc tcaagtaaga                                                   20


<210>  1297
<211>  20
<212>  DNA
<213>  Artificial

<220>
<223>  synthetic DNA

<400>  1297
tgtgattatg ccgaatgagg                                                   20


<210>  1298
<211>  20
<212>  DNA
<213>  Artificial

<220>
<223>  synthetic DNA

<400>  1298
gagtaataat ccaatcagta                                                   20


<210>  1299
<211>  20
<212>  DNA
<213>  Artificial

<220>
<223>  synthetic DNA

<400>  1299
ctccttggcg acagctgaac                                                   20


<210>  1300
<211>  20
<212>  DNA
<213>  Artificial

<220>
<223>  synthetic DNA

<400>  1300
ttacgcacac atacacagac                                                   20


<210>  1301
<211>  20

<212>  DNA
<213>  Artificial

<220>
<223>  synthetic DNA

<400>  1301
acgccgtatg gcgacttagg                                                   20


<210>  1302
<211>  20
<212>  DNA
<213>  Artificial

<220>
<223>  synthetic DNA

<400>  1302
agaacgacaa ttacgatggc                                                   20


<210>  1303
<211>  20
<212>  DNA
<213>  Artificial

<220>
<223>  synthetic DNA

<400>  1303
tgctaacgta ccactgccac                                                   20


<210>  1304
<211>  20
<212>  DNA
<213>  Artificial

<220>
<223>  synthetic DNA

<400>  1304
catccagaat gtctatcata                                                   20


<210>  1305
<211>  20
<212>  DNA
<213>  Artificial

<220>
<223>  synthetic DNA

<400>  1305
ggagaacgcc tatagcactc                                                   20


<210>  1306
<211>  20
<212>  DNA
<213>  Artificial

<220>
<223>  synthetic DNA

<400>  1306
acctcttgtg acggccagtc                                                   20


<210>  1307
<211>  20
<212>  DNA
<213>  Artificial

<220>
<223>  synthetic DNA

<400>  1307
tgccataact tggcataaga                                                   20


<210>  1308
<211>  20
<212>  DNA
<213>  Artificial

<220>
<223>  synthetic DNA

<400>  1308
acaattgtct gaccacgctc                                                   20


<210>  1309
<211>  20
<212>  DNA
<213>  Artificial

<220>
<223>  synthetic DNA

<400>  1309
tcgtcacctt cacagaacga                                                   20


<210>  1310
<211>  20
<212>  DNA
<213>  Artificial

<220>
<223>  synthetic DNA

<400>  1310
agcagcagat gatgatccaa                                                   20


<210>  1311
<211>  20
<212>  DNA
<213>  Artificial

<220>
<223>  synthetic DNA

<400>  1311
tcgtgccttg gattccagga                                                   20


<210>  1312
<211>  20
<212>  DNA
<213>  Artificial

<220>
<223>  synthetic DNA

<400>  1312
tgttatagcc acgatactat                                                   20


<210>  1313
<211>  20
<212>  DNA
<213>  Artificial

<220>
<223>  synthetic DNA

<400>  1313
aatctcacct gtaccttccg                                                   20


<210>  1314
<211>  20
<212>  DNA
<213>  Artificial

<220>
<223>  synthetic DNA

<400>  1314
gagtagcgga agcgttagcg                                                   20


<210>  1315
<211>  20
<212>  DNA
<213>  Artificial

<220>
<223>  synthetic DNA

<400>  1315
aatactccgg cgaggtatac                                                   20


<210>  1316
<211>  20
<212>  DNA
<213>  Artificial

<220>
<223>  synthetic DNA

<400>  1316
ttcgcatcct tgcacgaaca                                                   20

<210>  1317
<211>  20
<212>  DNA
<213>  Artificial

<220>
<223>  synthetic DNA

<400>  1317
aaccggctaa tactactggc                                                   20


<210>  1318
<211>  20
<212>  DNA
<213>  Artificial

<220>
<223>  synthetic DNA

<400>  1318
ctagcatctt agacaccaga                                                   20


<210>  1319
<211>  20
<212>  DNA
<213>  Artificial

<220>
<223>  synthetic DNA

<400>  1319
tagttgcgtg atacaagata                                                   20


<210>  1320
<211>  20
<212>  DNA
<213>  Artificial

<220>
<223>  synthetic DNA

<400>  1320
tcgtctcgac acagttggtc                                                   20


<210>  1321
<211>  20
<212>  DNA
<213>  Artificial

<220>
<223>  synthetic DNA

<400>  1321
tccgttcgcg tgcgaactga                                                   20


<210>  1322
<211>  20

<212>  DNA
<213>  Artificial

<220>
<223>  synthetic DNA

<400>  1322
tctgactctg gtgtacagtc                                                   20


<210>  1323
<211>  20
<212>  DNA
<213>  Artificial

<220>
<223>  synthetic DNA

<400>  1323
acagcgcaat tatatcctgt                                                   20


<210>  1324
<211>  20
<212>  DNA
<213>  Artificial

<220>
<223>  synthetic DNA

<400>  1324
agatccgtac gtgagactag                                                   20


<210>  1325
<211>  20
<212>  DNA
<213>  Artificial

<220>
<223>  synthetic DNA

<400>  1325
tacattgaag catccgaaca                                                   20


<210>  1326
<211>  20
<212>  DNA
<213>  Artificial

<220>
<223>  synthetic DNA

<400>  1326
ctcctgagag atcaacgcca                                                   20


<210>  1327
<211>  20
<212>  DNA
<213>  Artificial

<220>
<223>  synthetic DNA

<400>  1327
tcacctcgaa tgagttcgtt                                                    20


<210>  1328
<211>  20
<212>  DNA
<213>  Artificial

<220>
<223>  synthetic DNA

<400>  1328
tagcgactta aggtccaagc                                                    20


<210>  1329
<211>  20
<212>  DNA
<213>  Artificial

<220>
<223>  synthetic DNA

<400>  1329
agtacgtatt gccgtgcaag                                                    20


<210>  1330
<211>  20
<212>  DNA
<213>  Artificial

<220>
<223>  synthetic DNA

<400>  1330
agccacgaac cgacgtcata                                                    20


<210>  1331
<211>  20
<212>  DNA
<213>  Artificial

<220>
<223>  synthetic DNA

<400>  1331
tgatgtgtac gctactacta                                                    20


<210>  1332
<211>  20
<212>  DNA
<213>  Artificial

<220>
<223>  synthetic DNA

<400>  1332
ccactgtgtg cagcagacga                                                   20


<210>  1333
<211>  20
<212>  DNA
<213>  Artificial

<220>
<223>  synthetic DNA

<400>  1333
ctattgtaca gcgaacgctg                                                   20


<210>  1334
<211>  20
<212>  DNA
<213>  Artificial

<220>
<223>  synthetic DNA

<400>  1334
ctccgatatc gcacggatcg                                                   20


<210>  1335
<211>  20
<212>  DNA
<213>  Artificial

<220>
<223>  synthetic DNA

<400>  1335
aacttatcgt cggacgcatg                                                   20


<210>  1336
<211>  20
<212>  DNA
<213>  Artificial

<220>
<223>  synthetic DNA

<400>  1336
tatcctaatt cgtgccggtc                                                   20


<210>  1337
<211>  20
<212>  DNA
<213>  Artificial

<220>
<223>  synthetic DNA

<400>  1337
acagccttcc tgtgtggact                                                   20

<210>  1338
<211>  20
<212>  DNA
<213>  Artificial

<220>
<223>  synthetic DNA

<400>  1338
cctccgtgag gatcgtacca                                                    20


<210>  1339
<211>  20
<212>  DNA
<213>  Artificial

<220>
<223>  synthetic DNA

<400>  1339
gctctaagta acagaactaa                                                    20


<210>  1340
<211>  20
<212>  DNA
<213>  Artificial

<220>
<223>  synthetic DNA

<400>  1340
gacttaccgc gcgttctggt                                                    20


<210>  1341
<211>  20
<212>  DNA
<213>  Artificial

<220>
<223>  synthetic DNA

<400>  1341
tctgaggata cacatgtgga                                                    20


<210>  1342
<211>  20
<212>  DNA
<213>  Artificial

<220>
<223>  synthetic DNA

<400>  1342
tgtaatcaca ctggtgtcgg                                                    20


<210>  1343
<211>  20

<212>  DNA
<213>  Artificial

<220>
<223>  synthetic DNA

<400>  1343
cactaggcgg cagacataca                                                   20


<210>  1344
<211>  20
<212>  DNA
<213>  Artificial

<220>
<223>  synthetic DNA

<400>  1344
ctagagcaca gtaccacgtt                                                   20


<210>  1345
<211>  22
<212>  DNA
<213>  Artificial

<220>
<223>  synthetic DNA

<400>  1345
ttcagaggtc tacgcttccg gt                                                22


<210>  1346
<211>  22
<212>  DNA
<213>  Artificial

<220>
<223>  synthetic DNA

<400>  1346
aacacagact gcgttatgcc aa                                                22


<210>  1347
<211>  22
<212>  DNA
<213>  Artificial

<220>
<223>  synthetic DNA

<400>  1347
tgctgagttc tatacagcag tg                                                22


<210>  1348
<211>  22
<212>  DNA
<213>  Artificial

<220>
<223>  synthetic DNA

<400>  1348
acctattata tgatagcgtc at                                                22


<210>  1349
<211>  22
<212>  DNA
<213>  Artificial

<220>
<223>  synthetic DNA

<400>  1349
atcgtgagct acagtgaatg ca                                                22


<210>  1350
<211>  22
<212>  DNA
<213>  Artificial

<220>
<223>  synthetic DNA

<400>  1350
cgtgatgtat ccggccttgc ag                                                22


<210>  1351
<211>  22
<212>  DNA
<213>  Artificial

<220>
<223>  synthetic DNA

<400>  1351
tcttctggtc ctagagttgt gc                                                22


<210>  1352
<211>  22
<212>  DNA
<213>  Artificial

<220>
<223>  synthetic DNA

<400>  1352
tgatgtcggc ggcggatcag at                                                22


<210>  1353
<211>  22
<212>  DNA
<213>  Artificial

<220>
<223>  synthetic DNA

<400>  1353
tcggccttag cgttcagcat cc                                                22


<210>  1354
<211>  22
<212>  DNA
<213>  Artificial

<220>
<223>  synthetic DNA

<400>  1354
ttaagtaggt cagccactgc ac                                                22


<210>  1355
<211>  22
<212>  DNA
<213>  Artificial

<220>
<223>  synthetic DNA

<400>  1355
ccaggtgagt tgatctgaca cc                                                22


<210>  1356
<211>  22
<212>  DNA
<213>  Artificial

<220>
<223>  synthetic DNA

<400>  1356
tatactatta ctgtgttcga tc                                                22


<210>  1357
<211>  22
<212>  DNA
<213>  Artificial

<220>
<223>  synthetic DNA

<400>  1357
ccgcagtatg tctagtgttg tc                                                22


<210>  1358
<211>  22
<212>  DNA
<213>  Artificial

<220>
<223>  synthetic DNA

<400>  1358
gtctaccgcg tacgaagctc tc                                                22

<210> 1359
<211> 22
<212> DNA
<213> Artificial

<220>
<223> synthetic DNA

<400> 1359
atgcgagtcc gtggtcgatc ct 22


<210> 1360
<211> 22
<212> DNA
<213> Artificial

<220>
<223> synthetic DNA

<400> 1360
tggtagattg gtgtgagaac ta 22


<210> 1361
<211> 22
<212> DNA
<213> Artificial

<220>
<223> synthetic DNA

<400> 1361
aggttcgtcg atcaactgct aa 22


<210> 1362
<211> 22
<212> DNA
<213> Artificial

<220>
<223> synthetic DNA

<400> 1362
acgacaagca tcctgcgata tc 22


<210> 1363
<211> 22
<212> DNA
<213> Artificial

<220>
<223> synthetic DNA

<400> 1363
ttgaatcaca gagagcgtga tt 22


<210> 1364
<211> 22

<212>  DNA
<213>  Artificial

<220>
<223>  synthetic DNA

<400>  1364
gtacttagtg cttacgtcag ct                                                22


<210>  1365
<211>  22
<212>  DNA
<213>  Artificial

<220>
<223>  synthetic DNA

<400>  1365
gattattaag gccaagctca ta                                                22


<210>  1366
<211>  22
<212>  DNA
<213>  Artificial

<220>
<223>  synthetic DNA

<400>  1366
gcatgcagag acgtactcat cg                                                22


<210>  1367
<211>  22
<212>  DNA
<213>  Artificial

<220>
<223>  synthetic DNA

<400>  1367
tagcggatgg tgtcctggca ct                                                22


<210>  1368
<211>  22
<212>  DNA
<213>  Artificial

<220>
<223>  synthetic DNA

<400>  1368
tacggctgcc aacttaataa ct                                                22


<210>  1369
<211>  22
<212>  DNA
<213>  Artificial

<220>
<223>  synthetic DNA

<400>  1369
ctcatatgac aacttctata gt                                                22


<210>  1370
<211>  22
<212>  DNA
<213>  Artificial

<220>
<223>  synthetic DNA

<400>  1370
caagcaatag ttgtcggcca cc                                                22


<210>  1371
<211>  22
<212>  DNA
<213>  Artificial

<220>
<223>  synthetic DNA

<400>  1371
ttcagcaatc cgtactgcta ga                                                22


<210>  1372
<211>  22
<212>  DNA
<213>  Artificial

<220>
<223>  synthetic DNA

<400>  1372
tgagacgttg ctgacattct cc                                                22


<210>  1373
<211>  22
<212>  DNA
<213>  Artificial

<220>
<223>  synthetic DNA

<400>  1373
gttccgatga gttagatgta ta                                                22


<210>  1374
<211>  22
<212>  DNA
<213>  Artificial

<220>
<223>  synthetic DNA

<400>  1374
ttgacgcttg gaggagtaca ag                                                22


<210>  1375
<211>  22
<212>  DNA
<213>  Artificial

<220>
<223>  synthetic DNA

<400>  1375
ttcatgttac ctccacattg tg                                                22


<210>  1376
<211>  22
<212>  DNA
<213>  Artificial

<220>
<223>  synthetic DNA

<400>  1376
gagcacgtgc cagattgcaa cc                                                22


<210>  1377
<211>  22
<212>  DNA
<213>  Artificial

<220>
<223>  synthetic DNA

<400>  1377
ggtcgacaag cacaagcctt ct                                                22


<210>  1378
<211>  22
<212>  DNA
<213>  Artificial

<220>
<223>  synthetic DNA

<400>  1378
taggcaggta agatgaccga ct                                                22


<210>  1379
<211>  22
<212>  DNA
<213>  Artificial

<220>
<223>  synthetic DNA

<400>  1379
cgaggcatgc caagtcgcca at                                                22

<210>  1380
<211>  22
<212>  DNA
<213>  Artificial

<220>
<223>  synthetic DNA

<400>  1380
agtgttgata ggcggatgag ag                                                22


<210>  1381
<211>  22
<212>  DNA
<213>  Artificial

<220>
<223>  synthetic DNA

<400>  1381
ttcggtctag acctctcaca at                                                22


<210>  1382
<211>  22
<212>  DNA
<213>  Artificial

<220>
<223>  synthetic DNA

<400>  1382
gtgacgctca tatcttgcca cc                                                22


<210>  1383
<211>  22
<212>  DNA
<213>  Artificial

<220>
<223>  synthetic DNA

<400>  1383
gatgtaattc tacgcgcgga ct                                                22


<210>  1384
<211>  22
<212>  DNA
<213>  Artificial

<220>
<223>  synthetic DNA

<400>  1384
gatggcgatg ttgcattaca tg                                                22


<210>  1385
<211>  22

<212>  DNA
<213>  Artificial

<220>
<223>  synthetic DNA

<400>  1385
tatgctctga attaacgtag aa                                                22


<210>  1386
<211>  22
<212>  DNA
<213>  Artificial

<220>
<223>  synthetic DNA

<400>  1386
aggcaatatg gtgatccgta gc                                                22


<210>  1387
<211>  22
<212>  DNA
<213>  Artificial

<220>
<223>  synthetic DNA

<400>  1387
tgacagcgat gcatacagta gt                                                22


<210>  1388
<211>  22
<212>  DNA
<213>  Artificial

<220>
<223>  synthetic DNA

<400>  1388
ttctgctaac ggtatccaat ac                                                22


<210>  1389
<211>  22
<212>  DNA
<213>  Artificial

<220>
<223>  synthetic DNA

<400>  1389
gagtcgtcca tacgatctag ga                                                22


<210>  1390
<211>  22
<212>  DNA
<213>  Artificial

<220>
<223>  synthetic DNA

<400>  1390
agacggactc aacgccaatt cc                                                22


<210>  1391
<211>  22
<212>  DNA
<213>  Artificial

<220>
<223>  synthetic DNA

<400>  1391
gtagtgttga gcggaccgag ct                                                22


<210>  1392
<211>  22
<212>  DNA
<213>  Artificial

<220>
<223>  synthetic DNA

<400>  1392
aatataacta gatcatagcc ag                                                22


<210>  1393
<211>  22
<212>  DNA
<213>  Artificial

<220>
<223>  synthetic DNA

<400>  1393
tcaatcggag aatacagaac gt                                                22


<210>  1394
<211>  22
<212>  DNA
<213>  Artificial

<220>
<223>  synthetic DNA

<400>  1394
atctccgtcg tccgaaccaa ca                                                22


<210>  1395
<211>  22
<212>  DNA
<213>  Artificial

<220>
<223>  synthetic DNA

<400>  1395
taggcgttca gcggtatgct ta                                                22


<210>  1396
<211>  22
<212>  DNA
<213>  Artificial

<220>
<223>  synthetic DNA

<400>  1396
tgcgtgctat acaacctata cg                                                22


<210>  1397
<211>  22
<212>  DNA
<213>  Artificial

<220>
<223>  synthetic DNA

<400>  1397
atggccggca tacatctgta tg                                                22


<210>  1398
<211>  22
<212>  DNA
<213>  Artificial

<220>
<223>  synthetic DNA

<400>  1398
tgatgctgac ataacactga at                                                22


<210>  1399
<211>  22
<212>  DNA
<213>  Artificial

<220>
<223>  synthetic DNA

<400>  1399
atccaaggta cctgaacatc ct                                                22


<210>  1400
<211>  22
<212>  DNA
<213>  Artificial

<220>
<223>  synthetic DNA

<400>  1400
tagtgacgac caggtgagcc tc                                                22

<210>  1401
<211>  22
<212>  DNA
<213>  Artificial

<220>
<223>  synthetic DNA

<400>  1401
aggaggatcc gtcaagtcga cc                                                22


<210>  1402
<211>  22
<212>  DNA
<213>  Artificial

<220>
<223>  synthetic DNA

<400>  1402
agagtatgcc agatcgtgag gc                                                22


<210>  1403
<211>  22
<212>  DNA
<213>  Artificial

<220>
<223>  synthetic DNA

<400>  1403
ccactcacta ggatggctgc gt                                                22


<210>  1404
<211>  22
<212>  DNA
<213>  Artificial

<220>
<223>  synthetic DNA

<400>  1404
tatccaacct gttatagcga tt                                                22


<210>  1405
<211>  22
<212>  DNA
<213>  Artificial

<220>
<223>  synthetic DNA

<400>  1405
tcttgcagtg agttgagtct gc                                                22


<210>  1406
<211>  22

<212>  DNA
<213>  Artificial

<220>
<223>  synthetic DNA

<400>  1406
ccactgttgt acatacacct gg                                                22


<210>  1407
<211>  22
<212>  DNA
<213>  Artificial

<220>
<223>  synthetic DNA

<400>  1407
atgcgcgtag gccactaagt cc                                                22


<210>  1408
<211>  22
<212>  DNA
<213>  Artificial

<220>
<223>  synthetic DNA

<400>  1408
acagcggtct acaaccgact gc                                                22


<210>  1409
<211>  22
<212>  DNA
<213>  Artificial

<220>
<223>  synthetic DNA

<400>  1409
tcgcgctcca gacaattgca gc                                                22


<210>  1410
<211>  22
<212>  DNA
<213>  Artificial

<220>
<223>  synthetic DNA

<400>  1410
ccggtagacc aggagtggtc at                                                22


<210>  1411
<211>  22
<212>  DNA
<213>  Artificial

<220>
<223>  synthetic DNA

<400>  1411
atctcctaac ctagagccat ct                                                22


<210>  1412
<211>  22
<212>  DNA
<213>  Artificial

<220>
<223>  synthetic DNA

<400>  1412
ccacatcgaa tctaacaact ac                                                22


<210>  1413
<211>  22
<212>  DNA
<213>  Artificial

<220>
<223>  synthetic DNA

<400>  1413
tagtcttatt gaatacgtcc ta                                                22


<210>  1414
<211>  22
<212>  DNA
<213>  Artificial

<220>
<223>  synthetic DNA

<400>  1414
tccttaagcc ttggaactgg cg                                                22


<210>  1415
<211>  22
<212>  DNA
<213>  Artificial

<220>
<223>  synthetic DNA

<400>  1415
ccgtgatgga ttgacgtaga gg                                                22


<210>  1416
<211>  22
<212>  DNA
<213>  Artificial

<220>
<223>  synthetic DNA

<400>  1416
gcctggataa cagatgtctt ag                                                22


<210>  1417
<211>  22
<212>  DNA
<213>  Artificial

<220>
<223>  synthetic DNA

<400>  1417
ctcgacctat aatcttctgc ca                                                22


<210>  1418
<211>  22
<212>  DNA
<213>  Artificial

<220>
<223>  synthetic DNA

<400>  1418
agctacttct ccttcctaat ca                                                22


<210>  1419
<211>  22
<212>  DNA
<213>  Artificial

<220>
<223>  synthetic DNA

<400>  1419
acacgctatt gccttccagt ta                                                22


<210>  1420
<211>  22
<212>  DNA
<213>  Artificial

<220>
<223>  synthetic DNA

<400>  1420
aagcctgtgc atgcaatgag aa                                                22


<210>  1421
<211>  22
<212>  DNA
<213>  Artificial

<220>
<223>  synthetic DNA

<400>  1421
tcgttggtta tagcacaact tc                                                22

<210>  1422
<211>  22
<212>  DNA
<213>  Artificial

<220>
<223>  synthetic DNA

<400>  1422
gcgatgcctt ccaacatacc aa                                                22


<210>  1423
<211>  22
<212>  DNA
<213>  Artificial

<220>
<223>  synthetic DNA

<400>  1423
ccaccgttag cacgtgctac gt                                                22


<210>  1424
<211>  22
<212>  DNA
<213>  Artificial

<220>
<223>  synthetic DNA

<400>  1424
gttaccacaa tgccgccatc aa                                                22


<210>  1425
<211>  22
<212>  DNA
<213>  Artificial

<220>
<223>  synthetic DNA

<400>  1425
ggtgcattaa gaacgaacta cc                                                22


<210>  1426
<211>  22
<212>  DNA
<213>  Artificial

<220>
<223>  synthetic DNA

<400>  1426
tccttccgga taatgccgat tc                                                22


<210>  1427
<211>  22

<212>  DNA
<213>  Artificial

<220>
<223>  synthetic DNA

<400>  1427
aaccgcaact tctagcggaa ga                                                22


<210>  1428
<211>  22
<212>  DNA
<213>  Artificial

<220>
<223>  synthetic DNA

<400>  1428
tccttaagca gttgaaccta gg                                                22


<210>  1429
<211>  22
<212>  DNA
<213>  Artificial

<220>
<223>  synthetic DNA

<400>  1429
tactaagtca gataagatca ga                                                22


<210>  1430
<211>  22
<212>  DNA
<213>  Artificial

<220>
<223>  synthetic DNA

<400>  1430
ttcgccataa ctagatgaat gc                                                22


<210>  1431
<211>  22
<212>  DNA
<213>  Artificial

<220>
<223>  synthetic DNA

<400>  1431
aagaagttag acgcggtggc tg                                                22


<210>  1432
<211>  22
<212>  DNA
<213>  Artificial

<220>
<223>  synthetic DNA

<400>  1432
gtatctgatc gaagagcggt gg                                                22


<210>  1433
<211>  22
<212>  DNA
<213>  Artificial

<220>
<223>  synthetic DNA

<400>  1433
tcaagagcta cgaagtaagt cc                                                22


<210>  1434
<211>  22
<212>  DNA
<213>  Artificial

<220>
<223>  synthetic DNA

<400>  1434
cgagtacaca gcagcatacc ta                                                22


<210>  1435
<211>  22
<212>  DNA
<213>  Artificial

<220>
<223>  synthetic DNA

<400>  1435
ctcgataagt tactctgcta ga                                                22


<210>  1436
<211>  22
<212>  DNA
<213>  Artificial

<220>
<223>  synthetic DNA

<400>  1436
atggtgctgg ttctccgtct gt                                                22


<210>  1437
<211>  22
<212>  DNA
<213>  Artificial

<220>
<223>  synthetic DNA

<400>  1437
tcaagcggtc caaggctgag ac                                                22


<210>  1438
<211>  22
<212>  DNA
<213>  Artificial

<220>
<223>  synthetic DNA

<400>  1438
tgtcctgctc tgttgctacc gt                                                22


<210>  1439
<211>  22
<212>  DNA
<213>  Artificial

<220>
<223>  synthetic DNA

<400>  1439
agtcatatcg cgtcacacgt tg                                                22


<210>  1440
<211>  22
<212>  DNA
<213>  Artificial

<220>
<223>  synthetic DNA

<400>  1440
ggtgaataag gacatgagaa gc                                                22


<210>  1441
<211>  24
<212>  DNA
<213>  Artificial

<220>
<223>  synthetic DNA

<400>  1441
cctgatctta tctagtagag actc                                              24


<210>  1442
<211>  24
<212>  DNA
<213>  Artificial

<220>
<223>  synthetic DNA

<400>  1442
ttctgtgtag gtgtgccaat cacc                                              24

<210>  1443
<211>  24
<212>  DNA
<213>  Artificial

<220>
<223>  synthetic DNA

<400>  1443
gacttccaga tgcttaagac gaca                                              24


<210>  1444
<211>  24
<212>  DNA
<213>  Artificial

<220>
<223>  synthetic DNA

<400>  1444
gtccttcgac ggagaacatc cgag                                              24


<210>  1445
<211>  24
<212>  DNA
<213>  Artificial

<220>
<223>  synthetic DNA

<400>  1445
cttggttagt gtaccgtcaa cgtc                                              24


<210>  1446
<211>  24
<212>  DNA
<213>  Artificial

<220>
<223>  synthetic DNA

<400>  1446
aagcggcatg tgcctaatcg acgt                                              24


<210>  1447
<211>  24
<212>  DNA
<213>  Artificial

<220>
<223>  synthetic DNA

<400>  1447
cgaccgtcgt tacacggaat ccga                                              24


<210>  1448
<211>  24

<212>  DNA
<213>  Artificial

<220>
<223>  synthetic DNA

<400>  1448
tcgcaagtgt gccgttctgt tcat                                              24


<210>  1449
<211>  24
<212>  DNA
<213>  Artificial

<220>
<223>  synthetic DNA

<400>  1449
cgtactgaag ttcggagtcg ccgt                                              24


<210>  1450
<211>  24
<212>  DNA
<213>  Artificial

<220>
<223>  synthetic DNA

<400>  1450
ccactacaga atggtagcag atca                                              24


<210>  1451
<211>  24
<212>  DNA
<213>  Artificial

<220>
<223>  synthetic DNA

<400>  1451
agtaggagag aggcctacac aaca                                              24


<210>  1452
<211>  24
<212>  DNA
<213>  Artificial

<220>
<223>  synthetic DNA

<400>  1452
agccaagata ctcgttcggt atgg                                              24


<210>  1453
<211>  24
<212>  DNA
<213>  Artificial

<220>
<223>  synthetic DNA

<400>  1453
gttccgagta cattgaatcc tggc                                              24


<210>  1454
<211>  24
<212>  DNA
<213>  Artificial

<220>
<223>  synthetic DNA

<400>  1454
aggcgtacga gttattgcca gagg                                              24


<210>  1455
<211>  24
<212>  DNA
<213>  Artificial

<220>
<223>  synthetic DNA

<400>  1455
gtggcatcac acatatctca gcat                                              24


<210>  1456
<211>  24
<212>  DNA
<213>  Artificial

<220>
<223>  synthetic DNA

<400>  1456
gagaccgata tgttgatgcc agaa                                              24


<210>  1457
<211>  24
<212>  DNA
<213>  Artificial

<220>
<223>  synthetic DNA

<400>  1457
caactgtagc cagtcgattg ctat                                              24


<210>  1458
<211>  24
<212>  DNA
<213>  Artificial

<220>
<223>  synthetic DNA

<400>  1458
tatcaatgca atgagaggat gcag                                              24


<210>  1459
<211>  24
<212>  DNA
<213>  Artificial

<220>
<223>  synthetic DNA

<400>  1459
gtatgctcgg ctccaagtac tgtt                                              24


<210>  1460
<211>  24
<212>  DNA
<213>  Artificial

<220>
<223>  synthetic DNA

<400>  1460
agagactctt ataggcttga cgga                                              24


<210>  1461
<211>  24
<212>  DNA
<213>  Artificial

<220>
<223>  synthetic DNA

<400>  1461
acttaacaga tatggatcat cgcc                                              24


<210>  1462
<211>  24
<212>  DNA
<213>  Artificial

<220>
<223>  synthetic DNA

<400>  1462
aatcagagcg agtctcgctt cagg                                              24


<210>  1463
<211>  24
<212>  DNA
<213>  Artificial

<220>
<223>  synthetic DNA

<400>  1463
accaccgagg aacaggtgcg acaa                                              24

<210>  1464
<211>  24
<212>  DNA
<213>  Artificial

<220>
<223>  synthetic DNA

<400>  1464
tggtacatgt caaccgtaag cctg                                              24


<210>  1465
<211>  24
<212>  DNA
<213>  Artificial

<220>
<223>  synthetic DNA

<400>  1465
cgtgccgcgg tgttcttgta tatg                                              24


<210>  1466
<211>  24
<212>  DNA
<213>  Artificial

<220>
<223>  synthetic DNA

<400>  1466
gacaagcgcg cgtgagacat atca                                              24


<210>  1467
<211>  24
<212>  DNA
<213>  Artificial

<220>
<223>  synthetic DNA

<400>  1467
agtgcactcc gaacaagagt tagt                                              24


<210>  1468
<211>  24
<212>  DNA
<213>  Artificial

<220>
<223>  synthetic DNA

<400>  1468
cctcattacc gcgttaggag tccg                                              24


<210>  1469
<211>  24

<212>  DNA
<213>  Artificial

<220>
<223>  synthetic DNA

<400>  1469
tgcttattgc ttagttgcta tctc                                              24


<210>  1470
<211>  24
<212>  DNA
<213>  Artificial

<220>
<223>  synthetic DNA

<400>  1470
gcgtgatcct gttctattcg ttag                                              24


<210>  1471
<211>  24
<212>  DNA
<213>  Artificial

<220>
<223>  synthetic DNA

<400>  1471
ggccagaact atgacgagta taag                                              24


<210>  1472
<211>  24
<212>  DNA
<213>  Artificial

<220>
<223>  synthetic DNA

<400>  1472
gatggcgact atctaattgc aatg                                              24


<210>  1473
<211>  24
<212>  DNA
<213>  Artificial

<220>
<223>  synthetic DNA

<400>  1473
tagtaaccat agctctgtac aact                                              24


<210>  1474
<211>  24
<212>  DNA
<213>  Artificial

<220>
<223>  synthetic DNA

<400>  1474
cgtgatcgcc aatacacatg tcgc                                              24


<210>  1475
<211>  24
<212>  DNA
<213>  Artificial

<220>
<223>  synthetic DNA

<400>  1475
taataacgga tcgatatgca cgcg                                              24


<210>  1476
<211>  24
<212>  DNA
<213>  Artificial

<220>
<223>  synthetic DNA

<400>  1476
atcatcgcgc taatactatc tgaa                                              24


<210>  1477
<211>  24
<212>  DNA
<213>  Artificial

<220>
<223>  synthetic DNA

<400>  1477
cacgtgcgtg caggtcacta gtat                                              24


<210>  1478
<211>  24
<212>  DNA
<213>  Artificial

<220>
<223>  synthetic DNA

<400>  1478
aggtccaatg ccgagcgatc agaa                                              24


<210>  1479
<211>  24
<212>  DNA
<213>  Artificial

<220>
<223>  synthetic DNA

<400>  1479
cagcataaca acgagccagg tcag                                              24


<210>  1480
<211>  24
<212>  DNA
<213>  Artificial

<220>
<223>  synthetic DNA

<400>  1480
atggcgtcca atactccgac ctat                                              24


<210>  1481
<211>  24
<212>  DNA
<213>  Artificial

<220>
<223>  synthetic DNA

<400>  1481
aggaacatcg tgaataatga agac                                              24


<210>  1482
<211>  24
<212>  DNA
<213>  Artificial

<220>
<223>  synthetic DNA

<400>  1482
tctcgacgtt catgtaatta agga                                              24


<210>  1483
<211>  24
<212>  DNA
<213>  Artificial

<220>
<223>  synthetic DNA

<400>  1483
tcgcggttaa ccttacttag acga                                              24


<210>  1484
<211>  24
<212>  DNA
<213>  Artificial

<220>
<223>  synthetic DNA

<400>  1484
atcatatcta cggctctggc gccg                                              24

<210>  1485
<211>  24
<212>  DNA
<213>  Artificial

<220>
<223>  synthetic DNA

<400>  1485
gcagatggag accagaggta cagg                                              24


<210>  1486
<211>  24
<212>  DNA
<213>  Artificial

<220>
<223>  synthetic DNA

<400>  1486
agacagaaga ttaccacgtg ctat                                              24


<210>  1487
<211>  24
<212>  DNA
<213>  Artificial

<220>
<223>  synthetic DNA

<400>  1487
ccacggacaa catgccgctt aact                                              24


<210>  1488
<211>  24
<212>  DNA
<213>  Artificial

<220>
<223>  synthetic DNA

<400>  1488
cttgaagtct caagctatga gaga                                              24


<210>  1489
<211>  24
<212>  DNA
<213>  Artificial

<220>
<223>  synthetic DNA

<400>  1489
acagcagtcg tgcttaggtc actg                                              24


<210>  1490
<211>  24

<212>  DNA
<213>  Artificial

<220>
<223>  synthetic DNA

<400>  1490
aggtgttaat gaacgtaggt gaga                                              24


<210>  1491
<211>  24
<212>  DNA
<213>  Artificial

<220>
<223>  synthetic DNA

<400>  1491
agccactatg ttcaaggctg agcc                                              24


<210>  1492
<211>  24
<212>  DNA
<213>  Artificial

<220>
<223>  synthetic DNA

<400>  1492
gcaggcggtg tcgtgtgaca atga                                              24


<210>  1493
<211>  24
<212>  DNA
<213>  Artificial

<220>
<223>  synthetic DNA

<400>  1493
agccattgct acagaggtta ctta                                              24


<210>  1494
<211>  24
<212>  DNA
<213>  Artificial

<220>
<223>  synthetic DNA

<400>  1494
acaatcgaac ctacactgag tccg                                              24


<210>  1495
<211>  24
<212>  DNA
<213>  Artificial

<220>
<223>  synthetic DNA

<400>  1495
ccgatctcaa taggtaccac gaac                                              24


<210>  1496
<211>  24
<212>  DNA
<213>  Artificial

<220>
<223>  synthetic DNA

<400>  1496
gatacgtggc gctatgctaa ttaa                                              24


<210>  1497
<211>  24
<212>  DNA
<213>  Artificial

<220>
<223>  synthetic DNA

<400>  1497
agagagatgg cacacattga cgtc                                              24


<210>  1498
<211>  24
<212>  DNA
<213>  Artificial

<220>
<223>  synthetic DNA

<400>  1498
ctcaactcat ccttgtagcc gatg                                              24


<210>  1499
<211>  24
<212>  DNA
<213>  Artificial

<220>
<223>  synthetic DNA

<400>  1499
gtggaataac gcgatacgac tctt                                              24


<210>  1500
<211>  24
<212>  DNA
<213>  Artificial

<220>
<223>  synthetic DNA

<400>  1500
atctaccatg cgaatgctct ctag                                              24


<210>  1501
<211>  24
<212>  DNA
<213>  Artificial

<220>
<223>  synthetic DNA

<400>  1501
atacgcacgc ctgacacaag gacc                                              24


<210>  1502
<211>  24
<212>  DNA
<213>  Artificial

<220>
<223>  synthetic DNA

<400>  1502
gtccactctc agtgtgtaga gtcc                                              24


<210>  1503
<211>  24
<212>  DNA
<213>  Artificial

<220>
<223>  synthetic DNA

<400>  1503
aatatatcca gattctctgt gcag                                              24


<210>  1504
<211>  24
<212>  DNA
<213>  Artificial

<220>
<223>  synthetic DNA

<400>  1504
ccttccgcca catgttcgac aagg                                              24


<210>  1505
<211>  24
<212>  DNA
<213>  Artificial

<220>
<223>  synthetic DNA

<400>  1505
actgtgccat catccgagga gcca                                              24

<210>  1506
<211>  24
<212>  DNA
<213>  Artificial

<220>
<223>  synthetic DNA

<400>  1506
tctatgccgc tatggcgtcg tgta                                              24


<210>  1507
<211>  24
<212>  DNA
<213>  Artificial

<220>
<223>  synthetic DNA

<400>  1507
cgtaacctaa ggtaatatgt ctgc                                              24


<210>  1508
<211>  24
<212>  DNA
<213>  Artificial

<220>
<223>  synthetic DNA

<400>  1508
tactgaccgt atcaagatta ctaa                                              24


<210>  1509
<211>  24
<212>  DNA
<213>  Artificial

<220>
<223>  synthetic DNA

<400>  1509
tcatcggagc gccatacggt acgt                                              24


<210>  1510
<211>  24
<212>  DNA
<213>  Artificial

<220>
<223>  synthetic DNA

<400>  1510
gcaagaggaa tgaacgaagt gatt                                              24


<210>  1511
<211>  24

<212>  DNA
<213>  Artificial

<220>
<223>  synthetic DNA

<400>  1511
ggctgattga catcctgact tagt                                              24


<210>  1512
<211>  24
<212>  DNA
<213>  Artificial

<220>
<223>  synthetic DNA

<400>  1512
aaggcgctag attggattaa cgta                                              24


<210>  1513
<211>  24
<212>  DNA
<213>  Artificial

<220>
<223>  synthetic DNA

<400>  1513
gctagctaga agaataggat tcgt                                              24


<210>  1514
<211>  24
<212>  DNA
<213>  Artificial

<220>
<223>  synthetic DNA

<400>  1514
caggtgacgg cctctataac tcat                                              24


<210>  1515
<211>  24
<212>  DNA
<213>  Artificial

<220>
<223>  synthetic DNA

<400>  1515
caggttacac ataccactat cttc                                              24


<210>  1516
<211>  24
<212>  DNA
<213>  Artificial

<220>
<223>  synthetic DNA

<400>  1516
ttgctacgta ccgtcttaat ccgt                                              24


<210>  1517
<211>  24
<212>  DNA
<213>  Artificial

<220>
<223>  synthetic DNA

<400>  1517
ctcaacatgt cttgcaagct tcga                                              24


<210>  1518
<211>  24
<212>  DNA
<213>  Artificial

<220>
<223>  synthetic DNA

<400>  1518
ggtgcggtac gtagaaccag atca                                              24


<210>  1519
<211>  24
<212>  DNA
<213>  Artificial

<220>
<223>  synthetic DNA

<400>  1519
aatgctctcc aagatcctga ccta                                              24


<210>  1520
<211>  24
<212>  DNA
<213>  Artificial

<220>
<223>  synthetic DNA

<400>  1520
gcttcgcagg tctggatgat ggag                                              24


<210>  1521
<211>  24
<212>  DNA
<213>  Artificial

<220>
<223>  synthetic DNA

<400>  1521
acattgacca gacagcacct tgcg                                              24


<210>  1522
<211>  24
<212>  DNA
<213>  Artificial

<220>
<223>  synthetic DNA

<400>  1522
aggtatcaat gtgcttaata ggcg                                              24


<210>  1523
<211>  24
<212>  DNA
<213>  Artificial

<220>
<223>  synthetic DNA

<400>  1523
tccggacaca cgattagtaa cgga                                              24


<210>  1524
<211>  24
<212>  DNA
<213>  Artificial

<220>
<223>  synthetic DNA

<400>  1524
tacgaagtac tacagatcgg tcag                                              24


<210>  1525
<211>  24
<212>  DNA
<213>  Artificial

<220>
<223>  synthetic DNA

<400>  1525
aattgtcaga cgaatactgc tgga                                              24


<210>  1526
<211>  24
<212>  DNA
<213>  Artificial

<220>
<223>  synthetic DNA

<400>  1526
tgaatcatga gccagaggtt atgc                                              24

<210>  1527
<211>  24
<212>  DNA
<213>  Artificial

<220>
<223>  synthetic DNA

<400>  1527
cacaagacac gtcattaaca tcaa                                              24


<210>  1528
<211>  24
<212>  DNA
<213>  Artificial

<220>
<223>  synthetic DNA

<400>  1528
gaatgactac attactccgc cagg                                              24


<210>  1529
<211>  24
<212>  DNA
<213>  Artificial

<220>
<223>  synthetic DNA

<400>  1529
agccagagat actggaactt gact                                              24


<210>  1530
<211>  24
<212>  DNA
<213>  Artificial

<220>
<223>  synthetic DNA

<400>  1530
tatcagacac atcacaatgg atac                                              24


<210>  1531
<211>  24
<212>  DNA
<213>  Artificial

<220>
<223>  synthetic DNA

<400>  1531
ctaggacacc gctagtcggt tgaa                                              24


<210>  1532
<211>  24

<212>  DNA
<213>  Artificial

<220>
<223>  synthetic DNA

<400>  1532
gtataactgc gtgtcctggt gtat                                              24


<210>  1533
<211>  24
<212>  DNA
<213>  Artificial

<220>
<223>  synthetic DNA

<400>  1533
atgcaatact aaggtggacc tccg                                              24


<210>  1534
<211>  24
<212>  DNA
<213>  Artificial

<220>
<223>  synthetic DNA

<400>  1534
atgcagacgc ttgcgataag tcat                                              24


<210>  1535
<211>  24
<212>  DNA
<213>  Artificial

<220>
<223>  synthetic DNA

<400>  1535
ttgctcgata cacgtagacc agtg                                              24


<210>  1536
<211>  24
<212>  DNA
<213>  Artificial

<220>
<223>  synthetic DNA

<400>  1536
tactggagga cgattgtcta tcat                                              24


<210>  1537
<211>  26
<212>  DNA
<213>  Artificial

<220>
<223>  synthetic DNA

<400>  1537
actaaggcac gctgattcga gcatta                                          26


<210>  1538
<211>  26
<212>  DNA
<213>  Artificial

<220>
<223>  synthetic DNA

<400>  1538
cggattctgg cacgtacaag tagcag                                          26


<210>  1539
<211>  26
<212>  DNA
<213>  Artificial

<220>
<223>  synthetic DNA

<400>  1539
ttatggctcc agatctagtc accagc                                          26


<210>  1540
<211>  26
<212>  DNA
<213>  Artificial

<220>
<223>  synthetic DNA

<400>  1540
catacactcc aggcatgtat gatagg                                          26


<210>  1541
<211>  26
<212>  DNA
<213>  Artificial

<220>
<223>  synthetic DNA

<400>  1541
agttgtaagc caacgagtgt agcgta                                          26


<210>  1542
<211>  26
<212>  DNA
<213>  Artificial

<220>
<223>  synthetic DNA

<400>  1542
gtatcagctc cttcctctga ttccgg                                          26


<210>  1543
<211>  26
<212>  DNA
<213>  Artificial

<220>
<223>  synthetic DNA

<400>  1543
aacatacaga atgtctatgg tcagct                                          26


<210>  1544
<211>  26
<212>  DNA
<213>  Artificial

<220>
<223>  synthetic DNA

<400>  1544
gactcatatt catgttcagt atagag                                          26


<210>  1545
<211>  26
<212>  DNA
<213>  Artificial

<220>
<223>  synthetic DNA

<400>  1545
agagtgaacg aacgtgaccg acgctc                                          26


<210>  1546
<211>  26
<212>  DNA
<213>  Artificial

<220>
<223>  synthetic DNA

<400>  1546
aattggcgtc cttgccacaa catctt                                          26


<210>  1547
<211>  26
<212>  DNA
<213>  Artificial

<220>
<223>  synthetic DNA

<400>  1547
tcgtagacgc ctcgtacatc cgagat                                          26

<210>  1548
<211>  26
<212>  DNA
<213>  Artificial

<220>
<223>  synthetic DNA

<400>  1548
ccggctcgtg aggcgataat catata                                          26


<210>  1549
<211>  26
<212>  DNA
<213>  Artificial

<220>
<223>  synthetic DNA

<400>  1549
agtcctgatc acgaccacga ctcacg                                          26


<210>  1550
<211>  26
<212>  DNA
<213>  Artificial

<220>
<223>  synthetic DNA

<400>  1550
ggcactcaat cctccatgga gaagct                                          26


<210>  1551
<211>  26
<212>  DNA
<213>  Artificial

<220>
<223>  synthetic DNA

<400>  1551
tcatcattcc tcacgttcac cggtga                                          26


<210>  1552
<211>  26
<212>  DNA
<213>  Artificial

<220>
<223>  synthetic DNA

<400>  1552
tcaactctgt gctaaccggt cgtaca                                          26


<210>  1553
<211>  26

<212>  DNA
<213>  Artificial

<220>
<223>  synthetic DNA

<400>  1553
tgttcttatg cattaatgcc aggctt                                          26


<210>  1554
<211>  26
<212>  DNA
<213>  Artificial

<220>
<223>  synthetic DNA

<400>  1554
gattcacgac ctcaacagca tcactc                                          26


<210>  1555
<211>  26
<212>  DNA
<213>  Artificial

<220>
<223>  synthetic DNA

<400>  1555
ggcgagttcg accagaatgc tggaca                                          26


<210>  1556
<211>  26
<212>  DNA
<213>  Artificial

<220>
<223>  synthetic DNA

<400>  1556
ttccgtatac aatgcgatta agatct                                          26


<210>  1557
<211>  26
<212>  DNA
<213>  Artificial

<220>
<223>  synthetic DNA

<400>  1557
gagtaatccg taaccggcca acgttg                                          26


<210>  1558
<211>  26
<212>  DNA
<213>  Artificial

<220>
<223>  synthetic DNA

<400>  1558
cgcttccatc atggtacggt acgtat                                          26


<210>  1559
<211>  26
<212>  DNA
<213>  Artificial

<220>
<223>  synthetic DNA

<400>  1559
ccgtcgtggt gtgttgactg gtcaac                                          26


<210>  1560
<211>  26
<212>  DNA
<213>  Artificial

<220>
<223>  synthetic DNA

<400>  1560
tattcgcatc tccgtattag ttgtag                                          26


<210>  1561
<211>  26
<212>  DNA
<213>  Artificial

<220>
<223>  synthetic DNA

<400>  1561
tattattgta ttctaggcgg tgcaac                                          26


<210>  1562
<211>  26
<212>  DNA
<213>  Artificial

<220>
<223>  synthetic DNA

<400>  1562
aggctgccta cttcctcgtc atctcg                                          26


<210>  1563
<211>  26
<212>  DNA
<213>  Artificial

<220>
<223>  synthetic DNA

<400>  1563
gtaacatacg gctcatcgaa tgcatc                                          26


<210>  1564
<211>  26
<212>  DNA
<213>  Artificial

<220>
<223>  synthetic DNA

<400>  1564
ttatggcacg gatattaccg tacgcc                                          26


<210>  1565
<211>  26
<212>  DNA
<213>  Artificial

<220>
<223>  synthetic DNA

<400>  1565
atagcacttc ctctaatgct ctgctg                                          26


<210>  1566
<211>  26
<212>  DNA
<213>  Artificial

<220>
<223>  synthetic DNA

<400>  1566
tcacaggcaa tagcctaata ttatat                                          26


<210>  1567
<211>  26
<212>  DNA
<213>  Artificial

<220>
<223>  synthetic DNA

<400>  1567
ggcggatgtt cgttaatatt ataagg                                          26


<210>  1568
<211>  26
<212>  DNA
<213>  Artificial

<220>
<223>  synthetic DNA

<400>  1568
tgcaatagcc gttgtctctg ccagcg                                          26

<210>  1569
<211>  26
<212>  DNA
<213>  Artificial

<220>
<223>  synthetic DNA

<400>  1569
tacagcgcgt tggcgagtac tgatag                                          26


<210>  1570
<211>  26
<212>  DNA
<213>  Artificial

<220>
<223>  synthetic DNA

<400>  1570
tgcagttagt accttctcac gccaac                                          26


<210>  1571
<211>  26
<212>  DNA
<213>  Artificial

<220>
<223>  synthetic DNA

<400>  1571
ccattggcta cctagcagac tctacc                                          26


<210>  1572
<211>  26
<212>  DNA
<213>  Artificial

<220>
<223>  synthetic DNA

<400>  1572
aacagtagct cgcgtcttgc tctcgt                                          26


<210>  1573
<211>  26
<212>  DNA
<213>  Artificial

<220>
<223>  synthetic DNA

<400>  1573
gcagtccatc agctctcgct tataga                                          26


<210>  1574
<211>  26

<212>  DNA
<213>  Artificial

<220>
<223>  synthetic DNA

<400>  1574
tatctctctg tcgccagctt gaccaa                                          26


<210>  1575
<211>  26
<212>  DNA
<213>  Artificial

<220>
<223>  synthetic DNA

<400>  1575
cagactgttc aagcttgctg taggag                                          26


<210>  1576
<211>  26
<212>  DNA
<213>  Artificial

<220>
<223>  synthetic DNA

<400>  1576
taaccggaac tcgttcagca acattc                                          26


<210>  1577
<211>  26
<212>  DNA
<213>  Artificial

<220>
<223>  synthetic DNA

<400>  1577
tcaattatgc atgtcgtccg atctct                                          26


<210>  1578
<211>  26
<212>  DNA
<213>  Artificial

<220>
<223>  synthetic DNA

<400>  1578
ttgtctaagt caacctgtgg ataatc                                          26


<210>  1579
<211>  26
<212>  DNA
<213>  Artificial

<220>
<223>  synthetic DNA

<400>  1579
tctaagagtg gtatgaccag gagtcc                                          26


<210>  1580
<211>  26
<212>  DNA
<213>  Artificial

<220>
<223>  synthetic DNA

<400>  1580
tcgtagtact actggaacag gtaatc                                          26


<210>  1581
<211>  26
<212>  DNA
<213>  Artificial

<220>
<223>  synthetic DNA

<400>  1581
atgtcaacat tctaatcatc tctcgg                                          26


<210>  1582
<211>  26
<212>  DNA
<213>  Artificial

<220>
<223>  synthetic DNA

<400>  1582
agcgcgcaac tgttacggtg atccga                                          26


<210>  1583
<211>  26
<212>  DNA
<213>  Artificial

<220>
<223>  synthetic DNA

<400>  1583
gcgatagaat aatggtgtca cacacg                                          26


<210>  1584
<211>  26
<212>  DNA
<213>  Artificial

<220>
<223>  synthetic DNA

<400>  1584
aaggctgcga tgagaggcgt acatcg                                          26


<210>  1585
<211>  26
<212>  DNA
<213>  Artificial

<220>
<223>  synthetic DNA

<400>  1585
ggttcatggt ctcagtcgtg atcgcg                                          26


<210>  1586
<211>  26
<212>  DNA
<213>  Artificial

<220>
<223>  synthetic DNA

<400>  1586
tagtgactct atgtcacctc ggagcc                                          26


<210>  1587
<211>  26
<212>  DNA
<213>  Artificial

<220>
<223>  synthetic DNA

<400>  1587
atgtgatagc aatggcacct ctagtc                                          26


<210>  1588
<211>  26
<212>  DNA
<213>  Artificial

<220>
<223>  synthetic DNA

<400>  1588
tcgcgaagtg taatgcatca tccgct                                          26


<210>  1589
<211>  26
<212>  DNA
<213>  Artificial

<220>
<223>  synthetic DNA

<400>  1589
atgtggcgac gatccaagtt caacgc                                          26

<210>  1590
<211>  26
<212>  DNA
<213>  Artificial

<220>
<223>  synthetic DNA

<400>  1590
accttgtatg agtcggagtg tccggc                                          26


<210>  1591
<211>  26
<212>  DNA
<213>  Artificial

<220>
<223>  synthetic DNA

<400>  1591
acctcaagag agtagacagt tgagtt                                          26


<210>  1592
<211>  26
<212>  DNA
<213>  Artificial

<220>
<223>  synthetic DNA

<400>  1592
ggtgtaatcc tgtgtgcgaa gctggt                                          26


<210>  1593
<211>  26
<212>  DNA
<213>  Artificial

<220>
<223>  synthetic DNA

<400>  1593
atagcggaac tgtacgacgc tccagt                                          26


<210>  1594
<211>  26
<212>  DNA
<213>  Artificial

<220>
<223>  synthetic DNA

<400>  1594
aagcacgagt cgaccattag cctgga                                          26


<210>  1595
<211>  26

<212>  DNA
<213>  Artificial

<220>
<223>  synthetic DNA

<400>  1595
attccggtaa catcagaagg tacaat                                          26


<210>  1596
<211>  26
<212>  DNA
<213>  Artificial

<220>
<223>  synthetic DNA

<400>  1596
gtgcaacggc agtccagtat cctggt                                          26


<210>  1597
<211>  26
<212>  DNA
<213>  Artificial

<220>
<223>  synthetic DNA

<400>  1597
ccatcttata cacggtgacc gaagat                                          26


<210>  1598
<211>  26
<212>  DNA
<213>  Artificial

<220>
<223>  synthetic DNA

<400>  1598
gcacttaatc aagcttgagt gatgct                                          26


<210>  1599
<211>  26
<212>  DNA
<213>  Artificial

<220>
<223>  synthetic DNA

<400>  1599
agtattacgt gagtacgaag atagca                                          26


<210>  1600
<211>  26
<212>  DNA
<213>  Artificial

<220>
<223>  synthetic DNA

<400>  1600
ttcttaggtt aagttccttc tggacc                                          26


<210>  1601
<211>  26
<212>  DNA
<213>  Artificial

<220>
<223>  synthetic DNA

<400>  1601
gtccttgcta gacactgacc gttgct                                          26


<210>  1602
<211>  26
<212>  DNA
<213>  Artificial

<220>
<223>  synthetic DNA

<400>  1602
gccgctatgt gtgctgcatc ctaagc                                          26


<210>  1603
<211>  26
<212>  DNA
<213>  Artificial

<220>
<223>  synthetic DNA

<400>  1603
ccatcaataa cagacttatg ttgtga                                          26


<210>  1604
<211>  26
<212>  DNA
<213>  Artificial

<220>
<223>  synthetic DNA

<400>  1604
cgcgtgtgct tacaagtgct aacaag                                          26


<210>  1605
<211>  26
<212>  DNA
<213>  Artificial

<220>
<223>  synthetic DNA

<400>  1605
cgatatgtgt tcgcaataag agagcc                                            26


<210>  1606
<211>  26
<212>  DNA
<213>  Artificial

<220>
<223>  synthetic DNA

<400>  1606
cgcggatgtg agcggctcaa ttagca                                            26


<210>  1607
<211>  26
<212>  DNA
<213>  Artificial

<220>
<223>  synthetic DNA

<400>  1607
gctgcatgac tatcggatgg aggcat                                            26


<210>  1608
<211>  26
<212>  DNA
<213>  Artificial

<220>
<223>  synthetic DNA

<400>  1608
ctatgccgtg tatggtacga gtggcg                                            26


<210>  1609
<211>  26
<212>  DNA
<213>  Artificial

<220>
<223>  synthetic DNA

<400>  1609
ccggctggag ttcattacgt aggctg                                            26


<210>  1610
<211>  26
<212>  DNA
<213>  Artificial

<220>
<223>  synthetic DNA

<400>  1610
tgtaggccta ctgagctagt attaga                                            26

<210>  1611
<211>  26
<212>  DNA
<213>  Artificial

<220>
<223>  synthetic DNA

<400>  1611
ccgtcaagtg actattcttc taatct                                          26


<210>  1612
<211>  26
<212>  DNA
<213>  Artificial

<220>
<223>  synthetic DNA

<400>  1612
ggtcttacgc cagagactgc gcttct                                          26


<210>  1613
<211>  26
<212>  DNA
<213>  Artificial

<220>
<223>  synthetic DNA

<400>  1613
cgaagtgtga ttattaactg taatct                                          26


<210>  1614
<211>  26
<212>  DNA
<213>  Artificial

<220>
<223>  synthetic DNA

<400>  1614
gcacgcgtgg ccgtaagcat cgatta                                          26


<210>  1615
<211>  26
<212>  DNA
<213>  Artificial

<220>
<223>  synthetic DNA

<400>  1615
atcctgcgtc ggaacgtact atagct                                          26


<210>  1616
<211>  26

<212>  DNA
<213>  Artificial

<220>
<223>  synthetic DNA

<400>  1616
agtatcatca tatccattcg cagtac                                          26


<210>  1617
<211>  26
<212>  DNA
<213>  Artificial

<220>
<223>  synthetic DNA

<400>  1617
agtcctgacg ttcatatata gactcc                                          26


<210>  1618
<211>  26
<212>  DNA
<213>  Artificial

<220>
<223>  synthetic DNA

<400>  1618
cttgcagtaa tctgaatctg aaggtt                                          26


<210>  1619
<211>  26
<212>  DNA
<213>  Artificial

<220>
<223>  synthetic DNA

<400>  1619
ataacttggt tccagtaacg catagt                                          26


<210>  1620
<211>  26
<212>  DNA
<213>  Artificial

<220>
<223>  synthetic DNA

<400>  1620
gataaggata tggctgtagc gaagtg                                          26


<210>  1621
<211>  26
<212>  DNA
<213>  Artificial

<220>
<223>  synthetic DNA

<400>  1621
gtggagcgtt acagacatgc tgaaca                                          26


<210>  1622
<211>  26
<212>  DNA
<213>  Artificial

<220>
<223>  synthetic DNA

<400>  1622
cgcttccggc aggcgtcata taagtc                                          26


<210>  1623
<211>  26
<212>  DNA
<213>  Artificial

<220>
<223>  synthetic DNA

<400>  1623
ataacattct aacctctata agccga                                          26


<210>  1624
<211>  26
<212>  DNA
<213>  Artificial

<220>
<223>  synthetic DNA

<400>  1624
acgatctatg atccatatgg acttcc                                          26


<210>  1625
<211>  26
<212>  DNA
<213>  Artificial

<220>
<223>  synthetic DNA

<400>  1625
tgaagctcag atatcatgcc tcgagc                                          26


<210>  1626
<211>  26
<212>  DNA
<213>  Artificial

<220>
<223>  synthetic DNA

<400>  1626
agacttcacc gcaataactc gtagat                                          26


<210>  1627
<211>  26
<212>  DNA
<213>  Artificial

<220>
<223>  synthetic DNA

<400>  1627
agactaagac atacgccatc accgct                                          26


<210>  1628
<211>  26
<212>  DNA
<213>  Artificial

<220>
<223>  synthetic DNA

<400>  1628
tgtagcgtga tgtatcgtaa ttctgt                                          26


<210>  1629
<211>  26
<212>  DNA
<213>  Artificial

<220>
<223>  synthetic DNA

<400>  1629
tgtgctattg gcacctcacg ctgacc                                          26


<210>  1630
<211>  26
<212>  DNA
<213>  Artificial

<220>
<223>  synthetic DNA

<400>  1630
tgtagataag tatccagcga ctctct                                          26


<210>  1631
<211>  26
<212>  DNA
<213>  Artificial

<220>
<223>  synthetic DNA

<400>  1631
aattcgccaa ttgtgtgtag gcgcaa                                          26

<210>  1632
<211>  26
<212>  DNA
<213>  Artificial

<220>
<223>  synthetic DNA

<400>  1632
cgattatgag tacttgtaga ccagct                                          26


<210>  1633
<211>  28
<212>  DNA
<213>  Artificial

<220>
<223>  synthetic DNA

<400>  1633
ttgcaagaac aacgtatctc atatgaac                                        28


<210>  1634
<211>  28
<212>  DNA
<213>  Artificial

<220>
<223>  synthetic DNA

<400>  1634
caccgtgctg ttattacttg gtattcgg                                        28


<210>  1635
<211>  28
<212>  DNA
<213>  Artificial

<220>
<223>  synthetic DNA

<400>  1635
cacgtgtatt gttgcaccag aacgacaa                                        28


<210>  1636
<211>  28
<212>  DNA
<213>  Artificial

<220>
<223>  synthetic DNA

<400>  1636
atgcacgtaa ttacttccgg agaagacg                                        28


<210>  1637
<211>  28

<212>  DNA
<213>  Artificial

<220>
<223>  synthetic DNA

<400>  1637
tatgttgtct gatatggttc atgtggca                                          28


<210>  1638
<211>  28
<212>  DNA
<213>  Artificial

<220>
<223>  synthetic DNA

<400>  1638
agcgcgacta gttgatgcca acattgta                                          28


<210>  1639
<211>  28
<212>  DNA
<213>  Artificial

<220>
<223>  synthetic DNA

<400>  1639
ataggcaggt ccaggctcgg aacaagtc                                          28


<210>  1640
<211>  28
<212>  DNA
<213>  Artificial

<220>
<223>  synthetic DNA

<400>  1640
gcggtagtcg gtcaagaact agaaccgt                                          28


<210>  1641
<211>  28
<212>  DNA
<213>  Artificial

<220>
<223>  synthetic DNA

<400>  1641
actatacact ctagctatta ggaagcat                                          28


<210>  1642
<211>  28
<212>  DNA
<213>  Artificial

<220>
<223>  synthetic DNA

<400>  1642
gatcatcttg cttctcctgt ggagataa                                          28


<210>  1643
<211>  28
<212>  DNA
<213>  Artificial

<220>
<223>  synthetic DNA

<400>  1643
ctactacgag tccataactg atagcctc                                          28


<210>  1644
<211>  28
<212>  DNA
<213>  Artificial

<220>
<223>  synthetic DNA

<400>  1644
gcacagacac ctgtcctatc tagcagga                                          28


<210>  1645
<211>  28
<212>  DNA
<213>  Artificial

<220>
<223>  synthetic DNA

<400>  1645
aagcgaggcg cgaaggagat ggaaggat                                          28


<210>  1646
<211>  28
<212>  DNA
<213>  Artificial

<220>
<223>  synthetic DNA

<400>  1646
ctgaagacgc cagtctggat aggtgcct                                          28


<210>  1647
<211>  28
<212>  DNA
<213>  Artificial

<220>
<223>  synthetic DNA

<400>  1647
gtaagctctg tccttcgaga ttgataag                                          28


<210>  1648
<211>  28
<212>  DNA
<213>  Artificial

<220>
<223>  synthetic DNA

<400>  1648
ggttagagag attattgtgc gcatccat                                          28


<210>  1649
<211>  28
<212>  DNA
<213>  Artificial

<220>
<223>  synthetic DNA

<400>  1649
ccaggaggac ctatgatctt gccgccat                                          28


<210>  1650
<211>  28
<212>  DNA
<213>  Artificial

<220>
<223>  synthetic DNA

<400>  1650
actattcgag ctactgtatg tgtatccg                                          28


<210>  1651
<211>  28
<212>  DNA
<213>  Artificial

<220>
<223>  synthetic DNA

<400>  1651
gacatcgcga tacgtaactc cggagtgt                                          28


<210>  1652
<211>  28
<212>  DNA
<213>  Artificial

<220>
<223>  synthetic DNA

<400>  1652
ccgcaattcg tctatatatt ctagcata                                          28

<210>  1653
<211>  28
<212>  DNA
<213>  Artificial

<220>
<223>  synthetic DNA

<400>  1653
ctacacttga ggttgatgct caagatca                                          28


<210>  1654
<211>  28
<212>  DNA
<213>  Artificial

<220>
<223>  synthetic DNA

<400>  1654
cgatcagttc tagttcaccg cggacaat                                          28


<210>  1655
<211>  28
<212>  DNA
<213>  Artificial

<220>
<223>  synthetic DNA

<400>  1655
aagaatgatg attggccgcg aaccaagc                                          28


<210>  1656
<211>  28
<212>  DNA
<213>  Artificial

<220>
<223>  synthetic DNA

<400>  1656
cacgaccgga actagactcc taccaatt                                          28


<210>  1657
<211>  28
<212>  DNA
<213>  Artificial

<220>
<223>  synthetic DNA

<400>  1657
agttgcctgt gagtgaggct actatctc                                          28


<210>  1658
<211>  28

<212>  DNA
<213>  Artificial

<220>
<223>  synthetic DNA

<400>  1658
gattcttccg atgatcatgc cactacaa                                          28


<210>  1659
<211>  28
<212>  DNA
<213>  Artificial

<220>
<223>  synthetic DNA

<400>  1659
cgctgaagtg aactatgcaa gcaccgca                                          28


<210>  1660
<211>  28
<212>  DNA
<213>  Artificial

<220>
<223>  synthetic DNA

<400>  1660
attatcgtga tggtgagact gagctcgt                                          28


<210>  1661
<211>  28
<212>  DNA
<213>  Artificial

<220>
<223>  synthetic DNA

<400>  1661
cgaggccact ctgagccagg taagtatc                                          28


<210>  1662
<211>  28
<212>  DNA
<213>  Artificial

<220>
<223>  synthetic DNA

<400>  1662
tgccgaggac agccgatcac atcttcgt                                          28


<210>  1663
<211>  28
<212>  DNA
<213>  Artificial

<220>
<223>  synthetic DNA

<400>  1663
gttgacatga aggttatcgt cgatattc                                          28


<210>  1664
<211>  28
<212>  DNA
<213>  Artificial

<220>
<223>  synthetic DNA

<400>  1664
gtggtccagg tcaagctctg atcgaatg                                          28


<210>  1665
<211>  28
<212>  DNA
<213>  Artificial

<220>
<223>  synthetic DNA

<400>  1665
ccagtccggt gtactcagac ctaataac                                          28


<210>  1666
<211>  28
<212>  DNA
<213>  Artificial

<220>
<223>  synthetic DNA

<400>  1666
cgagacactg catgagcgta gtcttatt                                          28


<210>  1667
<211>  28
<212>  DNA
<213>  Artificial

<220>
<223>  synthetic DNA

<400>  1667
gacggcttgt atacttctct acggtctg                                          28


<210>  1668
<211>  28
<212>  DNA
<213>  Artificial

<220>
<223>  synthetic DNA

<400>  1668
ttagctggat ggaagccata ttccgtag                                          28


<210>  1669
<211>  28
<212>  DNA
<213>  Artificial

<220>
<223>  synthetic DNA

<400>  1669
cagcctacac ttgattactc aacaactc                                          28


<210>  1670
<211>  28
<212>  DNA
<213>  Artificial

<220>
<223>  synthetic DNA

<400>  1670
gtacgtagtg tcacgcgcct acgttcgt                                          28


<210>  1671
<211>  28
<212>  DNA
<213>  Artificial

<220>
<223>  synthetic DNA

<400>  1671
ctacaacttc tcaatcatgc ctctgttg                                          28


<210>  1672
<211>  28
<212>  DNA
<213>  Artificial

<220>
<223>  synthetic DNA

<400>  1672
cgaggacaga attcgacata aggagaga                                          28


<210>  1673
<211>  28
<212>  DNA
<213>  Artificial

<220>
<223>  synthetic DNA

<400>  1673
gccgaacgac acagtgagtt gataggta                                          28

<210>  1674
<211>  28
<212>  DNA
<213>  Artificial

<220>
<223>  synthetic DNA

<400>  1674
gaacactata tgctgtcgct gtctgagg                                          28


<210>  1675
<211>  28
<212>  DNA
<213>  Artificial

<220>
<223>  synthetic DNA

<400>  1675
gttaagttct tcggcggtca tgctcatt                                          28


<210>  1676
<211>  28
<212>  DNA
<213>  Artificial

<220>
<223>  synthetic DNA

<400>  1676
ttgcttacag atcgcgtatc catagtat                                          28


<210>  1677
<211>  28
<212>  DNA
<213>  Artificial

<220>
<223>  synthetic DNA

<400>  1677
gaggaccacc tctgcgaagt tcactgtg                                          28


<210>  1678
<211>  28
<212>  DNA
<213>  Artificial

<220>
<223>  synthetic DNA

<400>  1678
aatcctagca tatcgagaac gacactga                                          28


<210>  1679
<211>  28

<212>  DNA
<213>  Artificial

<220>
<223>  synthetic DNA

<400>  1679
tgaatactat agccatagtc gacttccg                                          28


<210>  1680
<211>  28
<212>  DNA
<213>  Artificial

<220>
<223>  synthetic DNA

<400>  1680
gacatccacg aagctggtaa tcggaacc                                          28


<210>  1681
<211>  28
<212>  DNA
<213>  Artificial

<220>
<223>  synthetic DNA

<400>  1681
ttagccgtct tagaagtgtc tgaccggc                                          28


<210>  1682
<211>  28
<212>  DNA
<213>  Artificial

<220>
<223>  synthetic DNA

<400>  1682
ctattctgcc gtaattgatt ccttcgtt                                          28


<210>  1683
<211>  28
<212>  DNA
<213>  Artificial

<220>
<223>  synthetic DNA

<400>  1683
acgcctctgg tcgaaggtag attagctc                                          28


<210>  1684
<211>  28
<212>  DNA
<213>  Artificial

<220>
<223>  synthetic DNA

<400>  1684
cagcctattg atcgtaagta gatggtcc                                          28


<210>  1685
<211>  28
<212>  DNA
<213>  Artificial

<220>
<223>  synthetic DNA

<400>  1685
ttaagtgagg tggacaacca tcaacttc                                          28


<210>  1686
<211>  28
<212>  DNA
<213>  Artificial

<220>
<223>  synthetic DNA

<400>  1686
aaggccttgc ggctaagtag tattcatc                                          28


<210>  1687
<211>  28
<212>  DNA
<213>  Artificial

<220>
<223>  synthetic DNA

<400>  1687
ttgtgatact aattcttctc aagagtca                                          28


<210>  1688
<211>  28
<212>  DNA
<213>  Artificial

<220>
<223>  synthetic DNA

<400>  1688
gcattaggtg acgaccttag tccatcac                                          28


<210>  1689
<211>  28
<212>  DNA
<213>  Artificial

<220>
<223>  synthetic DNA

<400>  1689
gcggatggac gtatacagtg agtcgtgc                                          28


<210>  1690
<211>  28
<212>  DNA
<213>  Artificial

<220>
<223>  synthetic DNA

<400>  1690
gaacatgcca gcctcaacta ggctaaga                                          28


<210>  1691
<211>  28
<212>  DNA
<213>  Artificial

<220>
<223>  synthetic DNA

<400>  1691
tccgtcatta gagtatgagt gactacta                                          28


<210>  1692
<211>  28
<212>  DNA
<213>  Artificial

<220>
<223>  synthetic DNA

<400>  1692
aacacttagt aaccagttcg gactggac                                          28


<210>  1693
<211>  28
<212>  DNA
<213>  Artificial

<220>
<223>  synthetic DNA

<400>  1693
cgctaactat tgcgtatatt cgcggctt                                          28


<210>  1694
<211>  28
<212>  DNA
<213>  Artificial

<220>
<223>  synthetic DNA

<400>  1694
gccatctacg atcttcggct tatcctag                                          28

<210>  1695
<211>  28
<212>  DNA
<213>  Artificial

<220>
<223>  synthetic DNA

<400>  1695
cctgagaatg ttgactaaga tcttgtga                                          28


<210>  1696
<211>  28
<212>  DNA
<213>  Artificial

<220>
<223>  synthetic DNA

<400>  1696
tcggttagtc taatcatcac gcaacgga                                          28


<210>  1697
<211>  28
<212>  DNA
<213>  Artificial

<220>
<223>  synthetic DNA

<400>  1697
attatctatt gaagcagtga cagcgatc                                          28


<210>  1698
<211>  28
<212>  DNA
<213>  Artificial

<220>
<223>  synthetic DNA

<400>  1698
gaggagaatc acggaacacg gtcacatg                                          28


<210>  1699
<211>  28
<212>  DNA
<213>  Artificial

<220>
<223>  synthetic DNA

<400>  1699
gctgcaagca ttatgaccat ggcatctg                                          28


<210>  1700
<211>  28

<212>  DNA
<213>  Artificial

<220>
<223>  synthetic DNA

<400>  1700
gaacaaccta taacgacgtt gtggacaa                                          28


<210>  1701
<211>  28
<212>  DNA
<213>  Artificial

<220>
<223>  synthetic DNA

<400>  1701
ttaatcatcg atagacgaca tggaatca                                          28


<210>  1702
<211>  28
<212>  DNA
<213>  Artificial

<220>
<223>  synthetic DNA

<400>  1702
tcgagtgtaa gcacactacg atctggaa                                          28


<210>  1703
<211>  28
<212>  DNA
<213>  Artificial

<220>
<223>  synthetic DNA

<400>  1703
gctacgcaca gtctctgcac agctacac                                          28


<210>  1704
<211>  28
<212>  DNA
<213>  Artificial

<220>
<223>  synthetic DNA

<400>  1704
cctgtatgta cgttctggct aatacctt                                          28


<210>  1705
<211>  28
<212>  DNA
<213>  Artificial

<220>
<223>  synthetic DNA

<400>  1705
tgaagcaccg gtacatggtg tatccgga                                          28


<210>  1706
<211>  28
<212>  DNA
<213>  Artificial

<220>
<223>  synthetic DNA

<400>  1706
tgctggaacc taactcggtg atgacgat                                          28


<210>  1707
<211>  28
<212>  DNA
<213>  Artificial

<220>
<223>  synthetic DNA

<400>  1707
cgctatctta ctgccaagtt ctcatata                                          28


<210>  1708
<211>  28
<212>  DNA
<213>  Artificial

<220>
<223>  synthetic DNA

<400>  1708
aacgcgcgcg tatcggcaat aatctcaa                                          28


<210>  1709
<211>  28
<212>  DNA
<213>  Artificial

<220>
<223>  synthetic DNA

<400>  1709
ccattaggat gaccatcgac tattagag                                          28


<210>  1710
<211>  28
<212>  DNA
<213>  Artificial

<220>
<223>  synthetic DNA

<400>  1710
tactgctaga ctgcgtgcat tcatggcg                                          28


<210>  1711
<211>  28
<212>  DNA
<213>  Artificial

<220>
<223>  synthetic DNA

<400>  1711
cattgcgcgc tccacgaact ctattgtc                                          28


<210>  1712
<211>  28
<212>  DNA
<213>  Artificial

<220>
<223>  synthetic DNA

<400>  1712
gacgcgccta gaactgtata gctctacg                                          28


<210>  1713
<211>  28
<212>  DNA
<213>  Artificial

<220>
<223>  synthetic DNA

<400>  1713
cattgcaact tgtcggtgat ggcaatcc                                          28


<210>  1714
<211>  28
<212>  DNA
<213>  Artificial

<220>
<223>  synthetic DNA

<400>  1714
ttaatgcaca tgcagtacgg caccacag                                          28


<210>  1715
<211>  28
<212>  DNA
<213>  Artificial

<220>
<223>  synthetic DNA

<400>  1715
agcggtacgt ggacgagtgg taattaat                                          28

<210>  1716
<211>  28
<212>  DNA
<213>  Artificial

<220>
<223>  synthetic DNA

<400>  1716
gacgtattgc tatgcattgg aagatgct                                          28


<210>  1717
<211>  28
<212>  DNA
<213>  Artificial

<220>
<223>  synthetic DNA

<400>  1717
aacacttcga ccattgcgcc tcaatggt                                          28


<210>  1718
<211>  28
<212>  DNA
<213>  Artificial

<220>
<223>  synthetic DNA

<400>  1718
cggtacgctc tagcggtcat aagatgca                                          28


<210>  1719
<211>  28
<212>  DNA
<213>  Artificial

<220>
<223>  synthetic DNA

<400>  1719
cctgaataac agccgcgcct aattagat                                          28


<210>  1720
<211>  28
<212>  DNA
<213>  Artificial

<220>
<223>  synthetic DNA

<400>  1720
aagcgtctaa tgtgccttaa gtcacatg                                          28


<210>  1721
<211>  28

<212>  DNA
<213>  Artificial

<220>
<223>  synthetic DNA

<400>  1721
gctctccaag aaccagaagt aagcatcg                                          28


<210>  1722
<211>  28
<212>  DNA
<213>  Artificial

<220>
<223>  synthetic DNA

<400>  1722
gaggagagtt gtccgagtgg tgtgatgt                                          28


<210>  1723
<211>  28
<212>  DNA
<213>  Artificial

<220>
<223>  synthetic DNA

<400>  1723
taacgagtgg tgcgtctaag caattgag                                          28


<210>  1724
<211>  28
<212>  DNA
<213>  Artificial

<220>
<223>  synthetic DNA

<400>  1724
ccaacagtat gctgacataa ctatgata                                          28


<210>  1725
<211>  28
<212>  DNA
<213>  Artificial

<220>
<223>  synthetic DNA

<400>  1725
gatccttgcc acgcctatga gatatcgc                                          28


<210>  1726
<211>  28
<212>  DNA
<213>  Artificial

<220>
<223>  synthetic DNA

<400>  1726
aacgcgctac cgtccttgtg catagagg                                          28


<210>  1727
<211>  28
<212>  DNA
<213>  Artificial

<220>
<223>  synthetic DNA

<400>  1727
ctacatgtgc cttatagtac agaggaac                                          28


<210>  1728
<211>  28
<212>  DNA
<213>  Artificial

<220>
<223>  synthetic DNA

<400>  1728
cagcctcgta gttagcgtga ttcatgcg                                          28


<210>  1729
<211>  29
<212>  DNA
<213>  Artificial

<220>
<223>  synthetic DNA

<400>  1729
ctcctcgccg attgaagtgc gtagaacta                                         29


<210>  1730
<211>  29
<212>  DNA
<213>  Artificial

<220>
<223>  synthetic DNA

<400>  1730
cagcaggcct caataggata agccaacta                                         29


<210>  1731
<211>  29
<212>  DNA
<213>  Artificial

<220>
<223>  synthetic DNA

<400>  1731
gaccatcaat ctcgaagact acgctctgt                                          29


<210>  1732
<211>  29
<212>  DNA
<213>  Artificial

<220>
<223>  synthetic DNA

<400>  1732
ggttgctccg tctgttcagc acactgtta                                          29


<210>  1733
<211>  29
<212>  DNA
<213>  Artificial

<220>
<223>  synthetic DNA

<400>  1733
aatgtcgact ggccattatc gccaagtgt                                          29


<210>  1734
<211>  29
<212>  DNA
<213>  Artificial

<220>
<223>  synthetic DNA

<400>  1734
gatagcttgc catgcgaatg gatctccag                                          29


<210>  1735
<211>  29
<212>  DNA
<213>  Artificial

<220>
<223>  synthetic DNA

<400>  1735
ccagaccgga gccaattggc tgccaatat                                          29


<210>  1736
<211>  29
<212>  DNA
<213>  Artificial

<220>
<223>  synthetic DNA

<400>  1736
aacgtcgctc catacgttac ctaatgcag                                          29

<210>  1737
<211>  29
<212>  DNA
<213>  Artificial

<220>
<223>  synthetic DNA

<400>  1737
gaatatgacg cgaacagtct attcggatc                                          29


<210>  1738
<211>  29
<212>  DNA
<213>  Artificial

<220>
<223>  synthetic DNA

<400>  1738
gacgagaatg tattaaggat aagcaaggt                                          29


<210>  1739
<211>  29
<212>  DNA
<213>  Artificial

<220>
<223>  synthetic DNA

<400>  1739
aagtcgtatg aatcgctatc acatgagtc                                          29


<210>  1740
<211>  29
<212>  DNA
<213>  Artificial

<220>
<223>  synthetic DNA

<400>  1740
gtcgtggaga ctacaattct cctcacgtt                                          29


<210>  1741
<211>  29
<212>  DNA
<213>  Artificial

<220>
<223>  synthetic DNA

<400>  1741
gttgccaccg ttacacgact atcgacagt                                          29


<210>  1742
<211>  29

<212>  DNA
<213>  Artificial

<220>
<223>  synthetic DNA

<400>  1742
aggataggct acgccttact ctcctaagc                                          29


<210>  1743
<211>  29
<212>  DNA
<213>  Artificial

<220>
<223>  synthetic DNA

<400>  1743
taatcatcct gttcgcctcg aggttgtta                                          29


<210>  1744
<211>  29
<212>  DNA
<213>  Artificial

<220>
<223>  synthetic DNA

<400>  1744
gacaagcagt aataattact gagtggacg                                          29


<210>  1745
<211>  29
<212>  DNA
<213>  Artificial

<220>
<223>  synthetic DNA

<400>  1745
tacagcgtta cgcaggtata tcaaggtag                                          29


<210>  1746
<211>  29
<212>  DNA
<213>  Artificial

<220>
<223>  synthetic DNA

<400>  1746
ctaacatcac ttactattag cggtctcgt                                          29


<210>  1747
<211>  29
<212>  DNA
<213>  Artificial

<220>
<223>  synthetic DNA

<400>  1747
ccgcgcttct tgacacgttc tccactagg                                         29


<210>  1748
<211>  29
<212>  DNA
<213>  Artificial

<220>
<223>  synthetic DNA

<400>  1748
caagtaacat gagatgctat cggtacatt                                         29


<210>  1749
<211>  29
<212>  DNA
<213>  Artificial

<220>
<223>  synthetic DNA

<400>  1749
cgaccactag gctgtgacca cgatacgct                                         29


<210>  1750
<211>  29
<212>  DNA
<213>  Artificial

<220>
<223>  synthetic DNA

<400>  1750
caggtcatgt gacgcagtcg gcagtcaac                                         29


<210>  1751
<211>  29
<212>  DNA
<213>  Artificial

<220>
<223>  synthetic DNA

<400>  1751
actccatcgt tagttcttcc gccgtgctg                                         29


<210>  1752
<211>  29
<212>  DNA
<213>  Artificial

<220>
<223>  synthetic DNA

<400>  1752
ctcaccacgt atgcgtcact cggttacgt                                          29


<210>  1753
<211>  29
<212>  DNA
<213>  Artificial

<220>
<223>  synthetic DNA

<400>  1753
tgcctatgct atggaccttg cgcgactct                                          29


<210>  1754
<211>  29
<212>  DNA
<213>  Artificial

<220>
<223>  synthetic DNA

<400>  1754
aatgaaggtc aacgctctgt agttacgcg                                          29


<210>  1755
<211>  29
<212>  DNA
<213>  Artificial

<220>
<223>  synthetic DNA

<400>  1755
caccattgat tcatggcttc catcactgc                                          29


<210>  1756
<211>  29
<212>  DNA
<213>  Artificial

<220>
<223>  synthetic DNA

<400>  1756
gacacgcaag gtaattcgag attgcagca                                          29


<210>  1757
<211>  29
<212>  DNA
<213>  Artificial

<220>
<223>  synthetic DNA

<400>  1757
caccgagagg aaggttcgat cgcttctcg                                          29

<210>  1758
<211>  29
<212>  DNA
<213>  Artificial

<220>
<223>  synthetic DNA

<400>  1758
cagttatcgg attgtgatat tcactcctg                                          29


<210>  1759
<211>  29
<212>  DNA
<213>  Artificial

<220>
<223>  synthetic DNA

<400>  1759
atactgtaac gcctcaacct atgctgact                                          29


<210>  1760
<211>  29
<212>  DNA
<213>  Artificial

<220>
<223>  synthetic DNA

<400>  1760
atctgtctta ttctggcaca ctcagactt                                          29


<210>  1761
<211>  29
<212>  DNA
<213>  Artificial

<220>
<223>  synthetic DNA

<400>  1761
tccaaccggt gacgtgctct tgatccaac                                          29


<210>  1762
<211>  29
<212>  DNA
<213>  Artificial

<220>
<223>  synthetic DNA

<400>  1762
cacactcagt tcggctatct ctgcgatag                                          29


<210>  1763
<211>  29

<212>  DNA
<213>  Artificial

<220>
<223>  synthetic DNA

<400>  1763
agctgtaagt caggtctacg actcgtact                                        29


<210>  1764
<211>  29
<212>  DNA
<213>  Artificial

<220>
<223>  synthetic DNA

<400>  1764
gtcggcggca cgcacagcta acattcgta                                        29


<210>  1765
<211>  29
<212>  DNA
<213>  Artificial

<220>
<223>  synthetic DNA

<400>  1765
atatggtagc cagccacgta tactgaaca                                        29


<210>  1766
<211>  29
<212>  DNA
<213>  Artificial

<220>
<223>  synthetic DNA

<400>  1766
tggacaatcc gactctaaca cagaggtag                                        29


<210>  1767
<211>  29
<212>  DNA
<213>  Artificial

<220>
<223>  synthetic DNA

<400>  1767
tccgccgctg acagttcaat ctatcaatt                                        29


<210>  1768
<211>  29
<212>  DNA
<213>  Artificial

<220>
<223>  synthetic DNA

<400>  1768
ggttccttag aatatgcacc tatcagcga                                          29


<210>  1769
<211>  29
<212>  DNA
<213>  Artificial

<220>
<223>  synthetic DNA

<400>  1769
cggctgtacg acatggatca taagagtgt                                          29


<210>  1770
<211>  29
<212>  DNA
<213>  Artificial

<220>
<223>  synthetic DNA

<400>  1770
tgcagatgta cgctgtggcc agtggagag                                          29


<210>  1771
<211>  29
<212>  DNA
<213>  Artificial

<220>
<223>  synthetic DNA

<400>  1771
cctactcact taacaataat cggttcggt                                          29


<210>  1772
<211>  29
<212>  DNA
<213>  Artificial

<220>
<223>  synthetic DNA

<400>  1772
cgcttcctac tgcctgtgcc gcgacataa                                          29


<210>  1773
<211>  29
<212>  DNA
<213>  Artificial

<220>
<223>  synthetic DNA

<400>  1773
ctagaccgac cggttatgcg ctattgttc                                          29


<210>  1774
<211>  29
<212>  DNA
<213>  Artificial

<220>
<223>  synthetic DNA

<400>  1774
ttgtgagcac gtctgcggca agcctatgg                                          29


<210>  1775
<211>  29
<212>  DNA
<213>  Artificial

<220>
<223>  synthetic DNA

<400>  1775
tcatcggccg gcgctgttgt tgttaccat                                          29


<210>  1776
<211>  29
<212>  DNA
<213>  Artificial

<220>
<223>  synthetic DNA

<400>  1776
gcggttaggt gcagttagga agactatca                                          29


<210>  1777
<211>  29
<212>  DNA
<213>  Artificial

<220>
<223>  synthetic DNA

<400>  1777
tatgcggtcg tgaggcgtag cattctaga                                          29


<210>  1778
<211>  29
<212>  DNA
<213>  Artificial

<220>
<223>  synthetic DNA

<400>  1778
ccatctattc gtcgaactct cagctcgta                                          29

<210>  1779
<211>  29
<212>  DNA
<213>  Artificial

<220>
<223>  synthetic DNA

<400>  1779
atcagatcta ctgatcgcgg tagagtatc                                          29


<210>  1780
<211>  29
<212>  DNA
<213>  Artificial

<220>
<223>  synthetic DNA

<400>  1780
tacacatagg cggcgcagcc ttctaatta                                          29


<210>  1781
<211>  29
<212>  DNA
<213>  Artificial

<220>
<223>  synthetic DNA

<400>  1781
ttaaccgtag ttcttagctt acgccgctc                                          29


<210>  1782
<211>  29
<212>  DNA
<213>  Artificial

<220>
<223>  synthetic DNA

<400>  1782
actatagagg acatggcact cctcttcta                                          29


<210>  1783
<211>  29
<212>  DNA
<213>  Artificial

<220>
<223>  synthetic DNA

<400>  1783
cagttcgtat taagattgaa tgtagcggt                                          29


<210>  1784
<211>  29

<212>  DNA
<213>  Artificial

<220>
<223>  synthetic DNA

<400>  1784
agttatcggt atccgcttat ccgtacgta                                          29


<210>  1785
<211>  29
<212>  DNA
<213>  Artificial

<220>
<223>  synthetic DNA

<400>  1785
agcttattca tacactgcac cacagcaag                                          29


<210>  1786
<211>  29
<212>  DNA
<213>  Artificial

<220>
<223>  synthetic DNA

<400>  1786
ccgtcggcta gtctatcctc taattagaa                                          29


<210>  1787
<211>  29
<212>  DNA
<213>  Artificial

<220>
<223>  synthetic DNA

<400>  1787
gtccgcttcc atgcctgctg tacgaacac                                          29


<210>  1788
<211>  29
<212>  DNA
<213>  Artificial

<220>
<223>  synthetic DNA

<400>  1788
tctcttcctc cttcattgtt cgctagctc                                          29


<210>  1789
<211>  29
<212>  DNA
<213>  Artificial

<220>
<223>  synthetic DNA

<400>  1789
tctcttgagc ggtcctcata caggtctgc                                          29


<210>  1790
<211>  29
<212>  DNA
<213>  Artificial

<220>
<223>  synthetic DNA

<400>  1790
gaccaagtgt aggtgatatc accggtact                                          29


<210>  1791
<211>  29
<212>  DNA
<213>  Artificial

<220>
<223>  synthetic DNA

<400>  1791
aagattgtga taggttggta gttaccaca                                          29


<210>  1792
<211>  29
<212>  DNA
<213>  Artificial

<220>
<223>  synthetic DNA

<400>  1792
tcgcctccga agagtatagc atcggcaga                                          29


<210>  1793
<211>  29
<212>  DNA
<213>  Artificial

<220>
<223>  synthetic DNA

<400>  1793
gaggtagtta tgagcatcga ggtcctgtt                                          29


<210>  1794
<211>  29
<212>  DNA
<213>  Artificial

<220>
<223>  synthetic DNA

<400>  1794
ggacgcaaga tcgcaggtac ttgtaagct                                          29


<210>  1795
<211>  29
<212>  DNA
<213>  Artificial

<220>
<223>  synthetic DNA

<400>  1795
actcgtacac gtcatcgtgc aggtctcag                                          29


<210>  1796
<211>  29
<212>  DNA
<213>  Artificial

<220>
<223>  synthetic DNA

<400>  1796
taatccgtca ggagtgagat ggctcgaca                                          29


<210>  1797
<211>  29
<212>  DNA
<213>  Artificial

<220>
<223>  synthetic DNA

<400>  1797
aagatggttc cgcgcattga ctagcaagt                                          29


<210>  1798
<211>  29
<212>  DNA
<213>  Artificial

<220>
<223>  synthetic DNA

<400>  1798
tccgcgatct gcggatcttg aatgctcac                                          29


<210>  1799
<211>  29
<212>  DNA
<213>  Artificial

<220>
<223>  synthetic DNA

<400>  1799
ttcacgagag tcaactgcta gtatcctag                                          29

<210>  1800
<211>  29
<212>  DNA
<213>  Artificial

<220>
<223>  synthetic DNA

<400>  1800
ttccaactgg attcttccaa ctcctcgaa                                          29


<210>  1801
<211>  29
<212>  DNA
<213>  Artificial

<220>
<223>  synthetic DNA

<400>  1801
cactactact caagttatac ggtgttgac                                          29


<210>  1802
<211>  29
<212>  DNA
<213>  Artificial

<220>
<223>  synthetic DNA

<400>  1802
caactggatt ctcaggatgc gtctctagc                                          29


<210>  1803
<211>  29
<212>  DNA
<213>  Artificial

<220>
<223>  synthetic DNA

<400>  1803
tggactagag tggagcgatt acgtaatat                                          29


<210>  1804
<211>  29
<212>  DNA
<213>  Artificial

<220>
<223>  synthetic DNA

<400>  1804
gaggtcattc aactggactc gccacggac                                          29


<210>  1805
<211>  29

<212>  DNA
<213>  Artificial

<220>
<223>  synthetic DNA

<400>  1805
caggtgtgta acgctgcaat cacatgaat                                        29


<210>  1806
<211>  29
<212>  DNA
<213>  Artificial

<220>
<223>  synthetic DNA

<400>  1806
tatgctgagg tattagttct aactatgcg                                        29


<210>  1807
<211>  29
<212>  DNA
<213>  Artificial

<220>
<223>  synthetic DNA

<400>  1807
cgtctgagtc ggataaggaa ggttaccgc                                        29


<210>  1808
<211>  29
<212>  DNA
<213>  Artificial

<220>
<223>  synthetic DNA

<400>  1808
gtactatcgt cgcaggcact atctctgcc                                        29


<210>  1809
<211>  29
<212>  DNA
<213>  Artificial

<220>
<223>  synthetic DNA

<400>  1809
gcttcctcct tgcaacttca ttgcttcga                                        29


<210>  1810
<211>  29
<212>  DNA
<213>  Artificial

<220>
<223>  synthetic DNA

<400>  1810
tgtctacgaa gtagaagaca cgaataatg                                          29


<210>  1811
<211>  29
<212>  DNA
<213>  Artificial

<220>
<223>  synthetic DNA

<400>  1811
ccgtcatcta aggcagagta catccgcga                                          29


<210>  1812
<211>  29
<212>  DNA
<213>  Artificial

<220>
<223>  synthetic DNA

<400>  1812
ccggaggcgt actaactgac cacaacacc                                          29


<210>  1813
<211>  29
<212>  DNA
<213>  Artificial

<220>
<223>  synthetic DNA

<400>  1813
aactcgtcgc tgcctgaata ggtcagagt                                          29


<210>  1814
<211>  29
<212>  DNA
<213>  Artificial

<220>
<223>  synthetic DNA

<400>  1814
ttataagatt aatgtcggtc agtgtcgga                                          29


<210>  1815
<211>  29
<212>  DNA
<213>  Artificial

<220>
<223>  synthetic DNA

<400>  1815
cgtctcgatg gatccacacg aacctgttg                                          29


<210>  1816
<211>  29
<212>  DNA
<213>  Artificial

<220>
<223>  synthetic DNA

<400>  1816
atgccatcat ggtcgtccta tcttaaggc                                          29


<210>  1817
<211>  29
<212>  DNA
<213>  Artificial

<220>
<223>  synthetic DNA

<400>  1817
gcgcttcagc gattcgtcat gcaaggcac                                          29


<210>  1818
<211>  29
<212>  DNA
<213>  Artificial

<220>
<223>  synthetic DNA

<400>  1818
ccaagcgata ccgaggtacg gttaacgag                                          29


<210>  1819
<211>  29
<212>  DNA
<213>  Artificial

<220>
<223>  synthetic DNA

<400>  1819
atatgacaga caggtggacc taagcaagc                                          29


<210>  1820
<211>  29
<212>  DNA
<213>  Artificial

<220>
<223>  synthetic DNA

<400>  1820
cactacatcg tcaggcctgg aagcctcag                                          29

<210>  1821
<211>  29
<212>  DNA
<213>  Artificial

<220>
<223>  synthetic DNA

<400>  1821
gccgtgtaga cgaggacatt atgtcgtat                                          29


<210>  1822
<211>  29
<212>  DNA
<213>  Artificial

<220>
<223>  synthetic DNA

<400>  1822
caacgtatat acacaccttg tgaagagaa                                          29


<210>  1823
<211>  29
<212>  DNA
<213>  Artificial

<220>
<223>  synthetic DNA

<400>  1823
tccaacgtaa ttccgccgtc tgtcgagac                                          29


<210>  1824
<211>  29
<212>  DNA
<213>  Artificial

<220>
<223>  synthetic DNA

<400>  1824
aattcgtgct tcgatcaccg tagactcag                                          29


<210>  1825
<211>  30
<212>  DNA
<213>  Artificial

<220>
<223>  synthetic DNA

<400>  1825
actatattgt attcacgtcc gacgactcgc                                         30


<210>  1826
<211>  30

<212>  DNA
<213>  Artificial

<220>
<223>  synthetic DNA

<400>  1826
gacgagcttg tggtacacta tacctatgag                                        30


<210>  1827
<211>  30
<212>  DNA
<213>  Artificial

<220>
<223>  synthetic DNA

<400>  1827
tgattcaagc accaggcatg cttaagctag                                        30


<210>  1828
<211>  30
<212>  DNA
<213>  Artificial

<220>
<223>  synthetic DNA

<400>  1828
cggtctccta taggaaggct cattctgacg                                        30


<210>  1829
<211>  30
<212>  DNA
<213>  Artificial

<220>
<223>  synthetic DNA

<400>  1829
agtcagtgtc gaatcaatca aggcgtcctt                                        30


<210>  1830
<211>  30
<212>  DNA
<213>  Artificial

<220>
<223>  synthetic DNA

<400>  1830
cgaacgtaat ggccatcacg cgctggccta                                        30


<210>  1831
<211>  30
<212>  DNA
<213>  Artificial

<220>
<223>  synthetic DNA

<400>  1831
cgaacctgga ccacctggca ttaccattac                                        30


<210>  1832
<211>  30
<212>  DNA
<213>  Artificial

<220>
<223>  synthetic DNA

<400>  1832
acattaggtt cctgtaatgt cttatcaacg                                        30


<210>  1833
<211>  30
<212>  DNA
<213>  Artificial

<220>
<223>  synthetic DNA

<400>  1833
cgtctaatgc accgtatcgt cttcgcgcat                                        30


<210>  1834
<211>  30
<212>  DNA
<213>  Artificial

<220>
<223>  synthetic DNA

<400>  1834
tctatgactt acaacggaat cttacttcgt                                        30


<210>  1835
<211>  30
<212>  DNA
<213>  Artificial

<220>
<223>  synthetic DNA

<400>  1835
gtaaccgatc ggtaccgtct gctattgttc                                        30


<210>  1836
<211>  30
<212>  DNA
<213>  Artificial

<220>
<223>  synthetic DNA

<400>  1836
ggtgattgat aagcaacaca tattaggagg                                        30


<210>  1837
<211>  30
<212>  DNA
<213>  Artificial

<220>
<223>  synthetic DNA

<400>  1837
aattatcgac gctaataggc gagctgttca                                        30


<210>  1838
<211>  30
<212>  DNA
<213>  Artificial

<220>
<223>  synthetic DNA

<400>  1838
ggaggtacat gacgagtgga cagacagacc                                        30


<210>  1839
<211>  30
<212>  DNA
<213>  Artificial

<220>
<223>  synthetic DNA

<400>  1839
ctctaatccg ttatgcggtg atgtaatccg                                        30


<210>  1840
<211>  30
<212>  DNA
<213>  Artificial

<220>
<223>  synthetic DNA

<400>  1840
gcaagcacgc ggcttggcga acttctatgc                                        30


<210>  1841
<211>  30
<212>  DNA
<213>  Artificial

<220>
<223>  synthetic DNA

<400>  1841
tagatgtagg cctggtaggc agaggagtaa                                        30

<210>  1842
<211>  30
<212>  DNA
<213>  Artificial

<220>
<223>  synthetic DNA

<400>  1842
ccgagtggcg accacacagg tacgcattaa                                        30


<210>  1843
<211>  30
<212>  DNA
<213>  Artificial

<220>
<223>  synthetic DNA

<400>  1843
gtcctggctc agattagtgc acttagttat                                        30


<210>  1844
<211>  30
<212>  DNA
<213>  Artificial

<220>
<223>  synthetic DNA

<400>  1844
gcggtaccta catgttatga ctcagacgac                                        30


<210>  1845
<211>  30
<212>  DNA
<213>  Artificial

<220>
<223>  synthetic DNA

<400>  1845
tctctgccaa tgctggtctc atcgaatcca                                        30


<210>  1846
<211>  30
<212>  DNA
<213>  Artificial

<220>
<223>  synthetic DNA

<400>  1846
tctctacaca gctacatact atactgtaac                                        30


<210>  1847
<211>  30

<212>  DNA
<213>  Artificial

<220>
<223>  synthetic DNA

<400>  1847
tacgacggac gctggtggtg taagagaagg                                        30


<210>  1848
<211>  30
<212>  DNA
<213>  Artificial

<220>
<223>  synthetic DNA

<400>  1848
gcctcgatat atctacgtat agttcaagtt                                        30


<210>  1849
<211>  30
<212>  DNA
<213>  Artificial

<220>
<223>  synthetic DNA

<400>  1849
ggctcctgca ttcattgaag gtcggccttg                                        30


<210>  1850
<211>  30
<212>  DNA
<213>  Artificial

<220>
<223>  synthetic DNA

<400>  1850
cagttcggtg attcaagaga acaatggtgg                                        30


<210>  1851
<211>  30
<212>  DNA
<213>  Artificial

<220>
<223>  synthetic DNA

<400>  1851
tataacgaag ccggctggaa cggtaactca                                        30


<210>  1852
<211>  30
<212>  DNA
<213>  Artificial

<220>
<223>  synthetic DNA

<400>  1852
ctgtatcaat tcaagtgaca gtggcacgtc                                        30


<210>  1853
<211>  30
<212>  DNA
<213>  Artificial

<220>
<223>  synthetic DNA

<400>  1853
agcaattgcg gttcataggc gtaattatat                                        30


<210>  1854
<211>  30
<212>  DNA
<213>  Artificial

<220>
<223>  synthetic DNA

<400>  1854
catatggacc tggagatcac cgttcagtcc                                        30


<210>  1855
<211>  30
<212>  DNA
<213>  Artificial

<220>
<223>  synthetic DNA

<400>  1855
gaaggccgtt ggtctatctc ttactggagc                                        30


<210>  1856
<211>  30
<212>  DNA
<213>  Artificial

<220>
<223>  synthetic DNA

<400>  1856
gtgcgttcat ctagcctaag acgctgacct                                        30


<210>  1857
<211>  30
<212>  DNA
<213>  Artificial

<220>
<223>  synthetic DNA

<400>  1857
gagtaactta tatcctctct acgacatcga                                        30


<210>  1858
<211>  30
<212>  DNA
<213>  Artificial

<220>
<223>  synthetic DNA

<400>  1858
attctacgct gatgtctccg ctgaacagga                                        30


<210>  1859
<211>  30
<212>  DNA
<213>  Artificial

<220>
<223>  synthetic DNA

<400>  1859
tcatcaacgt tactcactag taccacggct                                        30


<210>  1860
<211>  30
<212>  DNA
<213>  Artificial

<220>
<223>  synthetic DNA

<400>  1860
aaccattctt gaacgttgag aacctggtgg                                        30


<210>  1861
<211>  30
<212>  DNA
<213>  Artificial

<220>
<223>  synthetic DNA

<400>  1861
acgacacctc cgcggaacat acctgattag                                        30


<210>  1862
<211>  30
<212>  DNA
<213>  Artificial

<220>
<223>  synthetic DNA

<400>  1862
gcgcacttat tgaagtaatc tcatggccaa                                        30

<210>  1863
<211>  30
<212>  DNA
<213>  Artificial

<220>
<223>  synthetic DNA

<400>  1863
gcgccaattc agccagttag cgtctccgtg                                        30


<210>  1864
<211>  30
<212>  DNA
<213>  Artificial

<220>
<223>  synthetic DNA

<400>  1864
agcaacaagt cgctgtatat cgactggccg                                        30


<210>  1865
<211>  30
<212>  DNA
<213>  Artificial

<220>
<223>  synthetic DNA

<400>  1865
ccttacaata gacctcgcgg cgttcatgcc                                        30


<210>  1866
<211>  30
<212>  DNA
<213>  Artificial

<220>
<223>  synthetic DNA

<400>  1866
ggatccaact tcagcgaagc accaacgtcg                                        30


<210>  1867
<211>  30
<212>  DNA
<213>  Artificial

<220>
<223>  synthetic DNA

<400>  1867
gcgccagttc tcgtactctc gagaagcgac                                        30


<210>  1868
<211>  30

<212>  DNA
<213>  Artificial

<220>
<223>  synthetic DNA

<400>  1868
gagtgcggcc aatctggaac tcatgacgtt                                        30


<210>  1869
<211>  30
<212>  DNA
<213>  Artificial

<220>
<223>  synthetic DNA

<400>  1869
cctgagagtg attcgtgtct gcgaagatgc                                        30


<210>  1870
<211>  30
<212>  DNA
<213>  Artificial

<220>
<223>  synthetic DNA

<400>  1870
gtgactggtt aaggcaatat tggtcgaccg                                        30


<210>  1871
<211>  30
<212>  DNA
<213>  Artificial

<220>
<223>  synthetic DNA

<400>  1871
ctatcaagcc ttacaaggtc acgtccacta                                        30


<210>  1872
<211>  30
<212>  DNA
<213>  Artificial

<220>
<223>  synthetic DNA

<400>  1872
actgcgtcct tgcgtcggaa ctccttgtgt                                        30


<210>  1873
<211>  30
<212>  DNA
<213>  Artificial

<220>
<223>  synthetic DNA

<400>  1873
tgcaactcag tggcggcgac accaagagct                                        30


<210>  1874
<211>  30
<212>  DNA
<213>  Artificial

<220>
<223>  synthetic DNA

<400>  1874
ttcggttcta ctaggatctc tatctgagct                                        30


<210>  1875
<211>  30
<212>  DNA
<213>  Artificial

<220>
<223>  synthetic DNA

<400>  1875
agctaatcta ttaagacaga ttagacagga                                        30


<210>  1876
<211>  30
<212>  DNA
<213>  Artificial

<220>
<223>  synthetic DNA

<400>  1876
ggaccgctct taggttatgc acctgcgtat                                        30


<210>  1877
<211>  30
<212>  DNA
<213>  Artificial

<220>
<223>  synthetic DNA

<400>  1877
ctctaatact agtccacagg ttagtacgaa                                        30


<210>  1878
<211>  30
<212>  DNA
<213>  Artificial

<220>
<223>  synthetic DNA

<400>  1878
atccatatat gctcgtcgtc agccagtgtt                                        30


<210>  1879
<211>  30
<212>  DNA
<213>  Artificial

<220>
<223>  synthetic DNA

<400>  1879
gctattactg tgttgatgtc cacaggagaa                                        30


<210>  1880
<211>  30
<212>  DNA
<213>  Artificial

<220>
<223>  synthetic DNA

<400>  1880
gctacggcgc agatctagac aactggaagt                                        30


<210>  1881
<211>  30
<212>  DNA
<213>  Artificial

<220>
<223>  synthetic DNA

<400>  1881
gcctcttgtg ttagccgaat accaatgacc                                        30


<210>  1882
<211>  30
<212>  DNA
<213>  Artificial

<220>
<223>  synthetic DNA

<400>  1882
tgaggacgat aacattacct ctcgagtcgc                                        30


<210>  1883
<211>  30
<212>  DNA
<213>  Artificial

<220>
<223>  synthetic DNA

<400>  1883
cgattaccaa tccgacgact tcgcagcagc                                        30

<210>  1884
<211>  30
<212>  DNA
<213>  Artificial

<220>
<223>  synthetic DNA

<400>  1884
atgacacgag tccagtacat atgcgaagac                                        30


<210>  1885
<211>  30
<212>  DNA
<213>  Artificial

<220>
<223>  synthetic DNA

<400>  1885
gcgctcgcat gcactagtgt agactgacga                                        30


<210>  1886
<211>  30
<212>  DNA
<213>  Artificial

<220>
<223>  synthetic DNA

<400>  1886
gcacatctca gaattgatgg tctatgtcgc                                        30


<210>  1887
<211>  30
<212>  DNA
<213>  Artificial

<220>
<223>  synthetic DNA

<400>  1887
ttcttcgacg ccgcgtacta ataggtcaat                                        30


<210>  1888
<211>  30
<212>  DNA
<213>  Artificial

<220>
<223>  synthetic DNA

<400>  1888
ggaagcgcct ctaacaaccg atgcttgtgg                                        30


<210>  1889
<211>  30

<212>  DNA
<213>  Artificial

<220>
<223>  synthetic DNA

<400>  1889
ctctagacgc gtcgtgactc caatctgttg                                        30


<210>  1890
<211>  30
<212>  DNA
<213>  Artificial

<220>
<223>  synthetic DNA

<400>  1890
gtagttcgtc ggagtgacct cgtactcact                                        30


<210>  1891
<211>  30
<212>  DNA
<213>  Artificial

<220>
<223>  synthetic DNA

<400>  1891
atgctgtcga gtgtccggca tagagcacac                                        30


<210>  1892
<211>  30
<212>  DNA
<213>  Artificial

<220>
<223>  synthetic DNA

<400>  1892
gcgcatcttg cagcgtcctg tagttctgaa                                        30


<210>  1893
<211>  30
<212>  DNA
<213>  Artificial

<220>
<223>  synthetic DNA

<400>  1893
gcgattgttg aggaaccaca gcggcaccta                                        30


<210>  1894
<211>  30
<212>  DNA
<213>  Artificial

<220>
<223>  synthetic DNA

<400>  1894
cacgcgtact ctgcttgctg tgtggtcggt                                        30


<210>  1895
<211>  30
<212>  DNA
<213>  Artificial

<220>
<223>  synthetic DNA

<400>  1895
catccaacgc aggacctagt agtcatgctt                                        30


<210>  1896
<211>  30
<212>  DNA
<213>  Artificial

<220>
<223>  synthetic DNA

<400>  1896
ttctagttgt gatgagaatc gctagcgtgc                                        30


<210>  1897
<211>  30
<212>  DNA
<213>  Artificial

<220>
<223>  synthetic DNA

<400>  1897
cattctgaat ctggtctctc tcgatcatcc                                        30


<210>  1898
<211>  30
<212>  DNA
<213>  Artificial

<220>
<223>  synthetic DNA

<400>  1898
attaatgtag aggatagttc cgttctctcc                                        30


<210>  1899
<211>  30
<212>  DNA
<213>  Artificial

<220>
<223>  synthetic DNA

<400>  1899
gtatcgcgct tacgaatgag gtgtggcttc                                        30


<210>  1900
<211>  30
<212>  DNA
<213>  Artificial

<220>
<223>  synthetic DNA

<400>  1900
gctggtgaga gagccagatt atcggtggag                                        30


<210>  1901
<211>  30
<212>  DNA
<213>  Artificial

<220>
<223>  synthetic DNA

<400>  1901
ggcacgagca ggtagaacta gaacctagat                                        30


<210>  1902
<211>  30
<212>  DNA
<213>  Artificial

<220>
<223>  synthetic DNA

<400>  1902
tgtattatct cgaagcggtg cgttagagtc                                        30


<210>  1903
<211>  30
<212>  DNA
<213>  Artificial

<220>
<223>  synthetic DNA

<400>  1903
cacgtgttct agctactaat ggcgtcaatt                                        30


<210>  1904
<211>  30
<212>  DNA
<213>  Artificial

<220>
<223>  synthetic DNA

<400>  1904
cgcgctacat tacttcctac accatgcgta                                        30

<210>  1905
<211>  30
<212>  DNA
<213>  Artificial

<220>
<223>  synthetic DNA

<400>  1905
tgaggcaact agtgttcgca agatgacgga                                        30


<210>  1906
<211>  30
<212>  DNA
<213>  Artificial

<220>
<223>  synthetic DNA

<400>  1906
ttattattgt ctgtggaacg cacgccagtc                                        30


<210>  1907
<211>  30
<212>  DNA
<213>  Artificial

<220>
<223>  synthetic DNA

<400>  1907
gctatagtat tatccatgaa ttccgtcggc                                        30


<210>  1908
<211>  30
<212>  DNA
<213>  Artificial

<220>
<223>  synthetic DNA

<400>  1908
gtatcaatag ctcaattcgt cagagttgtg                                        30


<210>  1909
<211>  30
<212>  DNA
<213>  Artificial

<220>
<223>  synthetic DNA

<400>  1909
tagtccatgc gtggatatat tgagagctga                                        30


<210>  1910
<211>  30

<212>  DNA
<213>  Artificial

<220>
<223>  synthetic DNA

<400>  1910
gcacagtacg acttataaca ggtctagatc                                        30


<210>  1911
<211>  30
<212>  DNA
<213>  Artificial

<220>
<223>  synthetic DNA

<400>  1911
actcaatggt ggcacgctcg gcgcagcata                                        30


<210>  1912
<211>  30
<212>  DNA
<213>  Artificial

<220>
<223>  synthetic DNA

<400>  1912
gtagtaccac tccgccttag gcagcttaag                                        30


<210>  1913
<211>  30
<212>  DNA
<213>  Artificial

<220>
<223>  synthetic DNA

<400>  1913
cgctcaactg atgcgtgcaa ccaatgttat                                        30


<210>  1914
<211>  30
<212>  DNA
<213>  Artificial

<220>
<223>  synthetic DNA

<400>  1914
gcagcttgac tgcctagaca gcagttacag                                        30


<210>  1915
<211>  30
<212>  DNA
<213>  Artificial

<220>
<223>  synthetic DNA

<400>  1915
gcaacttctt agtacgaatt catcgtccaa                                        30


<210>  1916
<211>  30
<212>  DNA
<213>  Artificial

<220>
<223>  synthetic DNA

<400>  1916
atccgtatgc tgcggcagtg gaggtggctt                                        30


<210>  1917
<211>  30
<212>  DNA
<213>  Artificial

<220>
<223>  synthetic DNA

<400>  1917
tgcggatcaa tccagttctg tgtactgtga                                        30


<210>  1918
<211>  30
<212>  DNA
<213>  Artificial

<220>
<223>  synthetic DNA

<400>  1918
ttatgattat caccggcgta acattccgaa                                        30


<210>  1919
<211>  30
<212>  DNA
<213>  Artificial

<220>
<223>  synthetic DNA

<400>  1919
gctacctaga ttcttcaact catcgctacc                                        30


<210>  1920
<211>  30
<212>  DNA
<213>  Artificial

<220>
<223>  synthetic DNA

<400>  1920
cagtgttaga atggcggtgt gtagccgcta                                        30


<210>  1921
<211>  35
<212>  DNA
<213>  Artificial

<220>
<223>  synthetic DNA

<400>  1921
gcttatagac tacagctgcg aggtataagg tcact                                  35


<210>  1922
<211>  35
<212>  DNA
<213>  Artificial

<220>
<223>  synthetic DNA

<400>  1922
cgctcagcag gatgctatcc taagttaatg tggtg                                  35


<210>  1923
<211>  35
<212>  DNA
<213>  Artificial

<220>
<223>  synthetic DNA

<400>  1923
gaactgagcg gacatcagct aggcctacaa tacat                                  35


<210>  1924
<211>  35
<212>  DNA
<213>  Artificial

<220>
<223>  synthetic DNA

<400>  1924
tcgtgaactt ctgcgttggt ctctaccaag gcggt                                  35


<210>  1925
<211>  35
<212>  DNA
<213>  Artificial

<220>
<223>  synthetic DNA

<400>  1925
taagtcaggt atcttatcag tggtacacgg tacga                                  35

<210>  1926
<211>  35
<212>  DNA
<213>  Artificial

<220>
<223>  synthetic DNA

<400>  1926
taataatgtt gcgcgtgacc gaggaggaat ccact                                35


<210>  1927
<211>  35
<212>  DNA
<213>  Artificial

<220>
<223>  synthetic DNA

<400>  1927
ctaggagttc tcgtaagctg gagtaccgta acgtg                                35


<210>  1928
<211>  35
<212>  DNA
<213>  Artificial

<220>
<223>  synthetic DNA

<400>  1928
ggactctcct cagaggatcc ttcttgcgca ggcat                                35


<210>  1929
<211>  35
<212>  DNA
<213>  Artificial

<220>
<223>  synthetic DNA

<400>  1929
gctagaggcc tgagtacacc ttctcgcatc aggat                                35


<210>  1930
<211>  35
<212>  DNA
<213>  Artificial

<220>
<223>  synthetic DNA

<400>  1930
atatcgcgag cactaacgtc gttgtcgttc tagga                                35


<210>  1931
<211>  35

<212>  DNA
<213>  Artificial

<220>
<223>  synthetic DNA

<400>  1931
agcggttact atacctggcg gctgacgttg ttagt                                35


<210>  1932
<211>  35
<212>  DNA
<213>  Artificial

<220>
<223>  synthetic DNA

<400>  1932
gagctaggta gatctccaag tgtagctaag aagag                                35


<210>  1933
<211>  35
<212>  DNA
<213>  Artificial

<220>
<223>  synthetic DNA

<400>  1933
ggagtcgctg gtgacgtatg ccgaggatga gcttc                                35


<210>  1934
<211>  35
<212>  DNA
<213>  Artificial

<220>
<223>  synthetic DNA

<400>  1934
cgccgacctc ctgttcacga agccgcctga tgtaa                                35


<210>  1935
<211>  35
<212>  DNA
<213>  Artificial

<220>
<223>  synthetic DNA

<400>  1935
agtaggcact tagttatcga ttacgttagt tagtc                                35


<210>  1936
<211>  35
<212>  DNA
<213>  Artificial

<220>
<223>  synthetic DNA

<400>  1936
ggatgacgtc tcagtctacc tcgcagtgtc gtcta                                35


<210>  1937
<211>  35
<212>  DNA
<213>  Artificial

<220>
<223>  synthetic DNA

<400>  1937
ctggttcgcg ttagcaatac taaggcagtc aggag                                35


<210>  1938
<211>  35
<212>  DNA
<213>  Artificial

<220>
<223>  synthetic DNA

<400>  1938
atatggtcat attggcctct tcgaacacag actgt                                35


<210>  1939
<211>  35
<212>  DNA
<213>  Artificial

<220>
<223>  synthetic DNA

<400>  1939
tatcagagga tagcaggtct gagttgcaag gctaa                                35


<210>  1940
<211>  35
<212>  DNA
<213>  Artificial

<220>
<223>  synthetic DNA

<400>  1940
ggtggtctga ccatagctgt tcttctcaca gagac                                35


<210>  1941
<211>  35
<212>  DNA
<213>  Artificial

<220>
<223>  synthetic DNA

<400>  1941
gcaataccaa cgagatgagt attcgttgaa gctct                                35


<210>  1942
<211>  35
<212>  DNA
<213>  Artificial

<220>
<223>  synthetic DNA

<400>  1942
ccaagtcgac gctgcatgaa tgagcgctat tcact                                35


<210>  1943
<211>  35
<212>  DNA
<213>  Artificial

<220>
<223>  synthetic DNA

<400>  1943
ccattagatc gcttcgagac aattaggaga catga                                35


<210>  1944
<211>  35
<212>  DNA
<213>  Artificial

<220>
<223>  synthetic DNA

<400>  1944
gatgactgta cctcctatca ttgagtgtgg accaa                                35


<210>  1945
<211>  35
<212>  DNA
<213>  Artificial

<220>
<223>  synthetic DNA

<400>  1945
atatctggat gaatagtggt taggtaagca agtaa                                35


<210>  1946
<211>  35
<212>  DNA
<213>  Artificial

<220>
<223>  synthetic DNA

<400>  1946
accgactatg ttaattcgtg tctggatggc agaat                                35

<210>  1947
<211>  35
<212>  DNA
<213>  Artificial

<220>
<223>  synthetic DNA

<400>  1947
gtggcagtct tgctagtatc ttagaccatc accaa                                  35


<210>  1948
<211>  35
<212>  DNA
<213>  Artificial

<220>
<223>  synthetic DNA

<400>  1948
cgctatctta gtcgagcaca atgtcttcgt atagg                                  35


<210>  1949
<211>  35
<212>  DNA
<213>  Artificial

<220>
<223>  synthetic DNA

<400>  1949
attagtacgg cacgaaccgg ccattcatgg cagct                                  35


<210>  1950
<211>  35
<212>  DNA
<213>  Artificial

<220>
<223>  synthetic DNA

<400>  1950
agtacgacta tcaagactcc agcgctctcc ttgga                                  35


<210>  1951
<211>  35
<212>  DNA
<213>  Artificial

<220>
<223>  synthetic DNA

<400>  1951
atgagcctcg gagcgaacgt tatcgatcag gctgt                                  35


<210>  1952
<211>  35

<212>  DNA
<213>  Artificial

<220>
<223>  synthetic DNA

<400>  1952
ttgcgtgcag tagcaccgat acacagcgct tgtat                                35


<210>  1953
<211>  35
<212>  DNA
<213>  Artificial

<220>
<223>  synthetic DNA

<400>  1953
aacggctgca tcacctacac tatactcaac atcta                                35


<210>  1954
<211>  35
<212>  DNA
<213>  Artificial

<220>
<223>  synthetic DNA

<400>  1954
gtcgctatgc gagaagtggc gtggaatgct atggt                                35


<210>  1955
<211>  35
<212>  DNA
<213>  Artificial

<220>
<223>  synthetic DNA

<400>  1955
catggatacc tactgacttg acttctagag gaccg                                35


<210>  1956
<211>  35
<212>  DNA
<213>  Artificial

<220>
<223>  synthetic DNA

<400>  1956
gagtgacgca gacaccgtaa cgtcgaatct tctag                                35


<210>  1957
<211>  35
<212>  DNA
<213>  Artificial

<220>
<223>  synthetic DNA

<400>  1957
agtaccgtct gtgtgaatat tgttcctacg ttaca                                35


<210>  1958
<211>  35
<212>  DNA
<213>  Artificial

<220>
<223>  synthetic DNA

<400>  1958
ggctaatcga tagtgacgag ttctgcacgc ctgaa                                35


<210>  1959
<211>  35
<212>  DNA
<213>  Artificial

<220>
<223>  synthetic DNA

<400>  1959
ggcgagcgct cgtggttctg agtcgctgtt agatg                                35


<210>  1960
<211>  35
<212>  DNA
<213>  Artificial

<220>
<223>  synthetic DNA

<400>  1960
tatctccagc gttataagct actggagccg ctcgg                                35


<210>  1961
<211>  35
<212>  DNA
<213>  Artificial

<220>
<223>  synthetic DNA

<400>  1961
ccttctgcgc aagtcaagga ttcgcttaga tggac                                35


<210>  1962
<211>  35
<212>  DNA
<213>  Artificial

<220>
<223>  synthetic DNA

<400>  1962
gttgctgaca gccgttgcgt acttgcctta agaac                                35


<210>  1963
<211>  35
<212>  DNA
<213>  Artificial

<220>
<223>  synthetic DNA

<400>  1963
gtggcctaat cactcgcgct tcataggccg atagg                                35


<210>  1964
<211>  35
<212>  DNA
<213>  Artificial

<220>
<223>  synthetic DNA

<400>  1964
tgcatctagc ctacatcgga ccttgttatg gtaat                                35


<210>  1965
<211>  35
<212>  DNA
<213>  Artificial

<220>
<223>  synthetic DNA

<400>  1965
ggacagctac tggacaccac cgaactggta gtgtc                                35


<210>  1966
<211>  35
<212>  DNA
<213>  Artificial

<220>
<223>  synthetic DNA

<400>  1966
aactggcgat ggacggccgc tcttccgcta catag                                35


<210>  1967
<211>  35
<212>  DNA
<213>  Artificial

<220>
<223>  synthetic DNA

<400>  1967
ggagcagtta gctatggagc aggccgataa cctga                                35

<210>  1968
<211>  35
<212>  DNA
<213>  Artificial

<220>
<223>  synthetic DNA

<400>  1968
actctacggt gcacctcagc cttcatgcaa taggc                                35


<210>  1969
<211>  35
<212>  DNA
<213>  Artificial

<220>
<223>  synthetic DNA

<400>  1969
cttgtagcac aatacattac tctccacgtg atagc                                35


<210>  1970
<211>  35
<212>  DNA
<213>  Artificial

<220>
<223>  synthetic DNA

<400>  1970
ggacgctatc gataccgtta ttcctactct gtcgg                                35


<210>  1971
<211>  35
<212>  DNA
<213>  Artificial

<220>
<223>  synthetic DNA

<400>  1971
ggatgatcgt caacgatcaa ctgacagtta gtcga                                35


<210>  1972
<211>  35
<212>  DNA
<213>  Artificial

<220>
<223>  synthetic DNA

<400>  1972
tgacagtagc aatgtctcac gtctgcacaa cggaa                                35


<210>  1973
<211>  35

<212>  DNA
<213>  Artificial

<220>
<223>  synthetic DNA

<400>  1973
gtcgcaggac ctcacggata gtagtgcgag gtcta                                35


<210>  1974
<211>  35
<212>  DNA
<213>  Artificial

<220>
<223>  synthetic DNA

<400>  1974
atatcggcgg acgcaatgac agttgttggc tgatg                                35


<210>  1975
<211>  35
<212>  DNA
<213>  Artificial

<220>
<223>  synthetic DNA

<400>  1975
aagcaccaag gaggtatgtt ccatcgaggc gctcg                                35


<210>  1976
<211>  35
<212>  DNA
<213>  Artificial

<220>
<223>  synthetic DNA

<400>  1976
gaccgcacct tatagctata tcctggtcta gtact                                35


<210>  1977
<211>  35
<212>  DNA
<213>  Artificial

<220>
<223>  synthetic DNA

<400>  1977
tctcagagga aggttgagcg tctgaccagg ttggc                                35


<210>  1978
<211>  35
<212>  DNA
<213>  Artificial

<220>
<223>  synthetic DNA

<400>  1978
tggacctaga gacctagctc gtctcttcgc gatcg                                35


<210>  1979
<211>  35
<212>  DNA
<213>  Artificial

<220>
<223>  synthetic DNA

<400>  1979
cggagtggtt ccacgcgacc tcgcaactaa tcctt                                35


<210>  1980
<211>  35
<212>  DNA
<213>  Artificial

<220>
<223>  synthetic DNA

<400>  1980
ggagccgcgc gcagactgac cttgcttgat ctact                                35


<210>  1981
<211>  35
<212>  DNA
<213>  Artificial

<220>
<223>  synthetic DNA

<400>  1981
actctaagta tatgcgcagt tagtatactg aacca                                35


<210>  1982
<211>  35
<212>  DNA
<213>  Artificial

<220>
<223>  synthetic DNA

<400>  1982
gagcattgct tcgcttcgat gtctattctg atcag                                35


<210>  1983
<211>  35
<212>  DNA
<213>  Artificial

<220>
<223>  synthetic DNA

<400>  1983
gcttgtattg ccactcgagt aggtcgtggc agtag                                35

<210>  1984
<211>  35
<212>  DNA
<213>  Artificial

<220>
<223>  synthetic DNA

<400>  1984
atctggacat tgcattcggt gtgtatacag aaggc                                35

<210>  1985
<211>  35
<212>  DNA
<213>  Artificial

<220>
<223>  synthetic DNA

<400>  1985
ggttgcgatc agcttgatag caggtcatat cctca                                35

<210>  1986
<211>  35
<212>  DNA
<213>  Artificial

<220>
<223>  synthetic DNA

<400>  1986
gcaggtacta acctgagatg cgtagctaac acagg                                35

<210>  1987
<211>  35
<212>  DNA
<213>  Artificial

<220>
<223>  synthetic DNA

<400>  1987
atctgcaagg acgtaacgtc ctcggaaggt gaggt                                35

<210>  1988
<211>  35
<212>  DNA
<213>  Artificial

<220>
<223>  synthetic DNA

<400>  1988
ataatcttac gagcctccag tgaataatgc aagca                                35

<210>  1989
<211>  35
<212>  DNA
<213>  Artificial

<220>
<223>  synthetic DNA

<400>  1989
caatctccgc acagtcttgt tcaggtacag actta                                35


<210>  1990
<211>  35
<212>  DNA
<213>  Artificial

<220>
<223>  synthetic DNA

<400>  1990
atgtgcgcaa ttcagcgtaa gtgcctattc ataat                                35


<210>  1991
<211>  35
<212>  DNA
<213>  Artificial

<220>
<223>  synthetic DNA

<400>  1991
tcggacgcac acatcctgtt gtcgagaaga ggaag                                35


<210>  1992
<211>  35
<212>  DNA
<213>  Artificial

<220>
<223>  synthetic DNA

<400>  1992
tcggaagcat cacatgagca tcaggagttc attgc                                35


<210>  1993
<211>  35
<212>  DNA
<213>  Artificial

<220>
<223>  synthetic DNA

<400>  1993
atctggttgt ggacttctat acagtaccag agtgg                                35


<210>  1994
<211>  35

<212>  DNA
<213>  Artificial

<220>
<223>  synthetic DNA

<400>  1994
cgtctgaata tagttagcta gtagtgtaat ccagg                                35


<210>  1995
<211>  35
<212>  DNA
<213>  Artificial

<220>
<223>  synthetic DNA

<400>  1995
taatatctga tccgacctat tatctaggac tactc                                35


<210>  1996
<211>  35
<212>  DNA
<213>  Artificial

<220>
<223>  synthetic DNA

<400>  1996
tatgcggccg tccgtacctc gtctgcttca gttgg                                35


<210>  1997
<211>  35
<212>  DNA
<213>  Artificial

<220>
<223>  synthetic DNA

<400>  1997
tggctcaagt tccatattgc caagacgacc tggag                                35


<210>  1998
<211>  35
<212>  DNA
<213>  Artificial

<220>
<223>  synthetic DNA

<400>  1998
gcagttctgc taggcggtcc gaggcaattg aagag                                35


<210>  1999
<211>  35
<212>  DNA
<213>  Artificial

<220>
<223>  synthetic DNA

<400>  1999
catggcacag acgaagtatg caccacgctc attaa                                35


<210>  2000
<211>  35
<212>  DNA
<213>  Artificial

<220>
<223>  synthetic DNA

<400>  2000
ggagcgtact acgaccattc aaccgaatat gttac                                35


<210>  2001
<211>  35
<212>  DNA
<213>  Artificial

<220>
<223>  synthetic DNA

<400>  2001
gcgtagatct cgcgacagag acaaggtgcg aatgg                                35


<210>  2002
<211>  35
<212>  DNA
<213>  Artificial

<220>
<223>  synthetic DNA

<400>  2002
tggactgagg ttctccggtc tatactcctg tagga                                35


<210>  2003
<211>  35
<212>  DNA
<213>  Artificial

<220>
<223>  synthetic DNA

<400>  2003
tggctatagc aacggcttct tgtgatcgca ttgca                                35


<210>  2004
<211>  35
<212>  DNA
<213>  Artificial

<220>
<223>  synthetic DNA

<400>  2004
ggcgaagaat catgcgagac ggagtagacg gacgt                                35

<210>  2005
<211>  35
<212>  DNA
<213>  Artificial

<220>
<223>  synthetic DNA

<400>  2005
gagcattgcg agttgcacac gtgatatcag actgt                                35

<210>  2006
<211>  35
<212>  DNA
<213>  Artificial

<220>
<223>  synthetic DNA

<400>  2006
ctgttgacct atgccagaat caatacctca gatta                                35

<210>  2007
<211>  35
<212>  DNA
<213>  Artificial

<220>
<223>  synthetic DNA

<400>  2007
gttaacaagt agatgccaag atacaacgag agacc                                35

<210>  2008
<211>  35
<212>  DNA
<213>  Artificial

<220>
<223>  synthetic DNA

<400>  2008
gagcaagatt atagttagga agatagttaa ctcgc                                35

<210>  2009
<211>  35
<212>  DNA
<213>  Artificial

<220>
<223>  synthetic DNA

<400>  2009
tccggagtcg agcatatgtg accaactctc aacgc                                35

<210>  2010
<211>  35
<212>  DNA
<213>  Artificial

<220>
<223>  synthetic DNA

<400>  2010
ggagctgcga tgccgttacc gacgtcatct tcaag                                35


<210>  2011
<211>  35
<212>  DNA
<213>  Artificial

<220>
<223>  synthetic DNA

<400>  2011
gctctatctt acacattggc gtactggact cgcga                                35


<210>  2012
<211>  35
<212>  DNA
<213>  Artificial

<220>
<223>  synthetic DNA

<400>  2012
ttctacatat tcatcgccta ccgagttgcg cgaag                                35


<210>  2013
<211>  35
<212>  DNA
<213>  Artificial

<220>
<223>  synthetic DNA

<400>  2013
tggacgtctg acctgtgtct acatcggtgg tgcta                                35


<210>  2014
<211>  35
<212>  DNA
<213>  Artificial

<220>
<223>  synthetic DNA

<400>  2014
ggcaggacag ctccgtgttc tactcgaacc gcact                                35


<210>  2015
<211>  35

<212>  DNA
<213>  Artificial

<220>
<223>  synthetic DNA

<400>  2015
tgacaacctc atgtctccga ccgcaggcat acaat                                35


<210>  2016
<211>  35
<212>  DNA
<213>  Artificial

<220>
<223>  synthetic DNA

<400>  2016
gcaggcctaa caagtggtca cgaggagtcc ttatt                                35


<210>  2017
<211>  90
<212>  DNA
<213>  Artificial

<220>
<223>  synthetic DNA

<400>  2017
cccatacaca caccatgaag cttgaactaa ttaacattct caaactaatt aacaagcatg      60

caagcatgtt tttacacaat gacaatatat                                      90


<210>  2018
<211>  90
<212>  DNA
<213>  Artificial

<220>
<223>  synthetic DNA

<400>  2018
atgggtgagg gcgcagaggc aaagacatgg aggtccggaa gggtagaagc tcacatcaag      60

tcgagtatgt tgaatgcaat cccatatata                                      90


<210>  2019
<211>  24
<212>  DNA
<213>  Artificial

<220>
<223>  synthetic DNA

<400>  2019
cccatacaca caccatgaag cttg                                            24


<210>  2020

<211>  24
<212>  DNA
<213>  Artificial

<220>
<223>  synthetic DNA

<400>  2020
ggtagaagct cacatcaagt cgag                                              24


<210>  2021
<211>  90
<212>  DNA
<213>  Artificial

<220>
<223>  synthetic DNA

<400>  2021
aatcacagaa cgaggtctgg acgagaacag agctggacat ctacacgcac cgcatggtag       60

tagagcatgt actgcaaaag cttgaagcgc                                        90


<210>  2022
<211>  90
<212>  DNA
<213>  Artificial

<220>
<223>  synthetic DNA

<400>  2022
gatgctgagg gcgaagttgt cagccaagtc ctcaatgtca taggcgagat cgcagtagtt       60

ctgtaaccat tccctgctaa actggtccat                                        90


<210>  2023
<211>  24
<212>  DNA
<213>  Artificial

<220>
<223>  synthetic DNA

<400>  2023
acgagaacag agctggacat ctac                                              24


<210>  2024
<211>  24
<212>  DNA
<213>  Artificial

<220>
<223>  synthetic DNA

<400>  2024
tcaatgtcat aggcgagatc gcag                                              24

<210>  2025
<211>  90
<212>  DNA
<213>  Artificial

<220>
<223>  synthetic DNA

<400>  2025
agaccaacaa gcagcaagta gtcagagaag tacaagagaa ggagagcaag aaggatagta        60

agttgcaagc ttaccgttac aaagatgata                                          90


<210>  2026
<211>  90
<212>  DNA
<213>  Artificial

<220>
<223>  synthetic DNA

<400>  2026
ggaggagcac aactaggcgt ttatcaagat gggtcatcga gctcttggtg tcttcaacct        60

tcttgacatc aacttctcca atcttcgtct                                          90


<210>  2027
<211>  26
<212>  DNA
<213>  Artificial

<220>
<223>  synthetic DNA

<400>  2027
ggagagcaag aaggatagta agttgc                                              26


<210>  2028
<211>  25
<212>  DNA
<213>  Artificial

<220>
<223>  synthetic DNA

<400>  2028
cgagctcttg gtgtcttcaa ccttc                                               25


<210>  2029
<211>  90
<212>  DNA
<213>  Artificial

<220>
<223>  synthetic DNA

<400>  2029
tggggtagtc ctgaagctct aggtatgcct cttcatctcc ctgcacctct ggtgctagca        60

cctcctgctc ttcgggcacc tctaccgggg 90

<210> 2030
<211> 90
<212> DNA
<213> Artificial

<220>
<223> synthetic DNA

<400> 2030
ggatactgat gtagctttca cccgggagta ttccaaggta tcgattttcc acggggaacg 60

cgaagtgcac tagttgaggt ttagattgcc 90

<210> 2031
<211> 25
<212> DNA
<213> Artificial

<220>
<223> synthetic DNA

<400> 2031
gaagctctag gtatgcctct tcatc 25

<210> 2032
<211> 25
<212> DNA
<213> Artificial

<220>
<223> synthetic DNA

<400> 2032
gtgcactagt tgaggtttag attgc 25

<210> 2033
<211> 90
<212> DNA
<213> Artificial

<220>
<223> synthetic DNA

<400> 2033
tcgggaaaac gaacgggcga actacagatg tcagtacgaa gtagtctatg gcaggaaata 60

cgtagtccat acgtggtgcc agcccaagcc 90

<210> 2034
<211> 90
<212> DNA
<213> Artificial

<220>
<223> synthetic DNA

<400>  2034
agcaggaggg agaaaggaaa cgtggcattc atcggctgtc tgccattgcc atgtgagaca      60

aggaaatcta cttcaccccc atctatcgag                                        90


<210>  2035
<211>  24
<212>  DNA
<213>  Artificial

<220>
<223>  synthetic DNA

<400>  2035
gggcgaacta cagatgtcag tacg                                              24


<210>  2036
<211>  24
<212>  DNA
<213>  Artificial

<220>
<223>  synthetic DNA

<400>  2036
ctgtctgcca ttgccatgtg agac                                              24


<210>  2037
<211>  90
<212>  DNA
<213>  Artificial

<220>
<223>  synthetic DNA

<400>  2037
agacataaga ttaactatga acaaattcac gggtccgatt cctttgggat ttgcagcttg      60

caagaacctt caaatactca ttatatcttc                                        90


<210>  2038
<211>  90
<212>  DNA
<213>  Artificial

<220>
<223>  synthetic DNA

<400>  2038
ttaagttgca gaatttgata cgaagaactt gaagcatggt gaggttgccg agctcattgg      60

ggatggttcc agaaaggcta ttgtagctta                                        90


<210>  2039
<211>  24
<212>  DNA
<213>  Artificial

<220>
<223>  synthetic DNA

<400>  2039
gaacaaattc acgggtccga ttcc                                              24


<210>  2040
<211>  24
<212>  DNA
<213>  Artificial

<220>
<223>  synthetic DNA

<400>  2040
cgaagaactt gaagcatggt gagg                                              24


<210>  2041
<211>  10
<212>  DNA
<213>  Artificial

<220>
<223>  synthetic DNA

<400>  2041
gttacacacg                                                              10


<210>  2042
<211>  70
<212>  DNA
<213>  Artificial

<220>
<223>  synthetic DNA

<400>  2042
aatgatacgg cgaccaccga gatctacacc tctctattcg tcggcagcgt cagatgtgta      60

taagagacag                                                              70


<210>  2043
<211>  66
<212>  DNA
<213>  Artificial

<220>
<223>  synthetic DNA

<400>  2043
caagcagaag acggcatacg agattaaggc gagtctcgtg ggctcggaga tgtgtataag      60

agacag                                                                  66


<210>  2044
<211>  12
<212>  DNA
<213>  Artificial

<220>
<223>  synthetic DNA

<400>  2044
taagagacag aa                                                          12


<210>  2045
<211>  12
<212>  DNA
<213>  Artificial

<220>
<223>  synthetic DNA

<400>  2045
taagagacag at                                                          12


<210>  2046
<211>  12
<212>  DNA
<213>  Artificial

<220>
<223>  synthetic DNA

<400>  2046
taagagacag ac                                                          12


<210>  2047
<211>  12
<212>  DNA
<213>  Artificial

<220>
<223>  synthetic DNA

<400>  2047
taagagacag ag                                                          12


<210>  2048
<211>  12
<212>  DNA
<213>  Artificial

<220>
<223>  synthetic DNA

<400>  2048
taagagacag ta                                                          12


<210>  2049
<211>  12
<212>  DNA
<213>  Artificial

<220>
<223>  synthetic DNA

<400>  2049
taagagacag tt                                                          12


<210>  2050
<211>  12
<212>  DNA
<213>  Artificial

<220>
<223>  synthetic DNA

<400>  2050
taagagacag tc                                                          12


<210>  2051
<211>  12
<212>  DNA
<213>  Artificial

<220>
<223>  synthetic DNA

<400>  2051
taagagacag tg                                                          12


<210>  2052
<211>  12
<212>  DNA
<213>  Artificial

<220>
<223>  synthetic DNA

<400>  2052
taagagacag ca                                                          12


<210>  2053
<211>  12
<212>  DNA
<213>  Artificial

<220>
<223>  synthetic DNA

<400>  2053
taagagacag ct                                                          12


<210>  2054
<211>  12
<212>  DNA
<213>  Artificial

<220>
<223>  synthetic DNA

<400>  2054
taagagacag cc                                                          12

<210>  2055
<211>  12
<212>  DNA
<213>  Artificial

<220>
<223>  synthetic DNA

<400>  2055
taagagacag cg                                                          12


<210>  2056
<211>  12
<212>  DNA
<213>  Artificial

<220>
<223>  synthetic DNA

<400>  2056
taagagacag ga                                                          12


<210>  2057
<211>  12
<212>  DNA
<213>  Artificial

<220>
<223>  synthetic DNA

<400>  2057
taagagacag gt                                                          12


<210>  2058
<211>  12
<212>  DNA
<213>  Artificial

<220>
<223>  synthetic DNA

<400>  2058
taagagacag gc                                                          12


<210>  2059
<211>  12
<212>  DNA
<213>  Artificial

<220>
<223>  synthetic DNA

<400>  2059
taagagacag gg                                                          12

## Description

### Technical Field

The present invention relates to a method for producing a DNA library that can be used for analyzing a DNA marker, for example, and a method for genomic DNA analysis using such DNA library.

### Background Art

In general, genomic analysis is performed to conduct comprehensive analysis of genetic information contained in the genome, such as nucleotide sequence information. However, an analysis aimed at determination of the nucleotide sequence for whole genome is disadvantageous in terms of the number of processes and the cost. In cases of organisms with large genomic sizes, in addition, genomic analysis based on nucleotide sequence analysis has limitations because of genome complexity.

Patent Literature 1 discloses an amplified fragment length polymorphism (AFLP) marker technique wherein a sample-specific index is incorporated into a restriction-enzyme-treated fragment that had been ligated to an adapter and only a part of the sequence of the restriction-enzyme-treated fragment is to be determined. According to the technique disclosed in Patent Literature 1, the complexity of genomic DNA is reduced by treating genomic DNA with a restriction enzyme, the nucleotide sequence of a target part of the restriction-enzyme-treated fragment is determined, and the target restriction-enzyme-treated fragment is thus determined sufficiently. The technique disclosed in Patent Literature 1, however, requires processes such as treatment of genomic DNA with a restriction enzyme and ligation reaction with the use of an adapter. Thus, it is difficult to achieve a cost reduction.

Meanwhile, Patent Literature 2 discloses as follows. That is, a DNA marker for identification that is highly correlated with the results of taste evaluation was found from among DNA bands obtained by amplifying DNAs extracted from a rice sample via PCR in the presence of adequate primers by the so-called RAPD (randomly amplified polymorphic DNA) technique. The method disclosed in Patent Literature 2 involves the use of a plurality of sequence-tagged sites (STSs, which are primers) identified by particular sequences. According to the method disclosed in Patent Literature 2, a DNA marker for identification amplified with the use of an STS primer is detected via electrophoresis. However, the RAPD technique disclosed in Patent Literature 2 yields significantly poor reproducibility of PCR amplification, and, accordingly, such technique cannot be generally adopted as a DNA marker technique.

Patent Literature 3 discloses a method for producing a genomic library wherein PCR is carried out with the use of a single type of primer designed on the basis of a sequence that appears relatively frequently in the target genome, the entire genomic region is substantially uniformly amplified, and a genomic library can be thus produced. While Patent Literature 3 describes that a genomic library can be produced by conducting PCR with the use of a random primer containing a random sequence, it does not describe any actual procedures or results of experimentation. Accordingly, the method described in Patent Literature 3 is deduced to require nucleotide sequence information of the genome so as to identify the genome appearing frequency, which would increase the number of procedures and the cost. According to the method described in Patent Literature 3, in addition, the entire genome is to be amplified, and complexity of genomic DNA cannot be reduced, disadvantageously.

### Citation List

### Patent Literature

Patent Literature 1: JP Patent No. 5389638
Patent Literature 2: JP Patent Publication (Kokai) No. 2003-79375 A
Patent Literature 3: JP Patent No. 3972106

### Summary of Invention

### Technical Problem

For a technique for genome information analysis, such as genetic linkage analysis conducted with the use of a DNA marker, production of a DNA library in a more convenient and highly reproducible manner is desired. As described above, a wide variety of techniques for producing a DNA library are known. To date, however, there have been no techniques known to be sufficient in terms of convenience and/or reproducibility. Under the above circumstances, it is an object of the present invention to provide a method for producing a DNA library with more convenience and higher reproducibility, and it is another object to provide a method for analyzing genomic DNA with the use of such DNA library.

### Solution to Problem

The present inventors have conducted concentrated studies in order to attain the above objects. As a result, they discovered that high reproducibility could be achieved by conducting PCR with the use of a random primer while designating the concentration of such random primer within a designated range in a reaction solution. This has led to the completion of the present invention.

The present invention includes the following.
(1) A method for producing a DNA library, comprising conducting a nucleic acid amplification reaction in a reaction solution containing genomic DNA and a random primer at a high concentration using genomic DNA as a template to obtain DNA fragments.
(2) The method for producing a DNA library according to (1), wherein the reaction solution comprises the random primer at a concentration of 4 to 200 µM.
(3) The method for producing a DNA library according to (1), wherein the reaction solution comprises the random primer at a concentration of 4 to 100 µM.
(4) The method for producing a DNA library according to (1), wherein the random primer comprises 9 to 30 nucleotides.
(5) The method for producing a DNA library according to (1), wherein the DNA fragments each comprise 100 to 500 nucleotides.
(6) A method for analyzing genomic DNA, comprising using a DNA library produced by the method for producing a DNA library according to any one of (1) to (5) as a DNA marker.
(7) The method for analyzing genomic DNA according to (6), which comprises determining the nucleotide sequence of the DNA library produced by the method for producing a DNA library according to any one of (1) to (5) and confirming the presence or absence of the DNA marker based on the nucleotide sequence.
(8) The method for analyzing genomic DNA according to (7), wherein the presence or absence of the DNA marker is confirmed based on the number of reads of the nucleotide sequence of the DNA library in the step of confirming the presence or absence of the DNA marker.
(9) The method for analyzing genomic DNA according to (7), wherein the nucleotide sequence of the DNA library is compared with known sequence information or with the nucleotide sequence of a DNA library produced using genomic DNA from a different organism or tissue, and the presence or absence of the DNA marker is confirmed based on differences in the nucleotide sequences.
(10) The method for analyzing genomic DNA according to (6), which comprises:
   a step of preparing a pair of primers for specifically amplifying the DNA marker based on the nucleotide sequence of the DNA marker;
   a step of conducting a nucleic acid amplification reaction using genomic DNA extracted from a target organism as a template and the pair of primers; and
   a step of confirming the presence or absence of the DNA marker in the genomic DNA based on the results of the nucleic acid amplification reaction.
(11) A method for producing a DNA library, comprising:
   a step of conducting a nucleic acid amplification reaction in a first reaction solution comprising genomic DNA and a random primer at a high concentration to obtain first DNA fragments by the nucleic acid amplification reaction using the genomic DNA as a template; and
   a step of conducting a nucleic acid amplification reaction in a second reaction solution comprising the obtained first DNA fragments and a nucleotide, as a primer, which has a 3'-end nucleotide sequence having 70% identity to at least a 5'-end nucleotide sequence of the random primer to ligate the nucleotides to the first DNA fragments, thereby obtaining second DNA fragments.
(12) The method for producing a DNA library according to (11), wherein the first reaction solution comprises the random primer at a concentration of 4 to 100 µM.
(13) The method for producing a DNA library according to (11), wherein the first reaction solution comprises the random primer at a concentration of 4 to 100 µM.
(14) The method for producing a DNA library according to (11), wherein the random primer comprises 9 to 30 nucleotides.
(15) The method for producing a DNA library according to (11), wherein the first DNA fragments each comprise 100 to 500 nucleotides.
(16) The method for producing a DNA library according to (11), wherein the primer for amplifying the second DNA fragments comprises a region used for a nucleotide sequencing reaction, or the primer used for a nucleic acid amplification reaction using the second DNA fragments as templates or a nucleic acid amplification reaction to be conducted repeatedly comprises a region used for a nucleotide sequencing reaction.
(17) A method for analyzing a DNA library, comprising a step of determining a nucleotide sequence for a second DNA fragment obtained by the method for producing a DNA library according to any one of (11) to (15) or a DNA fragment obtained using a primer comprising a region complementary to a sequencer primer to be used in a nucleotide sequencing reaction in the method for producing a DNA library according to (16).
(18) A method for analyzing genomic DNA, comprising using a DNA library produced by the method for producing a DNA library according to any one of (11) to (17) as a DNA marker.
(19) The method for analyzing genomic DNA according to (18), which comprises determining the nucleotide sequence of the DNA library produced by the method for producing a DNA library according to any one of (11) to (17) and confirming the presence or absence of the DNA marker based on the nucleotide sequence.
(20) The method for analyzing genomic DNA according to (19), wherein the presence or absence of the DNA marker is confirmed based on the number of reads of the nucleotide sequence of the DNA library in the step of confirming the presence or absence of the DNA marker.
(21) The method for analyzing genomic DNA according to (19), wherein the nucleotide sequence of the DNA library is compared with known sequence information or with the nucleotide sequence of a DNA library produced using genomic DNA from a different organism or tissue, and the presence or absence of the DNA marker is confirmed based on differences in the nucleotide sequences.
(22) The method for analyzing genomic DNA according to (18), which comprises: a step of preparing a pair of primers for specifically amplifying the DNA marker based on the nucleotide sequence of the DNA marker; a step of conducting a nucleic acid amplification reaction using genomic DNA extracted from a target organism as a template and the pair of primers; and a step of confirming the presence or absence of the DNA marker in the genomic DNA based on the results of the nucleic acid amplification reaction.
(23) A DNA library, which is produced by the method for producing a DNA library according to any one of (1) to (5) and (11) to (16).

The present description includes part or all of the contents as disclosed in the descriptions and/or drawings of Japanese Patent Application Nos. 2016-129048, 2016-178528, and 2017-071020, which are priority documents of the present application.

### Advantageous Effects of Invention

A DNA library can be produced in a very convenient manner by the method for producing a DNA library according to the present invention because the method is based on a nucleic acid amplification method using random primers. In addition, reproducibility of a nucleic acid fragment to be amplified is excellent in the method for producing a DNA library according to the present invention even though the method is a nucleic acid amplification method using random primers. Therefore, according to the method for producing a DNA library of the present invention, the produced DNA library can be used as a DNA marker and thus can be used for genomic DNA analysis such as genetic linkage analysis.

The method for analyzing genomic DNA with the use of a DNA library according to the present invention involves the use of a DNA library produced in a simple manner with excellent reproducibility. Accordingly, genomic DNA can be analyzed in a cost-effective manner with high accuracy.

### Brief Description of Drawings

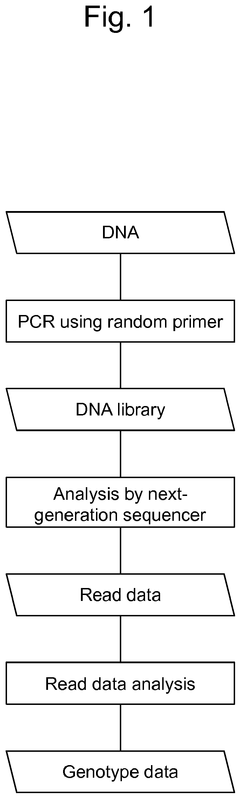

Fig. 1 shows a flow chart demonstrating the method for producing a DNA library and the method for genomic DNA analysis with the use of the DNA library according to the present invention.

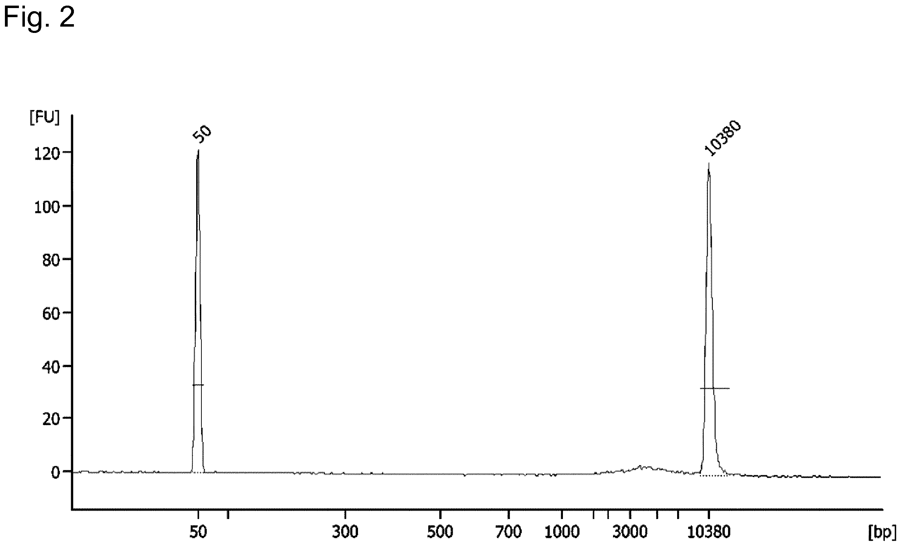

Fig. 2 shows a characteristic diagram demonstrating a correlation between an amplified fragment length and a fluorescence unit (FU) in which the amplified fragment length is determined based on an electrophoretic pattern of the DNA library amplified via PCR using DNA of the sugarcane variety NiF8 as a template under general conditions.

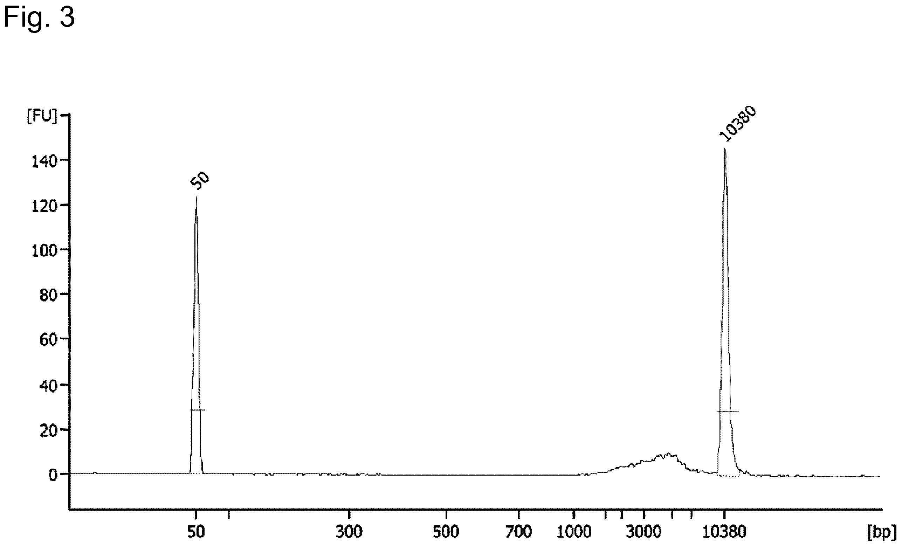

Fig. 3 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template at an annealing temperature of 45°C.

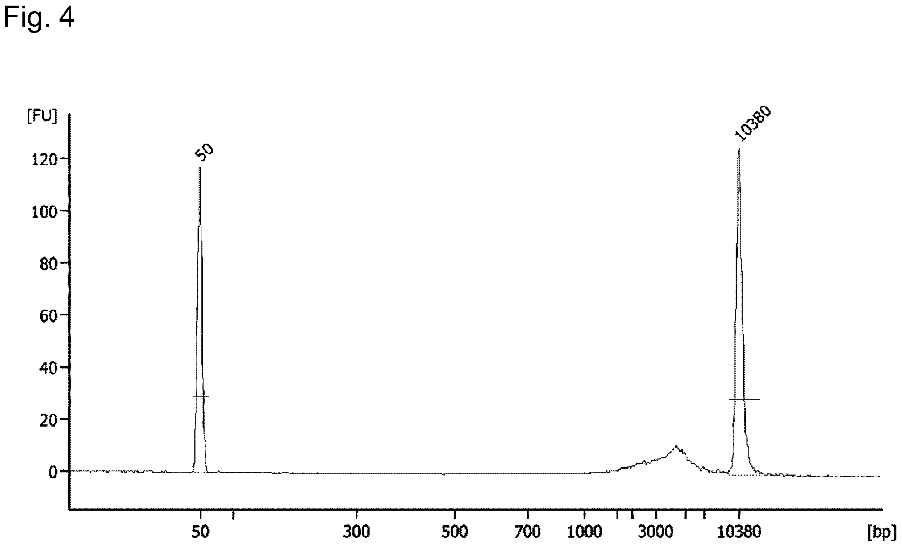

Fig. 4 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template at an annealing temperature of 40°C.

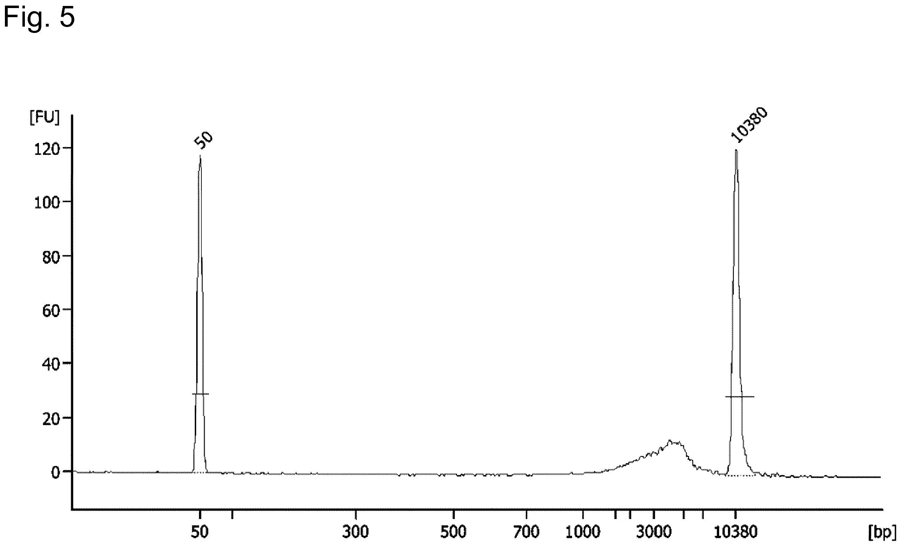

Fig. 5 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template at an annealing temperature of 37°C.

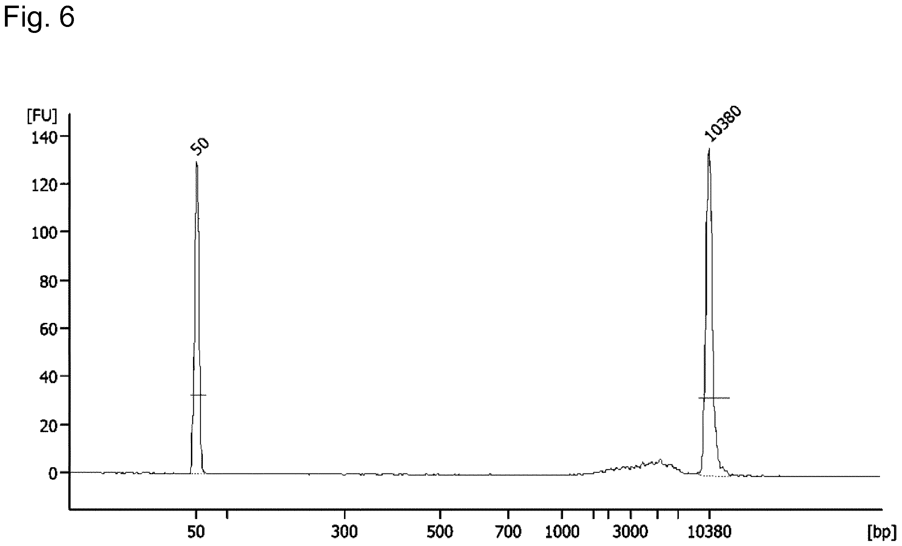

Fig. 6 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and 2.5 units of an enzyme.

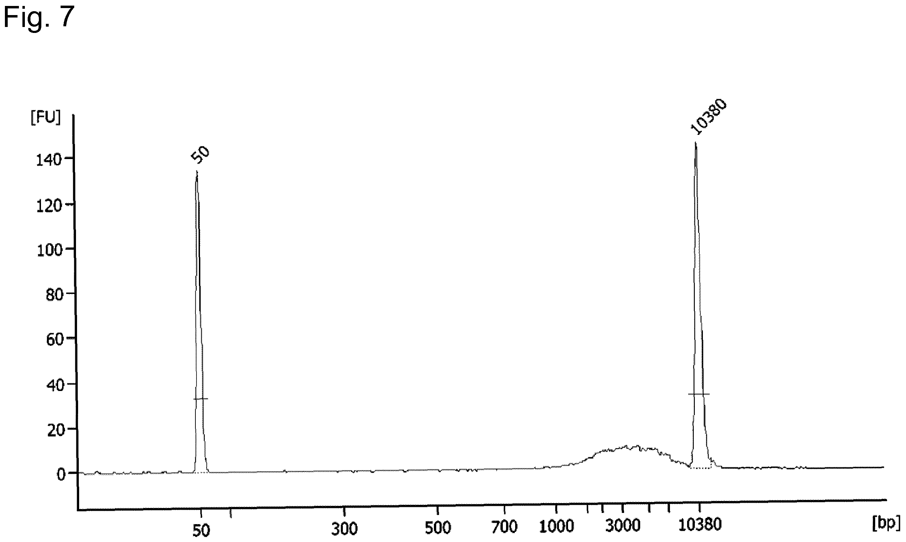

Fig. 7 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and 12.5 units of an enzyme.

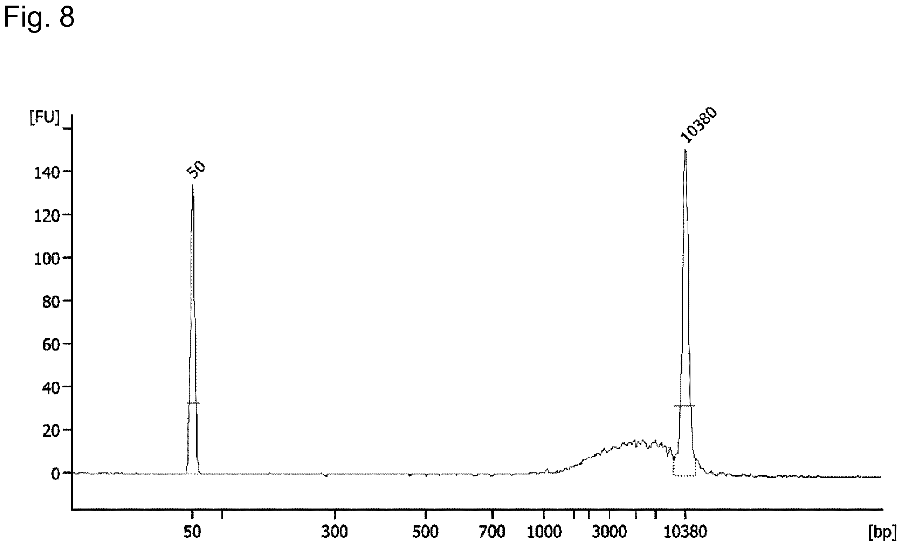

Fig. 8 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and MgCl₂ at the concentration doubled from the original level.

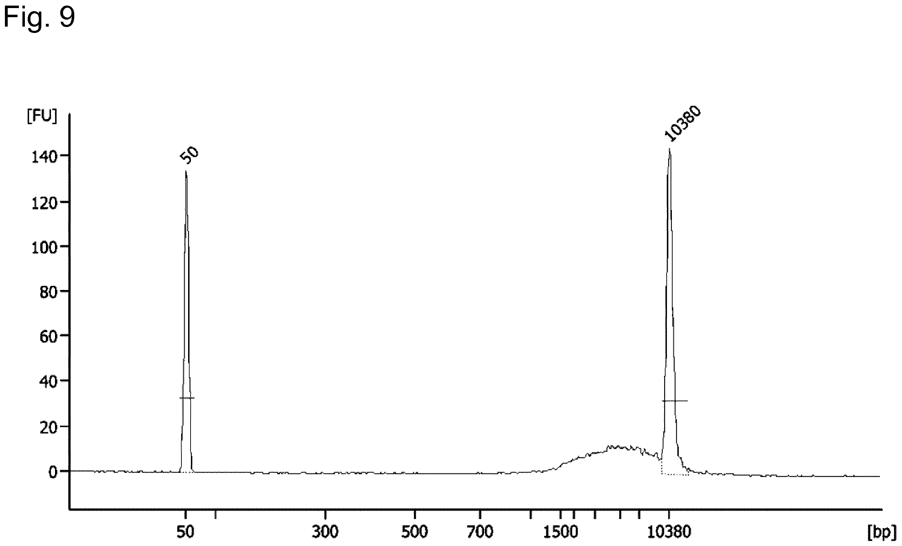

Fig. 9 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and MgCl₂ at the concentration tripled from the original level.

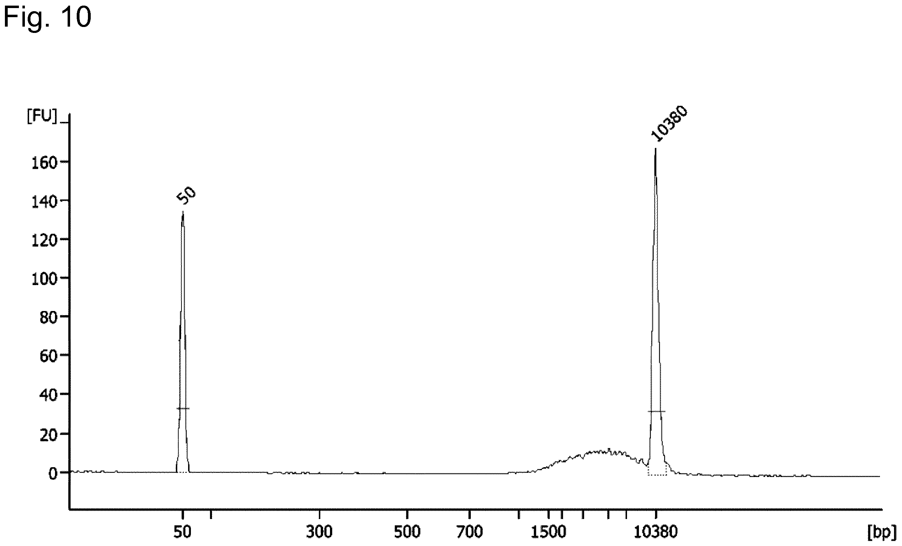

Fig. 10 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and MgCl₂ at the concentration quadrupled from the original level.

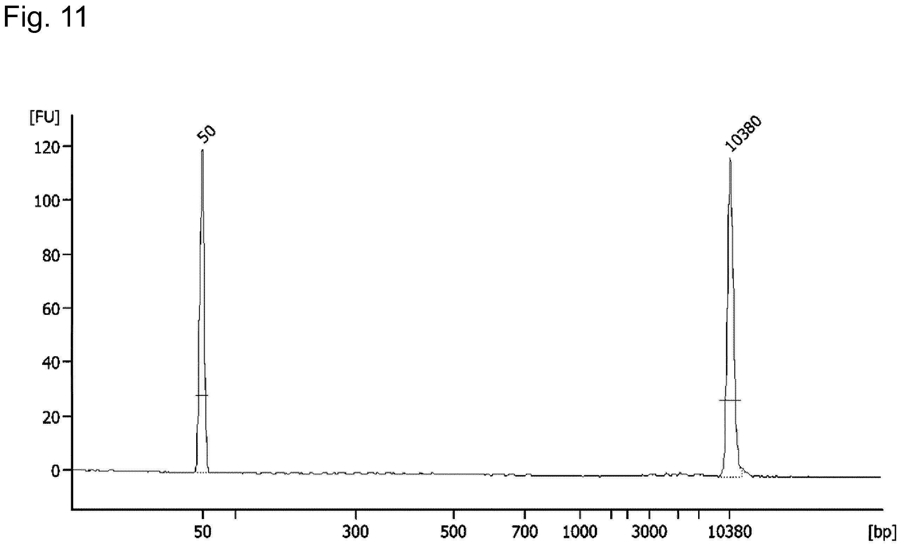

Fig. 11 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 8 nucleotides.

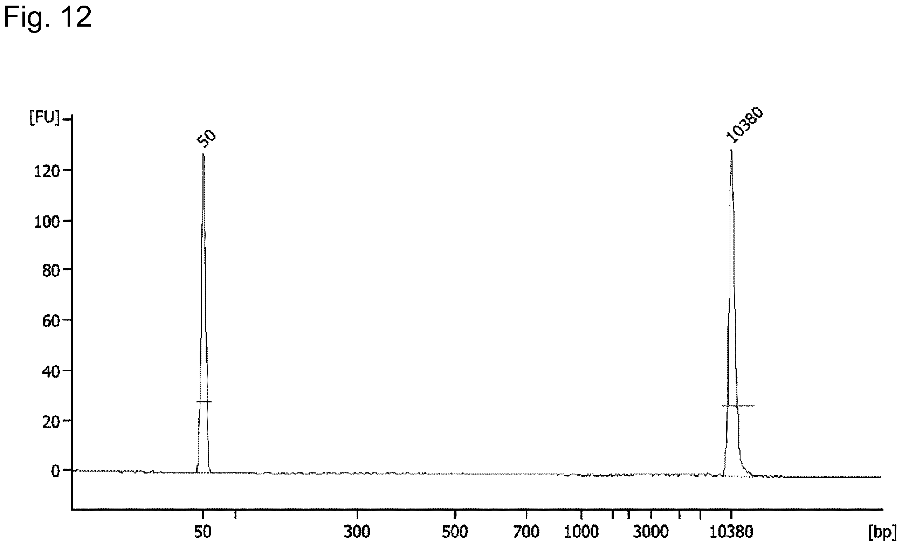

Fig. 12 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 9 nucleotides.

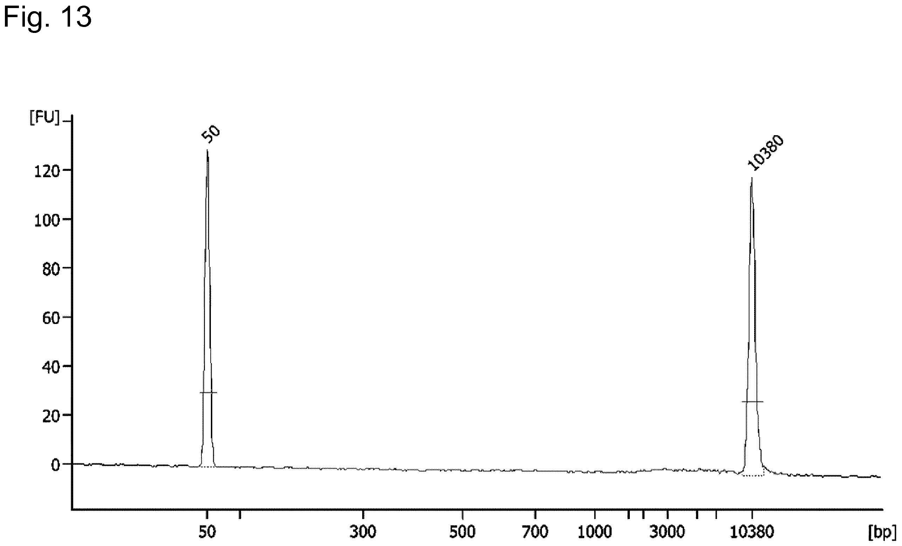

Fig. 13 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 11 nucleotides.

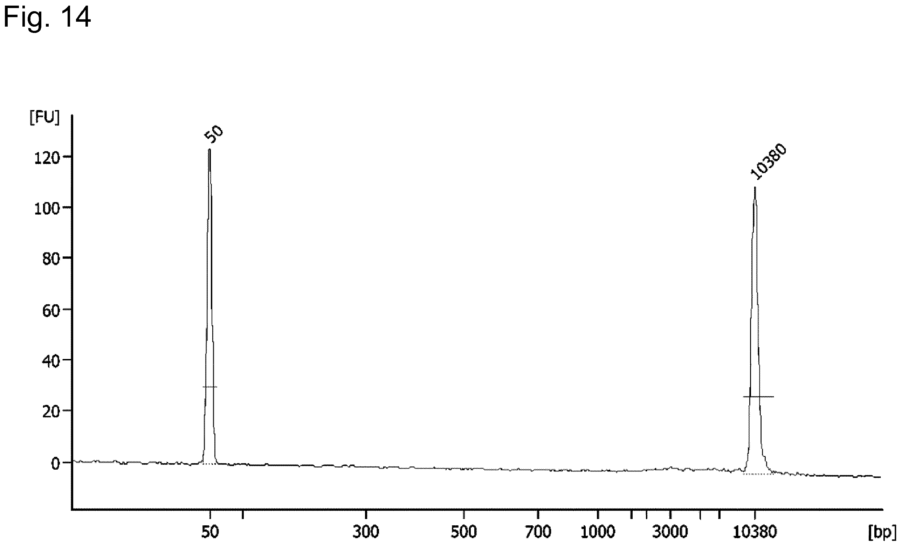

Fig. 14 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 12 nucleotides.

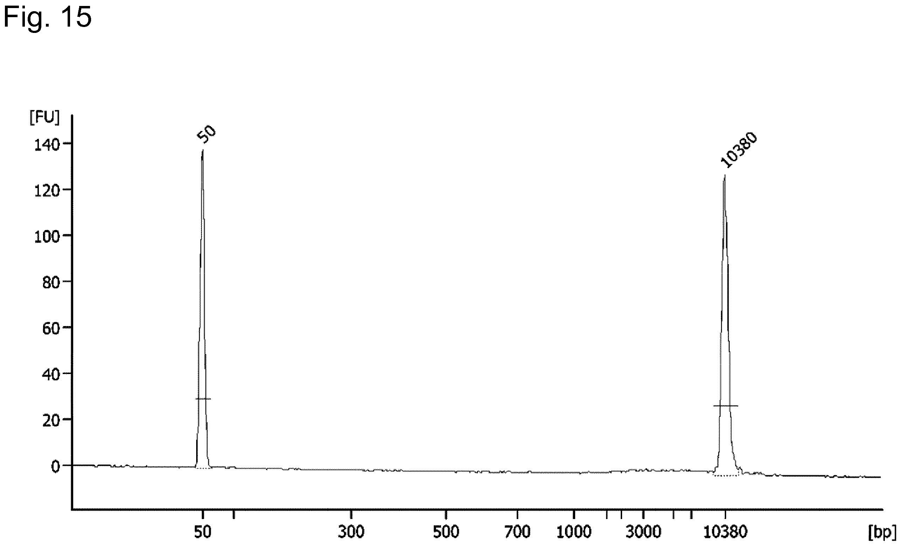

Fig. 15 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 14 nucleotides.

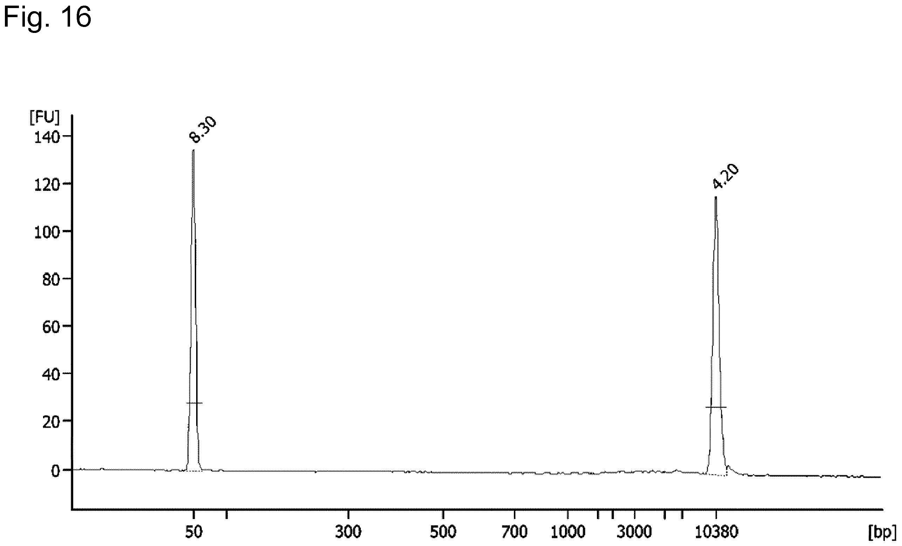

Fig. 16 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 16 nucleotides.

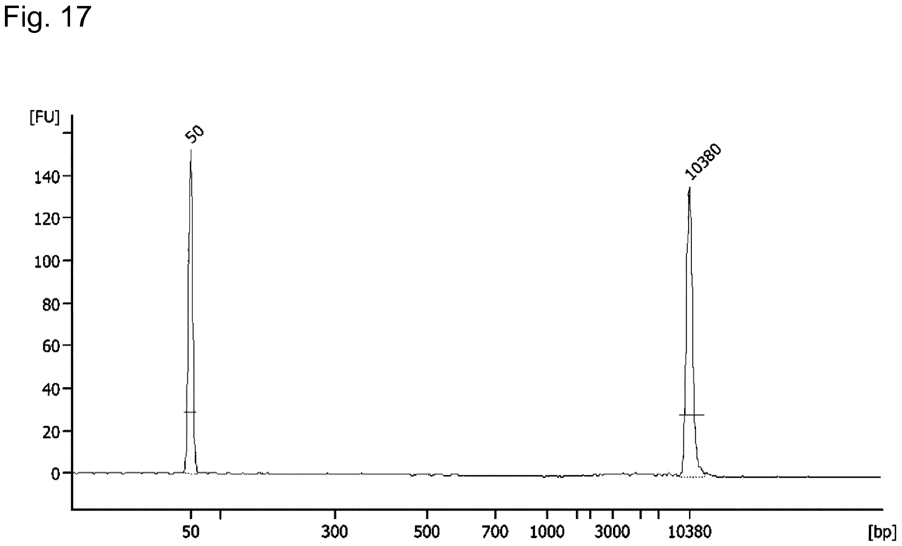

Fig. 17 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 18 nucleotides.

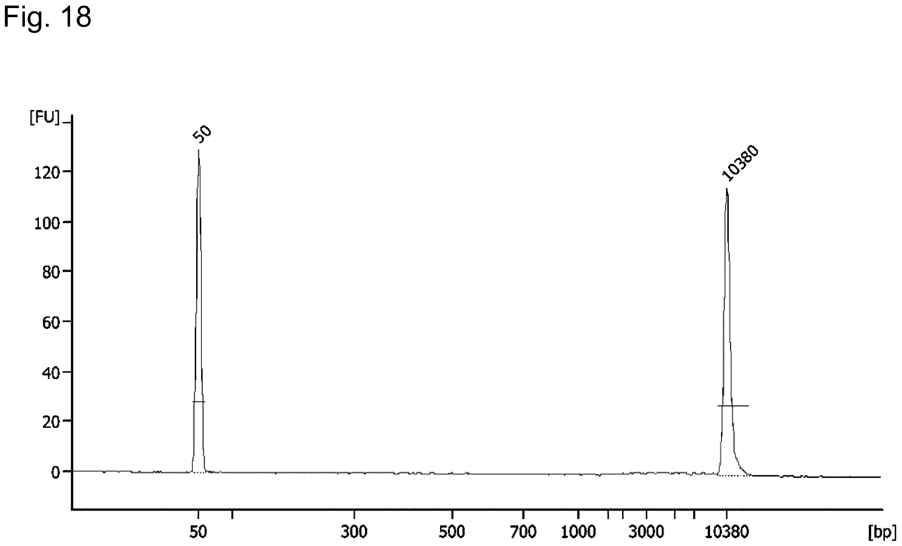

Fig. 18 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 20 nucleotides.

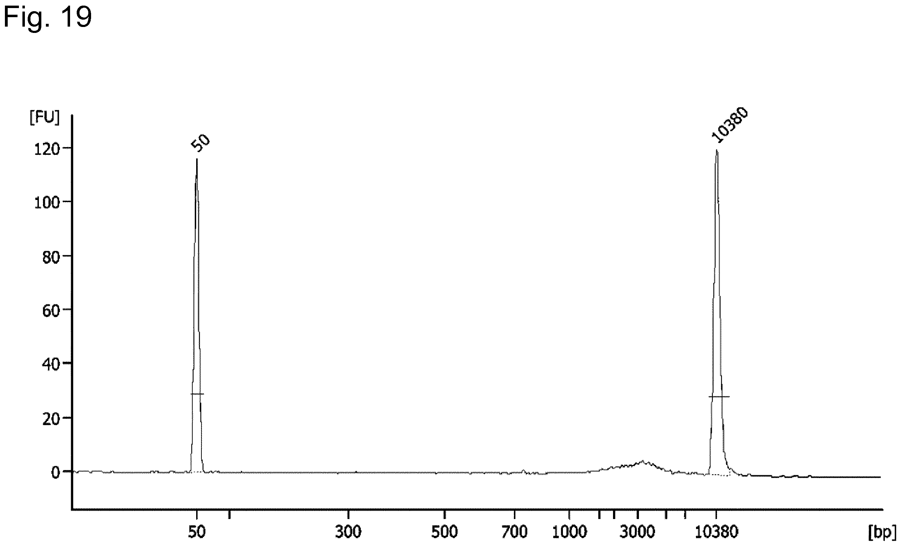

Fig. 19 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 2 µM.

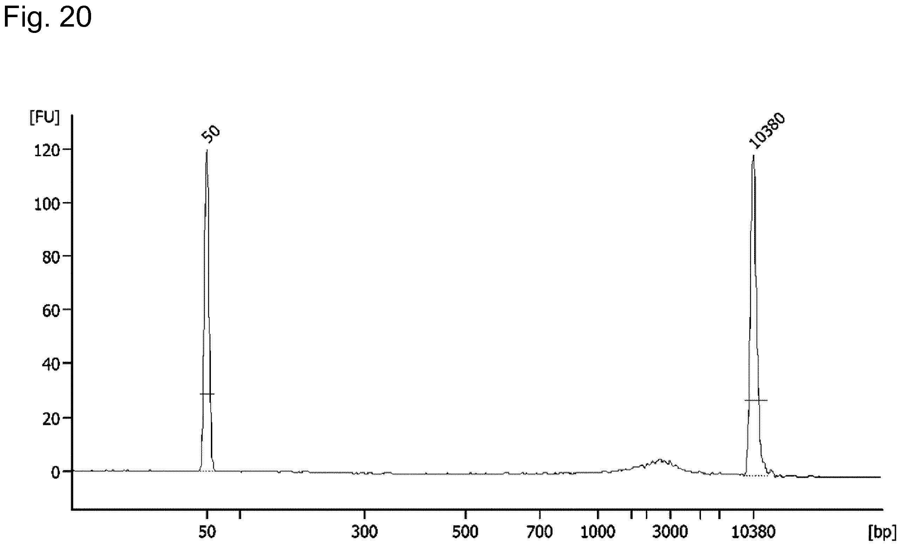

Fig. 20 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 4 µM.

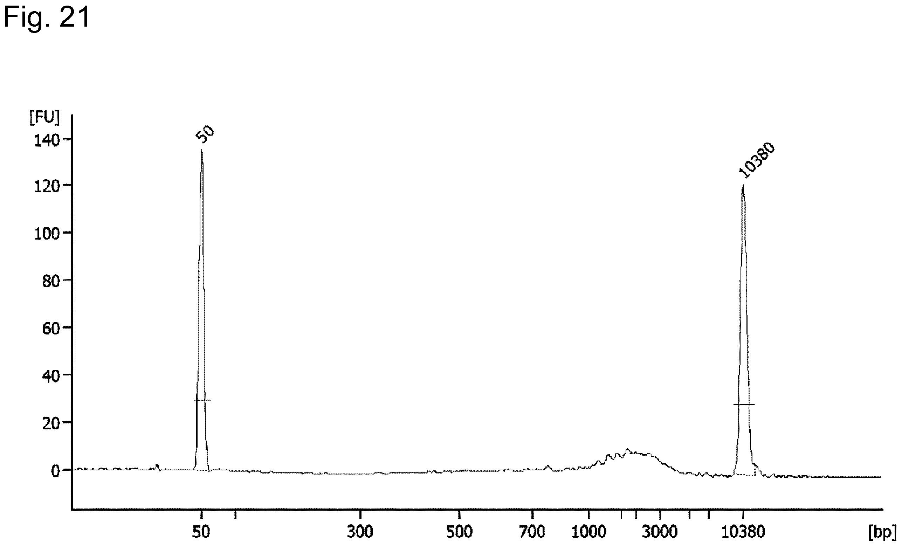

Fig. 21 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 6 µM.

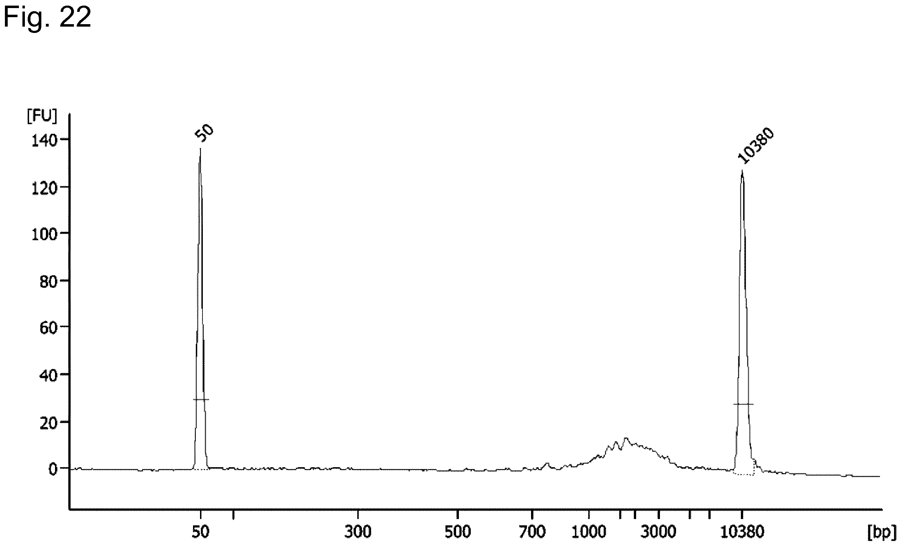

Fig. 22 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 6 µM.

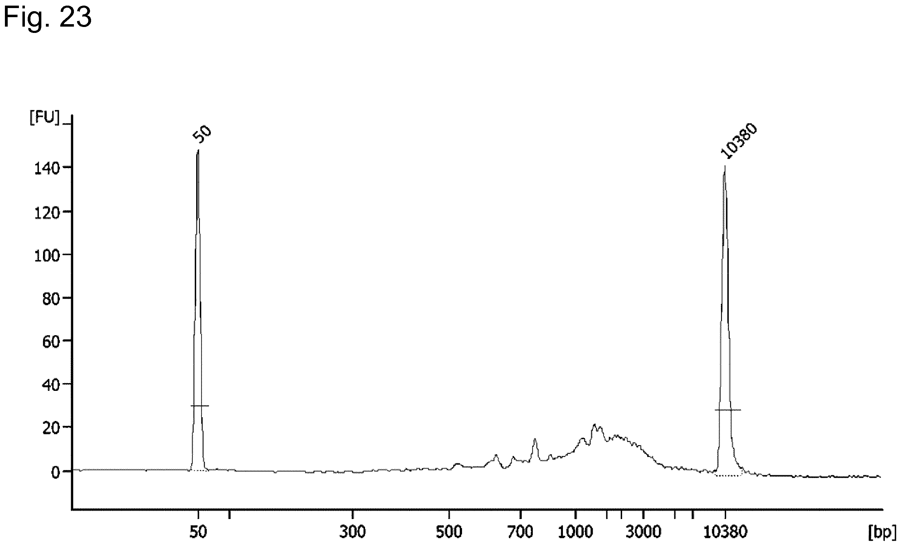

Fig. 23 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 8 µM.

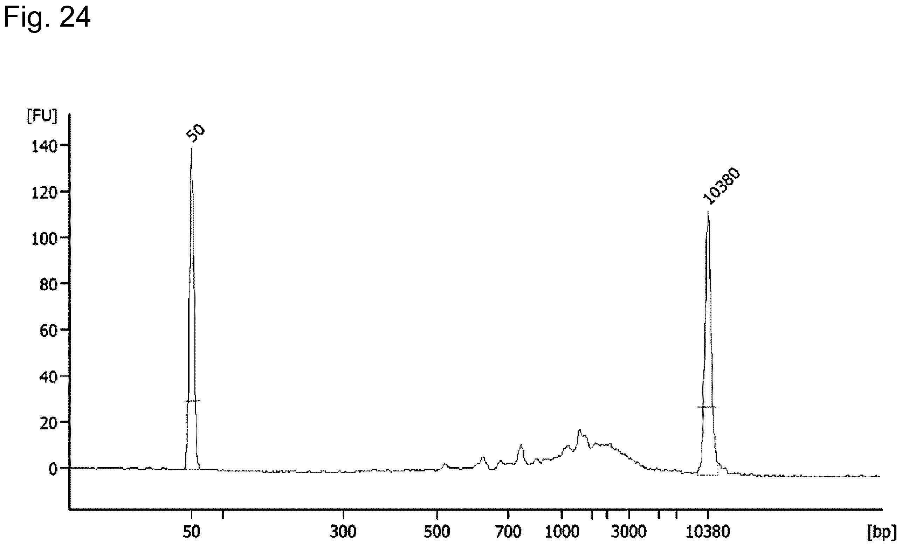

Fig. 24 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 8 µM.

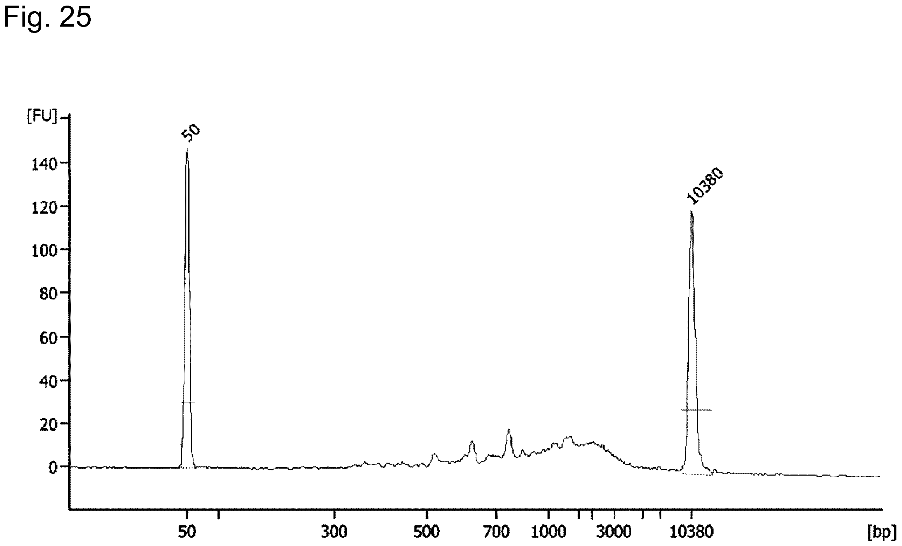

Fig. 25 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 10 µM.

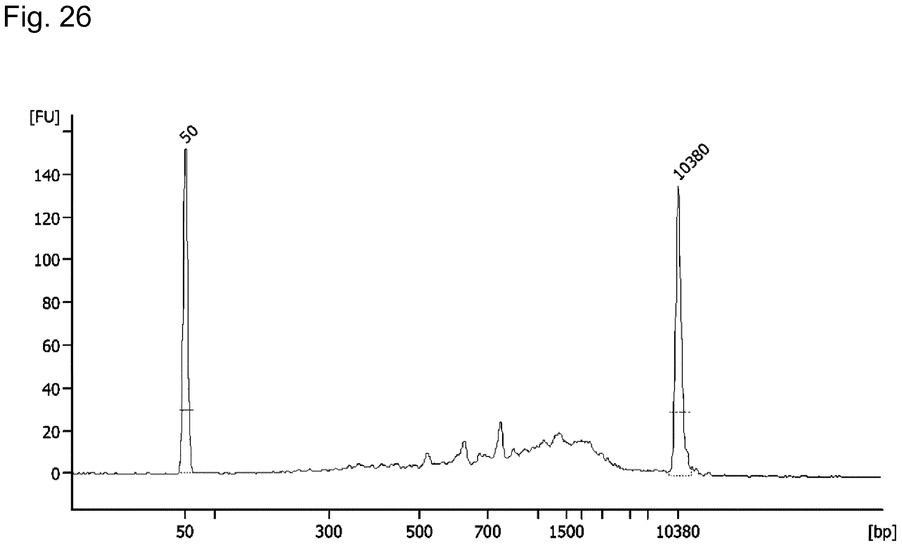

Fig. 26 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 10 µM.

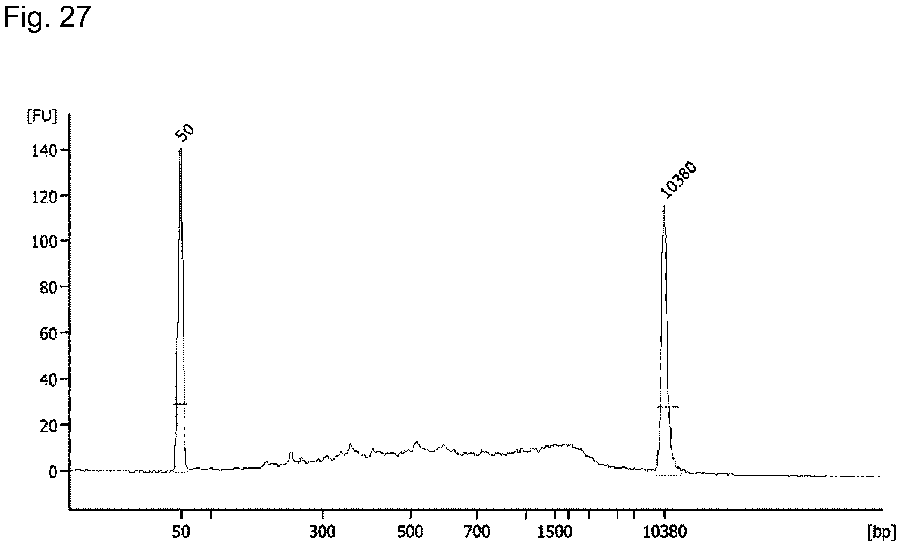

Fig. 27 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 20 µM.

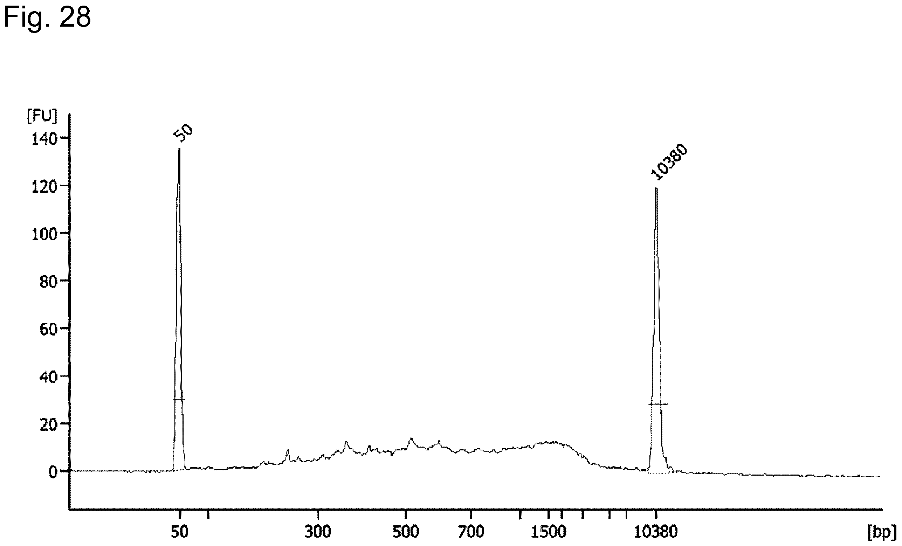

Fig. 28 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 20 µM.

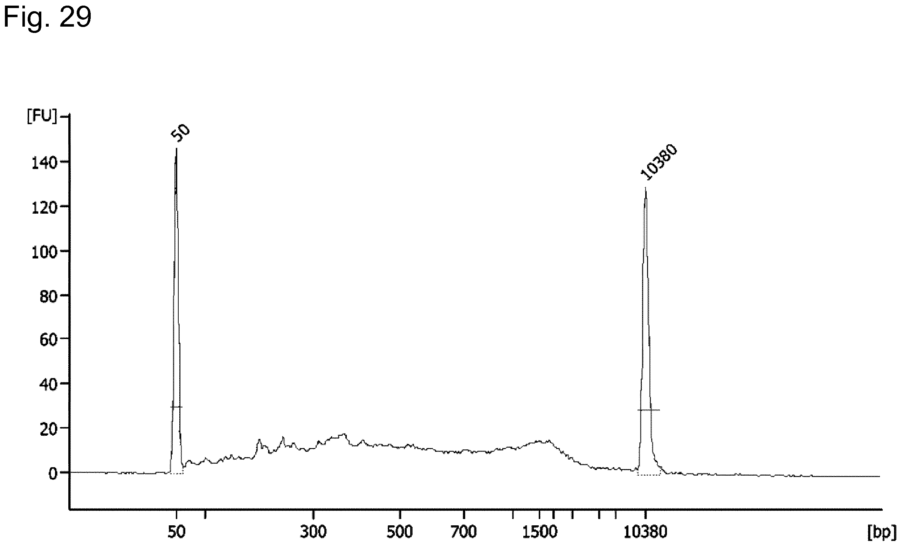

Fig. 29 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 40 µM.

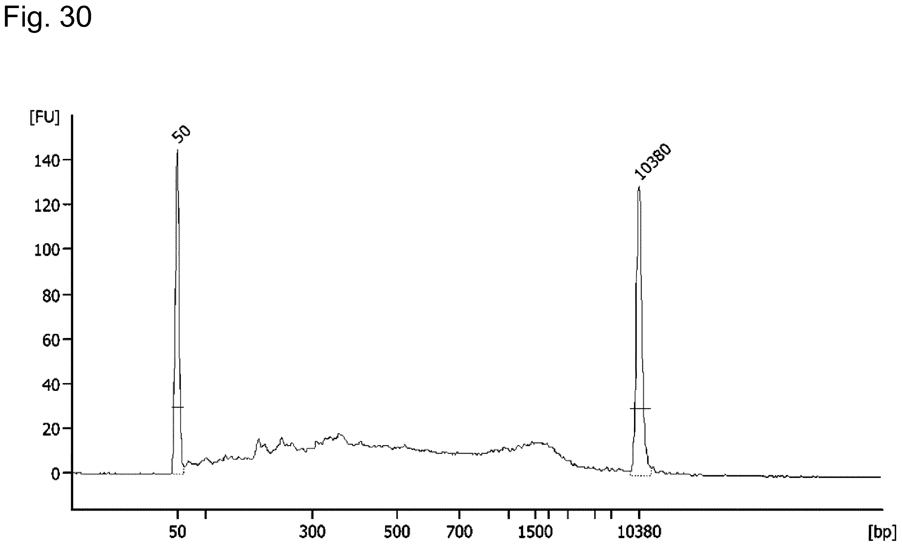

Fig. 30 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 40 µM.

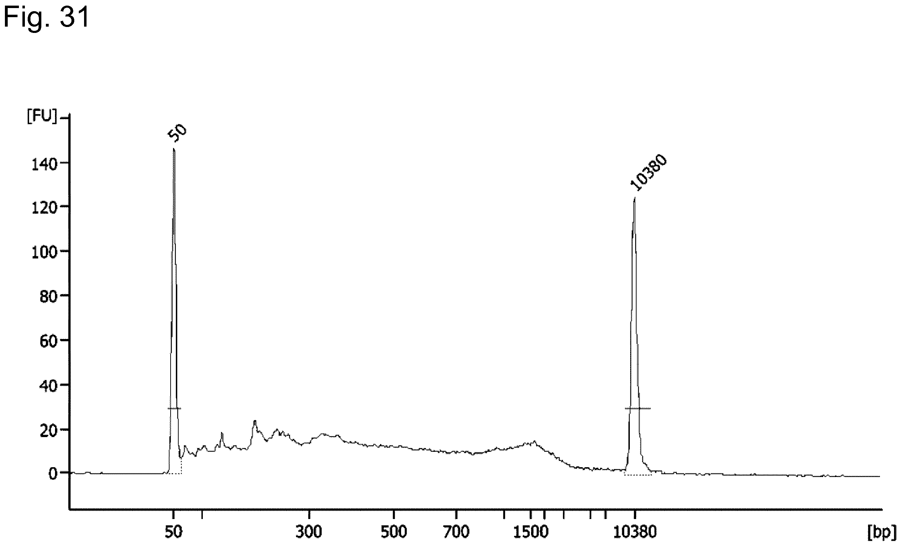

Fig. 31 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 60 µM.

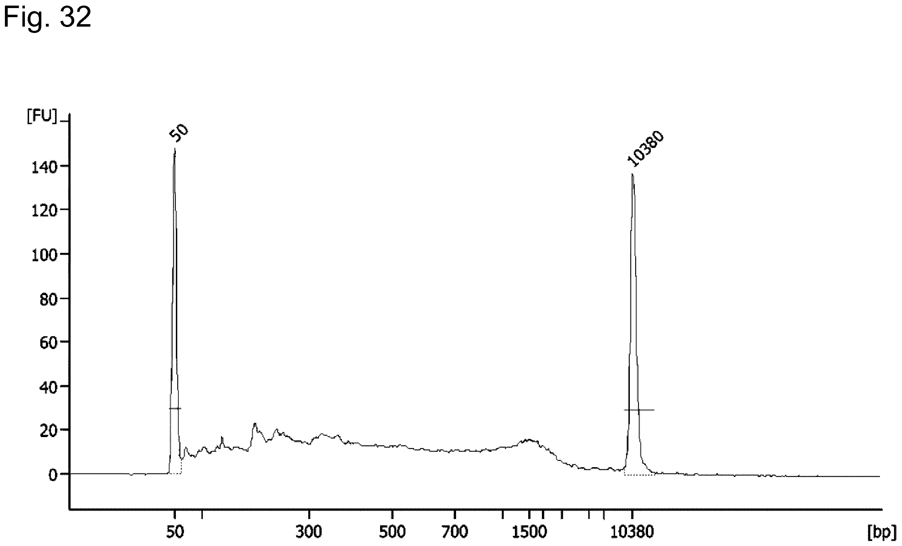

Fig. 32 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 60 µM.

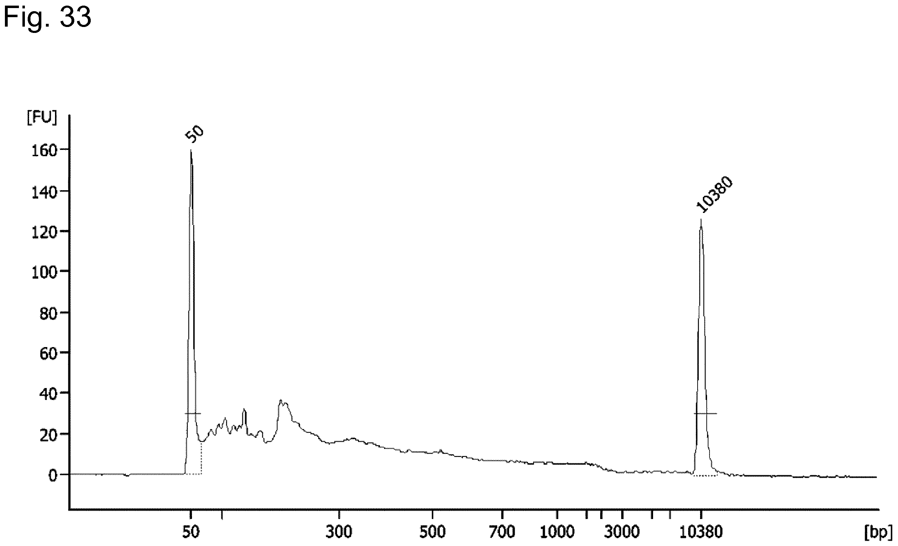

Fig. 33 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 100 µM.

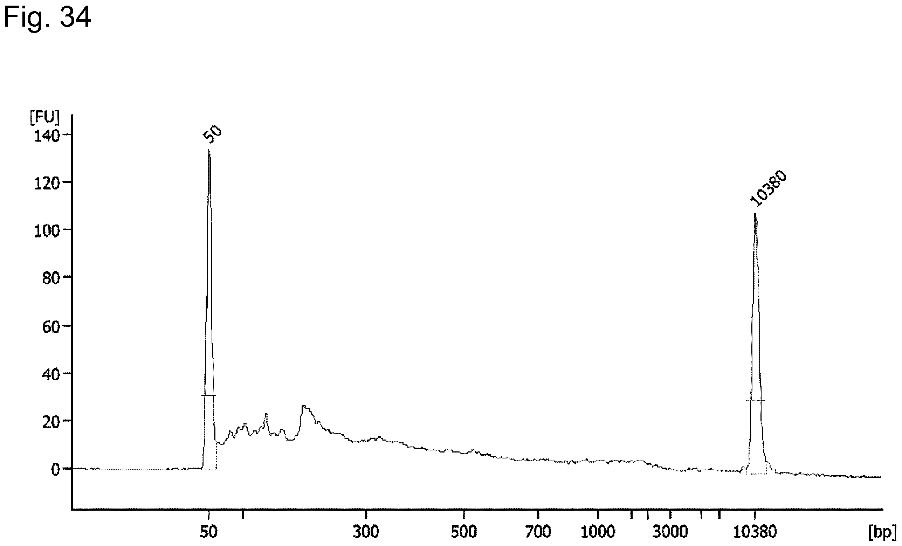

Fig. 34 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 100 µM.

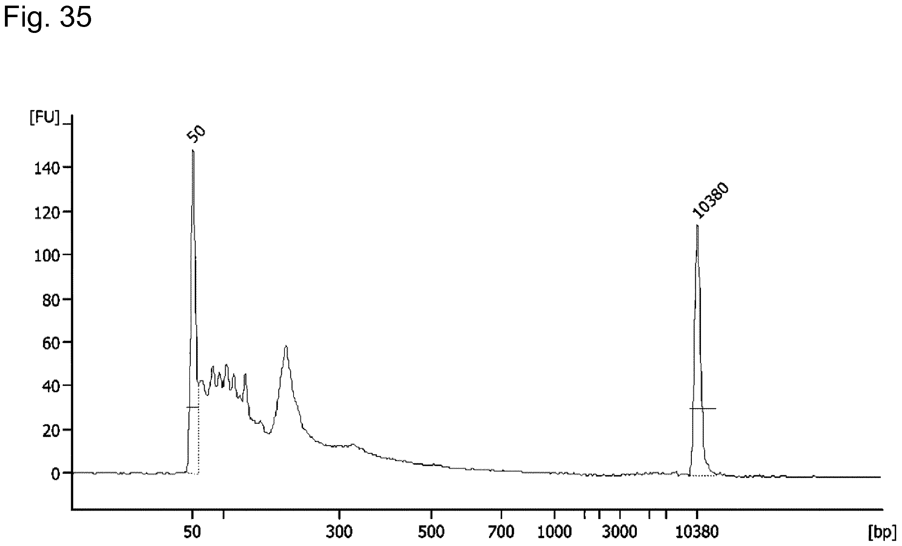

Fig. 35 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 200 µM.

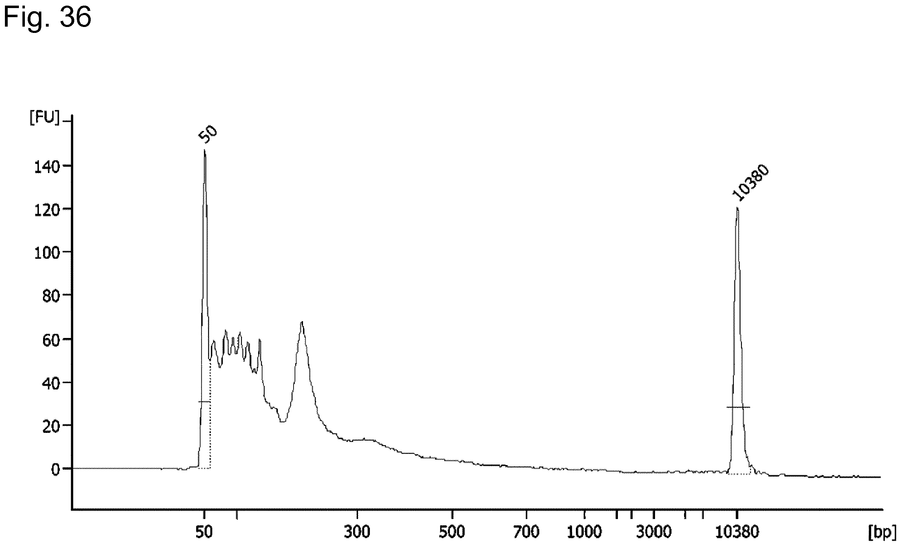

Fig. 36 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 200 µM.

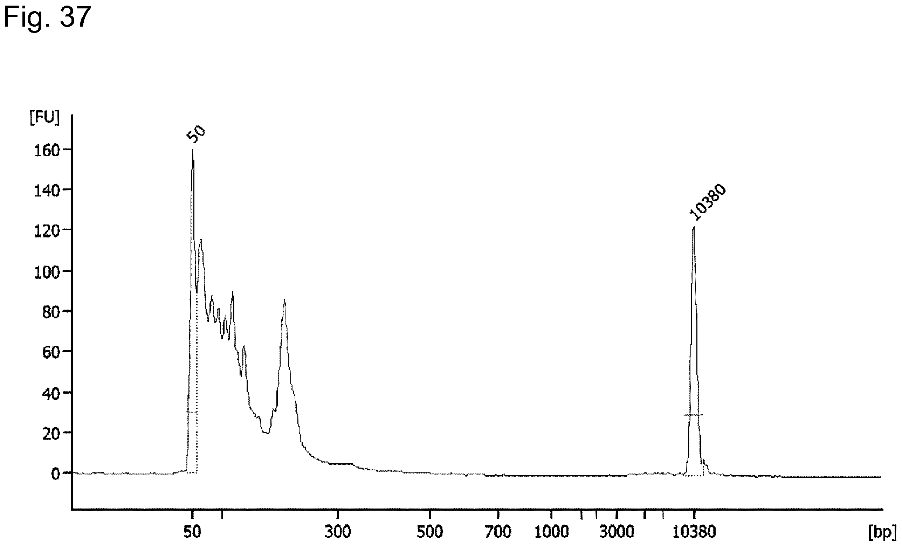

Fig. 37 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 300 µM.

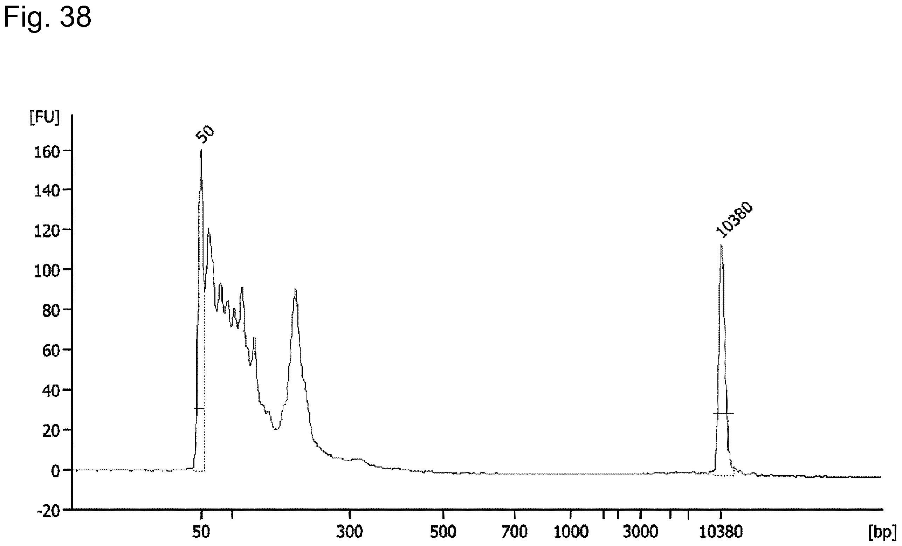

Fig. 38 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 300 µM.

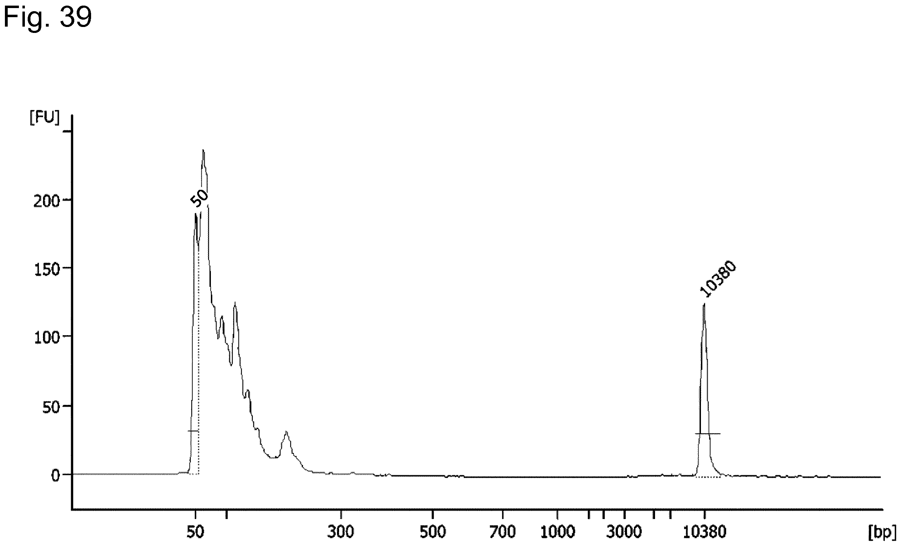

Fig. 39 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 400 µM.

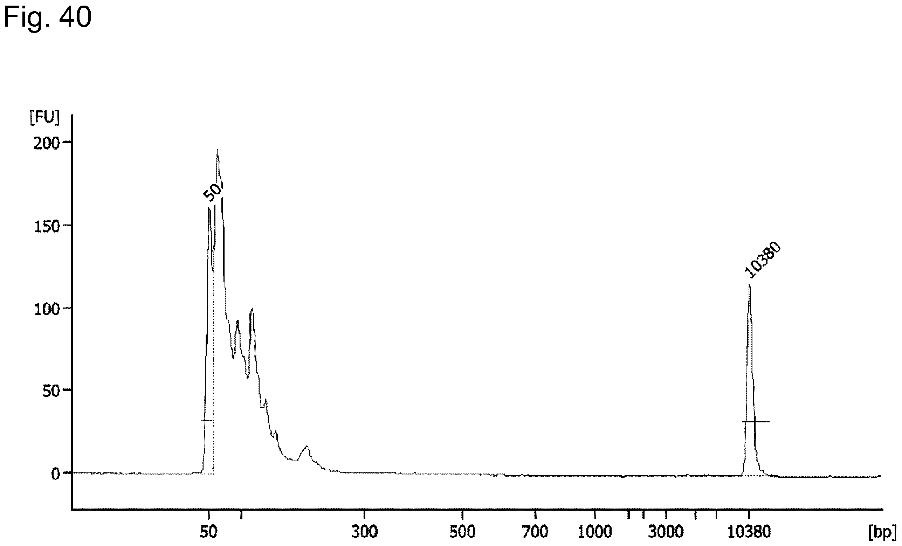

Fig. 40 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 400 µM.

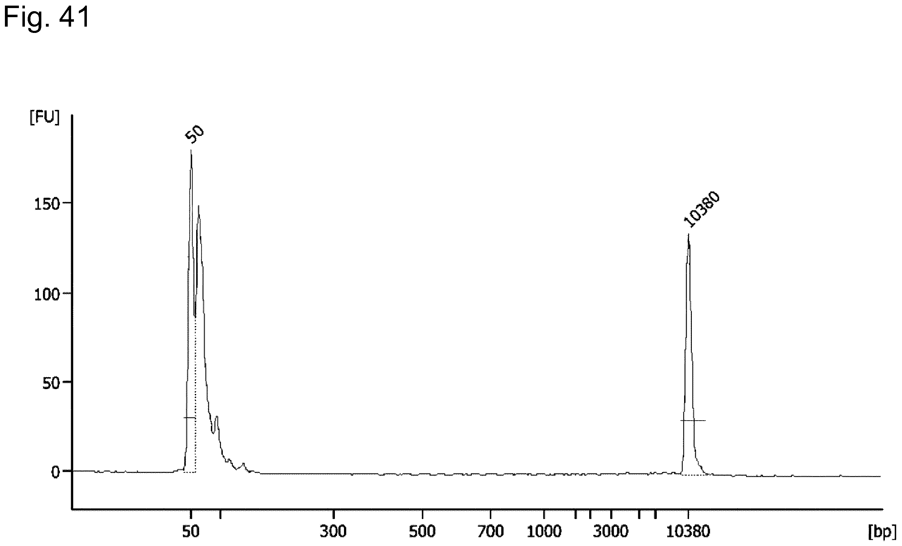

Fig. 41 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 500 µM.

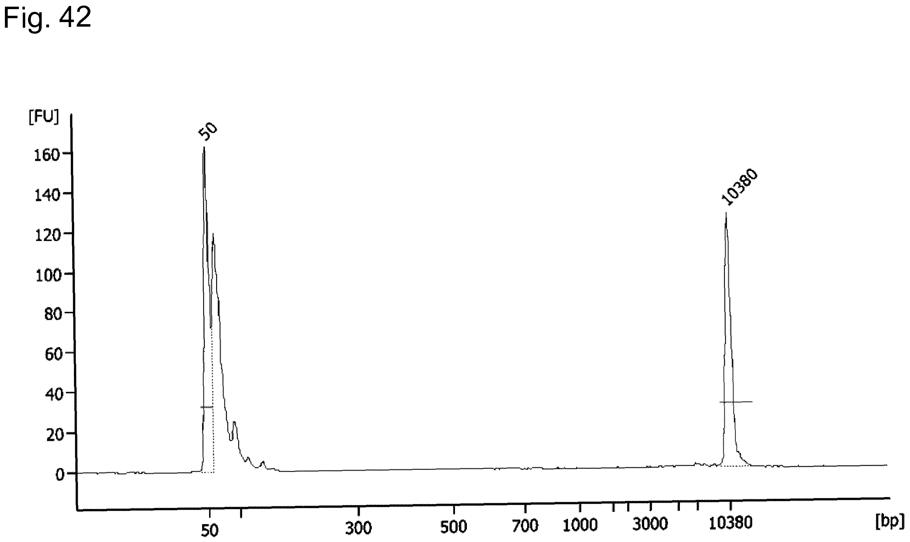

Fig. 42 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 500 µM.

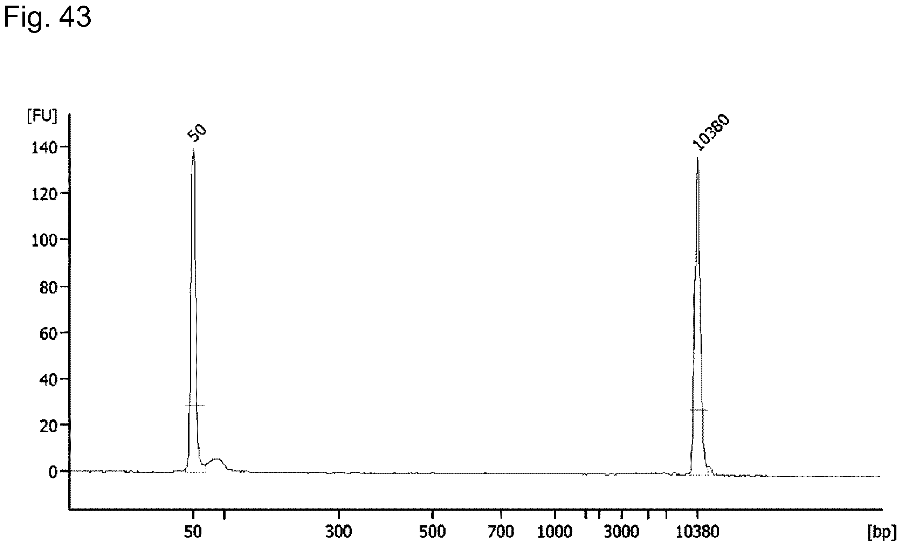

Fig. 43 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 600 µM.

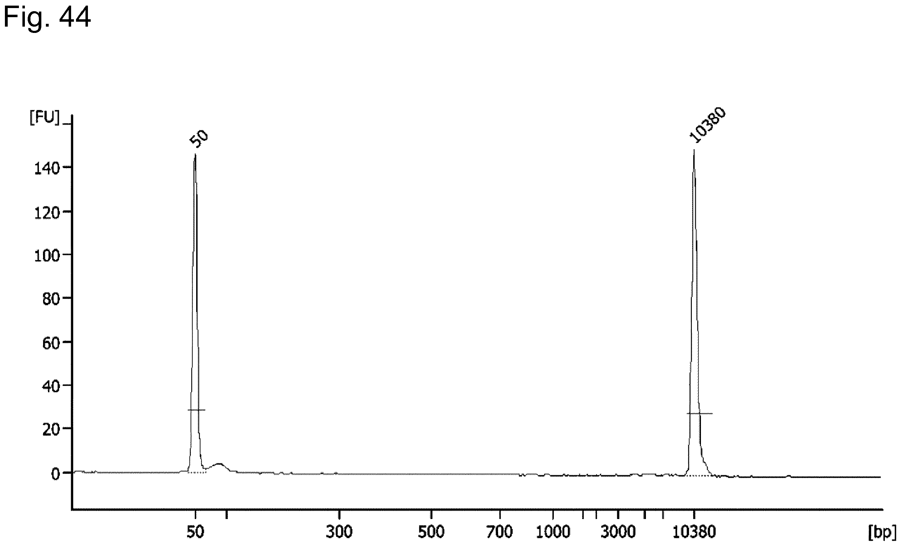

Fig. 44 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 700 µM.

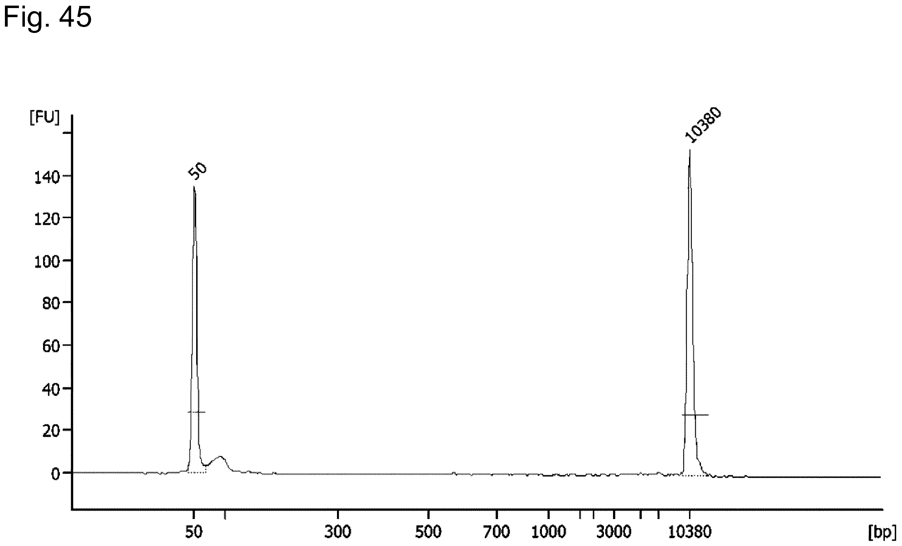

Fig. 45 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 800 µM.

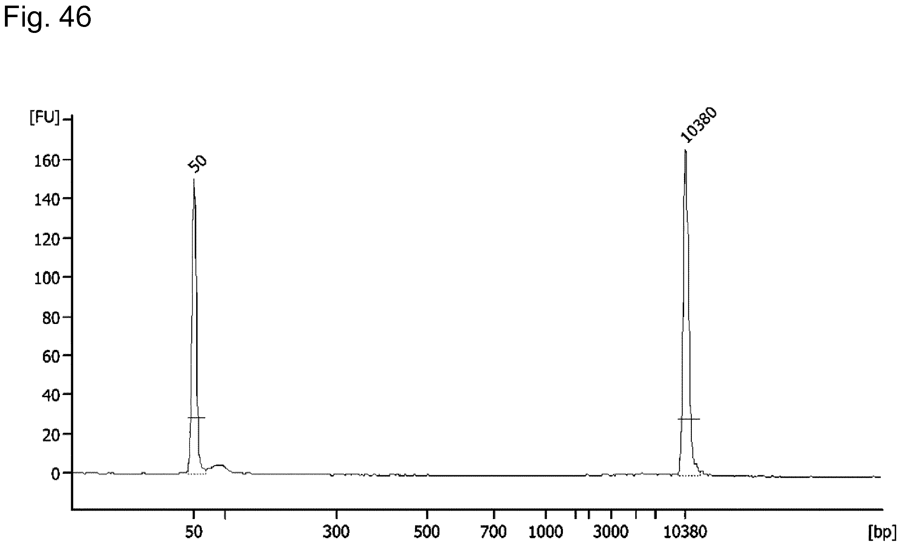

Fig. 46 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 900 µM.

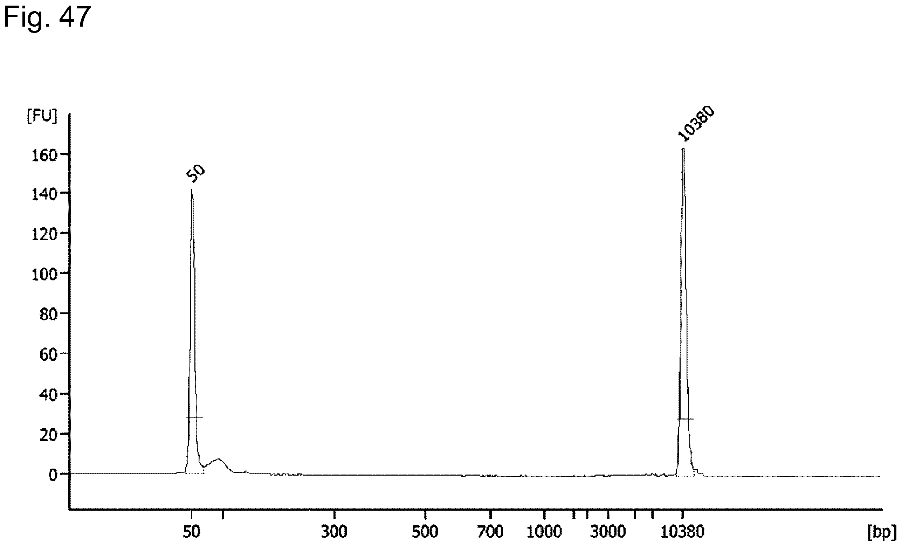

Fig. 47 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 1000 µM.

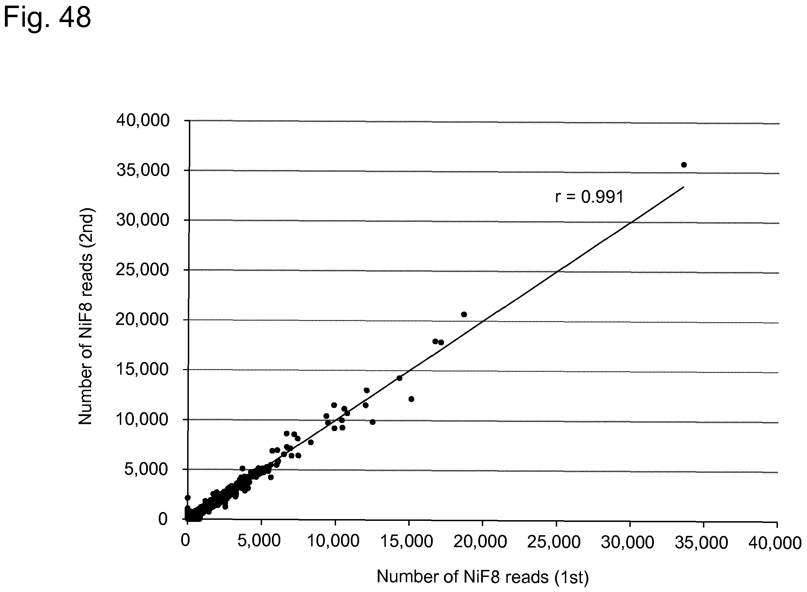

Fig. 48 shows a characteristic diagram demonstrating the results of MiSeq analysis of a DNA library amplified using DNA of the sugarcane variety NiF8 as a template and a random primer.

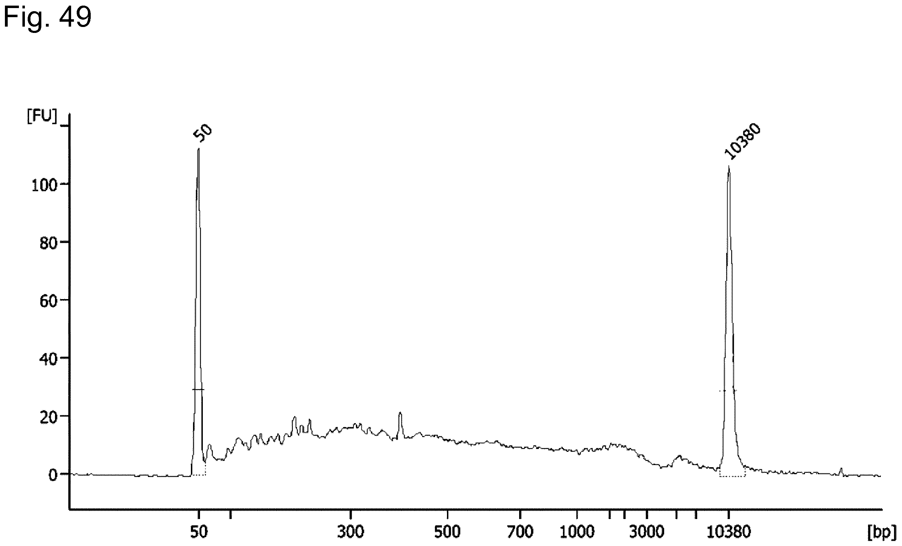

Fig. 49 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the rice variety Nipponbare as a template and a random primer.

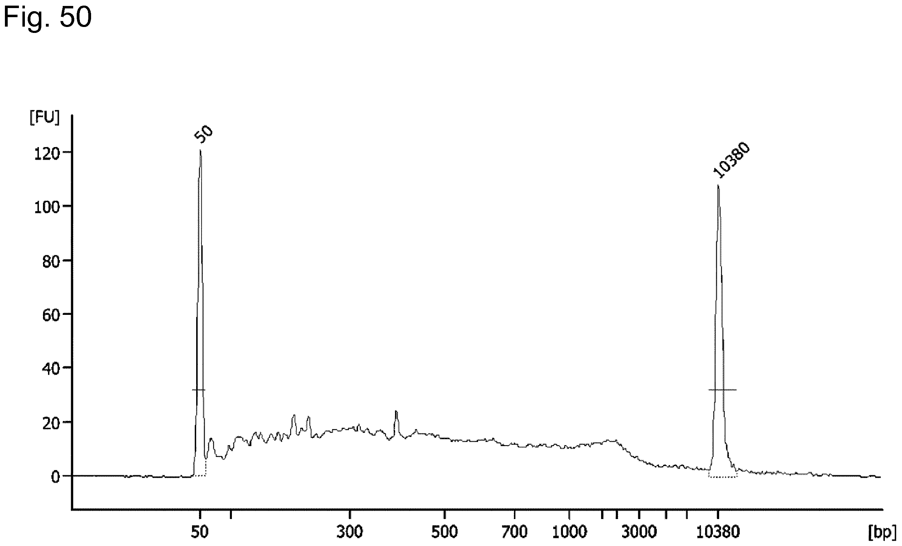

Fig. 50 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the rice variety Nipponbare as a template and a random primer.

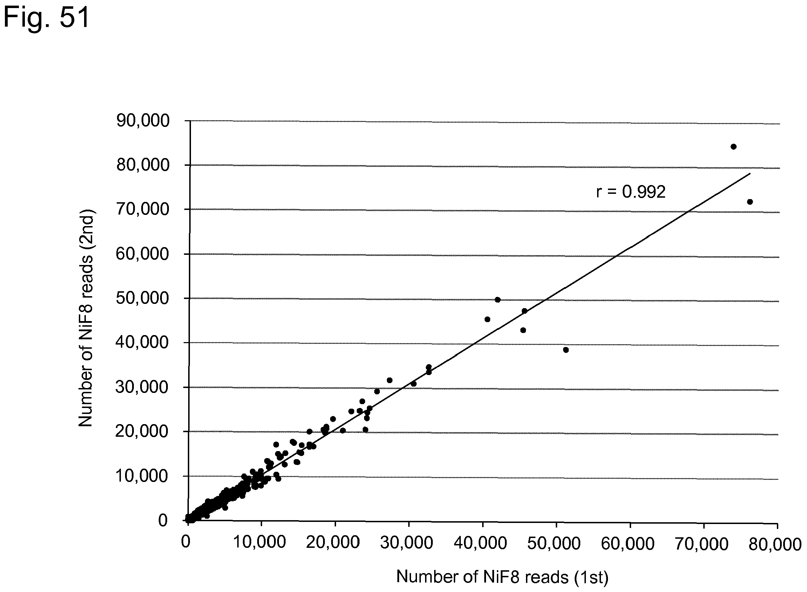

Fig. 51 shows a characteristic diagram demonstrating the results of MiSeq analysis of a DNA library amplified using DNA of the rice variety Nipponbare as a template and a random primer.

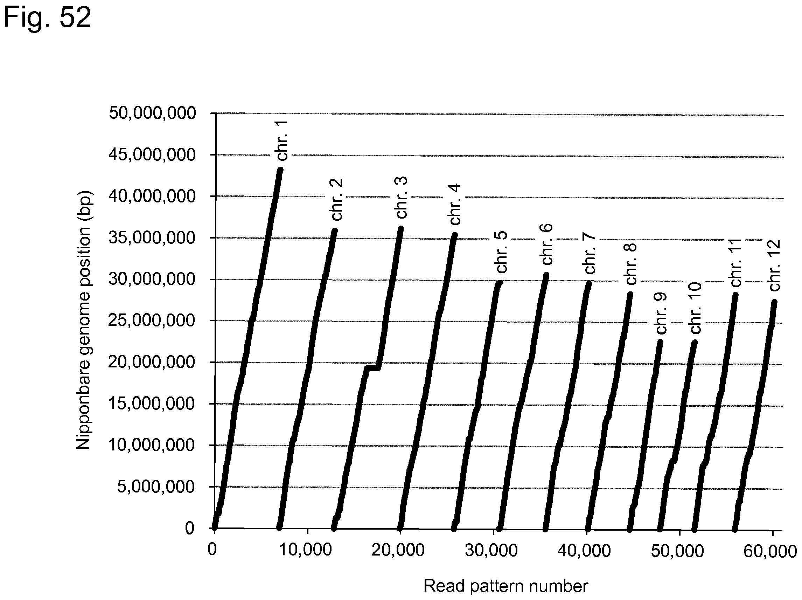

Fig. 52 shows a characteristic diagram demonstrating positions of MiSeq read patterns in the genome information of the rice variety Nipponbare.

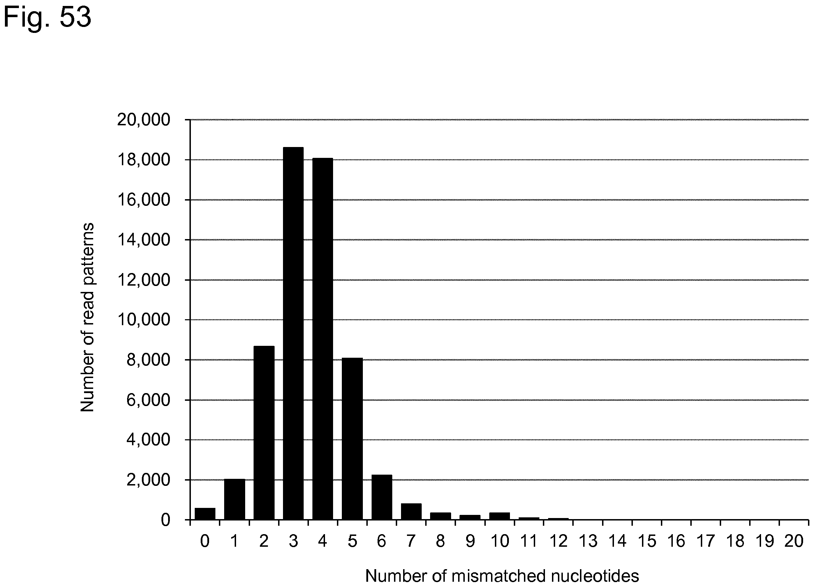

Fig. 53 shows a characteristic diagram demonstrating the frequency distribution of the number of mismatched nucleotides between the random primer and the rice genome.

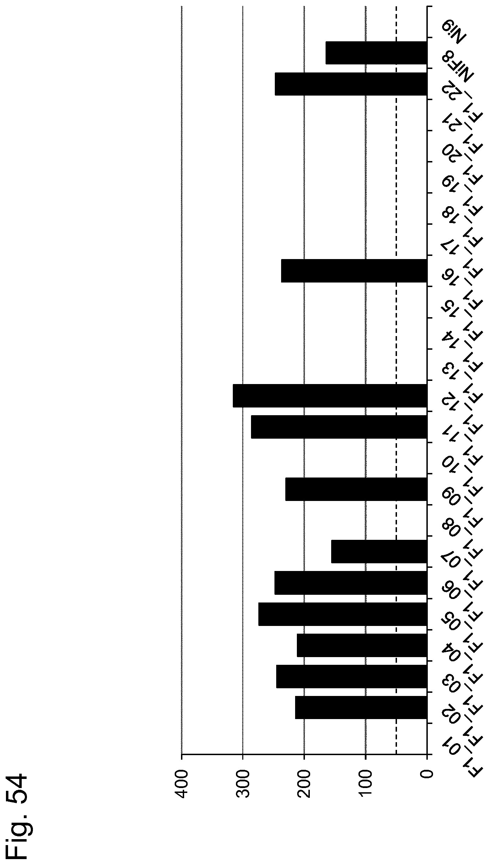

Fig. 54 shows a characteristic diagram demonstrating the number of reads of the sugarcane varieties NiF8 and Ni9 and hybrid progeny lines thereof at the marker N80521152.

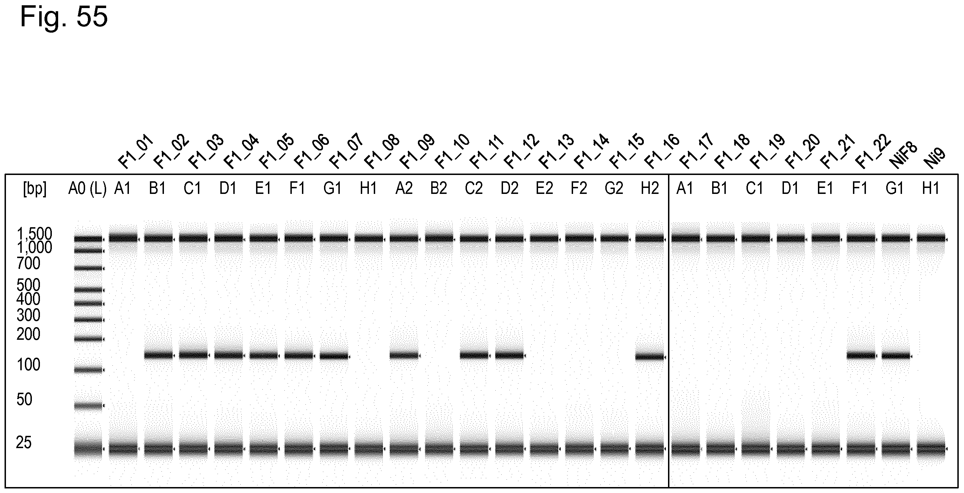

Fig. 55 shows a photograph demonstrating electrophoretic patterns of the sugarcane varietiesNiF8 and Ni9 and hybrid progeny lines thereof at the PCR marker N80521152.

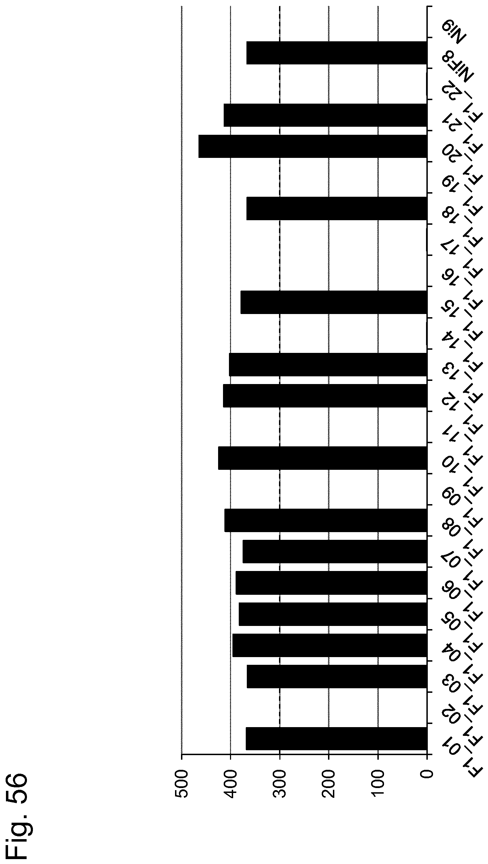

Fig. 56 shows a characteristic diagram demonstrating the number of reads of the sugarcane varieties NiF8 and Ni9 and hybrid progeny lines thereof at the marker N80997192.

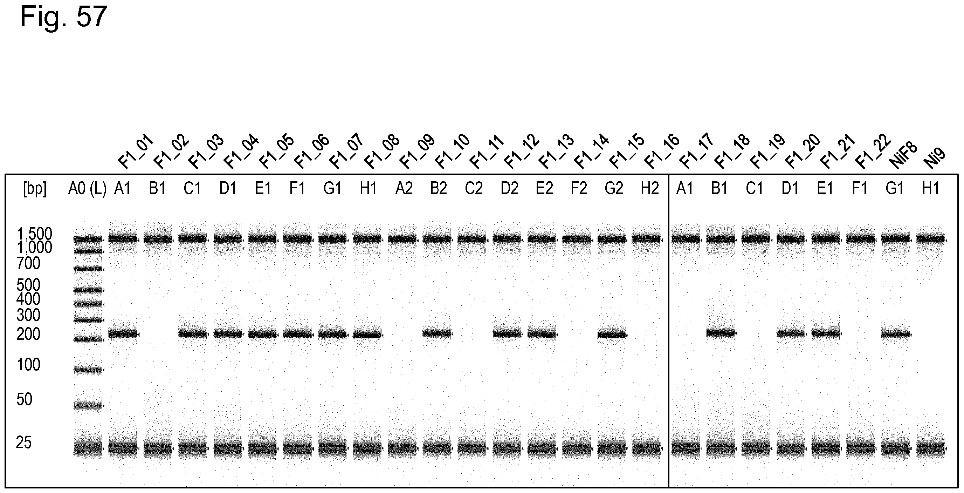

Fig. 57 shows a photograph demonstrating electrophoretic patterns of the sugarcane varietiesNiF8 and Ni9 and hybrid progeny lines thereof at the PCR marker N80997192.

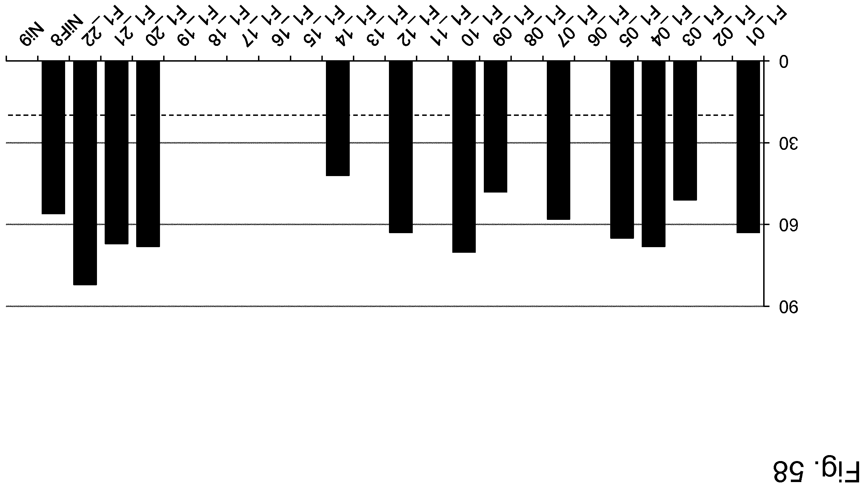

Fig. 58 shows a characteristic diagram demonstrating the number of reads of the sugarcane varieties NiF8 and Ni9 and hybrid progeny lines thereof at the marker N80533142.

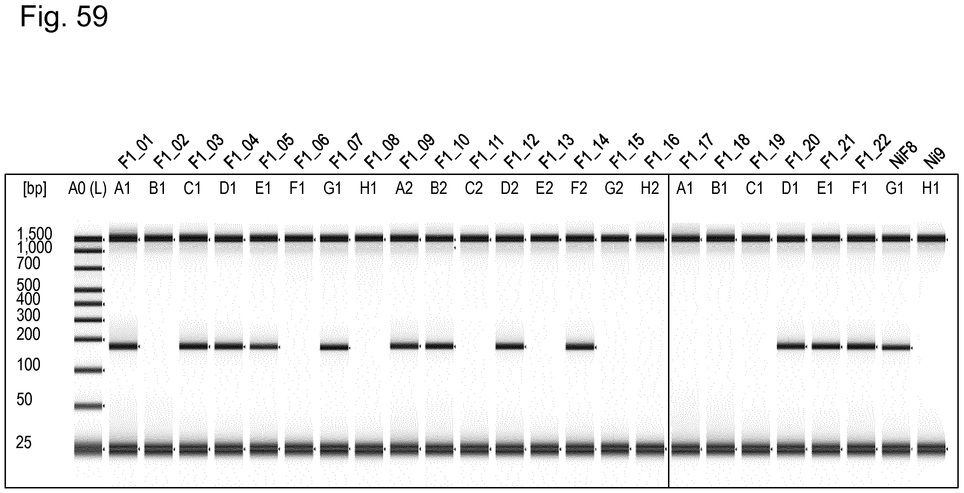

Fig. 59 shows a photograph demonstrating electrophoretic patterns of the sugarcane varietiesNiF8 and Ni9 and hybrid progeny lines thereof at the PCR marker N80533142.

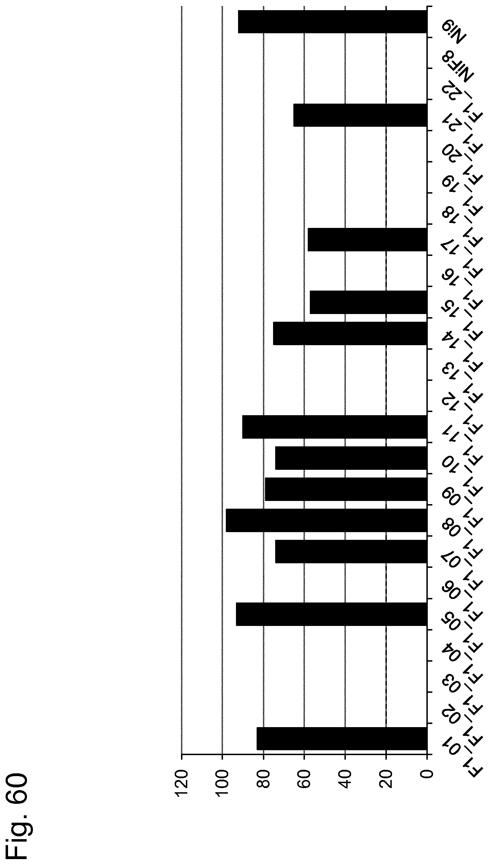

Fig. 60 shows a characteristic diagram demonstrating the number of reads of the sugarcane varieties NiF8 and Ni9 and hybrid progeny lines thereof at the marker N91552391.

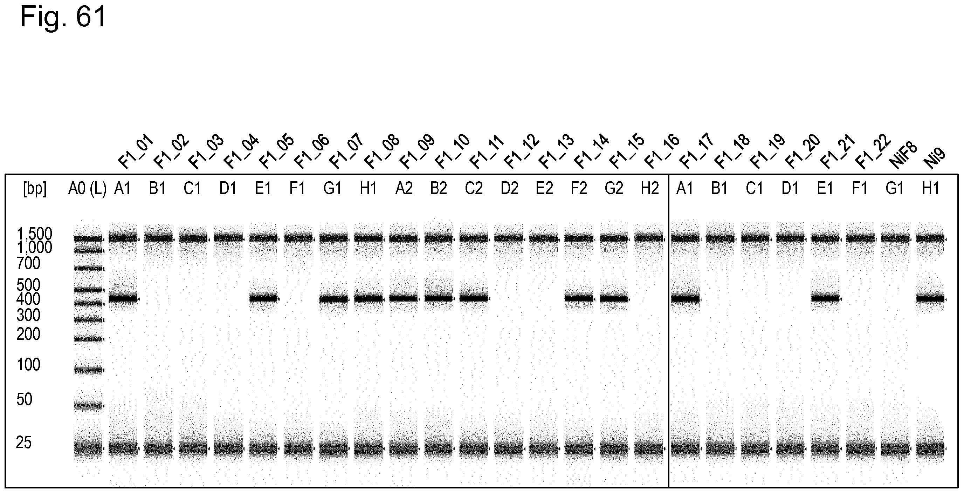

Fig. 61 shows a photograph demonstrating electrophoretic patterns of the sugarcane varieties NiF8 and Ni9 and hybrid progeny lines thereof at the PCR marker N91552391.

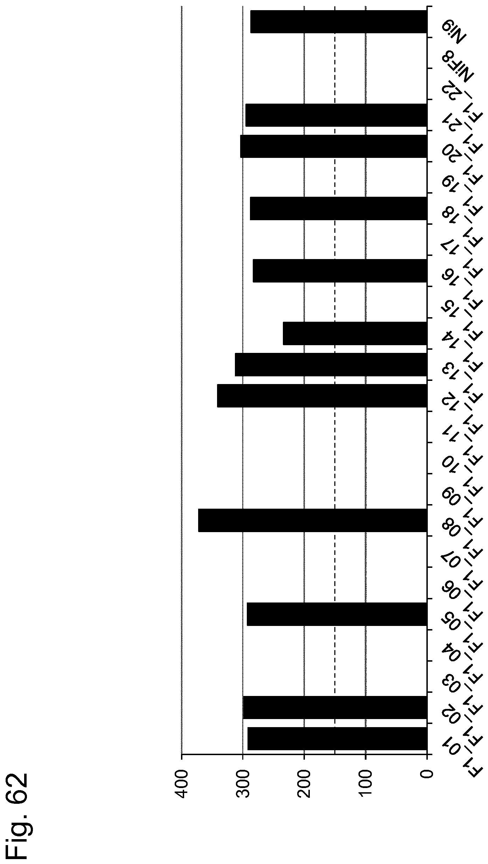

Fig. 62 shows a characteristic diagram demonstrating the number of reads of the sugarcane varieties NiF8 and Ni9 and hybrid progeny lines thereof at the marker N91653962.

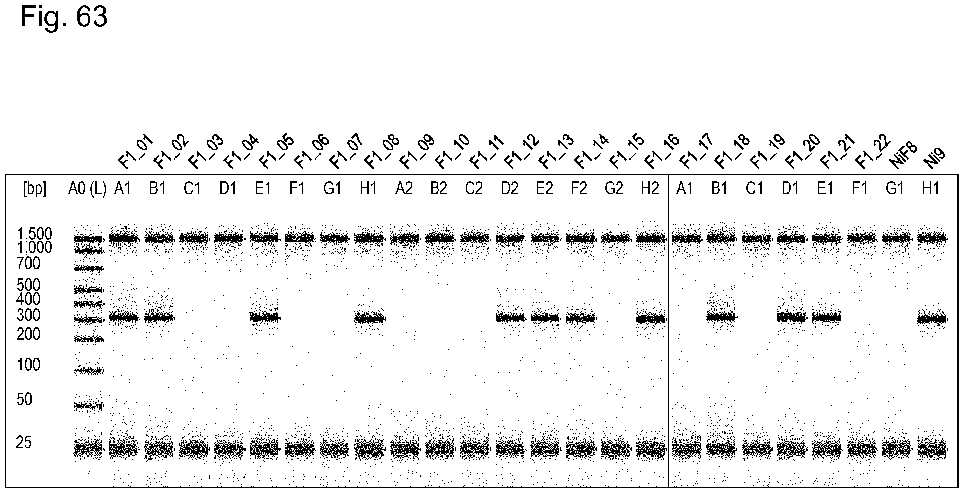

Fig. 63 shows a photograph demonstrating electrophoretic patterns of the sugarcane varietiesNiF8 and Ni9 and hybrid progeny lines thereof at the PCR marker N91653962.

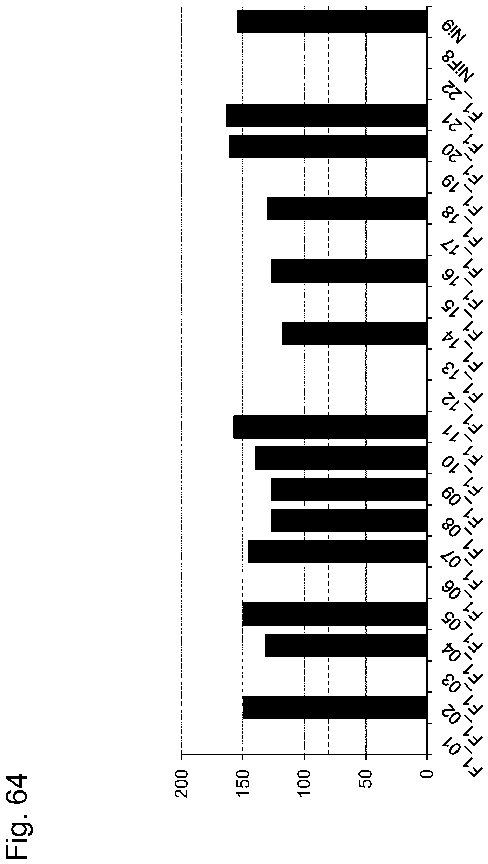

Fig. 64 shows a characteristic diagram demonstrating the number of reads of the sugarcane varieties NiF8 and Ni9 and hybrid progeny lines thereof at the marker N91124801.

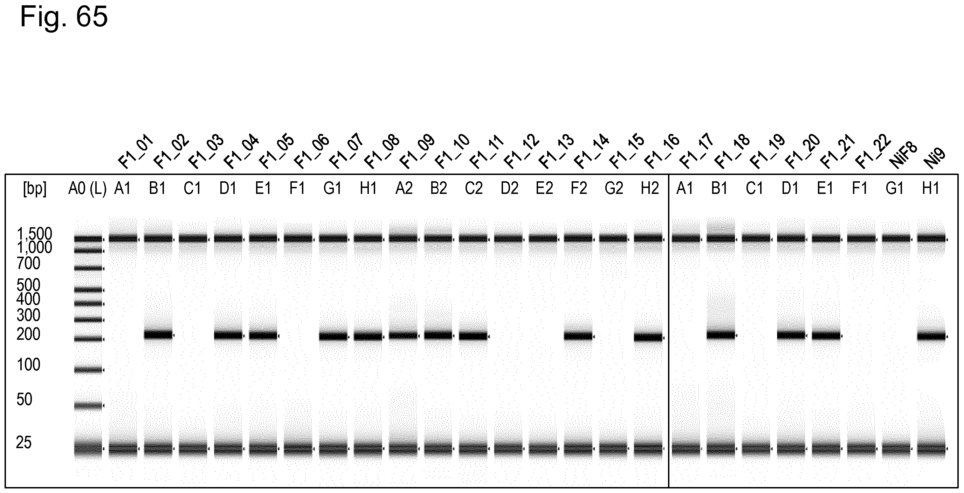

Fig. 65 shows a photograph demonstrating electrophoretic patterns of the sugarcane varietiesNiF8 and Ni9 and hybrid progeny lines thereof at the PCR marker N91124801.

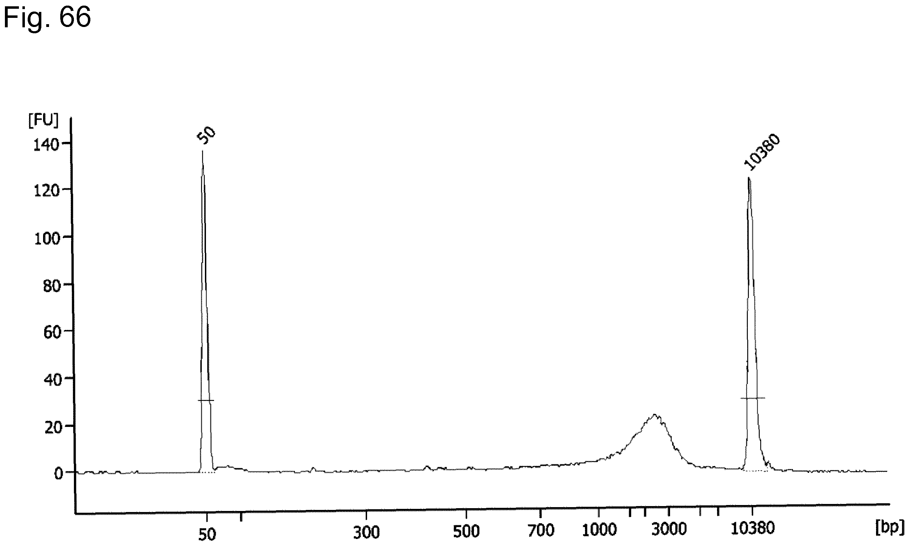

Fig. 66 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 9 nucleotides.

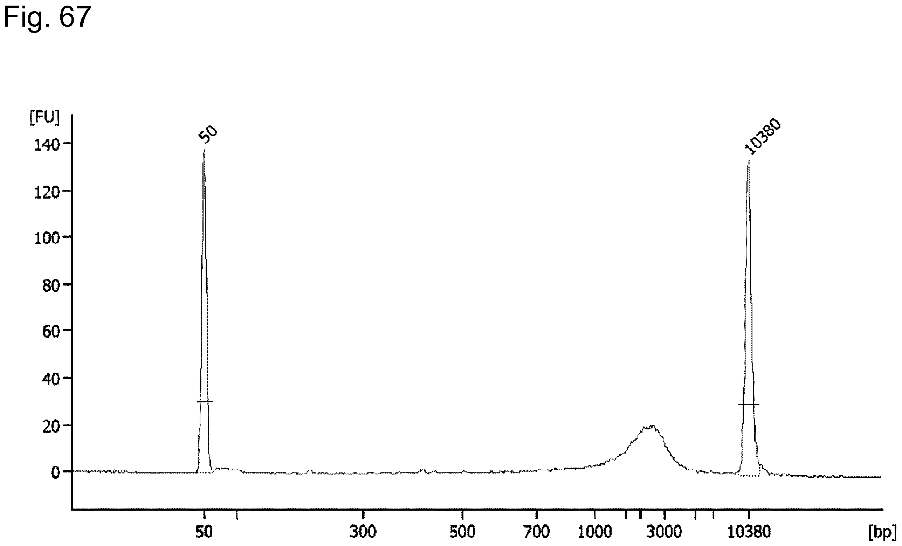

Fig. 67 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 9 nucleotides.

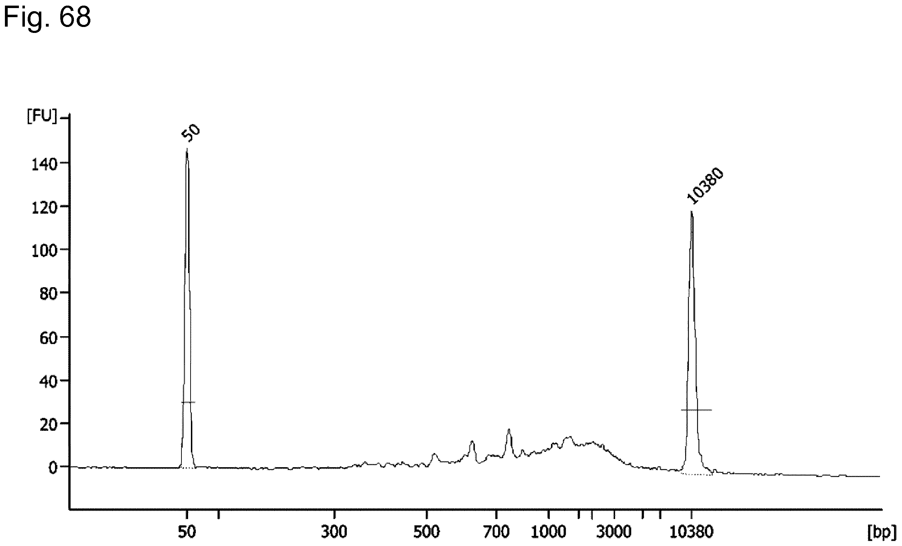

Fig. 68 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 10 nucleotides.

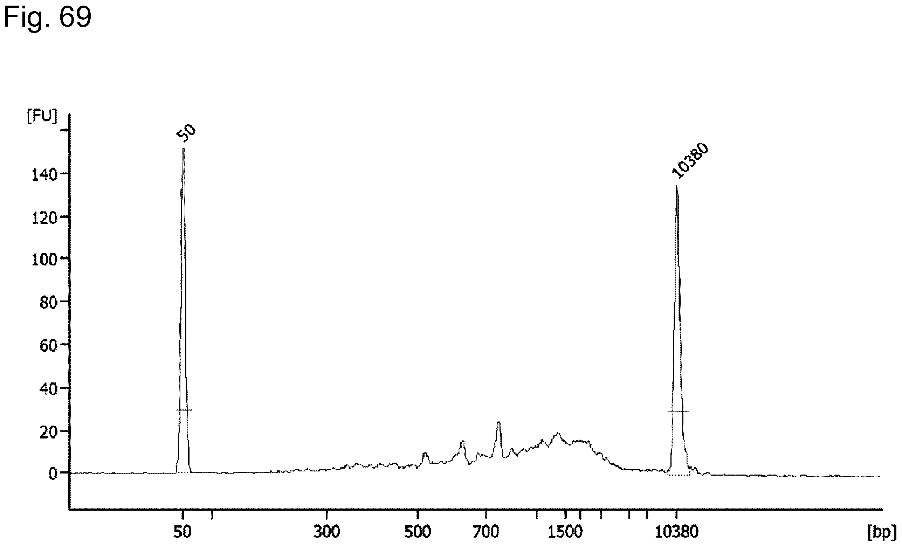

Fig. 69 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 10 nucleotides.

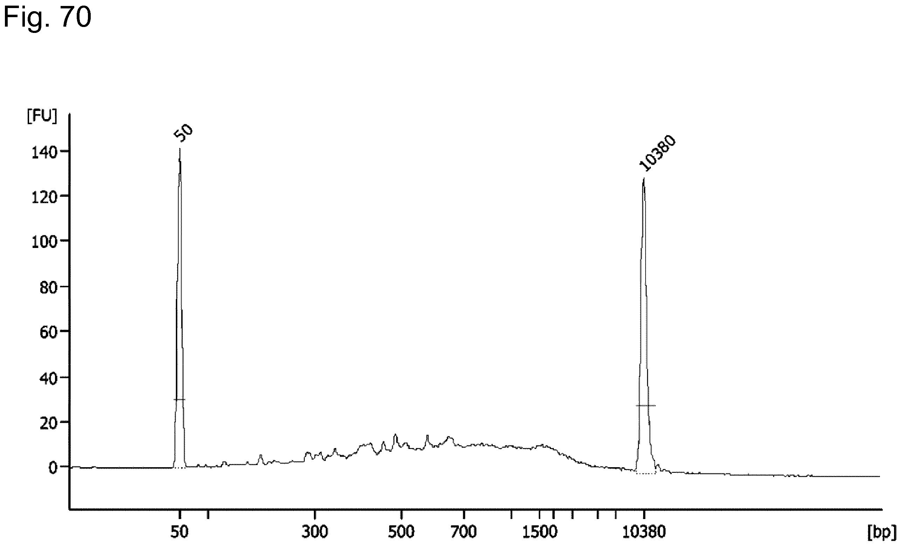

Fig. 70 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 11 nucleotides.

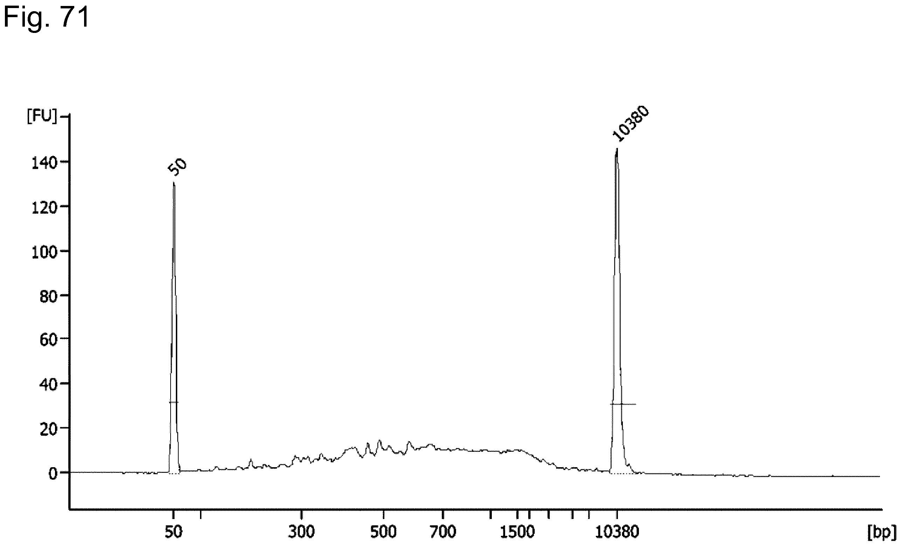

Fig. 71 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 11 nucleotides.

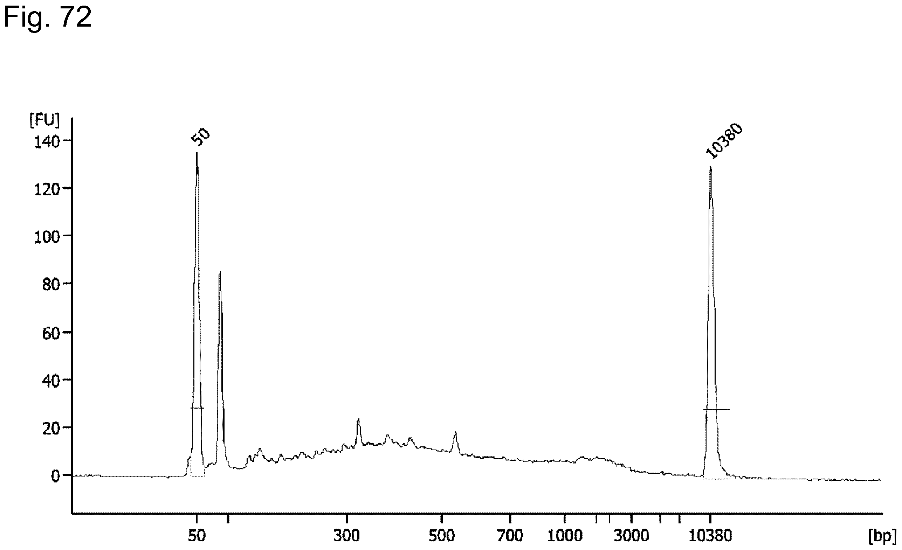

Fig. 72 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 12 nucleotides.

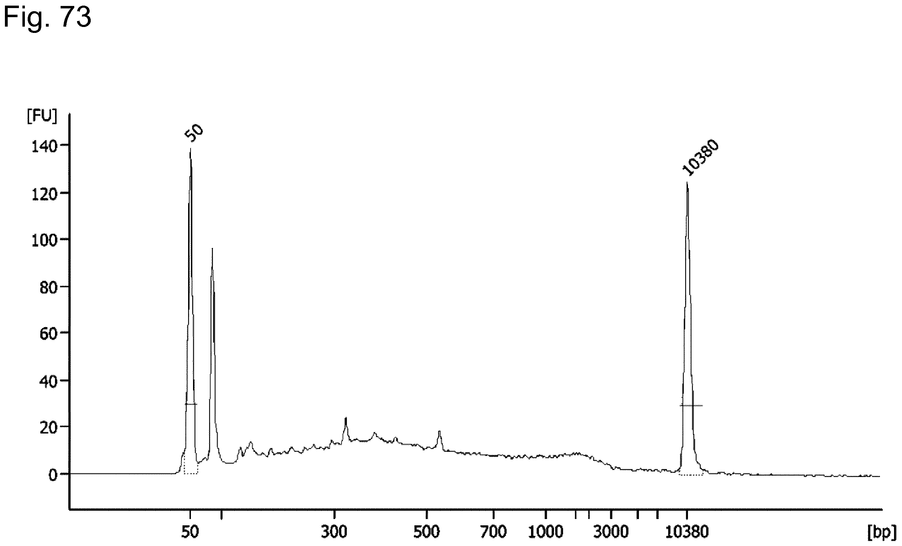

Fig. 73 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 12 nucleotides.

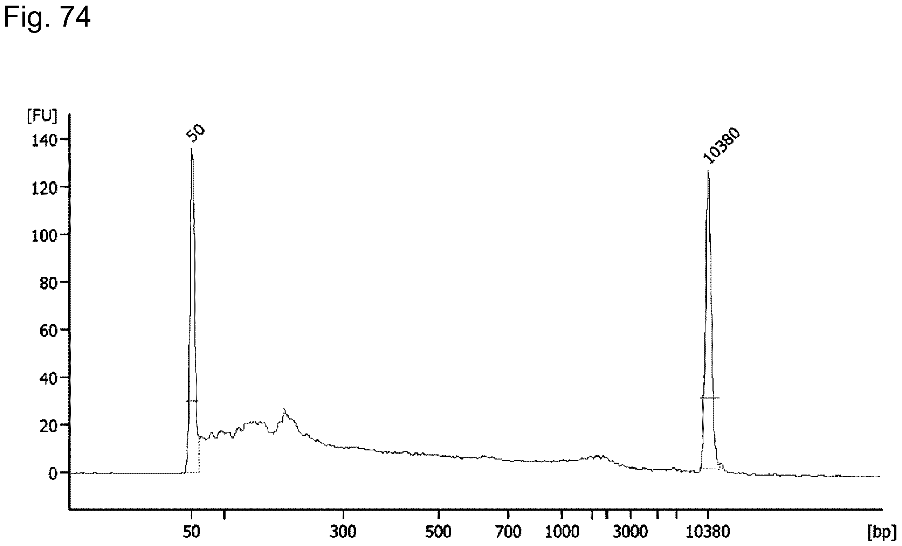

Fig. 74 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 14 nucleotides.

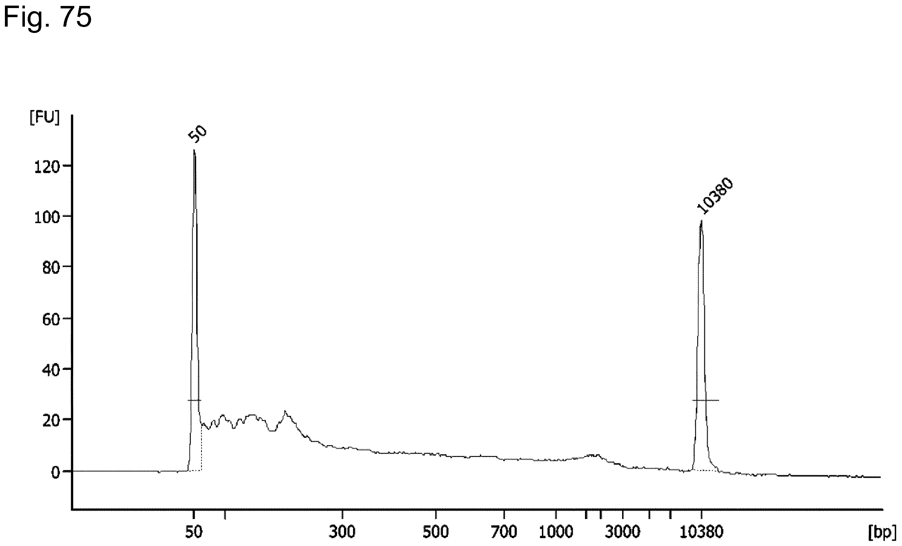

Fig. 75 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 14 nucleotides.

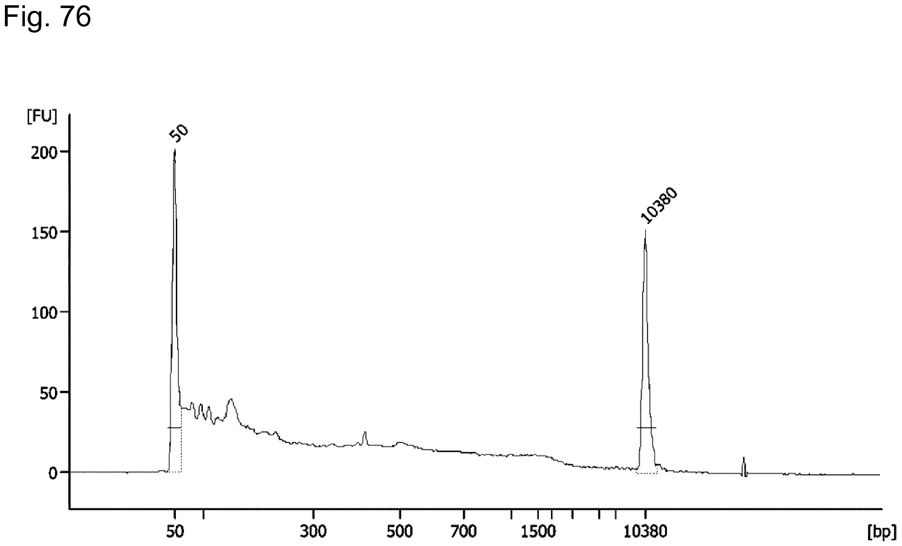

Fig. 76 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 16 nucleotides.

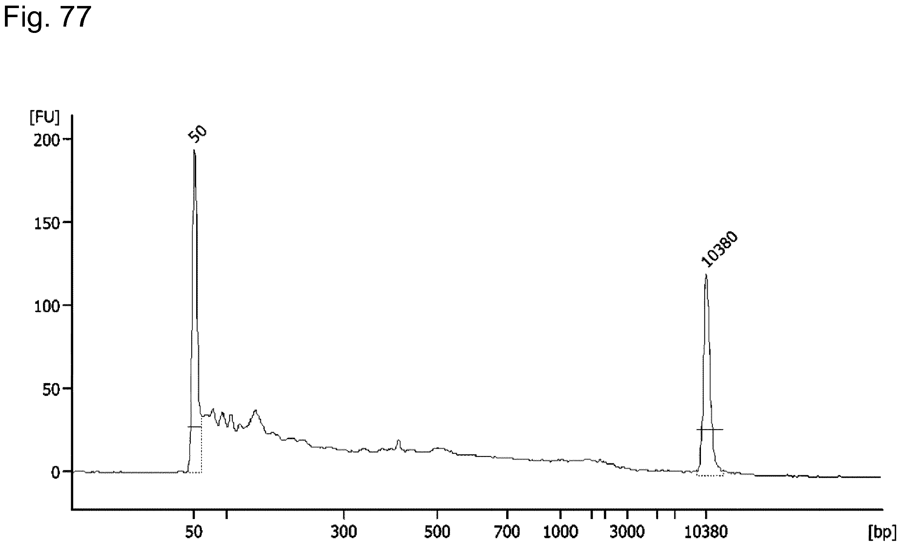

Fig. 77 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 16 nucleotides.

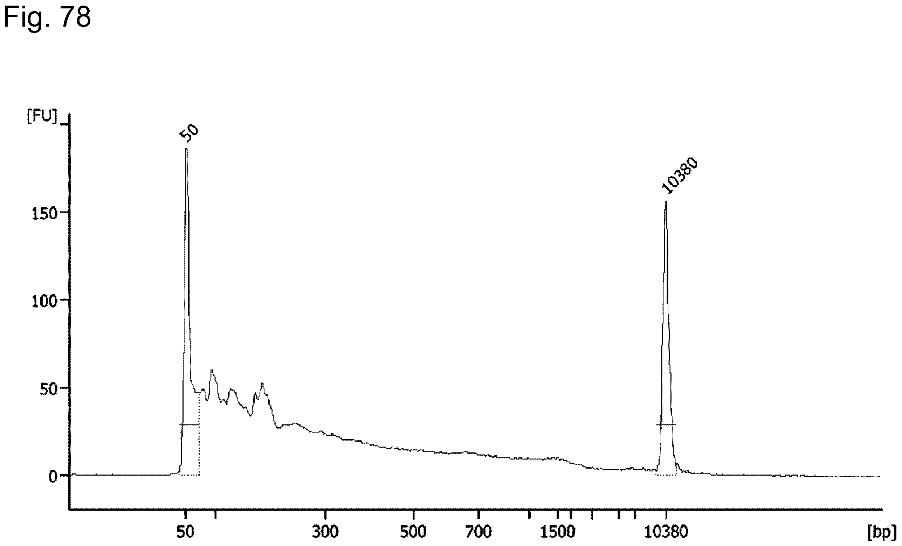

Fig. 78 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 18 nucleotides.

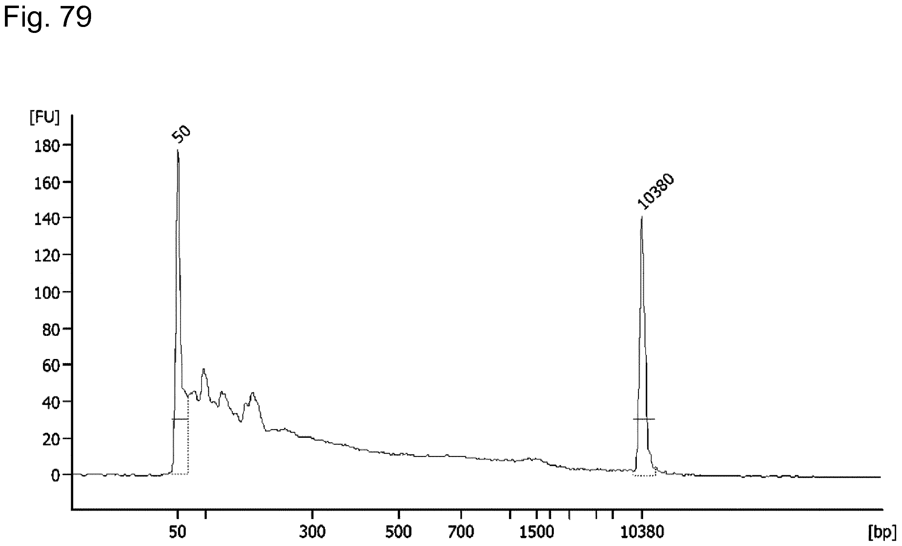

Fig. 79 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 18 nucleotides.

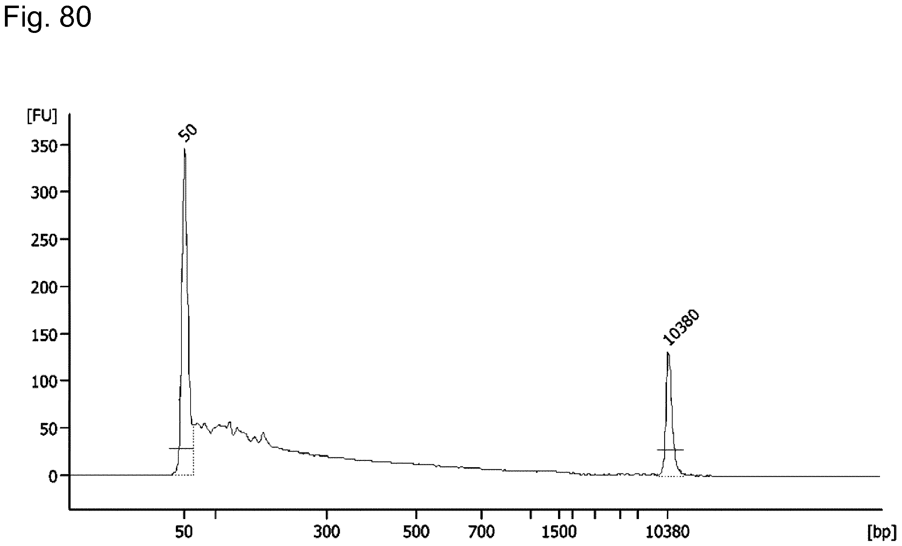

Fig. 80 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 20 nucleotides.

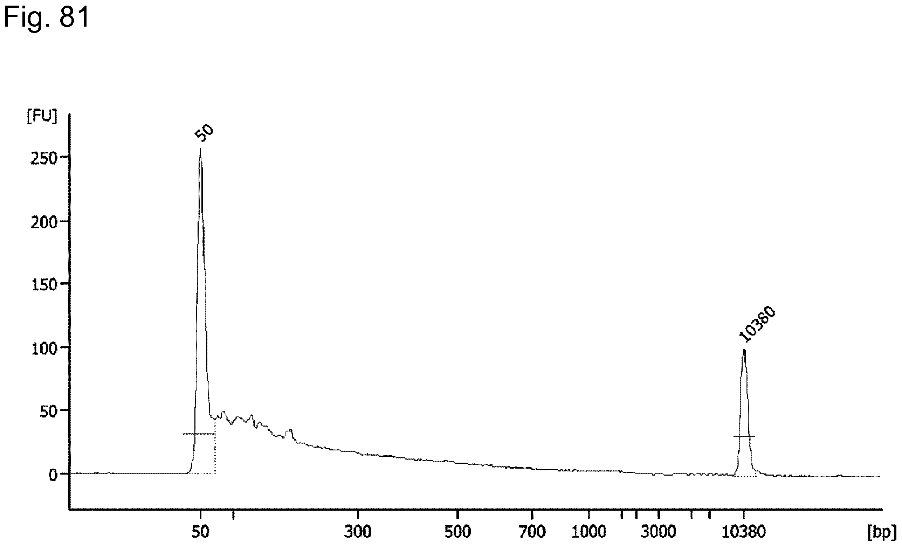

Fig. 81 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 20 nucleotides.

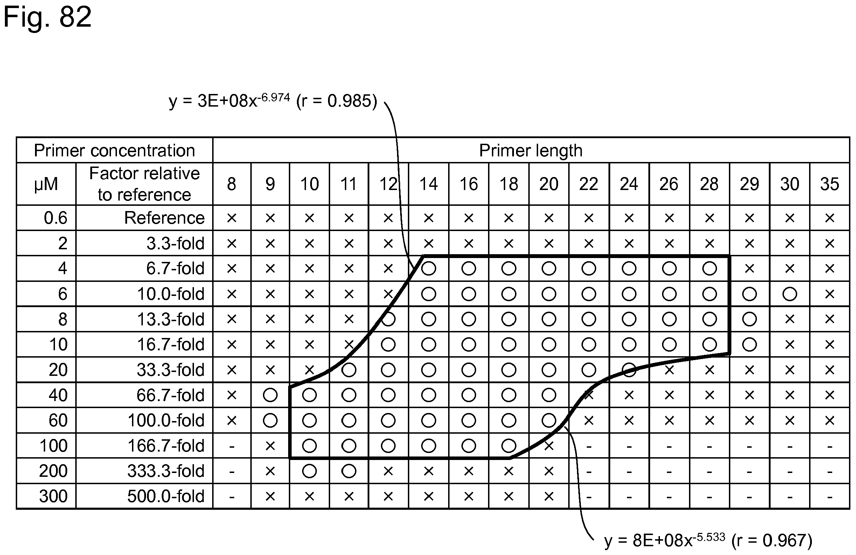

Fig. 82 shows a characteristic diagram demonstrating the results of investigating the reproducibility of the DNA library amplified using DNA of the sugarcane variety NiF8 as a template and random primers each comprising 8 to 35 nucleotides used at a concentration of 0.6 to 300 µM.

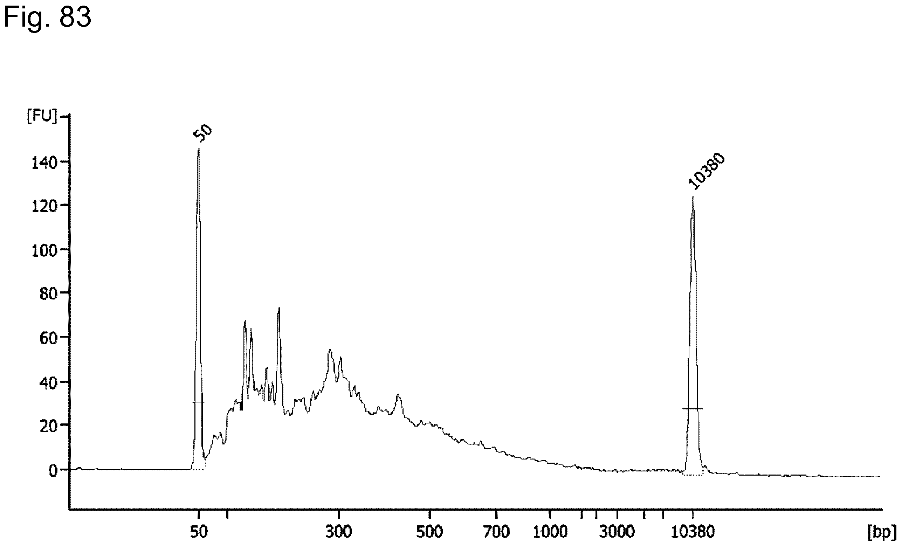

Fig. 83 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and 1 type of random primer.

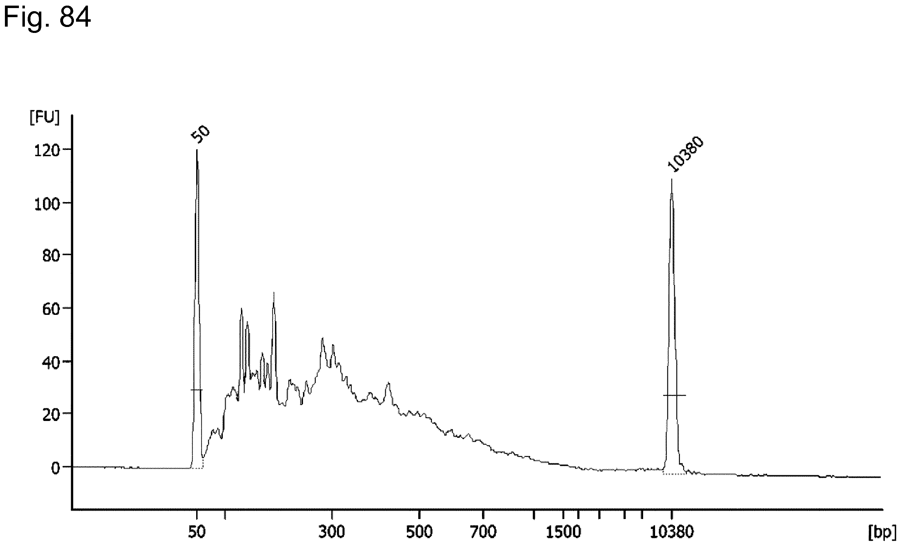

Fig. 84 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and 1 type of random primer.

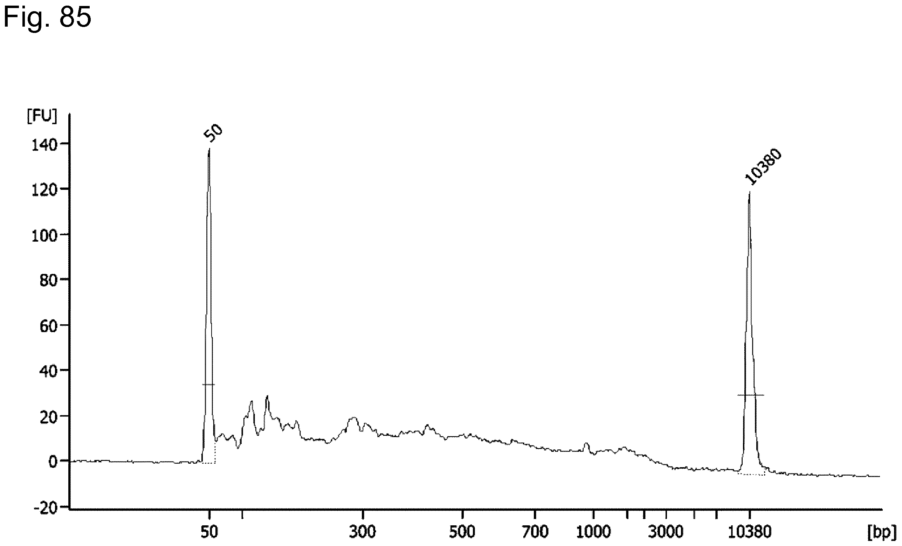

Fig. 85 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and 2 types of random primers.

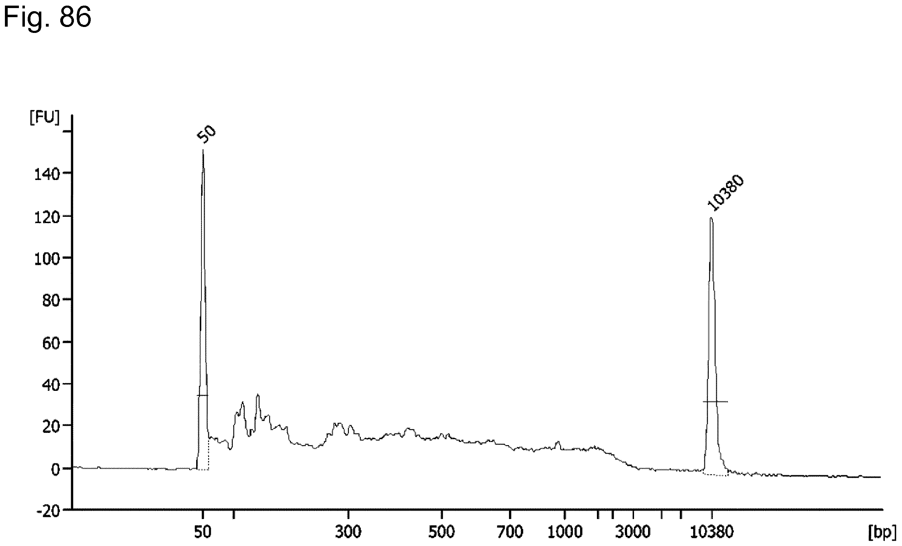

Fig. 86 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and 2 types of random primers.

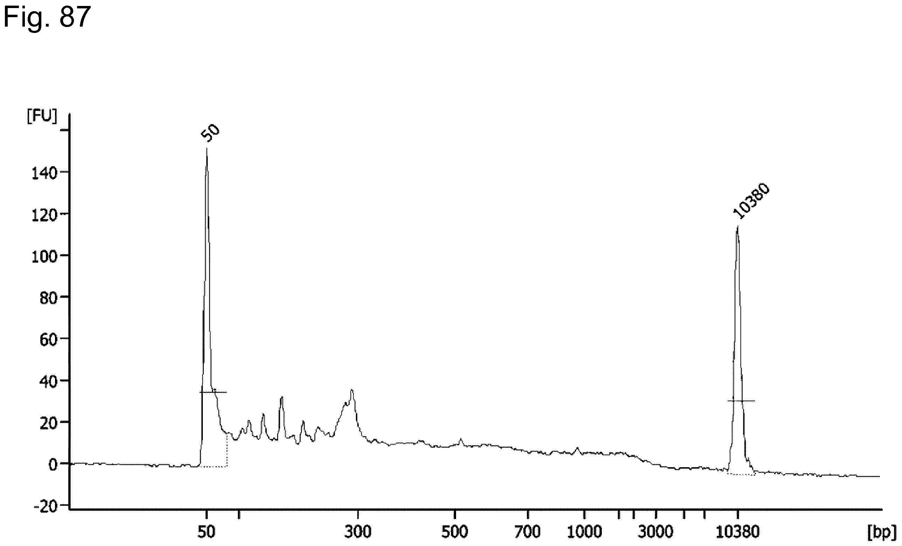

Fig. 87 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and 3 types of random primers.

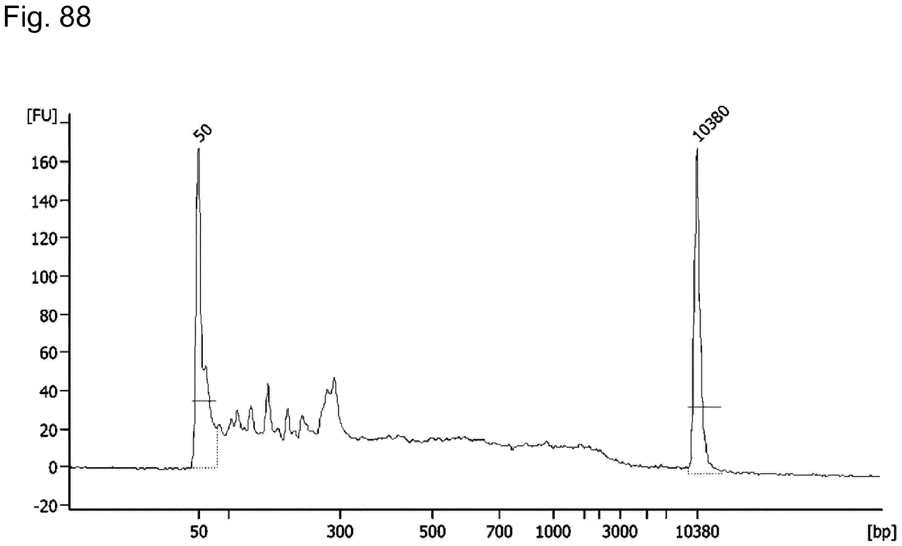

Fig. 88 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and 3 types of random primers.

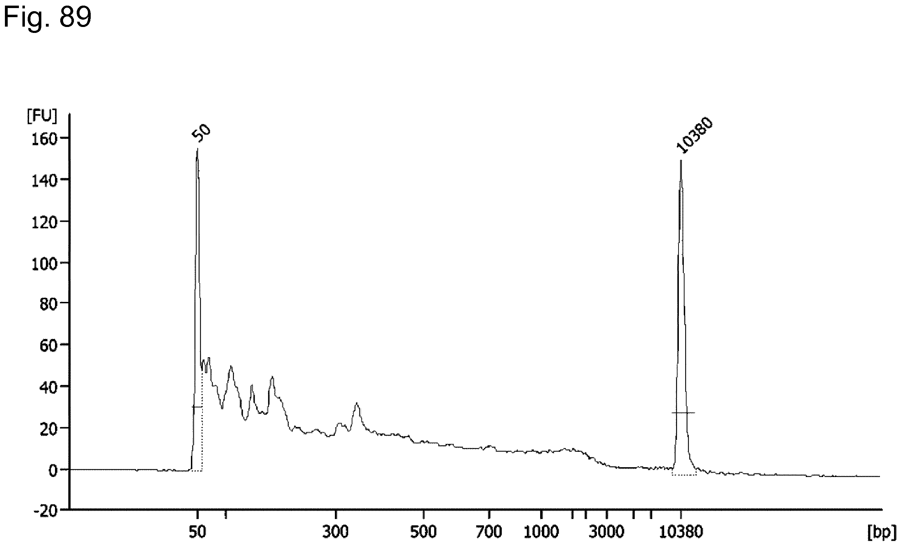

Fig. 89 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and 12 types of random primers.

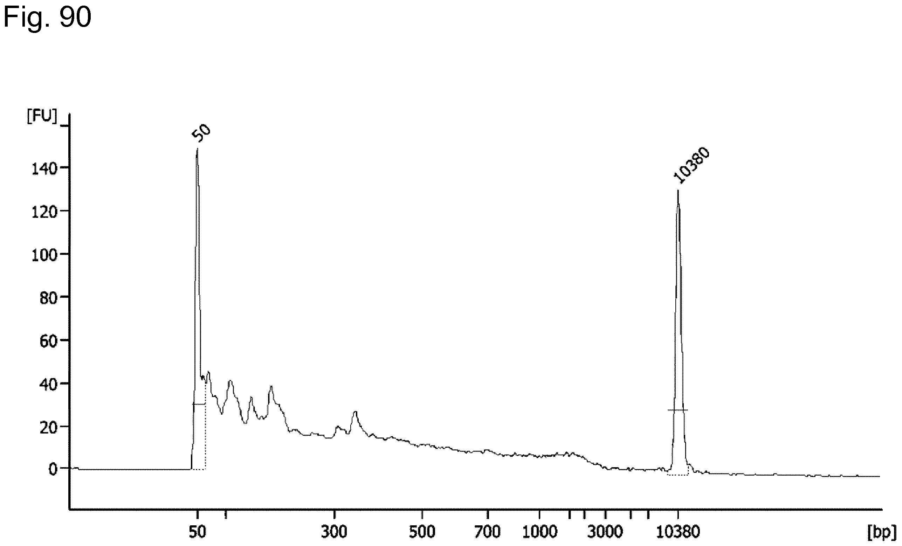

Fig. 90 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and 12 types of random primers.

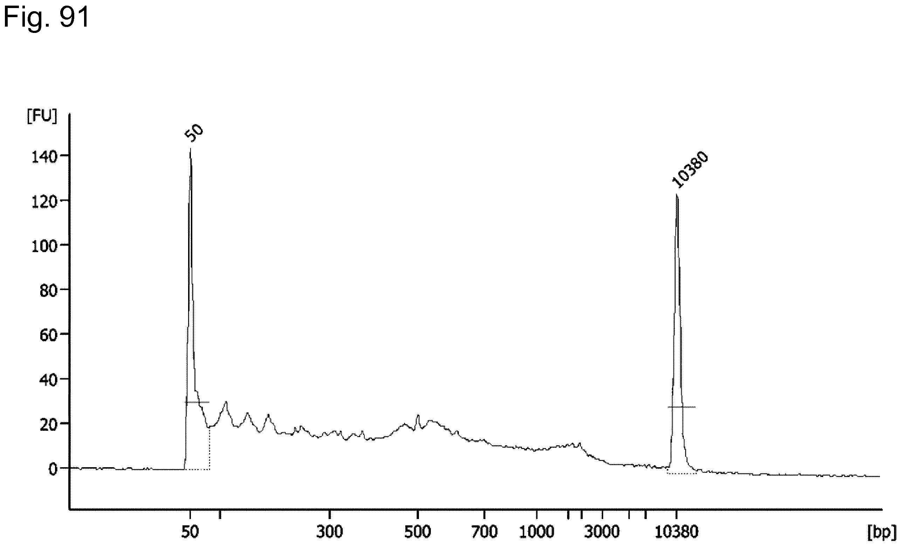

Fig. 91 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and 24 types of random primers.

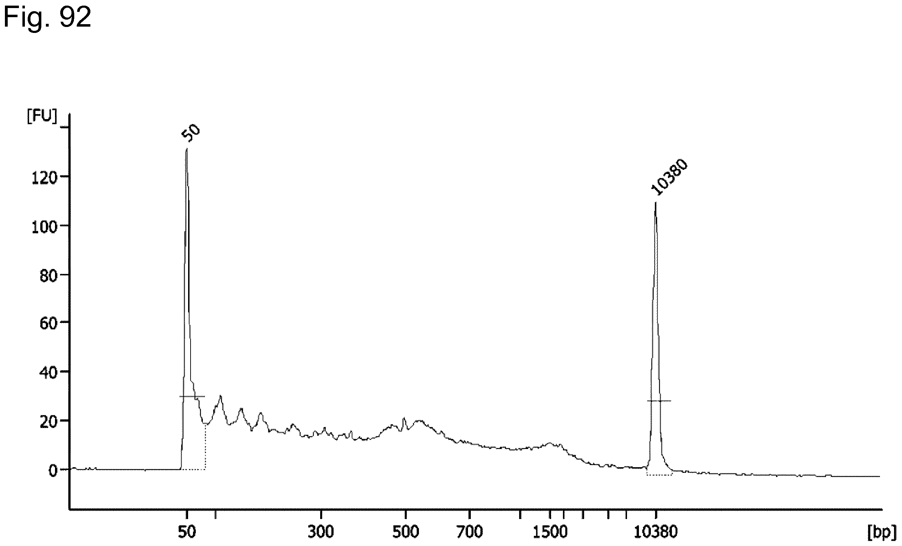

Fig. 92 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and 24 types of random primers.

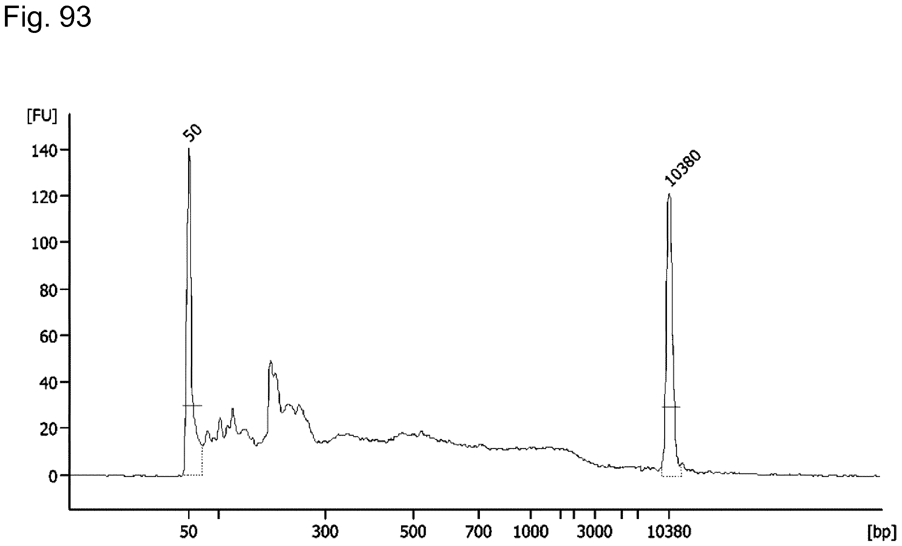

Fig. 93 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and 48 types of random primers.

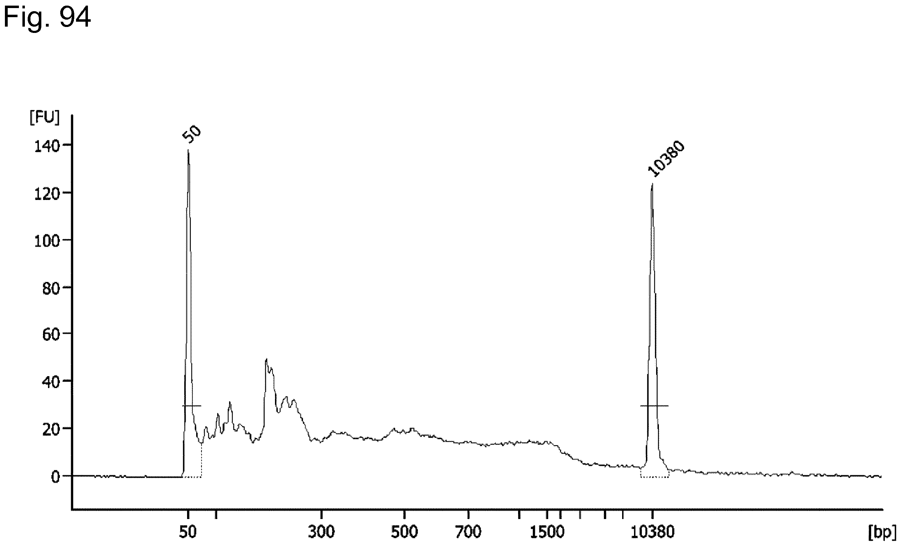

Fig. 94 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and 48 types of random primers.

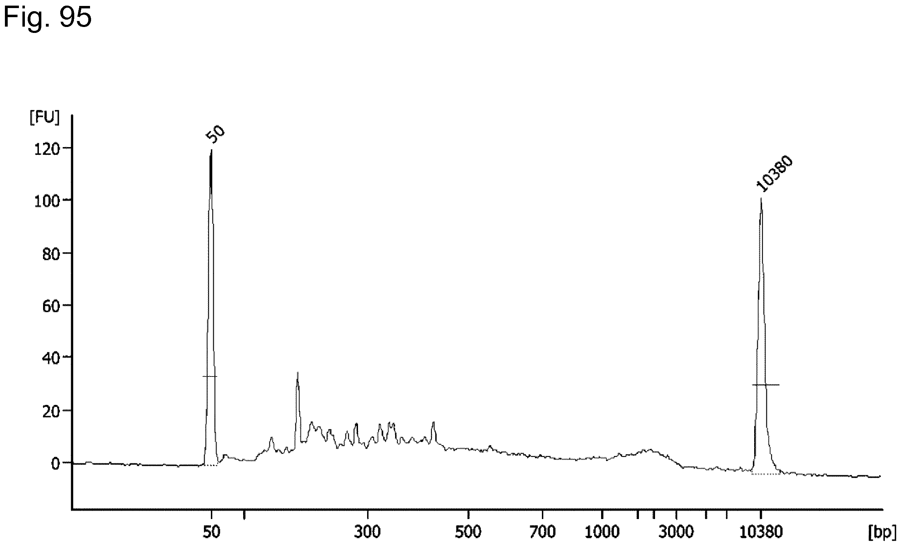

Fig. 95 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer B comprising 10 nucleotides.

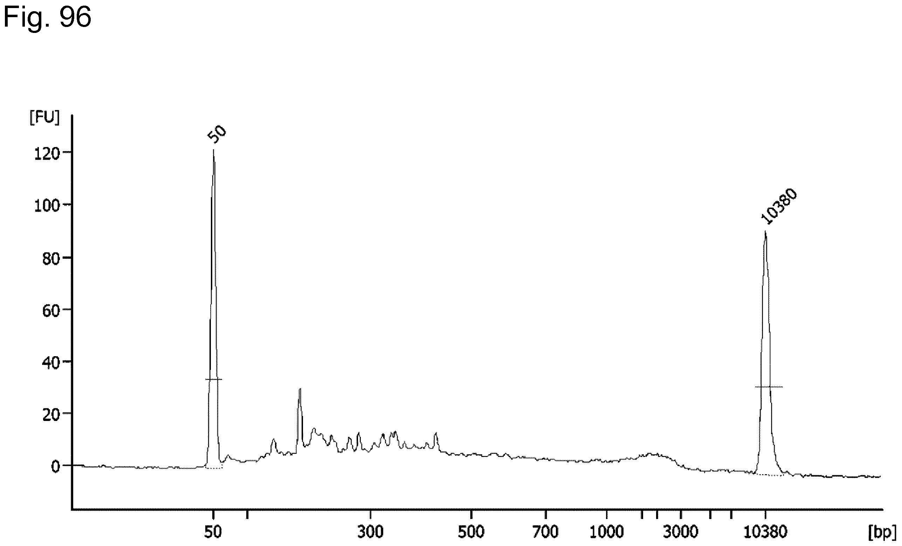

Fig. 96 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer B comprising 10 nucleotides.

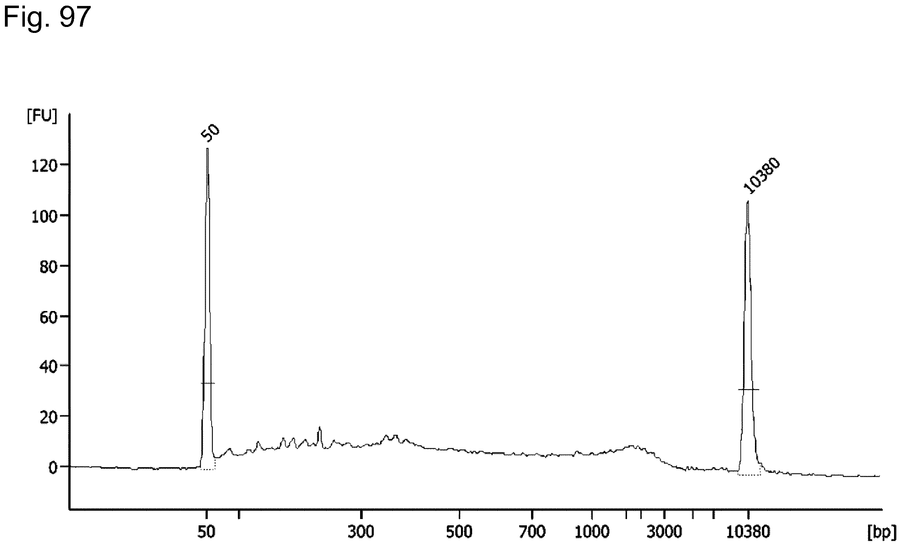

Fig. 97 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer C comprising 10 nucleotides.

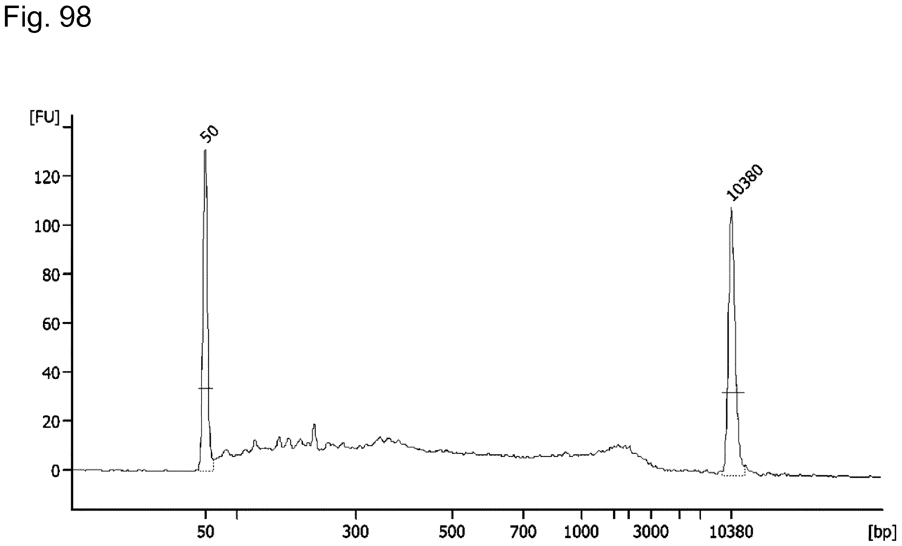

Fig. 98 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer C comprising 10 nucleotides.

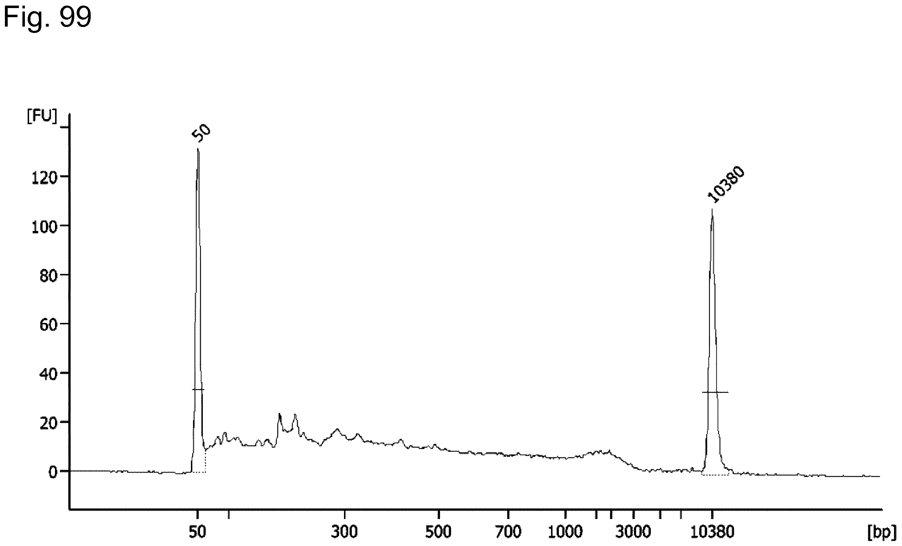

Fig. 99 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer D comprising 10 nucleotides.

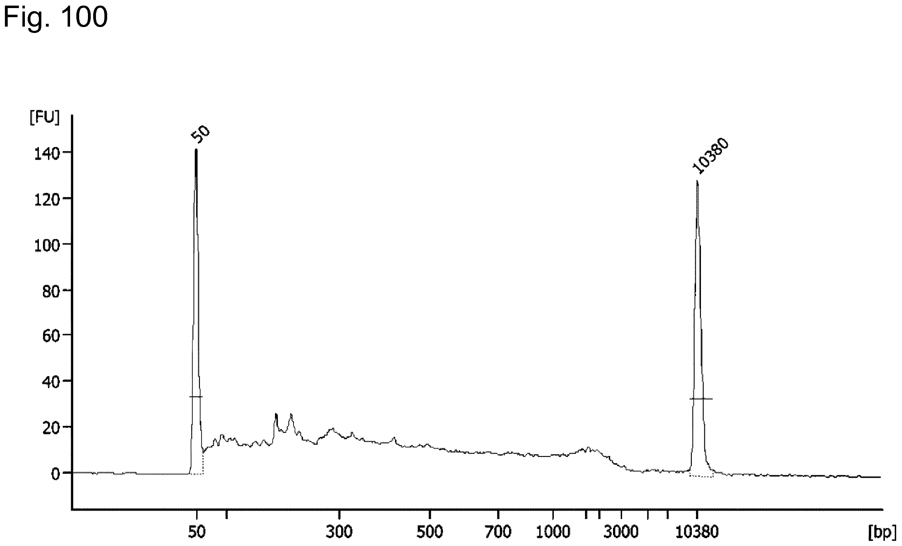

Fig. 100 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer D comprising 10 nucleotides.

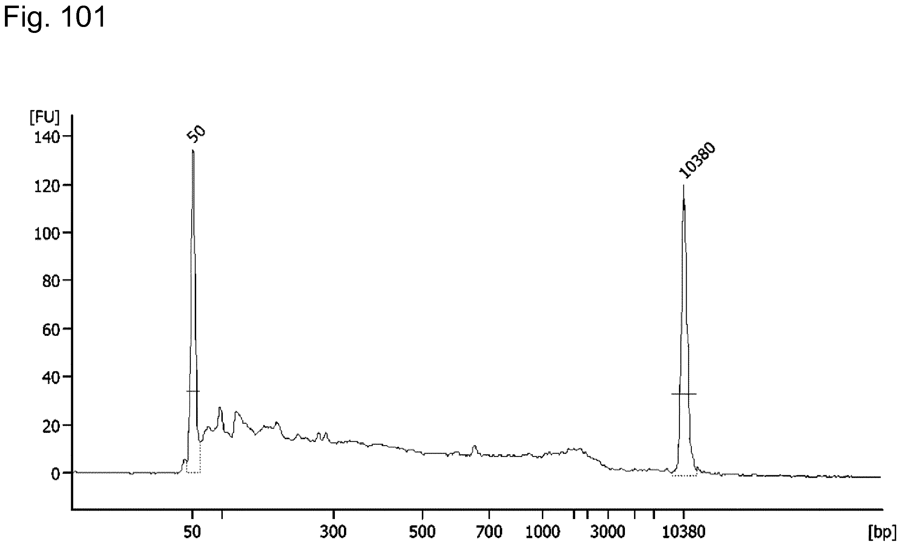

Fig. 101 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer E comprising 10 nucleotides.

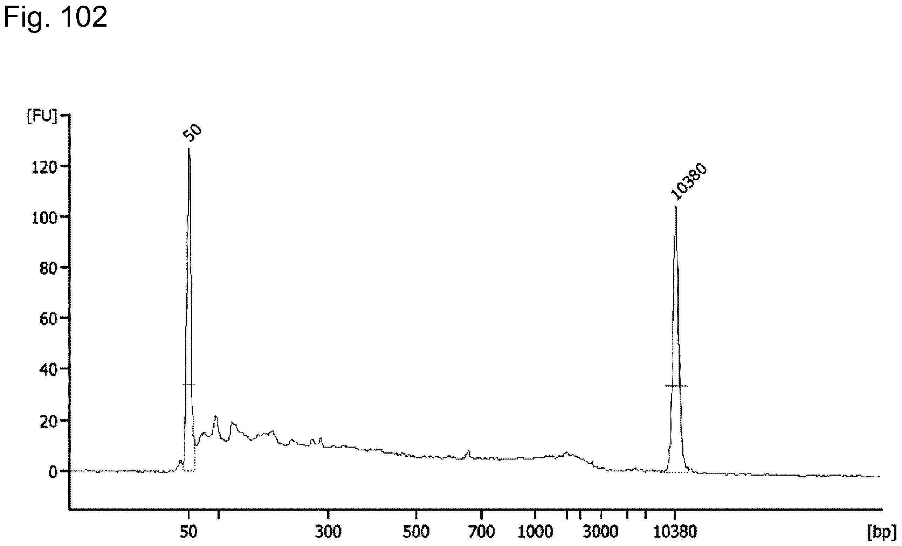

Fig. 102 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer E comprising 10 nucleotides.

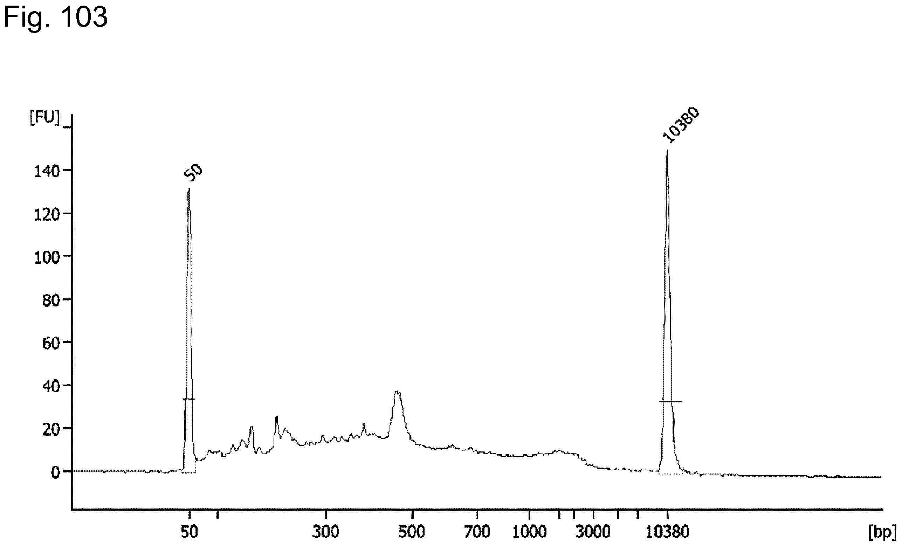

Fig. 103 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer F comprising 10 nucleotides.

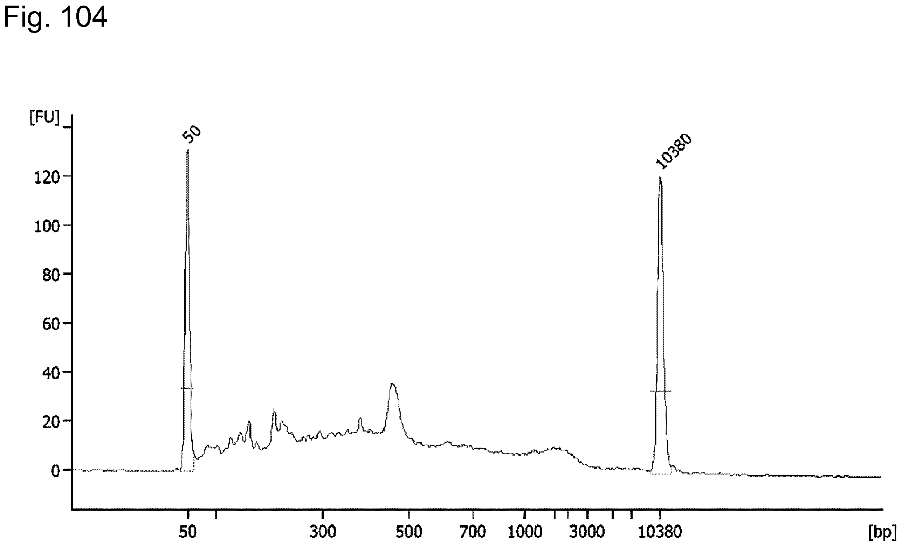

Fig. 104 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer F comprising 10 nucleotides.

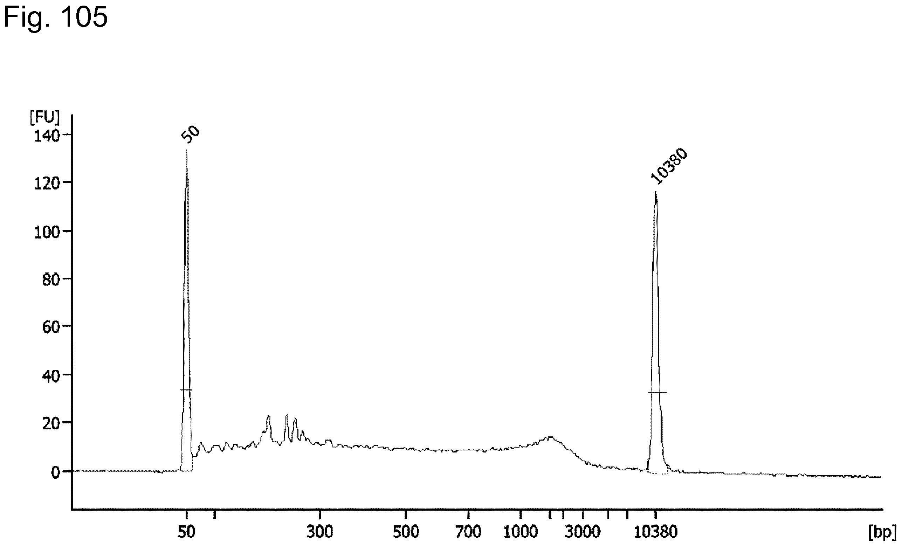

Fig. 105 shows a characteristic diagram demonstrating a correlation between an amplified fragment length and a fluorescence unit (FU) in which the amplified fragment length is determined based on an electrophoretic pattern (appeared for the first time) of the DNA library amplified using human genomic DNA as a template and a random primer A comprising 10 nucleotides.

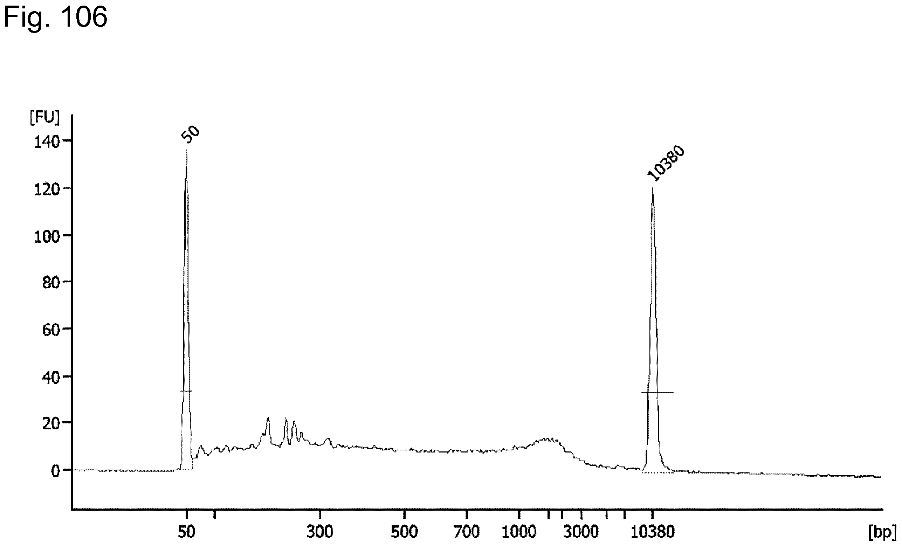

Fig. 106 shows a characteristic diagram demonstrating a correlation between an amplified fragment length and a fluorescence unit (FU) in which the amplified fragment length is determined based on an electrophoretic pattern (appeared for the second time) of the DNA library amplified using human genomic DNA as a template and a random primer A comprising 10 nucleotides.

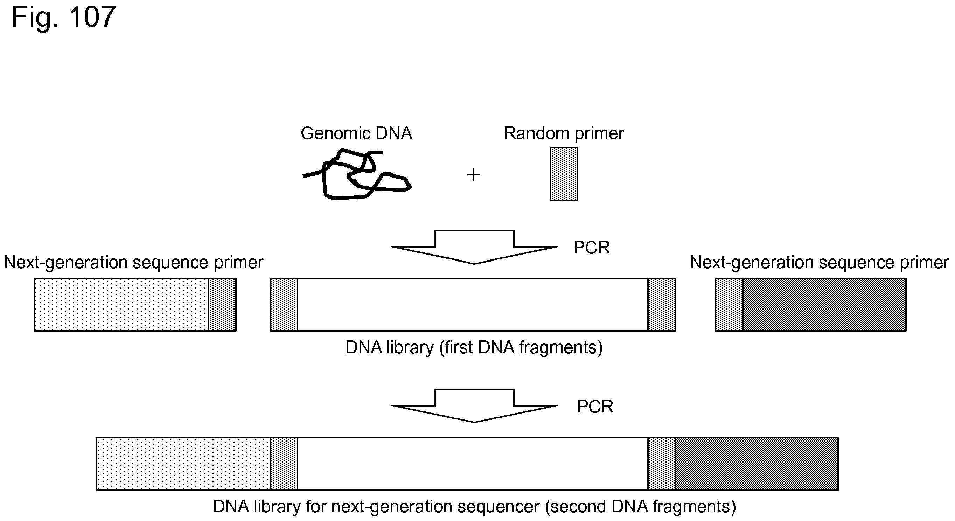

Fig. 107 schematically shows a characteristic diagram of a method for producing a DNA library applied to a next-generation sequencer.

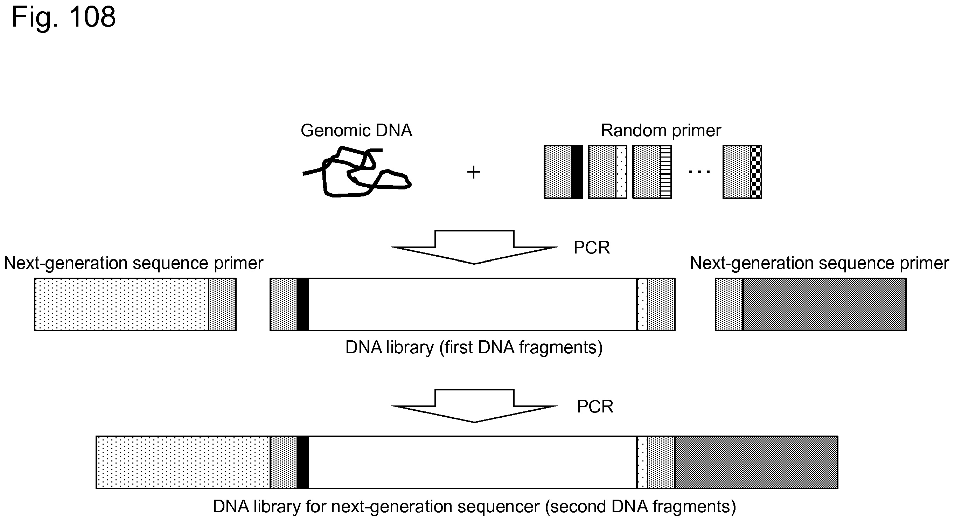

Fig. 108 schematically shows a characteristic diagram of a method for producing a DNA library applied to a next-generation sequencer.

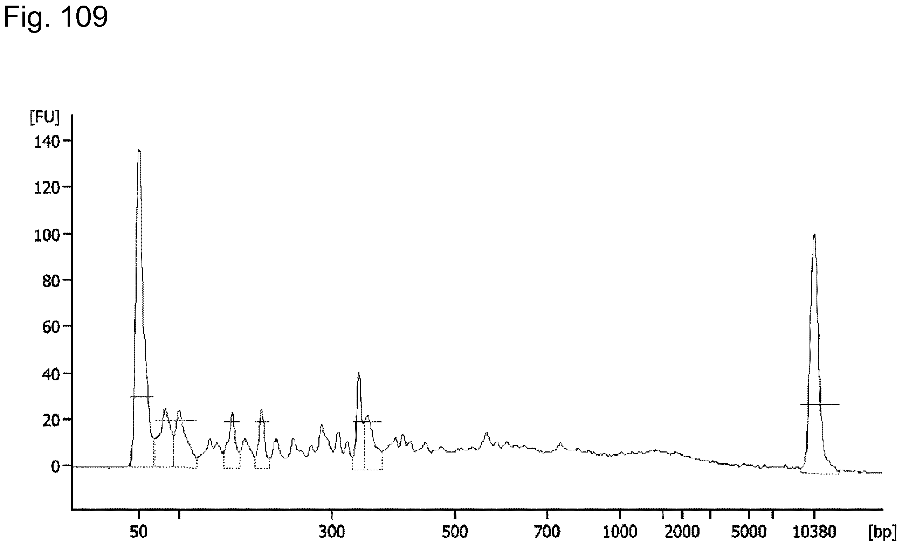

Fig. 109 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer G comprising 10 nucleotides.

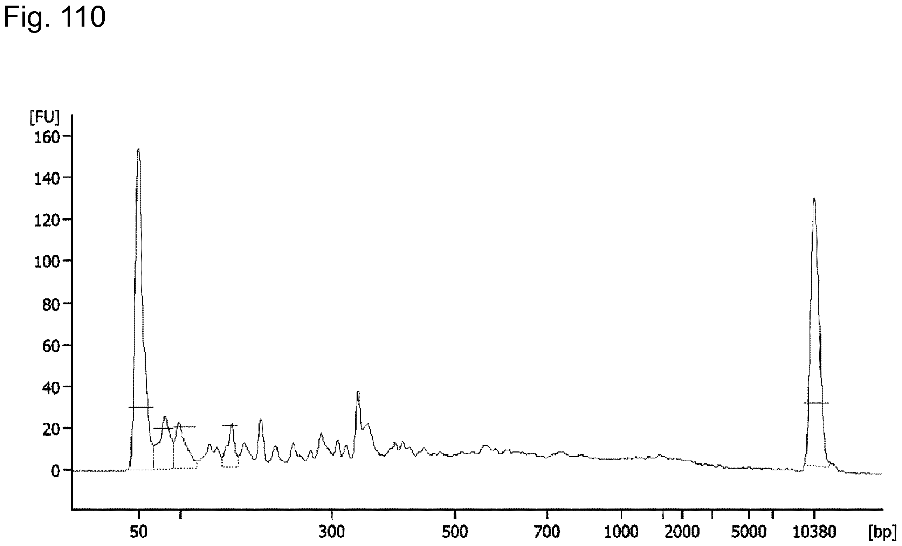

Fig. 110 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer G comprising 10 nucleotides.

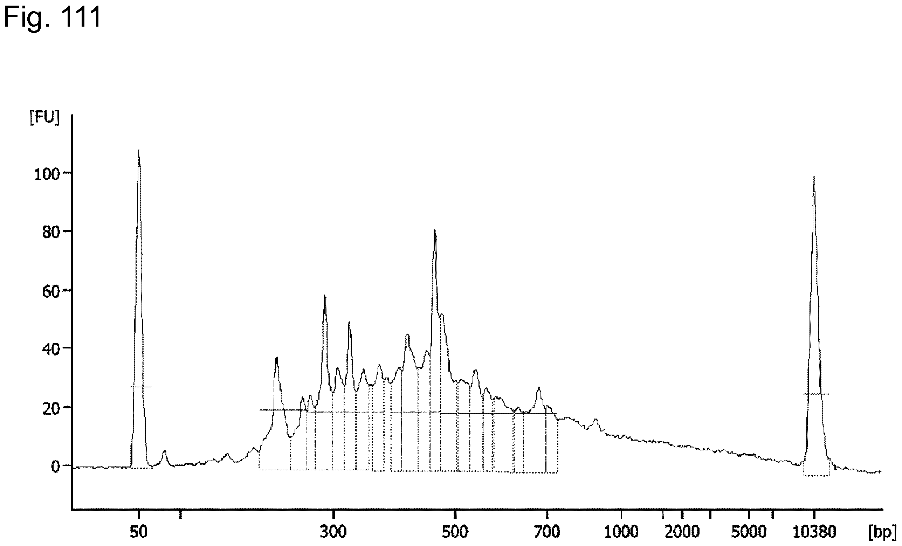

Fig. 111 shows a characteristic diagram demonstrating a correlation between an amplified fragment length and a fluorescence unit (FU) in which the amplified fragment length is determined based on an electrophoretic pattern (appeared for the first time) of the DNA library amplified using a DNA library of the sugarcane variety NiF8 produced using a random primer G comprising 10 nucleotides as a template and a next-generation sequencer.

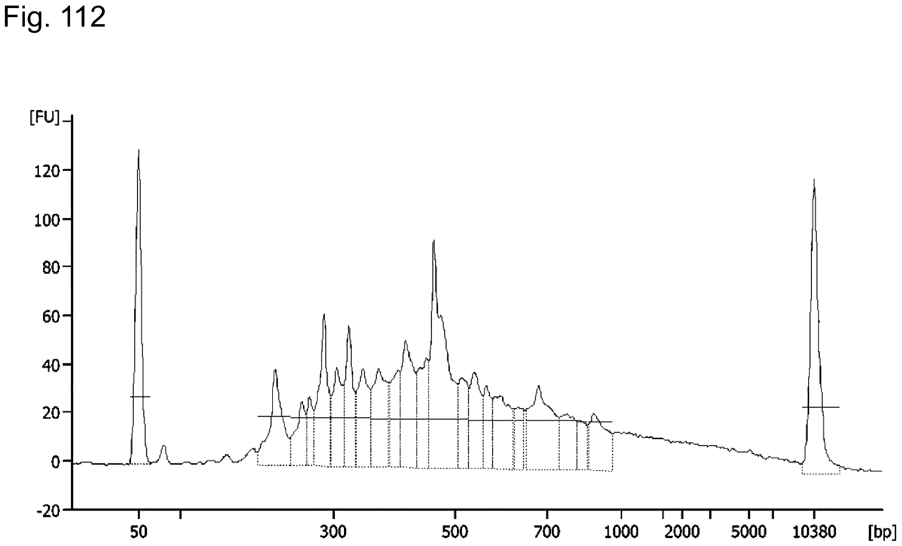

Fig. 112 shows a characteristic diagram demonstrating a correlation between an amplified fragment length and a fluorescence unit (FU) in which the amplified fragment length is determined based on an electrophoretic pattern (appeared for the second time) of the DNA library amplified using a DNA library of the sugarcane variety NiF8 produced using a random primer G comprising 10 nucleotides as a template and a next-generation sequencer.

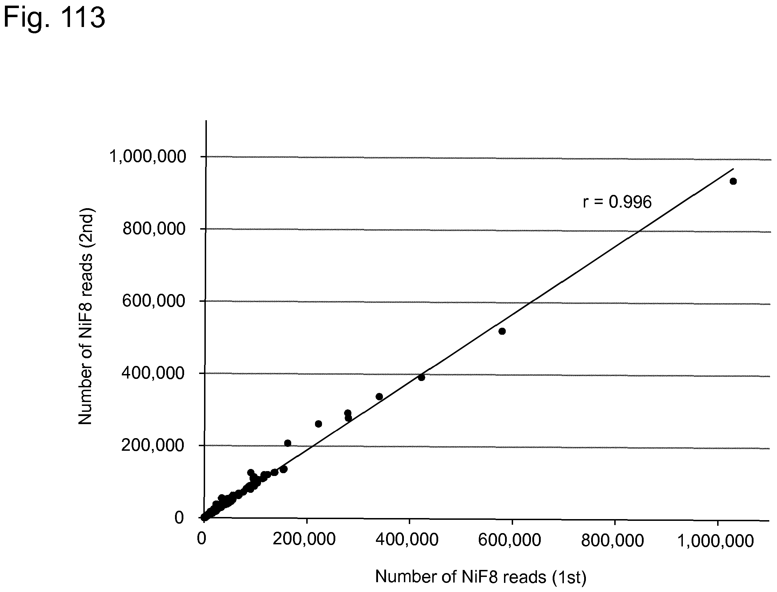

Fig. 113 shows a characteristic diagram demonstrating the results of MiSeq analysis of a DNA library amplified using DNA of the sugarcane variety NiF8 as a template and a random primer G comprising 10 nucleotides.

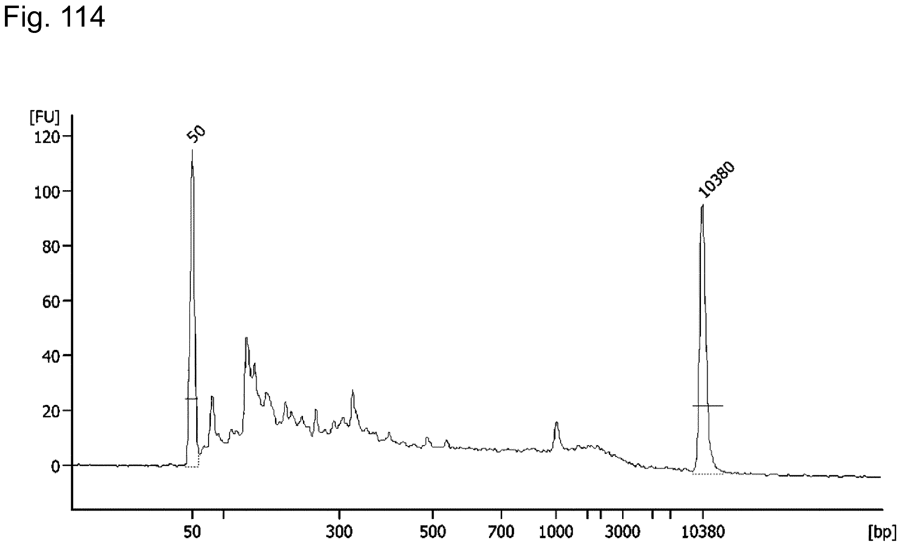

Fig. 114 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the rice variety Nipponbare as a template and a random primer B comprising 12 nucleotides.

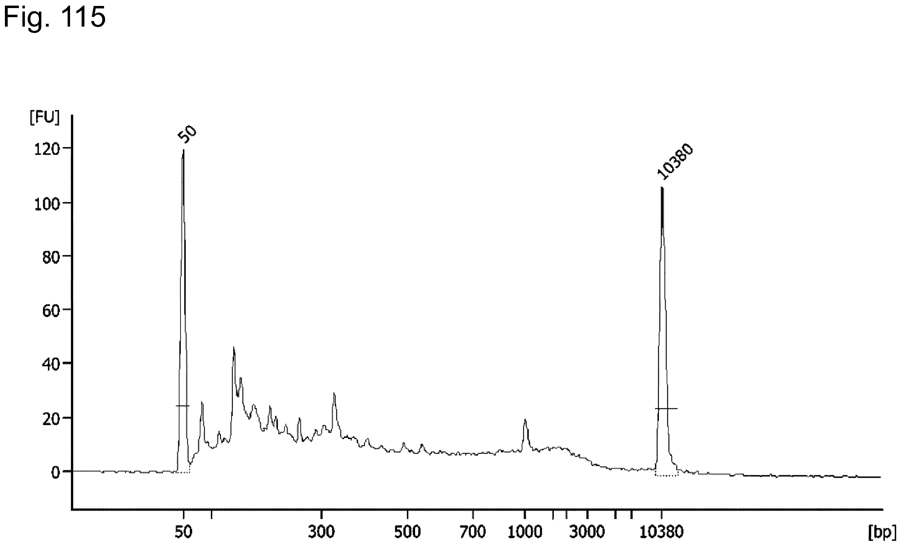

Fig. 115 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the rice variety Nipponbare as a template and a random primer B comprising 12 nucleotides.

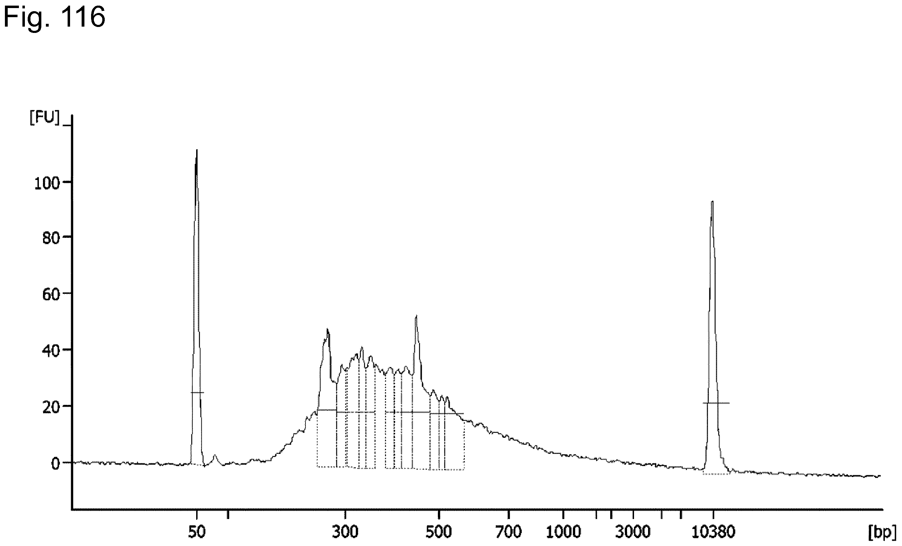

Fig. 116 shows a characteristic diagram demonstrating a correlation between an amplified fragment length and a fluorescence unit (FU) in which the amplified fragment length is determined based on an electrophoretic pattern (appeared for the first time) of the DNA library amplified using a DNA library of the rice variety Nipponbare produced using a random primer B comprising 12 nucleotides as a template and a next-generation sequencer.

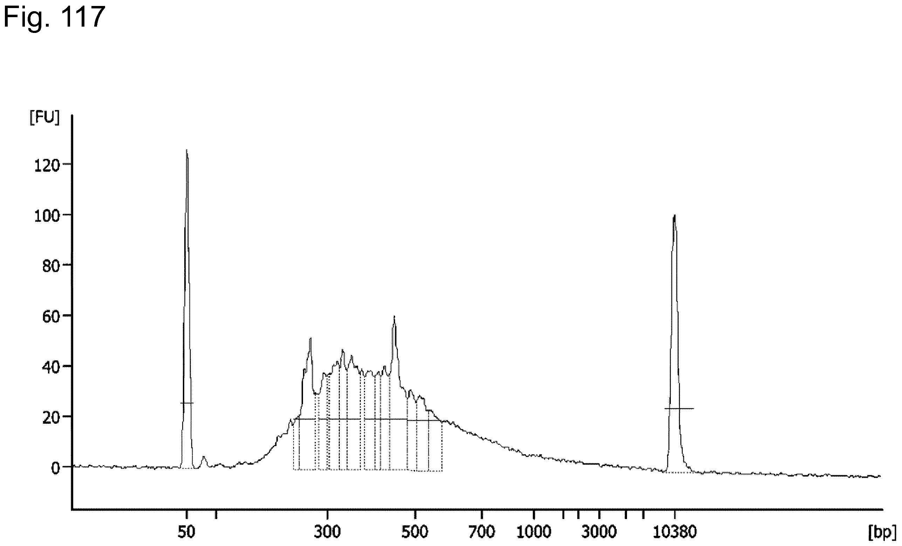

Fig. 117 shows a characteristic diagram demonstrating a correlation between an amplified fragment length and a fluorescence unit (FU) in which the amplified fragment length is determined based on an electrophoretic pattern (appeared for the second time) of the DNA library amplified using a DNA library of the rice variety Nipponbare produced using a random primer B comprising 12 nucleotides as a template and a next-generation sequencer.

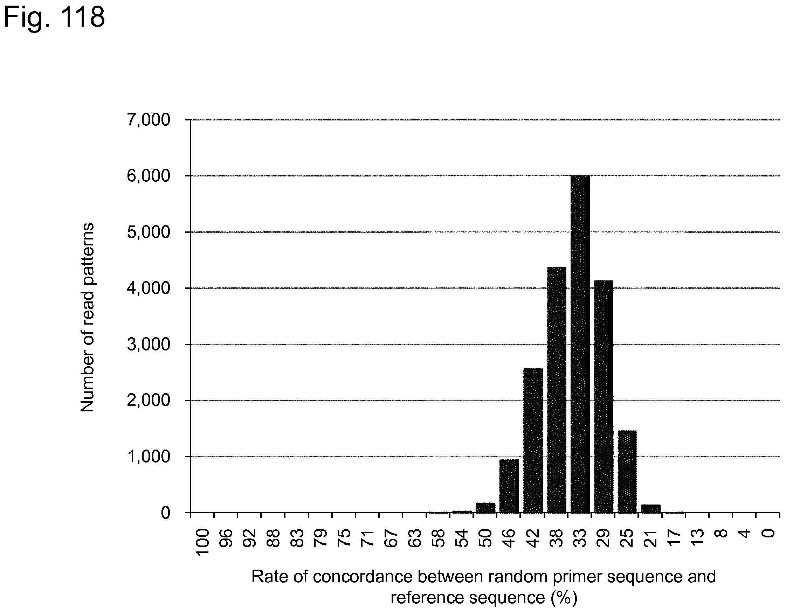

Fig. 118 shows a characteristic diagram demonstrating a distribution of the read pattern obtained by MiSeq analysis of a DNA library amplified using DNA of the rice variety Nipponbare as a template and a random primer B comprising 12 nucleotides and the degree of consistency between the random primer sequence and the reference sequence of rice variety Nipponbare.

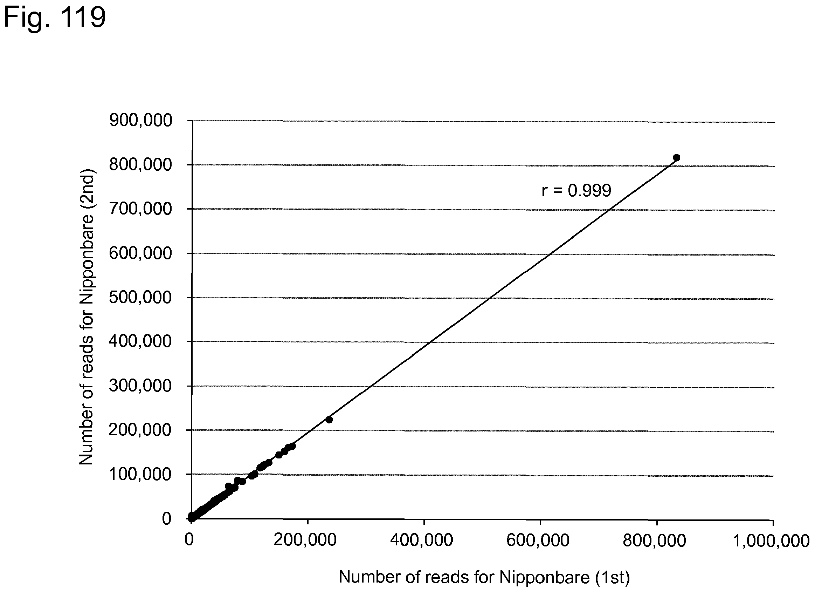

Fig. 119 shows a characteristic diagram demonstrating the results of MiSeq analysis of a DNA library amplified using DNA of the rice variety Nipponbare as a template and a random primer B comprising 12 nucleotides.

### Description of Embodiments

Hereafter, the present invention is described in detail.

According to the method for producing a DNA library of the present invention, a nucleic acid amplification reaction is conducted in a reaction solution, which is prepared to contain a primer having an arbitrary nucleotide sequence (hereafter, referred to as "random primer") at a high concentration, and the amplified nucleic acid fragment is determined to be a DNA library. The expression "high concentration" used herein means that the concentration is higher than the primer concentration in a general nucleic acid amplification reaction. Specifically, the method for producing a DNA library of the present invention is characterized in that a random primer is used at a higher concentration than a primer used in a general nucleic acid amplification reaction. As a template contained in a reaction solution, genomic DNA prepared from a target organism for which a DNA library is produced can be used.

In the method for producing a DNA library of the present invention, a target organism species is not particularly limited, and a target organism species can be any organism species such as an animal including a human, a plant, a microorganism, or a virus. In other words, according to the method for producing a DNA library of the present invention, a DNA library can be produced from any organism species.

In the method for producing a DNA library of the present invention, the concentration of a random primer is specified as described above. Thus, a nucleic acid fragment (or nucleic acid fragments) can be amplified with high reproducibility. The term "reproducibility" used herein means an extent of concordance among nucleic acid fragments amplified by a plurality of nucleic acid amplification reactions carried out with the use of the same template and the same random primer. That is, the term "high reproducibility (or the expression "reproducibility is high")" means that the extent of concordance among nucleic acid fragments amplified by a plurality of nucleic acid amplification reactions carried out with the use of the same template and the same random primer is high.

The extent of reproducibility can be evaluated by, for example, conducting a plurality of nucleic acid amplification reactions with the use of the same template and the same random primer, calculating the Spearman's rank correlation coefficient for the fluorescence unit (FU) obtained as a result of electrophoresis of the resulting amplified fragments, and evaluating the extent of reproducibility on the basis of such coefficient. The Spearman's rank correlation coefficient is generally represented by the symbol ρ. When ρ is greater than 0.9, for example, the reproducibility of the amplification reaction of interest can be evaluated to be sufficient.

### [Random primer]

A sequence constituting a random primer that can be used in the method for producing a DNA library according to the present invention is not particularly limited. For example, a random primer comprising nucleotides comprising 9 to 30 nucleotides can be used. In particular, a random primer may be composed of any nucleotide sequence comprising 9 to 30 nucleotides, a nucleotide type (i.e., a sequence type) is not particularly limited, and a random primer may be composed of 1 or more types of nucleotide sequences, preferably 1 to 10,000 types of nucleotide sequences, more preferably 1 to 1,000 types of nucleotide sequences, further preferably 1 to 100 types of nucleotide sequences, and most preferably 1 to 96 types of nucleotide sequences. With the use of nucleotides (or a group of nucleotides)within the range mentioned above for a random primer, an amplified nucleic acid fragment can be obtained with higher reproducibility. When a random primer comprises a plurality of nucleotide sequences, it is not necessary that all nucleotide sequences comprise the same number of nucleotides (9 to 30 nucleotides). A random primer may comprise a plurality of nucleotide sequences composed of a different number of nucleotides.

In general, in order to obtain a specific amplicon by a nucleic acid amplification reaction, the nucleotide sequence of a primer corresponding to the amplicon is designed. For example, a pair of primers are designed such that the primers sandwich a site corresponding to an amplicon of a template DNA of genomic DNA or the like. In such case, as the primers are designed to be hybridized to a specific region included in a template, they may be referred to as "specific primers."

Meanwhile, a random primer is different from a primer that is designed to obtain a specific amplicon, and it is designed to obtain a random amplicon but not to be hybridized to a specific region of a template DNA. A random primer may have any nucleotide sequence and can contribute to random amplicon amplification when it is incidentally hybridized to a region included in template DNA.

In other words, a random primer can be regarded as nucleotides involved in random amplicon amplification comprsing an arbitrary sequence as described above. Here, such arbitrary sequence is not particularly limited. However, it may be designed as, for example, a nucleotide sequence randomly selected from the group consisting of adenine, guanine, cytosine, and thymine or a specific nucleotide sequence. Examples of a specific nucleotide sequence include a nucleotide sequence including a restriction enzyme recognition sequence or a nucleotide sequence having an adapter sequence used for a next-generation sequencer.

When designing plural types of nucleotides for random primers, it is possible to use a method for designing a plurality of nucleotide sequences having certain lengths by randomly selecting from the group consisting of adenine, guanine, cytosine, and thymine. In addition, when designing different types of nucleotides for random primers, it is also possible to use a method for designing a plurality of nucleotide sequences each comprising a common part consisting of a specific nucleotide sequence and a non-common part consisting of an arbitrary nucleotide sequence. Here, the non-common part may consist of a nucleotide sequence randomly selected from the group consisting of adenine, guanine, cytosine, and thymine or all or one of combinations of four types of nucleotides which are adenine, guanine, cytosine, and thymine. The common part is not particularly limited, and it may consist of any nucleotide sequence. It may consist of, for example, a nucleotide sequence including a restriction enzyme recognition sequence, a nucleotide sequence having an adapter sequence used for a next-generation sequencer, or a nucleotide sequence common in a specific gene family.

When designing plural types of nucleotide sequences having certain lengths by randomly selecting nucleotides from four types of nucleotides for a plurality of random primers, 30% or more, preferably 50% or more, more preferably 70% or more, and further preferably 90% or more of the entire such sequences exhibit 70% or less, preferably 60% or less, more preferably 50% or less, and most preferably 40% or less identity. By designing different types of nucleotide sequences having certain lengths by randomly selecting nucleotides from different types of nucleotides for a plurality of random primers exhibiting the identity within such range, an amplified fragment can be obtained over the entire genomic DNA of the target organism species. Thus, uniformity of the amplified fragment can be enhanced.

When designing a plurality of nucleotide sequences each comprising a common part consisting of a specific nucleotide sequence and a non-common part consisting of an arbitrary nucleotide sequence for a plurality of random primers, it is possible to design, for example, a nucleotide sequence comprising a non-common part consisting of several nucleotides on the 3' end side and a common part consisting of the remaining nucleotides on the 5' end side. By allowing a non-common part to consist of n number of nucleotides on the 3' end side, it is possible to design 4ⁿ types of random primers. Here, the expression "n number" may refer to 1 to 5, preferably 2 to 4, and more preferably 2 to 3.

For example, it is possible to design, as a random primer comprising a common part and a non-common part, 16 types of random primers in total, each of which has an adapter sequence (common part) used for a next-generation sequencer on the 5' end side and two nucleotides (non-common part) on the 3' end side in total. It is possible to design 64 types of random primers in total by setting the number of nucleotides on the 3'end side to 3 nucleotides (non-common part). The more types of random primers, the more comprehensively the amplified fragments can be obtained throughout the genomic DNA of the target organism species. Therefore, when designing a random primer consisting of a common part and a non-common part, it is preferable that 3 nucleotides exist on the 3 'end side.

However, for example, after designing 64 types of nucleotide sequences each comprising a common part and a non-common part consisting of 3 nucleotides, not more than 63 types of random primers selected from these 64 types of nucleotide sequences may be used. In other words, as compared with the case of using all 64 types of random primers, in the case of using not more than 63 types of random primers, excellent results can be obtained in a nucleic acid amplification reaction or analysis using a next generation sequencer. Specifically, when 64 types of random primers are used, the number of reads of a specific nucleic acid amplification fragment might become remarkably large. In such case, favorable analysis results can be obtained by using the remaining 63 random primers excluding one or more random primers involved in the amplification of the specific nucleic acid amplification fragment from 64 types of random primers.

Similarly, in the case of designing 16 types of random primers each comprising a common part and a non-common part of 2 nucleotides, when not more than 15 types of random primers selected from 16 types of random primers are used, favorable analysis results may be obtained in a nucleic acid amplification reaction or analysis using a next generation sequencer.

Nucleotides constituting a random primer are preferably designed such that the G-C content is 5% to 95%, more preferably 10% to 90%, further preferably 15% to 80%, and most preferably 20% to 70%. With the use of a set of nucleotides having a G-C content within the above range as a random primer, amplified nucleic acid fragments can be obtained with enhanced reproducibility. The G-C content is the percentage of guanine and cytosine contained in the whole nucleotide chain.

Further, nucleotides constituting a random primer are designed such that consecutive nucleotides account for preferably 80% or less, more preferably 70% or less, further preferably 60% or less, and most preferably 50% or less with respect to the entire sequence length. Alternatively, nucleotides constituting a random primer are designed such that the number of consecutive nucleotides is preferably 8 or less, more preferably 7 or less, further preferably 6 or less, and most preferably 5 or less. An amplified nucleic acid fragment can be obtained with enhanced reproducibility with the use of a set of nucleotides constituting a random primer, for which the number of consecutive nucleotides falls within the above range.

In addition, it is preferable that nucleotides constituting a random primer be designed not to constitute a complementary region of 6 or more, more preferably 5 or more, and further preferably 4 or more nucleotides in a molecule. When the nucleotides designed not to constitute a complementary region within the above range, double strand formation occurring in a molecule can be prevented, and amplified nucleic acid fragments can be obtained with enhanced reproducibility.

Further, when plural types of nucleotides are designed for a random primer, in particular, it is preferable that a plurality of nucleotides be designed not to constitute a complementary region of 6 or more, more preferably 5 or more, and further preferably 4 or more nucleotides while forming a plurality of nucleotide sequences. When different types of nucleotide sequences are designed Thus, double strand formation occurring between nucleotide sequences can be prevented, and amplified nucleic acid fragments can be obtained with enhanced reproducibility.

When plural types of nucleotides are designed for random primers, it is preferable that the nucleotides be designed not to constitute a complementary sequence of 6 or more, more preferably 5 or more, and further preferably 4 or more nucleotides at the 3' end side. When they are designed not to form a complementary sequence within the above range at the 3' end side, double strand formation occurring between nucleotide sequences can be prevented, and amplified nucleic acid fragments can be obtained with enhanced reproducibility.

The terms "complementary region" and "complementary sequence" refer to, for example, a region and a sequence exhibiting 80% to 100% identity (e.g., a region and a sequence each comprising 5 nucleotides in which 4 or 5 nucleotides are complementary to each other) or a region and a sequence exhibiting 90% to 100% identity (e.g., a region and a sequence each comprising 5 nucleotides in which 5 nucleotides are complementary to each other).

Further, nucleotides constituting a random primer are preferably designed to have a Tm value suitable for thermal cycle conditions (in particular, an annealing temperature) in a nucleic acid amplification reaction. A Tm value can be calculated by a conventional method, such as the nearest neighbor base pair approach, the Wallace method, or the GC% method, although a method of calculation is not particularly limited thereto. Specifically, nucleotides used for a random primer are preferably designed to have a Tm value of 10°C to 85°C, more preferably 12°C to 75°C, further preferably 14°C to 70°C, and most preferably 16°C to 65°C. By designing Tm values for nucleotides within the above range, amplified nucleic acid fragments can be obtained with enhanced reproducibility under given thermal cycle conditions (in particular, at a given annealing temperature) in a nucleic acid amplification reaction.

Furthermore, when different types of nucleotides constituting a random primer are designed, in particular, a variation for Tm among a plurality of nucleotides is preferably 50°C or less, more preferably 45°C or less, further preferably 40°C or less, and most preferably 35°C or less. When the nucleotides are designed such that a variation for Tm among a plurality of nucleotides falls within the above range, amplified nucleic acid fragments can be obtained with enhanced reproducibility under given thermal cycle conditions (in particular, at a given annealing temperature) in a nucleic acid amplification reaction.

### [Nucleic acid amplification reaction]

According to the method for producing a DNA library of the present invention, many amplification fragments are obtained via a nucleic acid amplification reaction conducted with the use of the random primer and genomic DNA as a template described above. In particular, in such a nucleic acid amplification reaction, the concentration of a random prime in a reaction solution is set higher than the primer concentration in a usual nucleic acid amplification reaction. Thus, many amplification fragments can be obtained using genomic DNA as a template while achieving high reproducibility. The thus obtained many amplification fragments can be used for a DNA library that can be applied to genotyping and the like.

A nucleic acid amplification reaction is a reaction for synthesizing amplification fragments in a reaction solution containing genomic DNA as a template, the above-mentioned random primers, DNA polymerase, deoxynucleoside triphosphate as a substrate (i.e., dNTP, which is a mixture of dATP, dCTP, dTTP, and dGTP), and a buffer under given thermal cycle conditions. As it is necessary to add Mg²⁺ at a given concentration to a reaction solution in a nucleic acid amplification reaction, the buffer of the above composition contains MgCl₂. When the buffer does not contain MgCl₂, MgCl₂ is further added to the above composition.

In particular, in a nucleic acid amplification reaction, it is preferable to adequately set the concentration of a random primer in accordance with the nucleotide length of the random primer. When different types of nucleotides constitute random primers with different nucleotide lengths, the average of nucleotide lengths of random primers may be set as the nucleotide length (the average may be a simple average or the weight average taking the amount of nucleotides into account).

Specifically, a nucleic acid amplification reaction is conducted using a random primer comprising 9 to 30 nucleotides at a random primer concentration of 4 to 200 µM and preferably at 4 to 100 µM. Under such conditions, many amplified fragments, and in particular, many amplified fragments comprising 100 to 500 nucleotides via a nucleic acid amplification reaction can be obtained while achieving high reproducibility.

More specifically, when a random primer comprises 9 to 10 nucleotides, the random primer concentration is preferably 40 to 60 µM. When a random primer comprises 10 to 14 nucleotides, it is preferable that the random primer concentration satisfy 100 µM or less and y > 3E + 08x^{-6.974}, provided that the nucleotide length of the random primer is represented by "y" and the concentration of the random primer is represented by "x." When a random primer comprises 14 to 18 nucleotides, the random primer concentration is preferably 4 to 100 µM. When a random primer comprises 18 to 28 nucleotides, the random primer concentration satisfies preferably 4 µM or more and y < 8E + 08x^{-5.533}. When a random primer comprises 28 to 29 nucleotides, the random primer concentration is preferably 6 to 10 µM. By setting the random primer concentration in accordance with the nucleotide length of a random primer as described above, many amplified fragments can be obtained with improved certainty while achieving high reproducibility.

As described in the Examples below, the above inequations (y > 3E + 08x^{-6.974} and y < 8E + 08x^{-5.533}) are developed to be able to represent the random primer concentration at which many DNA fragments comprising 100 to 500 nucleotides can be obtained with favorable reproducibility as a result of thorough inspection of the correlation between the random primer length and the random primer concentration.

The amount of genomic DNA as a template in a nucleic acid amplification reaction is not particularly limited. However, it is preferably 0.1 to 1000 ng, more preferably 1 to 500 ng, further preferably 5 to 200 ng, and most preferably 10 to 100 ng, when the amount of the reaction solution is 50 µl. By setting the amount of genomic DNA as a template within the above range, many amplified fragments can be obtained without inhibiting the amplification reaction with a random primer, while achieving high reproducibility.

Genomic DNA can be prepared in accordance with a conventional technique without particular limitations. With the use of a commercially available kit, genomic DNA can be easily prepared from a target organism species. Genomic DNA extracted from an organism in accordance with a conventional technique or with the use of a commercially available kit may be used as is, genomic DNA extracted from an organism and purified may be used, or genomic DNA subjected to restriction enzyme treatment or ultrasonic treatment may be used.

DNA polymerase used in a nucleic acid amplification reaction is not particularly limited, and an enzyme having DNA polymerase activity under thermal cycle conditions for a nucleic acid amplification reaction can be used. Specifically, heat-stable DNA polymerase used for a general nucleic acid amplification reaction can be used. Examples of DNA polymerase include thermophilic bacteria-derived DNA polymerase such as Taq DNA polymerase, and hyperthermophilic Archaea-derived DNA polymerase such as KOD DNA polymerase or Pfu DNA polymerase. In a nucleic acid amplification reaction, it is particularly preferable to use Pfu DNA polymerase as DNA polymerase in combination with the random primer described above. With the use of such DNA polymerases, many amplified fragments can be obtained with improved certainty while achieving high reproducibility.

In a nucleic acid amplification reaction, the concentration of deoxynucleoside triphosphate as a substrate (i.e., dNTP, which is a mixture of dATP, dCTP, dTTP, and dGTP) is not particularly limited, and it can be 5 µM to 0.6 mM, preferably 10 µM to 0.4 mM, and more preferably 20 µM to 0.2 mM. By setting the concentration of dNTP as a substrate within such range, errors caused by incorrect incorporation by DNA polymerase can be prevented, and many amplified fragments can be obtained while achieving high reproducibility.

A buffer used in a nucleic acid amplification reaction is not particularly limited. For example, a solution comprising MgCl₂ as described above, Tris-HCl (pH 8.3), and KCl can be used. The concentration of Mg²⁺ is not particularly limited. For example, it can be 0.1 to 4.0 mM, preferably 0.2 to 3.0 mM, more preferably 0.3 to 2.0 mM, and further preferably 0.5 to 1.5 mM. By designating the concentration of Mg²⁺ in the reaction solution within such range, many amplified fragments can be obtained while achieving high reproducibility.

Thermal cycling conditions of a nucleic acid amplification reaction are not particularly limited, and a common thermal cycle can be adopted. A specific example of a thermal cycle comprises a first step of thermal denaturation in which genomic DNA as a template is dissociated into single strands, a cycle comprising thermal denaturation, annealing, and extension repeated a plurality of times (e.g., 20 to 40 times), a step of extension for a given period of time according to need, and the final step of storage.

Thermal denaturation can be performed at, for example, 93°C to 99°C, preferably 95°C to 98°C, and more preferably 97°C to 98°C. Annealing can be performed at, for example, 30°C to 70°C, preferably 35°C to 68°C, and more preferably 37°C to 65°C, although it varies depending on the Tm value of a random primer. Extension can be performed at, for example, 70°C to 76°C, preferably 71°C to 75°C, and more preferably 72°C to 74°C. Storage can be performed at, for example, 4°C.

The first step of thermal denaturation can be performed within the temperature range described above for a period of, for example, 5 seconds to 10 minutes, preferably 10 seconds to 5 minutes, and more preferably 30 seconds to 2 minutes. In the cycle comprising "thermal denaturation, annealing, and extension," thermal denaturation can be carried out within the temperature range described above for a period of, for example, 2 seconds to 5 minutes, preferably5 seconds to 2 minutes, and more preferably 10 seconds to 1 minute. In the cycle comprising "thermal denaturation, annealing, and extension," annealing can be carried out within the temperature range described above for a period of, for example, 1 second to 3 minutes, preferably 3 seconds to 2 minutes, and more preferably 5 seconds to 1 minute. In the cycle comprising "thermal denaturation, annealing, and extension," extension can be carried out within the temperature range described above for a period of, for example, 1 second to 3 minutes, preferably 3 seconds to 2 minutes, and more preferably 5 seconds to 1 minute.

According to the method for producing a DNA library of the present invention, amplified fragments may be obtained by a nucleic acid amplification reaction that employs a hot start method. The hot start method is intended to prevent mis-priming or non-specific amplification caused by primer-dimer formation prior the cycle comprising "thermal denaturation, annealing, and extension." The hot start method involves the use of an enzyme in which DNA polymerase activity has been suppressed by binding an anti-DNA polymerase antibody thereto or chemical modification thereof. Thus, DNA polymerase activity can be suppressed and a non-specific reaction prior to the thermal cycle can be prevented. According to the hot start method, a temperature is set high in the first thermal cycle, DNA polymerase activity is thus recovered, and the subsequent nucleic acid amplification reaction is then allowed to proceed.

As described above, many amplified fragments can be obtained with the use of genomic DNA as a template and a random primer by conducting a nucleic acid amplification reaction with the use of a random primer comprising 9 to 30 nucleotides and setting the concentration thereof to 4 to 200 µM in a reaction solution. With the use of the random primer comprising 9 to 30 nucleotides while setting the concentration thereof to 4 to 200 µM in a reaction solution, a nucleic acid amplification reaction can be performed with very high reproducibility. According to the nucleic acid amplification reaction, specifically, many amplified fragments can be obtained while achieving very high reproducibility. Therefore, the thus obtained many amplified fragments can be used for a DNA library in genetic analysis targeting genomic DNA.

By performing a nucleic acid amplification reaction with the use of the random primer comprising 9 to 30 nucleotides and setting the concentration thereof in a reaction solution to 4 to 200 µM, in particular, many amplified fragments comprising about 100 to 500 nucleotides can be obtained with the use of genomic DNA as a template. Such many amplified fragments comprising about 100 to 500 nucleotides are suitable for mass analysis of nucleotide sequences with the use of, for example, a next-generation sequencer, and highly accurate sequence information can thus be obtained. According to the present invention, a DNA library including DNA fragments comprising about 100 to 500 nucleotides can be produced.

By performing a nucleic acid amplification reaction with the use of the random primer comprising 9 to 30 nucleotides and setting the concentration thereof to 4 to 200 µM in a reaction solution, in particular, amplified fragments can be obtained uniformly across genomic DNA. In other words, DNA fragments are amplified in a distributed manner across the genome but not in a localized manner in a specific region of genomic DNA in a nucleic acid amplification reaction with the use of such random primer. That is, according to the present invention, a DNA library can be produced uniformly across the entire genome.

After performing the nucleic acid amplification reaction using the above-mentioned random primer, restriction enzyme treatment, size selection treatment, sequence capture treatment, and the like can be performed on the obtained amplified fragments. By carrying out restriction enzyme treatment, size selection treatment, and sequence capture treatment on the amplified fragments, specific amplified fragments (a fragment having a specific restriction enzyme site, an amplified fragment with a specific size range, and an amplified fragment having a specific sequence) can be obtained from among the obtained amplified fragments. Then, specific amplified fragments obtained by these treatments can be used for a DNA library.

### [Method of genomic DNA analysis]

With the use of the DNA library produced in the manner described above, genomic DNA analysis such as genotyping can be performed. Such DNA library has very high reproducibility, the size thereof is suitable for a next-generation sequencer, and it has uniformity across the entire genome. Accordingly, the DNA library can be used as a DNA marker (also referred to as "genetic marker" or "gene marker"). The term "DNA marker" refers to a wide range of characteristic nucleotide sequences present in genomic DNA. In addition, a DNA marker may be especially a nucleotide sequence on the genome serving as a marker associated with genetic traits. A DNA marker can be used for, for example, genotype identification, linkage mapping, gene mapping, breeding comprising a step of selection with the use of a marker, back crossing using a marker, quantitative trait locus mapping, bulked segregant analysis, variety identification, or discontinuous imbalance mapping.

For example, the nucleotide sequence of a DNA library prepared as described above is determined using a next generation sequencer or the like, and the presence or absence of a DNA marker can be confirmed based on the obtained nucleotide sequence.

As an example, the presence or absence of a DNA marker can be confirmed from the number of reads of the obtained nucleotide sequence. While a next-generation sequencer is not particularly limited, such sequencer is also referred to as a "second-generation sequencer," and such sequencer is an apparatus for nucleotide sequencing that allows simultaneous determination of nucleotide sequences of several tens of millions of DNA fragments. The sequencing principle of a next-generation sequencer is not particularly limited. For example, sequencing can be carried out in accordance with a method in which sequencing is carried out while amplifying and synthesizing target DNA on flow cells by bridge PCR method and the sequencing-by-synthesis method, or in accordance with a method in which sequencing is carried out by emulsion PCR and the pyrosequencing method for assaying the amount of pyrophosphoric acids released upon DNA synthesis. More specific examples of next-generation sequencers include MiniSeq, MiSeq, NextSeq, HiSeq, and HiSeq X Series (Illumina, Inc.) and Roche 454 GS FLX sequencers (Roche).

In another example, the presence or absence of a DNA marker can be confirmed by comparing the nucleotide sequence obtained for the DNA library prepared as described above with the reference nucleotide sequence. Here, the reference nucleotide sequence means a known sequence as a reference, and it can be, for example, a known sequence stored in a database. That is, a DNA library is prepared as described above for a given organism, its nucleotide sequence is determined, and the nucleotide sequence of the DNA library is compared with the reference nucleotide sequence. A nucleotide sequence that differs from the reference nucleotide sequence can be designated as a DNA marker (a characteristic nucleotide sequence existing in the genomic DNA) related to the organism. For each specified DNA marker, the relevance to the genetic trait (phenotype) can be determined by further analysis according to a conventional method. In other words, a DNA marker related to a phenotype (sometimes referred to as a "selective marker") can be identified from among the DNA markers identified as described above.

Furthermore, in another example, the presence or absence of a DNA marker can be confirmed by comparing the nucleotide sequence obtained for the DNA library prepared as described above with the nucleotide sequence of a DNA library prepared as described above using genomic DNA from a different organism or tissue. In other words, a DNA library is prepared as described above for each of two or more organisms or two different tissues, the nucleotide sequences thereof are determined, and the nucleotide sequences of the DNA libraries are compared with each other. Then, a nucleotide sequence that differs between the DNA libraries can be designated as a DNA marker (a characteristic nucleotide sequence existing in the genomic DNA) related to the sampled organism or tissue. For each specified DNA marker, the relevance to the genetic trait (phenotype) can be determined by further analysis according to a conventional method. In other words, a DNA marker related to a phenotype (sometimes referred to as a "selective marker") can be identified from among the DNA markers identified as described above.

As an aside, it is also possible to design a pair of primers which specifically amplify the DNA marker based on the obtained nucleotide sequence. It is also possible to confirm the presence or absence of the DNA marker in the extracted genomic DNA by performing a nucleic acid amplification reaction using a pair of designed primers and genomic DNA extracted from a target organism as a template.

Alternatively, DNA libraries prepared as described above can be used for metagenomic analysis for examining a wide variety of microorganisms and the like, genome mutation analysis of somatic cells of tumor tissue or the like, genotyping using microarrays, determination and analysis of ploidy, calculation and analysis of the number of chromosomes, analysis of the increase and decrease of chromosomes, analysis of partial insertion/deletion/replication/translocation of chromosomes, analysis of contamination with foreign genome, parentage discrimination analysis, and testing and analysis of crossed seed purity.

### [Application to next generation sequencing technology]

As described above, by conducting a nucleic acid amplification reaction with a random primer contained at a high concentration in a reaction solution, it is possible to obtain many amplified fragments with favorable reproducibility using genomic DNA as a template. Since each obtained amplified fragment has nucleotide sequence at both ends thereof which are the same as those of the random primer, it can be easily applied to the next generation sequence technology by utilizing the nucleotide sequence.

Specifically, as described above, a nucleic acid amplification reaction is conducted in a reaction solution (first reaction solution) containing genomic DNA and a random primer at a high concentration to obtain many amplified fragments (first DNA fragments) using the genomic DNA as a template. Next, a nucleic acid amplification reaction is conducted in a reaction solution (second reaction solution) containing the obtained many amplified fragments (first DNA fragments) and a primer designed based on the nucleotide sequence of the random primer (referred to as "next generation sequencer primer"). A next generation sequencer primer to be used herein is a nucleotide sequence including a region used for a nucleotide sequencing reaction. More specifically, for example, the next-generation sequencer primer may be a nucleotide sequence having a region necessary for a nucleotide sequencing reaction (sequence reaction) by a next-generation sequencer, in which the nucleotide sequence at the 3' end of the primer is a nucleotide sequence having 70% or more identity, preferably 80% or more identity, more preferably 90% or more identity, still more preferably 95% or more identity, further preferably 97% or more identity, and most preferably 100% identity to the nucleotide sequence on the 5' end side of the first DNA fragment.

Here, the "region used for a nucleotide sequencing reaction" included in a next-generation sequencer primer is not particularly limited because it varies depending on type of the next-generation sequencer. However, in the case of conducting a nucleotide sequencing reaction using a next-generation sequencer with a sequence primer, such region may be, for example, a nucleotide sequence complementary to the nucleotide sequence of the sequence primer. In a case in which a sequencing reaction is conducted by a next-generation sequencer using capture beads bound to given DNA, the "region used for a nucleotide sequencing reaction" refers to a nucleotide sequence complementary to the nucleotide sequence of the DNA bound to capture beads. Further, in a case in which a next-generation sequencer reads a sequence based on a current change when a DNA chain having a terminal hairpin loop passes through a protein having nano-sized pores, the "region used for a nucleotide sequencing reaction" may be a nucleotide sequence complementary to the nucleotide sequence forming the hairpin loop.

By designing the nucleotide sequence at the 3 'end of a next-generation sequencer primer as described above, the next-generation sequencer primer can be hybridized to the 3' end of the first DNA fragment under stringent conditions, and the second DNA fragment can be amplified using the first DNA fragment as a template. Stringent conditions mean conditions under which a so-called specific hybrid is formed while a nonspecific hybrid is not formed. For example, such conditions can be appropriately determined with reference to Molecular Cloning: A Laboratory Manual (Third Edition). Specifically, stringency can be determined by setting the temperature and the salt concentration in a solution upon Southern hybridization, and the temperature and the salt concentration in a solution in the washing step of Southern hybridization. More specifically, for example, the sodium concentration is set to 25 to 500 mM and preferably 25 to 300 mM and the temperature is set to 42°C to 68°C and preferably 42°C to 65°C under stringent conditions. More specifically, the sodium concentration is 5 × SSC (83 mM NaCl, 83 mM sodium citrate) and the temperature is 42°C.

In particular, when different types of random primers are used to obtain a first DNA fragment, next-generation sequencer primers may be prepared to correspond to all or some of random primers.

For example, in a case in which a set of different types of random primers (each having an arbitrary 3'-end sequence of several nucleotides) each comprising a common nucleotide sequence except several nucleotides (e.g., about 1 to 3 nucleotides) at the 3' end is used, all of the obtained many first DNA fragments have a common 5'-end sequence. Accordingly, the 3'-end nucleotide sequence of a next generation sequencer primer is designated to be a nucleotide sequence having 70% or more identity to the 5'-end nucleotide sequence common to the first DNA fragments. By designing next-generation sequencer primers as described above, it is possible to obtain next generation sequencer primers corresponding to all random primers. By using such next generation sequencer primers, it is possible to amplify second DNA fragments using all of the first DNA fragments as templates.

Similarly, even in a case in which a set of different types of random primers (each having an arbitrary 3'-end sequence of several nucleotides) each comprising a common nucleotide sequence except several nucleotides (e.g., about 1 to 3 nucleotides) at the 3' end is used, it is also possible to obtain second DNA fragments using some of the obtained many first DNA fragments as templates. Specifically, the 3'-end nucleotide sequence of a next generation sequencer primer is designated to be a nucleotide sequence having 70% or more identity to the 5'-end nucleotide sequence common to the first DNA fragments and the sequence comprising several nucleotides following the nucleotide sequence (corresponding to several nucleotides (arbitrary sequence) at the 3' end of the random primer) such that second DNA fragments can be amplified using some of the first DNA fragments as templates.

Meanwhile, in a case in which first DNA fragments are obtained using different types of random primers each consisting of an arbitrary nucleotide sequence, it is possible to obtain second DNA fragments using different types of next-generation sequencer primers such that the second DNA fragments correspond to all of the first DNA fragments, or it is also possible to obtain second DNA fragments using different types of next-generation sequencer primers such that the second DNA fragments correspond to some of the first DNA fragments.

As described above, the second DNA fragments amplified using next-generation sequencer primers have a region necessary for a nucleotide sequencing reaction (sequence reaction) by a next-generation sequencer, which is included in the next-generation sequencer primers. The region necessary for a sequence reaction is not particularly limited as it varies depending on a next generation sequencer. For example, when a next-generation sequencer primer is used in a next-generation sequencer based on the principle that sequencing is carried out while amplifying and synthesizing target DNA on flow cells by bridge PCR method and the sequencing-by-synthesis method, the next-generation sequencer primer needs to contain a region necessary for bridge PCR and a region necessary for the sequencing-by-synthesis method. The region necessary for bridge PCR is a region that is hybridized to an oligonucleotide immobilized on flow cells and has a length of 9 nucleotides including the 5 'end of the next generation sequencer primer. In addition, a region necessary for the sequencing-by-synthesis method is a region to which a sequence primer used in a sequence reaction is hybridized, and is a region in the middle of the next generation sequencer primer.

In addition, a next-generation sequencer may be an Ion Torrent sequencer. In the case of using the Ion Torrent sequencer, a next-generation sequencer primer has a so-called ion adapter on the 5 'end side and binds to a particle for conducting emulsion PCR. In addition, in the Ion Torrent sequencer, particles coated with a template amplified by emulsion PCR are placed on an ion chip and subjected to a sequence reaction.

Here, a nucleic acid amplification reaction using a next-generation sequencer primer and a second reaction solution containing the first DNA is not particularly limited, and conventional conditions for nucleic acid amplification reaction can be applied. That is, the conditions in [Nucleic acid amplification reaction] described above can be used. For example, the second reaction solution contains first DNA fragments as templates, the above-described next-generation sequencer primer, DNA polymerase, deoxynucleoside triphosphate as a substrate (i.e., dNTP, which is a mixture of dATP, dCTP, dTTP, and dGTP), and a buffer.

In particular, the concentration of the next-generation sequencer primer can be set to 0.01 to 5.0 µM, preferably 0.1 to 2.5 µM, and most preferably 0.3 to 0.7 µM.

While the amount of the first DNA fragments serving as templates in a nucleic acid amplification reaction is not particularly limited, it is preferably 0.1 to 1000 ng, more preferably 1 to 500 ng, further preferably 5 to 200 ng, and most preferably 10 to 100 ng when the amount of the reaction solution is 50 µl.

A method for preparing first DNA fragments as templates is not particularly limited. In the method, the reaction solution obtained after the completion of the nucleic acid amplification reaction using the above-described random primers may be used as is, or the reaction solution may be used after purifying the first DNA fragments therefrom.

Regarding the type of DNA polymerase, the concentration of deoxynucleoside triphosphate as a substrate (dNTP, i.e., a mixture of dATP, dCTP, dTTP and dGTP), the buffer composition, and temperature cycle conditions used for the nucleic acid amplification reaction, the conditions in [Nucleic acid amplification reaction] described above can be used. In addition, in a nucleic acid amplification reaction using next-generation sequencer primers, a hot start method may be employed, or amplified fragments may be obtained by a nucleic acid amplification reaction.

As described above, by using the first DNA fragments obtained using random primers as templates and using the second DNA fragments amplified using next-generation sequencer primers, it is possible to readily prepare a DNA library that can be applied to a next-generation sequencer.

In the above examples, a DNA library is prepared using the first DNA fragments obtained using random primers as templates and amplifying the second DNA fragments using next-generation sequencer primers. However, the scope of the present invention is not limited to Such examples. For example, the DNA library according to the present invention may be prepared by amplifying second DNA fragments using first DNA fragments obtained using random primers as templates and further obtaining third DNA fragments using the second DNA fragments as templates and next-generation sequencer primers, thereby obtaining a DNA library of the third DNA fragments applicable to a next generation sequencer.

Similarly, in order to prepare a DNA library applicable to a next-generation sequencer, after a nucleic acid amplification reaction using second DNA fragments as templates, a nucleic acid amplification reaction is repeatedly conducted using the obtained DNA fragments as templates, and next-generation sequencer primers are used for the final nucleic acid amplification reaction. In such case, the number of nucleic acid amplification reactions to be repeated is not particularly limited, but it is 2 to 10 times, preferably 2 to 5 times, and more preferably 2 to 3 times.

### Examples

Hereafter, the present invention is described in greater detail with reference to the Examples below, although the scope of the present invention is not limited to these Examples.

### [Example 1]

### 1. Flowchart

In this Example, a DNA library was prepared via PCR using genomic DNAs extracted from various types of organism species as templates and various sets of random primers in accordance with the flow chart shown in Fig. 1. In addition, with the use of the prepared DNA library, sequence analysis was performed by a so-called next-generation sequencer, and the genotype was analyzed based on the obtained read data.

### 2. Materials

In this Example, genomic DNAs were extracted from the sugarcane varieties NiF8 and Ni9, 22 hybrid progeny lines thereof, and the rice variety Nipponbare using the DNeasy Plant Mini Kit (QIAGEN), and the extracted genomic DNAs were purified. The purified genomic DNAs were used as NiF8-derived genomic DNA, Ni9-derived genomic DNA, genomic DNAs from 22 hybrid progeny lines, and Nipponbare-derived genomic DNA, respectively. In this Example, Human Genomic DNA was purchased as human DNA from TakaraBio and used as human-derived genomic DNA.

### 3. Method

### 3.1 Correlation between PCR conditions and DNA fragment sizes

### 3.1.1 Random primer designing

In order to design random primers, the GC content was set between 20% and 70%, and the number of consecutive nucleotides was adjusted to 5 or less. The nucleotide length was set at 16 levels (i.e., 8, 9, 10, 11, 12, 14, 16, 18, 20, 22, 24, 26, 28, 29, 30, and 35 nucleotides). For each nucleotide length, 96 types of nucleotide sequences were designed, and a set of 96 types of random primers was prepared for each nucleotide length. Concerning 10-nucleotide primers, 6 sets (each comprising 96 types of random primers) were designed (these 6 sets are referred to as 10-nucleotide primer A to 10-nucleotide primer F). In this Example, specifically, 21 different sets of random primers were prepared.

Tables 1 to 21 show nucleotide sequences of random primers contained in these 21 different sets of random primers.

### [Table 1-1]

**Table 1. Random primer list (10-nucleotide A)**

| No. | Primer sequence | SEQ ID NO: |
|---|---|---|
| 1 | AGACGTCGTT | 1 |
| 2 | GAGGCGATAT | 2 |
| 3 | GTGCGAACGT | 3 |
| 4 | TTATACTGCC | 4 |
| 5 | CAAGTTCGCA | 5 |
| 6 | ACAAGGTAGT | 6 |
| 7 | ACACAGCGAC | 7 |
| 8 | TTACCGATGT | 8 |
| 9 | CACAGAGTCG | 9 |
| 10 | TTCAGCGCGT | 10 |
| 11 | AGGACCGTGA | 11 |
| 12 | GTCTGTTCGC | 12 |
| 13 | ACCTGTCCAC | 13 |
| 14 | CCGCAATGAC | 14 |
| 15 | CTGCCGATCA | 15 |
| 16 | TACACGGAGC | 16 |
| 17 | CCGCATTCAT | 17 |
| 18 | GACTCTAGAC | 18 |
| 19 | GGAGAACTTA | 19 |
| 20 | TCCGGTATGC | 20 |
| 21 | GGTCAGGAGT | 21 |
| 22 | ACATTGGCAG | 22 |
| 23 | CGTAGACTGC | 23 |
| 24 | AGACTGTACT | 24 |
| 25 | TAGACGCAGT | 25 |
| 26 | CCGATAATCT | 26 |
| 27 | GAGAGCTAGT | 27 |
| 28 | GTACCGCGTT | 28 |
| 29 | GACTTGCGCA | 29 |
| 30 | CGTGATTGCG | 30 |
| 31 | ATCGTCTCTG | 31 |
| 32 | CGTAGCTACG | 32 |
| 33 | GCCGAATAGT | 33 |
| 34 | GTACCTAGGC | 34 |
| 35 | GCTTACATGA | 35 |
| 36 | TCCACGTAGT | 36 |
| 37 | AGAGGCCATC | 37 |
| 38 | CGGTGATGCT | 38 |
| 39 | CACTGTGCTT | 39 |
| 40 | CATGATGGCT | 40 |
| 41 | GCCACACATG | 41 |
| 42 | CACACACTGT | 42 |
| 43 | CAGAATCATA | 43 |
| 44 | ATCGTCTACG | 44 |
| 45 | CGAGCAATAC | 45 |
| 46 | ACAAGCGCAC | 46 |
| 47 | GCTTAGATGT | 47 |
| 48 | TGCATTCTGG | 48 |
| 49 | TGTCGGACCA | 49 |
| 50 | AGGCACTCGT | 50 |
| 51 | CTGCATGTGA | 51 |
| 52 | ACCACGCCTA | 52 |
| 53 | GAGGTCGTAC | 53 |
| 54 | AATACTCTGT | 54 |
| 55 | TGCCAACTGA | 55 |
| 56 | CCTGTTCGGT | 56 |
| 57 | GTAGAGAGTT | 57 |
| 58 | TACAGCGTAA | 58 |
| 59 | TGACGTGATG | 59 |
| 60 | AGACGTCGGT | 60 |
| 61 | CGCTAGGTTC | 61 |
| 62 | GCCTTATAGC | 62 |
| 63 | CCTTCGATCT | 63 |
| 64 | AGGCAACGTG | 64 |

**[Table 1-2]**

| No. | Primer sequence | SEQ ID NO: |
|---|---|---|
| 65 | TGAGCGGTGT | 65 |
| 66 | GTGTCGAACG | 66 |
| 67 | CGATGTTGCG | 67 |
| 68 | AACAAGACAC | 68 |
| 69 | GATGCTGGTT | 69 |
| 70 | ACCGGTAGTC | 70 |
| 71 | GTGACTAGCA | 71 |
| 72 | AGCCTATATT | 72 |
| 73 | TCGTGAGCTT | 73 |
| 74 | ACACTATGGC | 74 |
| 75 | GACTCTGTCG | 75 |
| 76 | TCGATGATGC | 76 |
| 77 | CTTGGACACT | 77 |
| 78 | GGCTGATCGT | 78 |
| 79 | ACTCACAGGC | 79 |
| 80 | ATGTGCGTAC | 80 |
| 81 | CACCATCGAT | 81 |
| 82 | AGCCATTAAC | 82 |
| 83 | AATCGACTGT | 83 |
| 84 | AATACTAGCG | 84 |
| 85 | TCGTCACTGA | 85 |
| 86 | CAGGCTCTTA | 86 |
| 87 | GGTCGGTGAT | 87 |
| 88 | CATTAGGCGT | 88 |
| 89 | ACTCGCGAGT | 89 |
| 90 | TTCCGAATAA | 90 |
| 91 | TGAGCATCGT | 91 |
| 92 | GCCACGTAAC | 92 |
| 93 | GAACTACATG | 93 |
| 94 | TCGTGAGGAC | 94 |
| 95 | GCGGCCTTAA | 95 |
| 96 | GCTAAGGACC | 96 |

### [Table 2-1]

**Table 2. Random primer list 10-nucleotide B)**

| No. | Primer sequence | SEQ ID NO: |
|---|---|---|
| 1 | ATAGCCATTA | 97 |
| 2 | CAGTAATCAT | 98 |
| 3 | ACTCCTTAAT | 99 |
| 4 | TCGAACATTA | 100 |
| 5 | ATTATGAGGT | 101 |
| 6 | AATCTTAGAG | 102 |
| 7 | TTAGATGATG | 103 |
| 8 | TACATATCTG | 104 |
| 9 | TCCTTAATCA | 105 |
| 10 | GTTGAGATTA | 106 |
| 11 | TGTTAACGTA | 107 |
| 12 | CATACAGTAA | 108 |
| 13 | CTTATACGAA | 109 |
| 14 | AGATCTATGT | 110 |
| 15 | AAGACTTAGT | 111 |
| 16 | TGCGCAATAA | 112 |
| 17 | TTGGCCATAT | 113 |
| 18 | TATTACGAGG | 114 |
| 19 | TTATGATCGC | 115 |
| 20 | AACTTAGGAG | 116 |
| 21 | TCACAATCGT | 117 |
| 22 | GAGTATATGG | 118 |
| 23 | ATCAGGACAA | 119 |
| 24 | GTACTGATAG | 120 |
| 25 | CTTATACTCG | 121 |
| 26 | TAACGGACTA | 122 |
| 27 | GCGTTGTATA | 123 |
| 28 | CTTAAGTGCT | 124 |
| 29 | ATACGACTGT | 125 |
| 30 | ACTGTTATCG | 126 |
| 31 | AATCTTGACG | 127 |
| 32 | ACATCACCTT | 128 |
| 33 | GGTATAGTAC | 129 |
| 34 | CTAATCCACA | 130 |
| 35 | GCACCTTATT | 131 |
| 36 | ATTGACGGTA | 132 |
| 37 | GACATATGGT | 133 |
| 38 | GATAGTCGTA | 134 |
| 39 | CAATTATCGC | 135 |
| 40 | CTTAGGTGAT | 136 |
| 41 | CATACTACTG | 137 |
| 42 | TAACGCGAAT | 138 |
| 43 | CAAGTTACGA | 139 |
| 44 | AATCTCAAGG | 140 |
| 45 | GCAATCATCA | 141 |
| 46 | TGTAACGTTC | 142 |
| 47 | TATCGTTGGT | 143 |
| 48 | CGCTTAAGAT | 144 |
| 49 | TTAGAACTGG | 145 |
| 50 | GTCATAACGT | 146 |
| 51 | AGAGCAGTAT | 147 |
| 52 | CAACATCACT | 148 |
| 53 | CAGAAGCTTA | 149 |
| 54 | AACTAACGTG | 150 |
| 55 | TTATACCGCT | 151 |
| 56 | GAATTCGAGA | 152 |
| 57 | TTACGTAACC | 153 |
| 58 | GCATGGTTAA | 154 |
| 59 | GCACCTAATT | 155 |
| 60 | TGTAGGTTGT | 156 |
| 61 | CCATCTGGAA | 157 |
| 62 | TTCGCGTTGA | 158 |
| 63 | AACCGAGGTT | 159 |
| 64 | GTACGCTGTT | 160 |

**[Table 2-2]**

| No. | Primer sequence | SEQ ID NO: |
|---|---|---|
| 65 | AGTATCCTGG | 161 |
| 66 | GGTTGTACAG | 162 |
| 67 | ACGTACACCA | 163 |
| 68 | TGTCGAGCAA | 164 |
| 69 | GTCGTGTTAC | 165 |
| 70 | GTGCAATAGG | 166 |
| 71 | ACTCGATGCT | 167 |
| 72 | GAATCGCGTA | 168 |
| 73 | CGGTCATTGT | 169 |
| 74 | ATCAGGCGAT | 170 |
| 75 | GTAAGATGCG | 171 |
| 76 | GGTCTCTTGA | 172 |
| 77 | TCCTCGCTAA | 173 |
| 78 | CTGCGTGATA | 174 |
| 79 | CATACTCGTC | 175 |
| 80 | ATCTGAGCTC | 176 |
| 81 | ACGGATAGTG | 177 |
| 82 | ACTGCAATGC | 178 |
| 83 | TAACGACGTG | 179 |
| 84 | TAGACTGTCG | 180 |
| 85 | CAGCACTTCA | 181 |
| 86 | AACATTCGCC | 182 |
| 87 | ACTAGTGCGT | 183 |
| 88 | ACGCTGTTCT | 184 |
| 89 | CGTCGAATGC | 185 |
| 90 | CTCTGACGGT | 186 |
| 91 | GTCGCCATGT | 187 |
| 92 | GGTCCACGTT | 188 |
| 93 | CGAGCGACTT | 189 |
| 94 | TTGACGCGTG | 190 |
| 95 | CTGAGAGCCT | 191 |
| 96 | CGCGCTAACT | 192 |

### [Table 3-1]

**Table 3. Random primer list (10-nucleotide C)**

| No. | Primer sequence | SEQ ID NO: |
|---|---|---|
| 1 | GGTCGTCAAG | 193 |
| 2 | AGGTTGACCA | 194 |
| 3 | TAACGGCAAC | 195 |
| 4 | GAGGCTGGAT | 196 |
| 5 | GTGCACACCT | 197 |
| 6 | TGAGGACCAG | 198 |
| 7 | TACTTGCGAG | 199 |
| 8 | AACTGTGAGA | 200 |
| 9 | CTCCATCAAC | 201 |
| 10 | CGGACTGTTA | 202 |
| 11 | TAGGACAGTC | 203 |
| 12 | AGAGGACACA | 204 |
| 13 | ACATTCGCGG | 205 |
| 14 | GCTTACTGCA | 206 |
| 15 | CAATACGTAA | 207 |
| 16 | AGACTTGCGC | 208 |
| 17 | GAGCGGTGTT | 209 |
| 18 | CGTGAGAGGT | 210 |
| 19 | AATCCGTCAG | 211 |
| 20 | ATACGTACCG | 212 |
| 21 | AACTGATTCC | 213 |
| 22 | CTGAGCGTAC | 214 |
| 23 | GTCGGATTCG | 215 |
| 24 | GCCGACCATA | 216 |
| 25 | GCAGAACTAA | 217 |
| 26 | CTAACGACCG | 218 |
| 27 | GCTGGACCAT | 219 |
| 28 | GACGCGGTTA | 220 |
| 29 | AGTGGTGAGC | 221 |
| 30 | CAGGCAGTCA | 222 |
| 31 | TCTGACGTCA | 223 |
| 32 | TACATGACGT | 224 |
| 33 | TGAGGCAACC | 225 |
| 34 | CAACTGCAGT | 226 |
| 35 | CGGAGATACG | 227 |
| 36 | CTTCGCAAGT | 228 |
| 37 | CTGGCATACG | 229 |
| 38 | TAACGTTCGC | 230 |
| 39 | CCGGCGTTAA | 231 |
| 40 | ACAAGACGCC | 232 |
| 41 | CCATTAGACT | 233 |
| 42 | GTCTGTGACA | 234 |
| 43 | GGCATTGGAC | 235 |
| 44 | TCTTCGCACG | 236 |
| 45 | TAGCCTGTGC | 237 |
| 46 | CACTGACCTA | 238 |
| 47 | CCGCACGATT | 239 |
| 48 | ATAGCACACG | 240 |
| 49 | GCACGTCATA | 241 |
| 50 | AAGCCGTTGG | 242 |
| 51 | CGGACCGTTA | 243 |
| 52 | TACACAGCGT | 244 |
| 53 | CGGACTTCAG | 245 |
| 54 | TAGAACGTCA | 246 |
| 55 | GGCATTGGAG | 247 |
| 56 | GGCACTCGTT | 248 |
| 57 | GTACCGTTAA | 249 |
| 58 | AATACGTGTC | 250 |
| 59 | CCATTGACGT | 251 |
| 60 | CGTGAATCGC | 252 |
| 61 | ATCAACGCGG | 253 |
| 62 | CGCCAAGGTA | 254 |
| 63 | AGAAGACGCC | 255 |
| 64 | CCGCATAGTC | 256 |

**[Table 3-2]**

| No. | Primer sequence | SEQ ID NO: |
|---|---|---|
| 65 | CTTATATGTG | 257 |
| 66 | GGTCTCATCG | 258 |
| 67 | CCACCATGTC | 259 |
| 68 | ACGAATGTGT | 260 |
| 69 | GGTAGTAACA | 261 |
| 70 | GCCACTTAAT | 262 |
| 71 | ATATTGCGCC | 263 |
| 72 | GACCAATAGT | 264 |
| 73 | AACAACACGG | 265 |
| 74 | ATAGCCGATG | 266 |
| 75 | CGAGAGCATA | 267 |
| 76 | CGAGACATGA | 268 |
| 77 | CGCCAAGTTA | 269 |
| 78 | TTATAATCGC | 270 |
| 79 | TAGAAGTGCA | 271 |
| 80 | GGAGGCATGT | 272 |
| 81 | GCCACTTCGA | 273 |
| 82 | TCCACGGTAC | 274 |
| 83 | CAACTATGCA | 275 |
| 84 | CAAGGAGGAC | 276 |
| 85 | GAGGTACCTA | 277 |
| 86 | GAGCGCATAA | 278 |
| 87 | TCGTCACGTG | 279 |
| 88 | AACTGTGACA | 280 |
| 89 | TCCACGTGAG | 281 |
| 90 | ACACTGCTCT | 282 |
| 91 | TACGGTGAGC | 283 |
| 92 | CGGACTAAGT | 284 |
| 93 | AAGCCACGTT | 285 |
| 94 | CAATTACTCG | 286 |
| 95 | TCTGGCCATA | 287 |
| 96 | TCAGGCTAGT | 288 |

### [Table 4-1]

**Table 4. Random primer list (10-nucleotide D)**

| No. | Primer sequence | SEQ ID NO: |
|---|---|---|
| 1 | TTGACCCGGA | 289 |
| 2 | TTTTTATGGT | 290 |
| 3 | ATGTGGTGCG | 291 |
| 4 | AAGGCGCTAG | 292 |
| 5 | TCCAACTTTG | 293 |
| 6 | CCATCCCATC | 294 |
| 7 | CAATACGAGG | 295 |
| 8 | GAGTGTTACC | 296 |
| 9 | GCCTCCTGTA | 297 |
| 10 | CGAAGGTTGC | 298 |
| 11 | GAGGTGCTAT | 299 |
| 12 | TAGGATAATT | 300 |
| 13 | CGTTGTCCTC | 301 |
| 14 | TGAGACCAGC | 302 |
| 15 | TGCCCAAGCT | 303 |
| 16 | TACTGAATCG | 304 |
| 17 | TTACATAGTC | 305 |
| 18 | ACAAAGGAAA | 306 |
| 19 | CTCGCTTGGG | 307 |
| 20 | CCTTGCGTCA | 308 |
| 21 | TAATTCCGAA | 309 |
| 22 | GTGAGCTTGA | 310 |
| 23 | ATGCCGATTC | 311 |
| 24 | GCTTGGGCTT | 312 |
| 25 | ACAAAGCGCC | 313 |
| 26 | GAAAGCTCTA | 314 |
| 27 | TACCGACCGT | 315 |
| 28 | TCGAAGAGAC | 316 |
| 29 | GTCGCTTACG | 317 |
| 30 | GGGCTCTCCA | 318 |
| 31 | GCGCCCTTGT | 319 |
| 32 | GGCAATAGGC | 320 |
| 33 | CAAGTCAGGA | 321 |
| 34 | GGGTCGCAAT | 322 |
| 35 | CAGCAACCTA | 323 |
| 36 | TTCCCGCCAC | 324 |
| 37 | TGTGCATTTT | 325 |
| 38 | ATCAACGACG | 326 |
| 39 | GTGACGTCCA | 327 |
| 40 | CGATCTAGTC | 328 |
| 41 | TTACATCCTG | 329 |
| 42 | AGCCTTCAAT | 330 |
| 43 | TCCATCCGAT | 331 |
| 44 | GACTGGGTCT | 332 |
| 45 | TTCGGTGGAG | 333 |
| 46 | GACCAGCACA | 334 |
| 47 | CATTAACGGA | 335 |
| 48 | TTTTTCTTGA | 336 |
| 49 | CATTGCACTG | 337 |
| 50 | TGCGGCGATC | 338 |
| 51 | ATATTGCGGT | 339 |
| 52 | GACGTCGCTC | 340 |
| 53 | TCGCTTATCG | 341 |
| 54 | GCGCAGACAC | 342 |
| 55 | CATGTATTGT | 343 |
| 56 | TCTATAACCT | 344 |
| 57 | GTGGAGACAA | 345 |
| 58 | CGAAGATTAT | 346 |
| 59 | TAGCAACTGC | 347 |
| 60 | ATAATCGGTA | 348 |
| 61 | CAGGATGGGT | 349 |
| 62 | GACGATTCCC | 350 |
| 63 | CACGCCTTAC | 351 |
| 64 | AGTTGGTTCC | 352 |

**[Table 4-2]**

| No. | Primer sequence | SEQ ID NO: |
|---|---|---|
| 65 | TCTTATCAGG | 353 |
| 66 | CGAGAAGTTC | 354 |
| 67 | GTGGTAGAAT | 355 |
| 68 | TAGGCTTGTG | 356 |
| 69 | ATGCGTTACG | 357 |
| 70 | ACTACCGAGG | 358 |
| 71 | CGAGTTGGTG | 359 |
| 72 | GGACGATCAA | 360 |
| 73 | AACAGTATGC | 361 |
| 74 | TTGGCTGATC | 362 |
| 75 | AGGATTGGAA | 363 |
| 76 | CATATGGAGA | 364 |
| 77 | CTGCAGGTTT | 365 |
| 78 | CTCTCTTTTT | 366 |
| 79 | AGTAGGGGTC | 367 |
| 80 | ACACCGCAAG | 368 |
| 81 | GAAGCGGGAG | 369 |
| 82 | GATACGGACT | 370 |
| 83 | TACGACGTGT | 371 |
| 84 | GTGCCTCCTT | 372 |
| 85 | GGTGACTGAT | 373 |
| 86 | ATATCTTACG | 374 |
| 87 | AATCATACGG | 375 |
| 88 | CTCTTGGGAC | 376 |
| 89 | GACGACAAAT | 377 |
| 90 | GTTGCGAGGT | 378 |
| 91 | AAACCGCACC | 379 |
| 92 | GCTAACACGT | 380 |
| 93 | ATCATGAGGG | 381 |
| 94 | GATTCACGTA | 382 |
| 95 | TCTCGAAAAG | 383 |
| 96 | CTCGTAACCA | 384 |

### [Table 5-1]

**Table 5. Random primer list (10-nucleotide E)**

| No. | Primer sequence | SEQ ID NO: |
|---|---|---|
| 1 | GTTACACACG | 385 |
| 2 | CGTGAAGGGT | 386 |
| 3 | ACGAGCATCT | 387 |
| 4 | ACGAGGGATT | 388 |
| 5 | GCAACGTCGG | 389 |
| 6 | CACGGCTAGG | 390 |
| 7 | CGTGACTCTC | 391 |
| 8 | TCTAGACGCA | 392 |
| 9 | CTGCGCACAT | 393 |
| 10 | ATGCTTGACA | 394 |
| 11 | TTTGTCGACA | 395 |
| 12 | ACGTGTCAGC | 396 |
| 13 | GAAAACATTA | 397 |
| 14 | ACATTAACGG | 398 |
| 15 | GTACAGGTCC | 399 |
| 16 | CTATGTGTAC | 400 |
| 17 | GCGTACATTA | 401 |
| 18 | GATTTGTGGC | 402 |
| 19 | TCGCGCGCTA | 403 |
| 20 | ACAAGGGCGA | 404 |
| 21 | AACGCGCGAT | 405 |
| 22 | CGTAAATGCG | 406 |
| 23 | TAGGCACTAC | 407 |
| 24 | GCGAGGATCG | 408 |
| 25 | CACGTTTACT | 409 |
| 26 | TACCACCACG | 410 |
| 27 | TTAACAGGAC | 411 |
| 28 | GCTGTATAAC | 412 |
| 29 | GTTGCTGGCA | 413 |
| 30 | AGTGTGGCCA | 414 |
| 31 | CTGCGGTTGT | 415 |
| 32 | TAGATCAGCG | 416 |
| 33 | TTCCGGTTAT | 417 |
| 34 | GATAAACTGT | 418 |
| 35 | TACAGTTGCC | 419 |
| 36 | CGATGGCGAA | 420 |
| 37 | CCGACGTCAG | 421 |
| 38 | TATGGTGCAA | 422 |
| 39 | GACGACAGTC | 423 |
| 40 | GTCACCGTCC | 424 |
| 41 | GGTTTTAACA | 425 |
| 42 | GAGGACAGTA | 426 |
| 43 | GTTACCTAAG | 427 |
| 44 | ATCACGTGTT | 428 |
| 45 | TAAGGCCTGG | 429 |
| 46 | TGTTCGTAGC | 430 |
| 47 | TGAGGACGTG | 431 |
| 48 | GTGCTGTGTA | 432 |
| 49 | GAGGGTACGC | 433 |
| 50 | CCGTGATTGT | 434 |
| 51 | AAAATCGCCT | 435 |
| 52 | CGATCGCAGT | 436 |
| 53 | ACGCAATAAG | 437 |
| 54 | AAGGTGCATC | 438 |
| 55 | CGCGTAGATA | 439 |
| 56 | CGAGCAGTGC | 440 |
| 57 | ATACGTGACG | 441 |
| 58 | AGATTGCGCG | 442 |
| 59 | ACGTGATGCC | 443 |
| 60 | GTACGCATCG | 444 |
| 61 | TCCCGACTTA | 445 |
| 62 | GTTTTTACAC | 446 |
| 63 | CCTGAGCGTG | 447 |
| 64 | CGGCATTGTA | 448 |

**[Table 5-2]**

| No. | Primer sequence | SEQ ID NO: |
|---|---|---|
| 65 | TAGAGTGCGT | 449 |
| 66 | ATGGCCAGAC | 450 |
| 67 | CTTAGCATGC | 451 |
| 68 | ACAACACCTG | 452 |
| 69 | AGTGACTATC | 453 |
| 70 | CATGCTACAC | 454 |
| 71 | AAAGCGGGCG | 455 |
| 72 | AGATCGCCGT | 456 |
| 73 | CGTAGATATT | 457 |
| 74 | AATGGCAGAC | 458 |
| 75 | GTATAACGTG | 459 |
| 76 | ATGTGCGTCA | 460 |
| 77 | CCTGCCAACT | 461 |
| 78 | TTTATAACTC | 462 |
| 79 | ACGGTTACGC | 463 |
| 80 | TAGCCTCTTG | 464 |
| 81 | TCGCGAAGTT | 465 |
| 82 | GTCTACAACC | 466 |
| 83 | GTCTACTGCG | 467 |
| 84 | GTTGCGTCTC | 468 |
| 85 | GGGCCGCTAA | 469 |
| 86 | GTACGTCGGA | 470 |
| 87 | AGCGAGAGAC | 471 |
| 88 | TGGCTACGGT | 472 |
| 89 | AGGCATCACG | 473 |
| 90 | TAGCTCCTCG | 474 |
| 91 | GGCTAGTCAG | 475 |
| 92 | CTCACTTTAT | 476 |
| 93 | ACGGCCACGT | 477 |
| 94 | AGCGTATATC | 478 |
| 95 | GACACGTCTA | 479 |
| 96 | GCCAGCGTAC | 480 |

### [Table 6-1]

**Table 6. Random primer list (10-nucleotide F)**

| No. | Primer sequence | SEQ ID NO: |
|---|---|---|
| 1 | AACATTAGCG | 481 |
| 2 | AGTGTGCTAT | 482 |
| 3 | CACGAGCGTT | 483 |
| 4 | GTAACGCCTA | 484 |
| 5 | CACATAGTAC | 485 |
| 6 | CGCGATATCG | 486 |
| 7 | CGTTCTGTGC | 487 |
| 8 | CTGATCGCAT | 488 |
| 9 | TGGCGTGAGA | 489 |
| 10 | TTGCCAGGCT | 490 |
| 11 | GTTATACACA | 491 |
| 12 | AGTGCCAACT | 492 |
| 13 | TCACGTAGCA | 493 |
| 14 | TAATTCAGCG | 494 |
| 15 | AAGTATCGTC | 495 |
| 16 | CACAGTTACT | 496 |
| 17 | CCTTACCGTG | 497 |
| 18 | ACGGTGTCGT | 498 |
| 19 | CGCGTAAGAC | 499 |
| 20 | TTCGCACCAG | 500 |
| 21 | CACGAACAGA | 501 |
| 22 | GTTGGACATT | 502 |
| 23 | GGTGCTTAAG | 503 |
| 24 | TCGGTCTCGT | 504 |
| 25 | TCTAGTACGC | 505 |
| 26 | TTAGGCCGAG | 506 |
| 27 | CGTCAAGAGC | 507 |
| 28 | ACATGTCTAC | 508 |
| 29 | ATCGTTACGT | 509 |
| 30 | ACGGATCGTT | 510 |
| 31 | AATCTTGGCG | 511 |
| 32 | AGTATCTGGT | 512 |
| 33 | CAACCGACGT | 513 |
| 34 | TGGTAACGCG | 514 |
| 35 | GTGCAGACAT | 515 |
| 36 | GTCTAGTTGC | 516 |
| 37 | CAATTCGACG | 517 |
| 38 | CTTAGCACCT | 518 |
| 39 | TAATGTCGCA | 519 |
| 40 | CAATCGGTAC | 520 |
| 41 | AGCACGCATT | 521 |
| 42 | AGGTCCTCGT | 522 |
| 43 | TTGTGCCTGC | 523 |
| 44 | ACCGCCTGTA | 524 |
| 45 | GTACGTCAGG | 525 |
| 46 | GCACACAACT | 526 |
| 47 | TGAGCACTTA | 527 |
| 48 | GTGCCGCATA | 528 |
| 49 | ATGTTTTCGC | 529 |
| 50 | ACACTTAGGT | 530 |
| 51 | CGTGCCGTGA | 531 |
| 52 | TTACTAATCA | 532 |
| 53 | GTGGCAGGTA | 533 |
| 54 | GCGCGATATG | 534 |
| 55 | GAACGACGTT | 535 |
| 56 | ATCAGGAGTG | 536 |
| 57 | GCCAGTAAGT | 537 |
| 58 | GCAAGAAGCA | 538 |
| 59 | AACTCCGCCA | 539 |
| 60 | ACTTGAGCCT | 540 |
| 61 | CGTGATCGTG | 541 |
| 62 | AATTAGCGAA | 542 |
| 63 | ACTTCCTTAG | 543 |
| 64 | TGTGCTGATA | 544 |

**[Table 6-2]**

| No. | Primer sequence | SEQ ID NO: |
|---|---|---|
| 65 | AGGCGGCTGA | 545 |
| 66 | CGTTTAGAGC | 546 |
| 67 | ACGCGTCTAA | 547 |
| 68 | GCGAATGTAC | 548 |
| 69 | CGTGATCCAA | 549 |
| 70 | CAACCAGATG | 550 |
| 71 | ACCATTAACC | 551 |
| 72 | CGATTCACGT | 552 |
| 73 | CTAGAACCTG | 553 |
| 74 | CCTAACGACA | 554 |
| 75 | GACGTGCATG | 555 |
| 76 | ATGTAACCTT | 556 |
| 77 | GATACAGTCG | 557 |
| 78 | CGTATGTCTC | 558 |
| 79 | AGATTATCGA | 559 |
| 80 | ATACTGGTAA | 560 |
| 81 | GTTGAGTAGC | 561 |
| 82 | ACCATTATCA | 562 |
| 83 | CACACTTCAG | 563 |
| 84 | GACTAGCGGT | 564 |
| 85 | AATTGTCGAG | 565 |
| 86 | CTAAGGACGT | 566 |
| 87 | ATTACGATGA | 567 |
| 88 | ATTGAAGACT | 568 |
| 89 | GCTTGTACGT | 569 |
| 90 | CCTACGTCAC | 570 |
| 91 | CACAACTTAG | 571 |
| 92 | GCGGTTCATC | 572 |
| 93 | GTACTCATCT | 573 |
| 94 | GTGCATCAGT | 574 |
| 95 | TCACATCCTA | 575 |
| 96 | CACGCGCTAT | 576 |

### [Table 7-1]

**Table 7. Random primer list 8-nucleotide**

| No. | Primer sequence | SEQ ID NO: |
|---|---|---|
| 1 | CTATCTTG | 577 |
| 2 | AAGTGCGT | 578 |
| 3 | ACATGCGA | 579 |
| 4 | ACCAATGG | 580 |
| 5 | TGCGTTGA | 581 |
| 6 | GACATGTC | 582 |
| 7 | TTGTGCGT | 583 |
| 8 | ACATCGCA | 584 |
| 9 | GAAGACGA | 585 |
| 10 | TCGATAGA | 586 |
| 11 | TCTTGCAA | 587 |
| 12 | AGCAAGTT | 588 |
| 13 | TTCATGGA | 589 |
| 14 | TCAATTCG | 590 |
| 15 | CGGTATGT | 591 |
| 16 | ACCACTAC | 592 |
| 17 | TCGCTTAT | 593 |
| 18 | TCTCGACT | 594 |
| 19 | GAATCGGT | 595 |
| 20 | GTTACAAG | 596 |
| 21 | CTGTGTAG | 597 |
| 22 | TGGTAGAA | 598 |
| 23 | ATACTGCG | 599 |
| 24 | AACTCGTC | 600 |
| 25 | ATATGTGC | 601 |
| 26 | AAGTTGCG | 602 |
| 27 | GATCATGT | 603 |
| 28 | TTGTTGCT | 604 |
| 29 | CCTCTTAG | 605 |
| 30 | TCACAGCT | 606 |
| 31 | AGATTGAC | 607 |
| 32 | AGCCTGAT | 608 |
| 33 | CGTCAAGT | 609 |
| 34 | AAGTAGAC | 610 |
| 35 | TCAGACAA | 611 |
| 36 | TCCTTGAC | 612 |
| 37 | GTAGCTGT | 613 |
| 38 | CGTCGTAA | 614 |
| 39 | CCAATGGA | 615 |
| 40 | TTGAGAGA | 616 |
| 41 | ACAACACC | 617 |
| 42 | TCTAGTAC | 618 |
| 43 | GAGGAAGT | 619 |
| 44 | GCGTATTG | 620 |
| 45 | AAGTAGCT | 621 |
| 46 | TGAACCTT | 622 |
| 47 | TGTGTTAC | 623 |
| 48 | TAACCTGA | 624 |
| 49 | GCTATTCC | 625 |
| 50 | GTTAGATG | 626 |
| 51 | CAGGATAA | 627 |
| 52 | ACCGTAGT | 628 |
| 53 | CCGTGTAT | 629 |
| 54 | TCCACTCT | 630 |
| 55 | TAGCTCAT | 631 |
| 56 | CGCTAATA | 632 |
| 57 | TACCTCTG | 633 |
| 58 | TGCACTAC | 634 |
| 59 | CTTGGAAG | 635 |
| 60 | AATGCACG | 636 |
| 61 | CACTGTTA | 637 |
| 62 | TCGACTAG | 638 |
| 63 | CTAGGTTA | 639 |
| 64 | GCAGATGT | 640 |

**[Table 7-2]**

| No. | Primer sequence | SEQ ID NO: |
|---|---|---|
| 65 | AGTTCAGA | 641 |
| 66 | CTCCATCA | 642 |
| 67 | TGGTTACG | 643 |
| 68 | ACGTAGCA | 644 |
| 69 | CTCTTCCA | 645 |
| 70 | CGTCAGAT | 646 |
| 71 | TGGATCAT | 647 |
| 72 | ATATCGAC | 648 |
| 73 | TTGTGGAG | 649 |
| 74 | TTAGAGCA | 650 |
| 75 | TAACTACC | 651 |
| 76 | CTATGAGG | 652 |
| 77 | CTTCTCAC | 653 |
| 78 | CGTTCTCT | 654 |
| 79 | GTCACTAT | 655 |
| 80 | TCGTTAGC | 656 |
| 81 | ATCGTGTA | 657 |
| 82 | GAGAGCAA | 658 |
| 83 | AGACGCAA | 659 |
| 84 | TCCAGTTA | 660 |
| 85 | AATGCCAC | 661 |
| 86 | ATCACGTG | 662 |
| 87 | ACTGTGCA | 663 |
| 88 | TCACTGCA | 664 |
| 89 | GCATCCAA | 665 |
| 90 | AGCACTAT | 666 |
| 91 | CGAAGGAT | 667 |
| 92 | CCTTGTGT | 668 |
| 93 | TGCGGATA | 669 |
| 94 | AGGAATGG | 670 |
| 95 | ATCGTAAC | 671 |
| 96 | GAATGTCT | 672 |

### [Table 8-1]

**Table 8. Random primer list (9-nucleotide)**

| No. | Primer sequence | SEQ ID NO: |
|---|---|---|
| 1 | TTGCTACAT | 673 |
| 2 | TAACGTATG | 674 |
| 3 | CAGTATGTA | 675 |
| 4 | TCAATAACG | 676 |
| 5 | CACACTTAT | 677 |
| 6 | GACTGTAAT | 678 |
| 7 | TATACACTG | 679 |
| 8 | ACTGCATTA | 680 |
| 9 | ACATTAAGC | 681 |
| 10 | CATATTACG | 682 |
| 11 | ATATCTACG | 683 |
| 12 | AGTAACTGT | 684 |
| 13 | ATGACGTTA | 685 |
| 14 | ATTATGCGA | 686 |
| 15 | AGTATACAC | 687 |
| 16 | TTAGCGTTA | 688 |
| 17 | TATGACACT | 689 |
| 18 | ATTAACGCT | 690 |
| 19 | TAGGACAAT | 691 |
| 20 | AAGACGTTA | 692 |
| 21 | TATAAGCGT | 693 |
| 22 | ATACCTGGC | 694 |
| 23 | CTCGAGATC | 695 |
| 24 | ATGGTGAGG | 696 |
| 25 | ATGTCGACG | 697 |
| 26 | GACGTCTGA | 698 |
| 27 | TACACTGCG | 699 |
| 28 | ATCGTCAGG | 700 |
| 29 | TGCACGTAC | 701 |
| 30 | GTCGTGCAT | 702 |
| 31 | GAGTGTTAC | 703 |
| 32 | AGACTGTAC | 704 |
| 33 | TGCGACTTA | 705 |
| 34 | TGTCCGTAA | 706 |
| 35 | GTAATCGAG | 707 |
| 36 | GTACCTTAG | 708 |
| 37 | ATCACGTGT | 709 |
| 38 | ACTTAGCGT | 710 |
| 39 | GTAATCGTG | 711 |
| 40 | ATGCCGTTA | 712 |
| 41 | ATAACGTGC | 713 |
| 42 | CTACGTTGT | 714 |
| 43 | TATGACGCA | 715 |
| 44 | CCGATAACA | 716 |
| 45 | ATGCGCATA | 717 |
| 46 | GATAAGCGT | 718 |
| 47 | ATATCTGCG | 719 |
| 48 | ACTTAGACG | 720 |
| 49 | ATCACCGTA | 721 |
| 50 | TAAGACACG | 722 |
| 51 | AATGCCGTA | 723 |
| 52 | AATCACGTG | 724 |
| 53 | TCGTTAGTC | 725 |
| 54 | CATCATGTC | 726 |
| 55 | TAAGACGGT | 727 |
| 56 | TGCATAGTG | 728 |
| 57 | GAGCGTTAT | 729 |
| 58 | TGCCTTACA | 730 |
| 59 | TTCGCGTTA | 731 |
| 60 | GTGTTAACG | 732 |
| 61 | GACACTGAA | 733 |
| 62 | CTGTTATCG | 734 |
| 63 | GGTCGTTAT | 735 |
| 64 | CGAGAGTAT | 736 |

**[Table 8-2]**

| No. | Primer sequence | SEQ ID NO: |
|---|---|---|
| 65 | ATACAGTCC | 737 |
| 66 | AATTCACGC | 738 |
| 67 | TATGTGCAC | 739 |
| 68 | GATGACGTA | 740 |
| 69 | GATGCGATA | 741 |
| 70 | GAGCGATTA | 742 |
| 71 | TGTCACAGA | 743 |
| 72 | TACTAACCG | 744 |
| 73 | CATAACGAG | 745 |
| 74 | CGTATACCT | 746 |
| 75 | TATCACGTG | 747 |
| 76 | GAACGTTAC | 748 |
| 77 | GTCGTATAC | 749 |
| 78 | ATGTCGACA | 750 |
| 79 | ATACAGCAC | 751 |
| 80 | TACTTACGC | 752 |
| 81 | AACTACGGT | 753 |
| 82 | TAGAACGGT | 754 |
| 83 | GAATGTCAC | 755 |
| 84 | TGTACGTCT | 756 |
| 85 | AACATTGCG | 757 |
| 86 | TTGAACGCT | 758 |
| 87 | AATCAGGAC | 759 |
| 88 | ATTCGCACA | 760 |
| 89 | CCATGTACT | 761 |
| 90 | TGTCCTGTT | 762 |
| 91 | TAATTGCGC | 763 |
| 92 | GATAGTGTG | 764 |
| 93 | ATAGACGCA | 765 |
| 94 | TGTACCGTT | 766 |
| 95 | ATTGTCGCA | 767 |
| 96 | GTCACGTAA | 768 |

### [Table 9-1]

**Table 9. Random primer list (11-nucleotide)**

| No. | Primer sequence | SEQ ID NO: |
|---|---|---|
| 1 | TTACACTATGC | 769 |
| 2 | GCGATAGTCGT | 770 |
| 3 | CTATTCACAGT | 771 |
| 4 | AGAGTCACTGT | 772 |
| 5 | AGAGTCGAAGC | 773 |
| 6 | CTGAATATGTG | 774 |
| 7 | ACTCCACAGGA | 775 |
| 8 | ATCCTCGTAAG | 776 |
| 9 | TACCATCGCCT | 777 |
| 10 | AACGCCTATAA | 778 |
| 11 | CTGTCGAACTT | 779 |
| 12 | TCAGATGTCCG | 780 |
| 13 | CTGCTTATCGT | 781 |
| 14 | ACATTCGCACA | 782 |
| 15 | CCTTAATGCAT | 783 |
| 16 | GGCTAGCTACT | 784 |
| 17 | TTCCAGTTGGC | 785 |
| 18 | GAGTCACAAGG | 786 |
| 19 | CAGAAGGTTCA | 787 |
| 20 | TCAACGTGCAG | 788 |
| 21 | CAAGCTTACTA | 789 |
| 22 | AGAACTCGTTG | 790 |
| 23 | CCGATACAGAG | 791 |
| 24 | GTACGCTGATC | 792 |
| 25 | TCCTCAGTGAA | 793 |
| 26 | GAGCCAACATT | 794 |
| 27 | GAGATCGATGG | 795 |
| 28 | ATCGTCAGCTG | 796 |
| 29 | GAAGCACACGT | 797 |
| 30 | ATCACGCAACC | 798 |
| 31 | TCGAATAGTCG | 799 |
| 32 | TATTACCGTCT | 800 |
| 33 | CAGTCACGACA | 801 |
| 34 | TTACTCGACGT | 802 |
| 35 | GCAATGTTGAA | 803 |
| 36 | GACACGAGCAA | 804 |
| 37 | CGAGATTACAA | 805 |
| 38 | TACCGACTACA | 806 |
| 39 | ACCGTTGCCAT | 807 |
| 40 | ATGTAATCGCC | 808 |
| 41 | AAGCCTGATGT | 809 |
| 42 | AAGTAACGTGG | 810 |
| 43 | GTAGAGGTTGG | 811 |
| 44 | CTCTTGCCTCA | 812 |
| 45 | ATCGTGAAGTG | 813 |
| 46 | ACCAGCACTAT | 814 |
| 47 | CACCAGAATGT | 815 |
| 48 | GAGTGAACAAC | 816 |
| 49 | TAACGTTACGC | 817 |
| 50 | CTTGGATCTTG | 818 |
| 51 | GTTCCAACGTT | 819 |
| 52 | CAAGGACCGTA | 820 |
| 53 | GACTTCACGCA | 821 |
| 54 | CACACTACTGG | 822 |
| 55 | TCAGATGAATC | 823 |
| 56 | TATGGATCTGG | 824 |
| 57 | TCTTAGGTGTG | 825 |
| 58 | TGTCAGCGTCA | 826 |
| 59 | GTCTAGGACAG | 827 |
| 60 | GCCTCTTCATA | 828 |
| 61 | AGAAGTGTTAC | 829 |
| 62 | CATGAGGCTTG | 830 |
| 63 | TGGATTGCTCA | 831 |
| 64 | ATCTACCTAAG | 832 |

**[Table 9-2]**

| No. | Primer sequence | SEQ ID NO: |
|---|---|---|
| 65 | ATGAGCAGTGA | 833 |
| 66 | CCAGGAGATAC | 834 |
| 67 | CCGTTATACTT | 835 |
| 68 | CTCAGTACAAG | 836 |
| 69 | GGTGATCGTAG | 837 |
| 70 | CGAACGAGACA | 838 |
| 71 | ACTACGAGCTT | 839 |
| 72 | TTGCCACAGCA | 840 |
| 73 | GTCAACTCTAC | 841 |
| 74 | TGGACTGTGTC | 842 |
| 75 | GGAATGGACTT | 843 |
| 76 | CGAGAACATAA | 844 |
| 77 | ACCTGGTCAGT | 845 |
| 78 | CGAACGACACA | 846 |
| 79 | AGTCTAGCCAT | 847 |
| 80 | AGGCCTAGATG | 848 |
| 81 | GGTGCGTTAGT | 849 |
| 82 | ATTGTGTCCGA | 850 |
| 83 | GCAGACATTAA | 851 |
| 84 | ATTGGCTCATG | 852 |
| 85 | GAGGTTACATG | 853 |
| 86 | CCTATAGGACC | 854 |
| 87 | TTAGACGGTCT | 855 |
| 88 | GATTGACGCAC | 856 |
| 89 | AAGACACCTCG | 857 |
| 90 | TCGAATAATCG | 858 |
| 91 | TCTATGTCGGA | 859 |
| 92 | TCGCATGAACC | 860 |
| 93 | TGTTATGTCTC | 861 |
| 94 | TGGATCCTACA | 862 |
| 95 | ATCGTTCAGCC | 863 |
| 96 | TACCGCAAGCA | 864 |

### [Table 10-1]

**Table 10. Random primer list (12-nucleotide)**

| No. | Primer sequence | SEQ ID NO: |
|---|---|---|
| 1 | GCTGTTGAACCG | 865 |
| 2 | ATACTCCGAGAT | 866 |
| 3 | CTTAAGGAGCGC | 867 |
| 4 | TATACTACAAGC | 868 |
| 5 | TAGTGGTCGTCA | 869 |
| 6 | GTGCTTCAGGAG | 870 |
| 7 | GACGCATACCTC | 871 |
| 8 | CCTACCTGTGGA | 872 |
| 9 | GCGGTCACATAT | 873 |
| 10 | CTGCATTCACGA | 874 |
| 11 | TGGATCCTTCAT | 875 |
| 12 | TTGTGCTGGACT | 876 |
| 13 | ATTGAGAGCTAT | 877 |
| 14 | TCGCTAATGTAG | 878 |
| 15 | CTACTGGCACAA | 879 |
| 16 | AGAGCCAGTCGT | 880 |
| 17 | AATACTGGCTAA | 881 |
| 18 | CTGCATGCATAA | 882 |
| 19 | TTGTCACAACTC | 883 |
| 20 | TGCTAACTCTCC | 884 |
| 21 | TCTCTAGTTCGG | 885 |
| 22 | TTACGTCCGCAA | 886 |
| 23 | GTGTTGCTACCA | 887 |
| 24 | CGCATGTATGCC | 888 |
| 25 | CCTGTTCTGATT | 889 |
| 26 | TAAGATGCTTGA | 890 |
| 27 | ATATATCTCAGC | 891 |
| 28 | TTCCTCGTGGTT | 892 |
| 29 | ATGTCGATCTAG | 893 |
| 30 | CATCCACTAATC | 894 |
| 31 | GCCTCTGGTAAC | 895 |
| 32 | AGTCAAGAGATT | 896 |
| 33 | ACTGAGGCGTTC | 897 |
| 34 | TAAGGCTGACAT | 898 |
| 35 | AGTTCGCATACA | 899 |
| 36 | GCAGAATTGCGA | 900 |
| 37 | GGTTATGAAGAA | 901 |
| 38 | AGAAGTCGCCTC | 902 |
| 39 | TTCGCGTTATTG | 903 |
| 40 | TACCTGGTCGGT | 904 |
| 41 | GGTTACCGAGGA | 905 |
| 42 | ACACACTTCTAG | 906 |
| 43 | GGAAGTGATTAA | 907 |
| 44 | TCCATCAGATAA | 908 |
| 45 | TGTCTGTATCAT | 909 |
| 46 | AATTGGCTATAG | 910 |
| 47 | ACGTCGGAAGGT | 911 |
| 48 | AGGCATCCGTTG | 912 |
| 49 | ACCGTCGCTTGA | 913 |
| 50 | TACCGTCAAGTG | 914 |
| 51 | CTCGATATAGTT | 915 |
| 52 | CGTCAACGTGGT | 916 |
| 53 | TAGTCAACGTAG | 917 |
| 54 | TGAGTAGGTCAG | 918 |
| 55 | CTTGGCATGTAC | 919 |
| 56 | TGCCGAGACTTC | 920 |
| 57 | CTAAGACTTAAG | 921 |
| 58 | TTCTCGTGTGCG | 922 |
| 59 | CACCTGCACGAT | 923 |
| 60 | ATTAAGCCTAAG | 924 |
| 61 | GGTGGAACCATG | 925 |
| 62 | ACTAACGCGACT | 926 |
| 63 | CAGTTGTGCTAT | 927 |
| 64 | ACGCTGTTAGCA | 928 |

**[Table 10-2]**

| No. | Primer sequence | SEQ ID NO: |
|---|---|---|
| 65 | GTCAACGCTAAG | 929 |
| 66 | AGCTTAGGTATG | 930 |
| 67 | CGCAGGACGATT | 931 |
| 68 | AACCGGCTGTCT | 932 |
| 69 | GTTGCTCACGTG | 933 |
| 70 | GAATCTTCCGCG | 934 |
| 71 | AGAGCGTACACG | 935 |
| 72 | AAGGCTAATGTC | 936 |
| 73 | TCTATGTAGACG | 937 |
| 74 | AGACGGTCTAGT | 938 |
| 75 | TTGGTCACACGC | 939 |
| 76 | GTCGATATATGG | 940 |
| 77 | AACATGGATACG | 941 |
| 78 | TTCGCAGTTCCT | 942 |
| 79 | CGCATGTTGTGC | 943 |
| 80 | TGTTAAGTTGGA | 944 |
| 81 | CAAGTGTGATGA | 945 |
| 82 | CTGGTACCACGT | 946 |
| 83 | CGCTAGGATCAC | 947 |
| 84 | TGCTCATTACGG | 948 |
| 85 | TGCTCAGTAACA | 949 |
| 86 | ACGATCATAGCC | 950 |
| 87 | ACGATACGTGGA | 951 |
| 88 | GTTCGATGATGG | 952 |
| 89 | AAGAGCTGTGCC | 953 |
| 90 | GGTTGGATCAAC | 954 |
| 91 | GCGCGCTTATGA | 955 |
| 92 | CGTCGATCATCA | 956 |
| 93 | GAGACTGCACTC | 957 |
| 94 | GATAGATCGCAT | 958 |
| 95 | GGCCATCATCAG | 959 |
| 96 | GGTGTTCCACTG | 960 |

### [Table 11-1]

**Table 11. Random primer list (14-nucleotide)**

| No. | Primer sequence | SEQ ID NO: |
|---|---|---|
| 1 | AGCTATACAGAGGT | 961 |
| 2 | AGGCCGTTCTGTCT | 962 |
| 3 | CATTGGTCTGCTAT | 963 |
| 4 | CTACATACGCGCCA | 964 |
| 5 | GCTTAACGGCGCTT | 965 |
| 6 | TACGATACTCCACC | 966 |
| 7 | ACCGGCATAAGAAG | 967 |
| 8 | GGATGCTTCGATAA | 968 |
| 9 | GTGTACCTGAATGT | 969 |
| 10 | CGCGGATACACAGA | 970 |
| 11 | TTCCACGGCACTGT | 971 |
| 12 | TAGCCAGGCAACAA | 972 |
| 13 | AGCGTCAACACGTA | 973 |
| 14 | TAACGCTACTCGCG | 974 |
| 15 | TAGATAGACGATCT | 975 |
| 16 | ACTCTTGCAATGCT | 976 |
| 17 | ACTCGGTTAGGTCG | 977 |
| 18 | CATTATCTAC G CAT | 978 |
| 19 | CACACCGGCGATTA | 979 |
| 20 | TACGCAGTACTGTG | 980 |
| 21 | CAAGCGCGTGAATG | 981 |
| 22 | GAATGGACTGACGA | 982 |
| 23 | CTAGCGCTGAAGTT | 983 |
| 24 | TGCGGCAGACCAAT | 984 |
| 25 | AAGGCATAGAGATT | 985 |
| 26 | TTCTCCTCGCCATG | 986 |
| 27 | TCATTGGTCGTGAA | 987 |
| 28 | ATTACGCTATACGA | 988 |
| 29 | ATGATCCTCCACGG | 989 |
| 30 | CGTCGTTAGTAATC | 990 |
| 31 | TGCACATAGTCTCA | 991 |
| 32 | GTCAAGGAGTCACG | 992 |
| 33 | GGTTGGAATCTTGC | 993 |
| 34 | CATCGGTGCACTCA | 994 |
| 35 | AATGCACTAGACGT | 995 |
| 36 | TACAGTCAGGCTCG | 996 |
| 37 | AGAGAAGCTTAGCC | 997 |
| 38 | CCATAGGATCGTAT | 998 |
| 39 | TTGTGCTACACCTG | 999 |
| 40 | CTCCAGTAATACTA | 1000 |
| 41 | TGATGCCGATGTGG | 1001 |
| 42 | GTCATACCGCTTAA | 1002 |
| 43 | ACGTTCTCTTGAGA | 1003 |
| 44 | CAGCCATATCGTGT | 1004 |
| 45 | TTGAACGTAGCAAT | 1005 |
| 46 | ACAATCGCGGTAAT | 1006 |
| 47 | GTTCCTGTAGATCC | 1007 |
| 48 | AGAGCCTTACGGCA | 1008 |
| 49 | AATATGGCGCCACC | 1009 |
| 50 | ACCATATAGGTTCG | 1010 |
| 51 | ATGCACCACAGCTG | 1011 |
| 52 | CTACTATTGAACAG | 1012 |
| 53 | TGCCATCACTCTAG | 1013 |
| 54 | GCGAACGAGAATCG | 1014 |
| 55 | GAATCAAGGAGACC | 1015 |
| 56 | CAACATCTATGCAG | 1016 |
| 57 | CAATCCGTCATGGA | 1017 |
| 58 | AGCTCTTAGCCATA | 1018 |
| 59 | AACAAGGCAACTGG | 1019 |
| 60 | GTCGTCGCTCCTAT | 1020 |
| 61 | GTCATCATTAGATG | 1021 |
| 62 | GCACTAAGTAGCAG | 1022 |
| 63 | ACCTTACCGGACCT | 1023 |
| 64 | GCTCAGGTATGTCA | 1024 |

**[Table 11-2]**

| No. | Primer sequence | SEQ ID NO: |
|---|---|---|
| 65 | TGTCACGAGTTAGT | 1025 |
| 66 | CAGATGACTTACGT | 1026 |
| 67 | GAAGTAGCGATTGA | 1027 |
| 68 | GCAGGCAATCTGTA | 1028 |
| 69 | CCTTATACAACAAG | 1029 |
| 70 | CCTTAGATTGATTG | 1030 |
| 71 | AGCCACGAGTGATA | 1031 |
| 72 | GGATGACTCGTGAC | 1032 |
| 73 | CTTCGTTCGCCATT | 1033 |
| 74 | TCTTGCGTATTGAT | 1034 |
| 75 | CTTAACGTGGTGGC | 1035 |
| 76 | TGCTGTTACGGAAG | 1036 |
| 77 | CTGAATTAGTTCTC | 1037 |
| 78 | CCTCCAAGTACAGA | 1038 |
| 79 | CTGGTAATTCGCGG | 1039 |
| 80 | CGACTGCAATCTGG | 1040 |
| 81 | TGGATCGCGATTGG | 1041 |
| 82 | CGACTATTCCTGCG | 1042 |
| 83 | CAAGTAGGTCCGTC | 1043 |
| 84 | AGTAATCAGTGTTC | 1044 |
| 85 | TTATTCTCACTACG | 1045 |
| 86 | CATGTCTTCTTCGT | 1046 |
| 87 | AGGCACATACCATC | 1047 |
| 88 | AGGTTAGAGGATGT | 1048 |
| 89 | CAACTGGCAAGTGC | 1049 |
| 90 | CGCTCACATAGAGG | 1050 |
| 91 | GCAATGTCGAGATC | 1051 |
| 92 | GTTCTGTGGTGCTC | 1052 |
| 93 | AAGTGATCAGACTA | 1053 |
| 94 | ATTGAAGGATTCCA | 1054 |
| 95 | ACGCCATGCTACTA | 1055 |
| 96 | CTGAAGATGTCTGC | 1056 |

### [Table 12-1]

**Table 12. Random primer list (16-nucleotide)**

| No. | Primer sequence | SEQ ID NO: |
|---|---|---|
| 1 | GACAATCTCTGCCGAT | 1057 |
| 2 | GGTCCGCCTAATGTAA | 1058 |
| 3 | AGCCACAGGCAATTCC | 1059 |
| 4 | ATCTCAAGTTCTCAAC | 1060 |
| 5 | TGTAACGCATACGACG | 1061 |
| 6 | TATCTCGAATACCAGC | 1062 |
| 7 | ACCGCAACACAGGCAA | 1063 |
| 8 | GGCCAGTAACATGACT | 1064 |
| 9 | GTGAACAGTTAAGGTG | 1065 |
| 10 | CCAGGATCCGTATTGC | 1066 |
| 11 | GACCTAGCACTAGACC | 1067 |
| 12 | CGCCATCCTATTCACG | 1068 |
| 13 | AAGTGCAGTAATGGAA | 1069 |
| 14 | TCAACGCGTTCGTCTA | 1070 |
| 15 | AGCGGCCACTATCTAA | 1071 |
| 16 | CTCGGCGCCATATAGA | 1072 |
| 17 | CGATAACTTAGAAGAA | 1073 |
| 18 | CATAGGATGTGACGCC | 1074 |
| 19 | GGCTTGTCGTCGTATC | 1075 |
| 20 | CTTGTCTGAATATTAG | 1076 |
| 21 | ACAGTTCGAGTGTCGG | 1077 |
| 22 | CTCTAACCTGTGACGT | 1078 |
| 23 | CGCGCTAATTCAACAA | 1079 |
| 24 | ACTCACGAATGCGGCA | 1080 |
| 25 | AATCTTCGGCATTCAT | 1081 |
| 26 | AAGTATCAGGATCGCG | 1082 |
| 27 | AGTAACTCTGCAGACA | 1083 |
| 28 | GGATTGAACATTGTGC | 1084 |
| 29 | GTGATGCTCACGCATC | 1085 |
| 30 | CGTAGCGTAACGGATA | 1086 |
| 31 | TGCGATGCACCGTTAG | 1087 |
| 32 | CCAGTATGCTCTCAGG | 1088 |
| 33 | AATGACGTTGAAGCCT | 1089 |
| 34 | TCGATTCTATAGGAGT | 1090 |
| 35 | CGATAGGTTCAGCTAT | 1091 |
| 36 | CCATGTTGATAGAATA | 1092 |
| 37 | GAGCCACTTCTACAGG | 1093 |
| 38 | GCGAACTCTCGGTAAT | 1094 |
| 39 | GACCTGAGTAGCTGGT | 1095 |
| 40 | CGAGTCTATTAGCCTG | 1096 |
| 41 | GTAGTGCCATACACCT | 1097 |
| 42 | CCAGTGGTCTATAGCA | 1098 |
| 43 | GTCAGTGCGTTATTGC | 1099 |
| 44 | AGTGTCGGAGTGACGA | 1100 |
| 45 | AATCTCCGCTATAGTT | 1101 |
| 46 | CGAGTAGGTCTGACTT | 1102 |
| 47 | CTGTCGCTCTAATAAC | 1103 |
| 48 | GCTGTCAATATAACTG | 1104 |
| 49 | AGCTCAAGTTGAATCC | 1105 |
| 50 | AATTCATGCTCCTAAC | 1106 |
| 51 | CCAAGGTCTGGTGATA | 1107 |
| 52 | CTCCACGTATCTTGAA | 1108 |
| 53 | TAGCCGAACAACACTT | 1109 |
| 54 | AGTACACGACATATGC | 1110 |
| 55 | ACGTTCTAGACTCCTG | 1111 |
| 56 | CGACTCAAGCACTGCT | 1112 |
| 57 | TGAAGCTCACGATTAA | 1113 |
| 58 | TATCTAACGTATGGTA | 1114 |
| 59 | TATACCATGTTCCTTG | 1115 |
| 60 | TTCCTACGATGACTTC | 1116 |
| 61 | CTCTCCAATATGTGCC | 1117 |
| 62 | GAGTAGAGTCTTGCCA | 1118 |
| 63 | GCGAGATGTGGTCCTA | 1119 |
| 64 | AAGCTACACGGACCAC | 1120 |

**[Table 12-2]**

| No. | Primer sequence | SEQ ID NO: |
|---|---|---|
| 65 | ATACAACTGGCAACCG | 1121 |
| 66 | CGGTAGATGCTATGCT | 1122 |
| 67 | TCTTGACCGGTCATCA | 1123 |
| 68 | AGATCGTGCATGCGAT | 1124 |
| 69 | TCCTCGAGACAGCCTT | 1125 |
| 70 | TAGCCGGTACCACTTA | 1126 |
| 71 | GTAAGGCAGCGTGCAA | 1127 |
| 72 | TAGTCTGCTCCTGGTC | 1128 |
| 73 | TGGATTATAGCAGCAG | 1129 |
| 74 | AAGAATGATCAGACAT | 1130 |
| 75 | CAGCGCTATATACCTC | 1131 |
| 76 | GAGTAGTACCTCCACC | 1132 |
| 77 | GACGTGATCCTCTAGA | 1133 |
| 78 | GTTCCGTTCACTACGA | 1134 |
| 79 | TGCAAGCACCAGGATG | 1135 |
| 80 | TTAGTTGGCGGCTGAG | 1136 |
| 81 | CAGATGCAGACATACG | 1137 |
| 82 | GACGCTTGATGATTAT | 1138 |
| 83 | TGGATCACGACTAGGA | 1139 |
| 84 | CTCGTCGGTATAACGC | 1140 |
| 85 | AAGCACGGATGCGATT | 1141 |
| 86 | AGATCTTCCGGTGAAC | 1142 |
| 87 | GGACAATAGCAACCTG | 1143 |
| 88 | GATAATCGGTTCCAAT | 1144 |
| 89 | CTCAAGCTACAGTTGT | 1145 |
| 90 | GTTGGCATGATGTAGA | 1146 |
| 91 | CAGCATGAGGTAAGTG | 1147 |
| 92 | GCCTCATCACACGTCA | 1148 |
| 93 | TCGATACTACACATCG | 1149 |
| 94 | TACACGAGGCTTGATC | 1150 |
| 95 | TTCTCGTGTCCGCATT | 1151 |
| 96 | GGTGAAGCAACAGCAT | 1152 |

### [Table 13-1]

**Table 13. Random primer list (18-nucleotide)**

| No. | Primer sequence | SEQ ID NO: |
|---|---|---|
| 1 | CGAACCGACTGTACAGTT | 1153 |
| 2 | CCGACTGCGGATAAGTTA | 1154 |
| 3 | CGACAGGTAGGTAAGCAG | 1155 |
| 4 | TGATACGTTGGTATACAG | 1156 |
| 5 | CTACTATAGAATACGTAG | 1157 |
| 6 | AGACTGTGGCAATGGCAT | 1158 |
| 7 | GGAAGACTGATACAACGA | 1159 |
| 8 | TATGCACATATAGCGCTT | 1160 |
| 9 | CATGGTAATCGACCGAGG | 1161 |
| 10 | GTCATTGCCGTCATTGCC | 1162 |
| 11 | CCTAAGAACTCCGAAGCT | 1163 |
| 12 | TCGCTCACCGTACTAGGA | 1164 |
| 13 | TATTACTGTCACAGCAGG | 1165 |
| 14 | TGAGACAGGCTACGAGTC | 1166 |
| 15 | AAGCTATGCGAACACGTT | 1167 |
| 16 | AACGGAGGAGTGAGCCAA | 1168 |
| 17 | CCACTATGGACATCATGG | 1169 |
| 18 | ATGGTGGTGGATAGCTCG | 1170 |
| 19 | TCACCGGTTACACATCGC | 1171 |
| 20 | AAGATACTGAGATATGGA | 1172 |
| 21 | GACCTGTTCTTGAACTAG | 1173 |
| 22 | AAGTAGAGCTCTCGGTTA | 1174 |
| 23 | CTATGTTCTTACTCTCTT | 1175 |
| 24 | CAAGGCTATAAGCGGTTA | 1176 |
| 25 | GAAGCTAATTAACCGATA | 1177 |
| 26 | TTCACGTCTGCCAAGCAC | 1178 |
| 27 | ATCGTATAGATCGAGACA | 1179 |
| 28 | GTCACAGATTCACATCAT | 1180 |
| 29 | GTGCCTGTGAACTATCAG | 1181 |
| 30 | CAGCGTACAAGATAGTCG | 1182 |
| 31 | GCATGGCATGGTAGACCT | 1183 |
| 32 | GGTATGCTACTCTTCGCA | 1184 |
| 33 | ATGTTCAGTCACAAGCGA | 1185 |
| 34 | TAGGAAGTGTGTAATAGC | 1186 |
| 35 | AATCCATGTAGCTGTACG | 1187 |
| 36 | CCAGATTCACTGGCATAG | 1188 |
| 37 | TTGTCTCTACGTAATATC | 1189 |
| 38 | GTGGTGCTTGTGACAATT | 1190 |
| 39 | CAGCCTACTTGGCTGAGA | 1191 |
| 40 | TACTCAATGCATCTGTGT | 1192 |
| 41 | TGTAGAGAGACGAATATA | 1193 |
| 42 | GCCTACAACCATCCTACT | 1194 |
| 43 | GCGTGGCATTGAGATTCA | 1195 |
| 44 | GCATGCCAGCTAACTGAG | 1196 |
| 45 | GCGAGTAATCCGGTTGGA | 1197 |
| 46 | GCCTCTACCAGAACGTCA | 1198 |
| 47 | GTCAGCAGAAGACTGACC | 1199 |
| 48 | GATAACAGACGTAGCAGG | 1200 |
| 49 | CAGGAGATCGCATGTCGT | 1201 |
| 50 | CTGGAAGGAATGGAGCCA | 1202 |
| 51 | ATTGGTTCTCTACCACAA | 1203 |
| 52 | CTCATTGTTGACGGCTCA | 1204 |
| 53 | TTCAGGACTGTAGTTCAT | 1205 |
| 54 | AGACCGCACTAACTCAAG | 1206 |
| 55 | GGAATATTGTGCAGACCG | 1207 |
| 56 | CCTATTACTAATAGCTCA | 1208 |
| 57 | ATGGCATGAGTACTTCGG | 1209 |
| 58 | GACACGTATGCGTCTAGC | 1210 |
| 59 | GAAGGTACGGAATCTGTT | 1211 |
| 60 | TATAACGTCCGACACTGT | 1212 |
| 61 | GCTAATACATTACCGCCG | 1213 |
| 62 | GAAGCCAACACTCCTGAC | 1214 |
| 63 | CGAATAACGAGCTGTGAT | 1215 |
| 64 | GCCTACCGATCGCACTTA | 1216 |

**[Table 13-2]**

| No. | Primer sequence | SEQ ID NO: |
|---|---|---|
| 65 | CTGAGGAGAATAGCCTGC | 1217 |
| 66 | CAGCATGGACAGTACTTC | 1218 |
| 67 | GGTATAGAGCCTTCCTTA | 1219 |
| 68 | CGCTCTGCATATATAGCA | 1220 |
| 69 | CGGCTCTACTATGCTCGT | 1221 |
| 70 | CCTAATGCGAAGCTCACC | 1222 |
| 71 | ACAACCGGTGAGGCAGTA | 1223 |
| 72 | TTGGTTCGAACCAACCGC | 1224 |
| 73 | ATACTAGGTTGAACTAAG | 1225 |
| 74 | GCGTTGAGAGTAACATAT | 1226 |
| 75 | AGTTGTATAATAAGCGTC | 1227 |
| 76 | GTATGATGCCGTCCAATT | 1228 |
| 77 | GGACTCTCTGAAGAGTCT | 1229 |
| 78 | GGACTCTCTTGACTTGAA | 1230 |
| 79 | GATAACAGTGCTTCGTCC | 1231 |
| 80 | GGCCATTATAGATGAACT | 1232 |
| 81 | ATAGAGAGCACAGAGCAG | 1233 |
| 82 | GTGTGAGTGTATCATAAC | 1234 |
| 83 | ATAACCTTAGTGCGCGTC | 1235 |
| 84 | CCGACTGATATGCATGGA | 1236 |
| 85 | GGATATCTGATCGCATCA | 1237 |
| 86 | CAGCATTAACGAGGCGAA | 1238 |
| 87 | GCGAGGCCTACATATTCG | 1239 |
| 88 | CGATAAGTGGTAAGGTCT | 1240 |
| 89 | AGATCCTGAGTCGAGCAA | 1241 |
| 90 | AAGATATAACGAGACCGA | 1242 |
| 91 | CCGACTGATTGAGAACGT | 1243 |
| 92 | TCGGCTTATATGACACGT | 1244 |
| 93 | AATAACGTACGCCGGAGG | 1245 |
| 94 | AACACAGCATTGCGCACG | 1246 |
| 95 | GTAGTCTGACAGCAACAA | 1247 |
| 96 | AGAATGACTTGAGCTGCT | 1248 |

### [Table 14-1]

**Table 14. Random primer list (20-nucleotide)**

| No. | Primer sequence | SEQ ID NO: |
|---|---|---|
| 1 | ACTGGTAGTAACGTCCACCT | 1249 |
| 2 | AGACTGGTTGTTATTCGCCT | 1250 |
| 3 | TATCATTGACAGCGAGCTCA | 1251 |
| 4 | TGGAGTCTGAAGAAGGACTC | 1252 |
| 5 | CATCTGGACTACGGCAACGA | 1253 |
| 6 | AACTGTCATAAGACAGACAA | 1254 |
| 7 | CCTCAACATGACATACACCG | 1255 |
| 8 | CAATACCGTTCGCGATTCTA | 1256 |
| 9 | GCGTCTACGTTGATTCGGCC | 1257 |
| 10 | TGAACAGAGGCACTTGCAGG | 1258 |
| 11 | CGACTAGAACCTACTACTGC | 1259 |
| 12 | GCACCGCACGTGGAGAGATA | 1260 |
| 13 | CTGAGAGACCGACTGATGCG | 1261 |
| 14 | TCGTCCTTCTACTTAATGAT | 1262 |
| 15 | CAAGCTATACCATCCGAATT | 1263 |
| 16 | CAATACGTATAGTCTTAGAT | 1264 |
| 17 | CCATCCACAGTGACCTATGT | 1265 |
| 18 | TATCCGTTGGAGAAGGTTCA | 1266 |
| 19 | CGCCTAGGTACCTGAGTACG | 1267 |
| 20 | CAGAGTGCTCGTGTTCGCGA | 1268 |
| 21 | CGCTTGGACATCCTTAAGAA | 1269 |
| 22 | GACCGCATGATTAGTCTTAC | 1270 |
| 23 | CTTGGCCGTAGTCACTCAGT | 1271 |
| 24 | GATAGCGATATTCAGTTCGC | 1272 |
| 25 | ATCCAACACTAAGACAACCA | 1273 |
| 26 | CCATTCTGTTGCGTGTCCTC | 1274 |
| 27 | ACATTCTGTACGCTTGCAGC | 1275 |
| 28 | TGCTGAACGCCAATCGCTTA | 1276 |
| 29 | TCCTCTACAAGAATATTGCG | 1277 |
| 30 | CGACCAACGCAGCCTGATTC | 1278 |
| 31 | ATTGCGAGCTTGAGTAGCGC | 1279 |
| 32 | AAGGTGCGAGCATAGGAATC | 1280 |
| 33 | CACTTAAGTGTGATATAGAT | 1281 |
| 34 | ATCGGTATGCTGACCTAGAC | 1282 |
| 35 | TACAATCTCGAATGCAGGAT | 1283 |
| 36 | CCATATGAAGCGCAGCCGTC | 1284 |
| 37 | CGTCTCGTGGACATTCGAGG | 1285 |
| 38 | CCGAGTACAGAAGCGTGGAA | 1286 |
| 39 | TTACGTGGTCGACAGGCAGT | 1287 |
| 40 | AGCTGCAATCTGCATGATTA | 1288 |
| 41 | ACCTGCCGAAGCAGCCTACA | 1289 |
| 42 | AACATGATAACCACATGGTT | 1290 |
| 43 | ATCCGACTGATTGAATTACC | 1291 |
| 44 | TCACGCTGACTCTTATCAGG | 1292 |
| 45 | GCGCGCTCGAAGTACAACAT | 1293 |
| 46 | ACAGCCAGATGCGTTGTTCC | 1294 |
| 47 | GGAGCTCTGACCTGCAAGAA | 1295 |
| 48 | AACATTAGCCTCAAGTAAGA | 1296 |
| 49 | TGTGATTATGCCGAATGAGG | 1297 |
| 50 | GAGTAATAATCCAATCAGTA | 1298 |
| 51 | CTCCTTGGCGACAGCTGAAC | 1299 |
| 52 | TTACGCACACATACACAGAC | 1300 |
| 53 | ACGCCGTATGGCGACTTAGG | 1301 |
| 54 | AGAACGACAATTACGATGGC | 1302 |
| 55 | TGCTAACGTACCACTGCCAC | 1303 |
| 56 | CATCCAGAATGTCTATCATA | 1304 |
| 57 | GGAGAACGCCTATAGCACTC | 1305 |
| 58 | ACCTCTTGTGACGGCCAGTC | 1306 |
| 59 | TGCCATAACTTGGCATAAGA | 1307 |
| 60 | ACAATTGTCTGACCACGCTC | 1308 |
| 61 | TCGTCACCTTCACAGAACGA | 1309 |
| 62 | AGCAGCAGATGATGATCCAA | 1310 |
| 63 | TCGTGCCTTGGATTCCAGGA | 1311 |
| 64 | TGTTATAGCCACGATACTAT | 1312 |

**[Table 14-2]**

| No. | Primer sequence | SEQ ID NO: |
|---|---|---|
| 65 | AATCTCACCTGTACCTTCCG | 1313 |
| 66 | GAGTAGCGGAAGCGTTAGCG | 1314 |
| 67 | AATACTCCGGCGAGGTATAC | 1315 |
| 68 | TTCGCATCCTTGCACGAACA | 1316 |
| 69 | AACCGGCTAATACTACTGGC | 1317 |
| 70 | CTAGCATCTTAGACACCAGA | 1318 |
| 71 | TAGTTGCGTGATACAAGATA | 1319 |
| 72 | TCGTCTCGACACAGTTGGTC | 1320 |
| 73 | TCCGTTCGCGTGCGAACTGA | 1321 |
| 74 | TCTGACTCTGGTGTACAGTC | 1322 |
| 75 | ACAGCGCAATTATATCCTGT | 1323 |
| 76 | AGATCCGTACGTGAGACTAG | 1324 |
| 77 | TACATTGAAGCATCCGAACA | 1325 |
| 78 | CTCCTGAGAGATCAACGCCA | 1326 |
| 79 | TCACCTCGAATGAGTTCGTT | 1327 |
| 80 | TAGCGACTTAAGGTCCAAGC | 1328 |
| 81 | AGTACGTATTGCCGTGCAAG | 1329 |
| 82 | AGCCACGAACCGACGTCATA | 1330 |
| 83 | TGATGTGTACGCTACTACTA | 1331 |
| 84 | CCACTGTGTGCAGCAGACGA | 1332 |
| 85 | CTATTGTACAGCGAACGCTG | 1333 |
| 86 | CTCCGATATCGCACGGATCG | 1334 |
| 87 | AACTTATCGTCGGACGCATG | 1335 |
| 88 | TATCCTAATTCGTGCCGGTC | 1336 |
| 89 | ACAGCCTTCCTGTGTGGACT | 1337 |
| 90 | CCTCCGTGAGGATCGTACCA | 1338 |
| 91 | GCTCTAAGTAACAGAACTAA | 1339 |
| 92 | GACTTACCGCGCGTTCTGGT | 1340 |
| 93 | TCTGAGGATACACATGTGGA | 1341 |
| 94 | TGTAATCACACTGGTGTCGG | 1342 |
| 95 | CACTAGGCGGCAGACATACA | 1343 |
| 96 | CTAGAGCACAGTACCACGTT | 1344 |

### [Table 15-1]

**Table 15. Random primer list 22-nucleotide**

| No. | Primer sequence | SEQ ID NO: |
|---|---|---|
| 1 | TTCAGAGGTCTACGCTTCCGGT | 1345 |
| 2 | AACACAGACTGCGTTATGCCAA | 1346 |
| 3 | TGCTGAGTTCTATACAGCAGTG | 1347 |
| 4 | ACCTATTATATGATAGCGTCAT | 1348 |
| 5 | ATCGTGAGCTACAGTGAATGCA | 1349 |
| 6 | CGTGATGTATCCGGCCTTGCAG | 1350 |
| 7 | TCTTCTGGTCCTAGAGTTGTGC | 1351 |
| 8 | TGATGTCGGCGGCGGATCAGAT | 1352 |
| 9 | TCGGCCTTAGCGTTCAGCATCC | 1353 |
| 10 | TTAAGTAGGTCAGCCACTGCAC | 1354 |
| 11 | CCAGGTGAGTTGATCTGACACC | 1355 |
| 12 | TATACTATTACTGTGTTCGATC | 1356 |
| 13 | CCGCAGTATGTCTAGTGTTGTC | 1357 |
| 14 | GTCTACCGCGTACGAAGCTCTC | 1358 |
| 15 | ATGCGAGTCCGTGGTCGATCCT | 1359 |
| 16 | TGGTAGATTGGTGTGAGAACTA | 1360 |
| 17 | AGGTTCGTCGATCAACTGCTAA | 1361 |
| 18 | ACGACAAGCATCCTGCGATATC | 1362 |
| 19 | TTGAATCACAGAGAGCGTGATT | 1363 |
| 20 | GTACTTAGTGCTTACGTCAGCT | 1364 |
| 21 | GATTATTAAGGCCAAGCTCATA | 1365 |
| 22 | GCATGCAGAGACGTACTCATCG | 1366 |
| 23 | TAGCGGATGGTGTCCTGGCACT | 1367 |
| 24 | TACGGCTGCCAACTTAATAACT | 1368 |
| 25 | CTCATATGACAACTTCTATAGT | 1369 |
| 26 | CAAGCAATAGTTGTCGGCCACC | 1370 |
| 27 | TTCAGCAATCCGTACTGCTAGA | 1371 |
| 28 | TGAGACGTTGCTGACATTCTCC | 1372 |
| 29 | GTTCCGATGAGTTAGATGTATA | 1373 |
| 30 | TTGACGCTTGGAGGAGTACAAG | 1374 |
| 31 | TTCATGTTACCTCCACATTGTG | 1375 |
| 32 | GAGCACGTGCCAGATTGCAACC | 1376 |

**[Table 15-2]**

| No. | Primer sequence | SEQ ID NO: |
|---|---|---|
| 33 | GGTCGACAAGCACAAGCCTTCT | 1377 |
| 34 | TAGGCAGGTAAGATGACCGACT | 1378 |
| 35 | CGAGGCATGCCAAGTCGCCAAT | 1379 |
| 36 | AGTGTTGATAGGCGGATGAGAG | 1380 |
| 37 | TTCGGTCTAGACCTCTCACAAT | 1381 |
| 38 | GTGACGCTCATATCTTGCCACC | 1382 |
| 39 | GATGTAATTCTACGCGCGGACT | 1383 |
| 40 | GATGGCGATGTTGCATTACATG | 1384 |
| 41 | TATGCTCTGAATTAACGTAGAA | 1385 |
| 42 | AGGCAATATGGTGATCCGTAGC | 1386 |
| 43 | TGACAGCGATGCATACAGTAGT | 1387 |
| 44 | TTCTGCTAACGGTATCCAATAC | 1388 |
| 45 | GAGTCGTCCATACGATCTAGGA | 1389 |
| 46 | AGACGGACTCAACGCCAATTCC | 1390 |
| 47 | GTAGTGTTGAGCGGACCGAGCT | 1391 |
| 48 | AATATAACTAGATCATAGCCAG | 1392 |
| 49 | TCAATCGGAGAATACAGAACGT | 1393 |
| 50 | ATCTCCGTCGTCCGAACCAACA | 1394 |
| 51 | TAGGCGTTCAGCGGTATGCTTA | 1395 |
| 52 | TGCGTGCTATACAACCTATACG | 1396 |
| 53 | ATGGCCGGCATACATCTGTATG | 1397 |
| 54 | TGATGCTGACATAACACTGAAT | 1398 |
| 55 | ATCCAAGGTACCTGAACATCCT | 1399 |
| 56 | TAGTGACGACCAGGTGAGCCTC | 1400 |
| 57 | AGGAGGATCCGTCAAGTCGACC | 1401 |
| 58 | AGAGTATGCCAGATCGTGAGGC | 1402 |
| 59 | CCACTCACTAGGATGGCTGCGT | 1403 |
| 60 | TATCCAACCTGTTATAGCGATT | 1404 |
| 61 | TCTTGCAGTGAGTTGAGTCTGC | 1405 |
| 62 | CCACTGTTGTACATACACCTGG | 1406 |
| 63 | ATGCGCGTAGGCCACTAAGTCC | 1407 |
| 64 | ACAGCGGTCTACAACCGACTGC | 1408 |

**[Table 15-3]**

| No. | Primer sequence | SEQ ID NO: |
|---|---|---|
| 65 | TCGCGCTCCAGACAATTGCAGC | 1409 |
| 66 | CCGGTAGACCAGGAGTGGTCAT | 1410 |
| 67 | ATCTCCTAACCTAGAGCCATCT | 1411 |
| 68 | CCACATCGAATCTAACAACTAC | 1412 |
| 69 | TAGTCTTATTGAATACGTCCTA | 1413 |
| 70 | TCCTTAAGCCTTGGAACTGGCG | 1414 |
| 71 | CCGTGATGGATTGACGTAGAGG | 1415 |
| 72 | GCCTGGATAACAGATGTCTTAG | 1416 |
| 73 | CTCGACCTATAATCTTCTGCCA | 1417 |
| 74 | AGCTACTTCTCCTTCCTAATCA | 1418 |
| 75 | ACACGCTATTGCCTTCCAGTTA | 1419 |
| 76 | AAGCCTGTGCATGCAATGAGAA | 1420 |
| 77 | TCGTTGGTTATAGCACAACTTC | 1421 |
| 78 | GCGATGCCTTCCAACATACCAA | 1422 |
| 79 | CCACCGTTAGCACGTGCTACGT | 1423 |
| 80 | GTTACCACAATGCCGCCATCAA | 1424 |
| 81 | GGTGCATTAAGAACGAACTACC | 1425 |
| 82 | TCCTTCCGGATAATGCCGATTC | 1426 |
| 83 | AACCGCAACTTCTAGCGGAAGA | 1427 |
| 84 | TCCTTAAGCAGTTGAACCTAGG | 1428 |
| 85 | TACTAAGTCAGATAAGATCAGA | 1429 |
| 86 | TTCGCCATAACTAGATGAATGC | 1430 |
| 87 | AAGAAGTTAGACGCGGTGGCTG | 1431 |
| 88 | GTATCTGATCGAAGAGCGGTGG | 1432 |
| 89 | TCAAGAGCTACGAAGTAAGTCC | 1433 |
| 90 | CGAGTACACAGCAGCATACCTA | 1434 |
| 91 | CTCGATAAGTTACTCTGCTAGA | 1435 |
| 92 | ATGGTGCTGGTTCTCCGTCTGT | 1436 |
| 93 | TCAAGCGGTCCAAGGCTGAGAC | 1437 |
| 94 | TGTCCTGCTCTGTTGCTACCGT | 1438 |
| 95 | AGTCATATCGCGTCACACGTTG | 1439 |
| 96 | GGTGAATAAGGACATGAGAAGC | 1440 |

### [Table 16-1]

**Table 16. Random primer list (24-nucleotide)**

| No. | Primer sequence | SEQ ID NO: |
|---|---|---|
| 1 | CCTGATCTTATCTAGTAGAGACTC | 1441 |
| 2 | TTCTGTGTAGGTGTGCCAATCACC | 1442 |
| 3 | GACTTCCAGATGCTTAAGACGACA | 1443 |
| 4 | GTCCTTCGACGGAGAACATCCGAG | 1444 |
| 5 | CTTGGTTAGTGTACCGTCAACGTC | 1445 |
| 6 | AAGCGGCATGTGCCTAATCGACGT | 1446 |
| 7 | CGACCGTCGTTACACGGAATCCGA | 1447 |
| 8 | TCGCAAGTGTGCCGTTCTGTTCAT | 1448 |
| 9 | CGTACTGAAGTTCGGAGTCGCCGT | 1449 |
| 10 | CCACTACAGAATGGTAGCAGATCA | 1450 |
| 11 | AGTAGGAGAGAGGCCTACACAACA | 1451 |
| 12 | AGCCAAGATACTCGTTCGGTATGG | 1452 |
| 13 | GTTCCGAGTACATTGAATCCTGGC | 1453 |
| 14 | AGGCGTACGAGTTATTGCCAGAGG | 1454 |
| 15 | GTGGCATCACACATATCTCAGCAT | 1455 |
| 16 | GAGACCGATATGTTGATGCCAGAA | 1456 |
| 17 | CAACTGTAGCCAGTCGATTGCTAT | 1457 |
| 18 | TATCAATGCAATGAGAGGATGCAG | 1458 |
| 19 | GTATGCTCGGCTCCAAGTACTGTT | 1459 |
| 20 | AGAGACTCTTATAGGCTTGACGGA | 1460 |
| 21 | ACTTAACAGATATGGATCATCGCC | 1461 |
| 22 | AATCAGAGCGAGTCTCGCTTCAGG | 1462 |
| 23 | ACCACCGAGGAACAGGTGCGACAA | 1463 |
| 24 | TGGTACATGTCAACCGTAAGCCTG | 1464 |
| 25 | CGTGCCGCGGTGTTCTTGTATATG | 1465 |
| 26 | GACAAGCGCGCGTGAGACATATCA | 1466 |
| 27 | AGTGCACTCCGAACAAGAGTTAGT | 1467 |
| 28 | CCTCATTACCGCGTTAGGAGTCCG | 1468 |
| 29 | TGCTTATTGCTTAGTTGCTATCTC | 1469 |
| 30 | GCGTGATCCTGTTCTATTCGTTAG | 1470 |
| 31 | GGCCAGAACTATGACGAGTATAAG | 1471 |
| 32 | GATGGCGACTATCTAATTGCAATG | 1472 |

**[Table 16-2]**

| No. | Primer sequence | SEQ ID NO: |
|---|---|---|
| 33 | TAGTAACCATAGCTCTGTACAACT | 1473 |
| 34 | CGTGATCGCCAATACACATGTCGC | 1474 |
| 35 | TAATAACGGATCGATATGCACGCG | 1475 |
| 36 | ATCATCGCGCTAATACTATCTGAA | 1476 |
| 37 | CACGTGCGTGCAGGTCACTAGTAT | 1477 |
| 38 | AGGTCCAATGCCGAGCGATCAGAA | 1478 |
| 39 | CAGCATAACAACGAGCCAGGTCAG | 1479 |
| 40 | ATGGCGTCCAATACTCCGACCTAT | 1480 |
| 41 | AGGAACATCGTGAATAATGAAGAC | 1481 |
| 42 | TCTCGACGTTCATGTAATTAAGGA | 1482 |
| 43 | TCGCGGTTAACCTTACTTAGACGA | 1483 |
| 44 | ATCATATCTACGGCTCTGGCGCCG | 1484 |
| 45 | GCAGATGGAGACCAGAGGTACAGG | 1485 |
| 46 | AGACAGAAGATTACCACGTGCTAT | 1486 |
| 47 | CCACGGACAACATGCCGCTTAACT | 1487 |
| 48 | CTTGAAGTCTCAAGCTATGAGAGA | 1488 |
| 49 | ACAGCAGTCGTGCTTAGGTCACTG | 1489 |
| 50 | AGGTGTTAATGAACGTAGGTGAGA | 1490 |
| 51 | AGCCACTATGTTCAAGGCTGAGCC | 1491 |
| 52 | GCAGGCGGTGTCGTGTGACAATGA | 1492 |
| 53 | AGCCATTGCTACAGAGGTTACTTA | 1493 |
| 54 | ACAATCGAACCTACACTGAGTCCG | 1494 |
| 55 | CCGATCTCAATAGGTACCACGAAC | 1495 |
| 56 | GATACGTGGCGCTATGCTAATTAA | 1496 |
| 57 | AGAGAGATGGCACACATTGACGTC | 1497 |
| 58 | CTCAACTCATCCTTGTAGCCGATG | 1498 |
| 59 | GTGGAATAACGCGATACGACTCTT | 1499 |
| 60 | ATCTACCATGCGAATGCTCTCTAG | 1500 |
| 61 | ATACGCACGCCTGACACAAGGACC | 1501 |
| 62 | GTCCACTCTCAGTGTGTAGAGTCC | 1502 |
| 63 | AATATATCCAGATTCTCTGTGCAG | 1503 |
| 64 | CCTTCCGCCACATGTTCGACAAGG | 1504 |

**[Table 16-3]**

| No. | Primer sequence | SEQ ID NO: |
|---|---|---|
| 65 | ACTGTGCCATCATCCGAGGAGCCA | 1505 |
| 66 | TCTATGCCGCTATGGCGTCGTGTA | 1506 |
| 67 | CGTAACCTAAGGTAATATGTCTGC | 1507 |
| 68 | TACTGACCGTATCAAGATTACTAA | 1508 |
| 69 | TCATCGGAGCGCCATACGGTACGT | 1509 |
| 70 | GCAAGAGGAATGAACGAAGTGATT | 1510 |
| 71 | GGCTGATTGACATCCTGACTTAGT | 1511 |
| 72 | AAGGCGCTAGATTGGATTAACGTA | 1512 |
| 73 | GCTAGCTAGAAGAATAGGATTCGT | 1513 |
| 74 | CAGGTGACGGCCTCTATAACTCAT | 1514 |
| 75 | CAGGTTACACATACCACTATCTTC | 1515 |
| 76 | TTGCTACGTACCGTCTTAATCCGT | 1516 |
| 77 | CTCAACATGTCTTGCAAGCTTCGA | 1517 |
| 78 | GGTGCGGTACGTAGAACCAGATCA | 1518 |
| 79 | AATGCTCTCCAAGATCCTGACCTA | 1519 |
| 80 | GCTTCGCAGGTCTGGATGATGGAG | 1520 |
| 81 | ACATTGACCAGACAGCACCTTGCG | 1521 |
| 82 | AGGTATCAATGTGCTTAATAGGCG | 1522 |
| 83 | TCCGGACACACGATTAGTAACGGA | 1523 |
| 84 | TACGAAGTACTACAGATCGGTCAG | 1524 |
| 85 | AATTGTCAGACGAATACTGCTGGA | 1525 |
| 86 | TGAATCATGAGCCAGAGGTTATGC | 1526 |
| 87 | CACAAGACACGTCATTAACATCAA | 1527 |
| 88 | GAATGACTACATTACTCCGCCAGG | 1528 |
| 89 | AGCCAGAGATACTGGAACTTGACT | 1529 |
| 90 | TATCAGACACATCACAATGGATAC | 1530 |
| 91 | CTAGGACACCGCTAGTCGGTTGAA | 1531 |
| 92 | GTATAACTGCGTGTCCTGGTGTAT | 1532 |
| 93 | ATGCAATACTAAGGTGGACCTCCG | 1533 |
| 94 | ATGCAGACGCTTGCGATAAGTCAT | 1534 |
| 95 | TTGCTCGATACACGTAGACCAGTG | 1535 |
| 96 | TACTGGAGGACGATTGTCTATCAT | 1536 |

### [Table 17-1]

**Table 17. Random primer list 26-nucleotide**

| No. | Primer sequence | SEQ ID NO: |
|---|---|---|
| 1 | ACTAAGGCACGCTGATTCGAGCATTA | 1537 |
| 2 | CGGATTCTGGCACGTACAAGTAGCAG | 1538 |
| 3 | TTATGGCTCCAGATCTAGTCACCAGC | 1539 |
| 4 | CATACACTCCAGGCATGTATGATAGG | 1540 |
| 5 | AGTTGTAAGCCAACGAGTGTAGCGTA | 1541 |
| 6 | GTATCAGCTCCTTCCTCTGATTCCGG | 1542 |
| 7 | AACATACAGAATGTCTATGGTCAGCT | 1543 |
| 8 | GACTCATATTCATGTTCAGTATAGAG | 1544 |
| 9 | AGAGTGAACGAACGTGACCGACGCTC | 1545 |
| 10 | AATTGGCGTCCTTGCCACAACATCTT | 1546 |
| 11 | TCGTAGACGCCTCGTACATCCGAGAT | 1547 |
| 12 | CCGGCTCGTGAGGCGATAATCATATA | 1548 |
| 13 | AGTCCTGATCACGACCACGACTCACG | 1549 |
| 14 | GGCACTCAATCCTCCATGGAGAAGCT | 1550 |
| 15 | TCATCATTCCTCACGTTCACCGGTGA | 1551 |
| 16 | TCAACTCTGTGCTAACCGGTCGTACA | 1552 |
| 17 | TGTTCTTATGCATTAATGCCAGGCTT | 1553 |
| 18 | GATTCACGACCTCAACAGCATCACTC | 1554 |
| 19 | GGCGAGTTCGACCAGAATGCTGGACA | 1555 |
| 20 | TTCCGTATACAATGCGATTAAGATCT | 1556 |
| 21 | GAGTAATCCGTAACCGGCCAACGTTG | 1557 |
| 22 | CGCTTCCATCATGGTACGGTACGTAT | 1558 |
| 23 | CCGTCGTGGTGTGTTGACTGGTCAAC | 1559 |
| 24 | TATTCGCATCTCCGTATTAGTTGTAG | 1560 |
| 25 | TATTATTGTATTCTAGGCGGTGCAAC | 1561 |
| 26 | AGGCTGCCTACTTCCTCGTCATCTCG | 1562 |
| 27 | GTAACATACGGCTCATCGAATGCATC | 1563 |
| 28 | TTATGGCACGGATATTACCGTACGCC | 1564 |
| 29 | ATAGCACTTCCTCTAATGCTCTGCTG | 1565 |
| 30 | TCACAG GCAATAG CCTAATATTATAT | 1566 |
| 31 | GGCGGATGTTCGTTAATATTATAAGG | 1567 |
| 32 | TGCAATAGCCGTTGTCTCTGCCAGCG | 1568 |

**[Table 17-2]**

| No. | Primer sequence | SEQ ID NO: |
|---|---|---|
| 33 | TACAGCGCGTTGGCGAGTACTGATAG | 1569 |
| 34 | TGCAGTTAGTACCTTCTCACGCCAAC | 1570 |
| 35 | CCATTGGCTACCTAGCAGACTCTACC | 1571 |
| 36 | AACAGTAGCTCGCGTCTTGCTCTCGT | 1572 |
| 37 | GCAGTCCATCAGCTCTCGCTTATAGA | 1573 |
| 38 | TATCTCTCTGTCGCCAGCTTGACCAA | 1574 |
| 39 | CAGACTGTTCAAGCTTGCTGTAGGAG | 1575 |
| 40 | TAACCGGAACTCGTTCAGCAACATTC | 1576 |
| 41 | TCAATTATGCATGTCGTCCGATCTCT | 1577 |
| 42 | TTGTCTAAGTCAACCTGTGGATAATC | 1578 |
| 43 | TCTAAGAGTGGTATGACCAGGAGTCC | 1579 |
| 44 | TCGTAGTACTACTGGAACAGGTAATC | 1580 |
| 45 | ATGTCAACATTCTAATCATCTCTCGG | 1581 |
| 46 | AGCGCGCAACTGTTACGGTGATCCGA | 1582 |
| 47 | GCGATAGAATAATGGTGTCACACACG | 1583 |
| 48 | AAGGCTGCGATGAGAGGCGTACATCG | 1584 |
| 49 | GGTTCATGGTCTCAGTCGTGATCGCG | 1585 |
| 50 | TAGTGACTCTATGTCACCTCGGAGCC | 1586 |
| 51 | ATGTGATAGCAATGGCACCTCTAGTC | 1587 |
| 52 | TCGCGAAGTGTAATGCATCATCCGCT | 1588 |
| 53 | ATGTGGCGACGATCCAAGTTCAACGC | 1589 |
| 54 | ACCTTGTATGAGTCGGAGTGTCCGGC | 1590 |
| 55 | ACCTCAAGAGAGTAGACAGTTGAGTT | 1591 |
| 56 | GGTGTAATCCTGTGTGCGAAGCTGGT | 1592 |
| 57 | ATAGCGGAACTGTACGACGCTCCAGT | 1593 |
| 58 | AAGCACGAGTCGACCATTAGCCTGGA | 1594 |
| 59 | ATTCCGGTAACATCAGAAGGTACAAT | 1595 |
| 60 | GTGCAACGGCAGTCCAGTATCCTGGT | 1596 |
| 61 | CCATCTTATACACGGTGACCGAAGAT | 1597 |
| 62 | GCACTTAATCAAGCTTGAGTGATGCT | 1598 |
| 63 | AGTATTACGTGAGTACGAAGATAGCA | 1599 |
| 64 | TTCTTAGGTTAAGTTCCTTCTGGACC | 1600 |

**[Table 17-3]**

| No. | Primer sequence | SEQ ID NO: |
|---|---|---|
| 65 | GTCCTTGCTAGACACTGACCGTTGCT | 1601 |
| 66 | GCCGCTATGTGTGCTGCATCCTAAGC | 1602 |
| 67 | CCATCAATAACAGACTTATGTTGTGA | 1603 |
| 68 | CGCGTGTGCTTACAAGTGCTAACAAG | 1604 |
| 69 | CGATATGTGTTCGCAATAAGAGAGCC | 1605 |
| 70 | CGCGGATGTGAGCGGCTCAATTAGCA | 1606 |
| 71 | GCTGCATGACTATCGGATGGAGGCAT | 1607 |
| 72 | CTATGCCGTGTATGGTACGAGTGGCG | 1608 |
| 73 | CCGGCTGGAGTTCATTACGTAGGCTG | 1609 |
| 74 | TGTAGGCCTACTGAGCTAGTATTAGA | 1610 |
| 75 | CCGTCAAGTGACTATTCTTCTAATCT | 1611 |
| 76 | GGTCTTACGCCAGAGACTGCGCTTCT | 1612 |
| 77 | CGAAGTGTGATTATTAACTGTAATCT | 1613 |
| 78 | GCACGCGTGGCCGTAAGCATCGATTA | 1614 |
| 79 | ATCCTGCGTCGGAACGTACTATAGCT | 1615 |
| 80 | AGTATCATCATATCCATTCGCAGTAC | 1616 |
| 81 | AGTCCTGACGTTCATATATAGACTCC | 1617 |
| 82 | CTTGCAGTAATCTGAATCTGAAGGTT | 1618 |
| 83 | ATAACTTGGTTCCAGTAACGCATAGT | 1619 |
| 84 | GATAAGGATATGGCTGTAGCGAAGTG | 1620 |
| 85 | GTGGAGCGTTACAGACATGCTGAACA | 1621 |
| 86 | CGCTTCCGGCAGGCGTCATATAAGTC | 1622 |
| 87 | ATAACATTCTAACCTCTATAAGCCGA | 1623 |
| 88 | ACGATCTATGATCCATATGGACTTCC | 1624 |
| 89 | TGAAGCTCAGATATCATGCCTCGAGC | 1625 |
| 90 | AGACTTCACCGCAATAACTCGTAGAT | 1626 |
| 91 | AGACTAAGACATACGCCATCACCGCT | 1627 |
| 92 | TGTAGCGTGATGTATCGTAATTCTGT | 1628 |
| 93 | TGTGCTATTGGCACCTCACGCTGACC | 1629 |
| 94 | TGTAGATAAGTATCCAGCGACTCTCT | 1630 |
| 95 | AATTCGCCAATTGTGTGTAGGCGCAA | 1631 |
| 96 | CGATTATGAGTACTTGTAGACCAGCT | 1632 |

### [Table 18-1]

**Table 18. Random primer list (28-nucleotide)**

| No. | Primer sequence | SEQ ID NO: |
|---|---|---|
| 1 | TTGCAAGAACAACGTATCTCATATGAAC | 1633 |
| 2 | CACCGTGCTGTTATTACTTGGTATTCGG | 1634 |
| 3 | CACGTGTATTGTTGCACCAGAACGACAA | 1635 |
| 4 | ATGCACGTAATTACTTCCGGAGAAGACG | 1636 |
| 5 | TATGTTGTCTGATATGGTTCATGTGGCA | 1637 |
| 6 | AGCGCGACTAGTTGATGCCAACATTGTA | 1638 |
| 7 | ATAGGCAGGTCCAGGCTCGGAACAAGTC | 1639 |
| 8 | GCGGTAGTCGGTCAAGAACTAGAACCGT | 1640 |
| 9 | ACTATACACTCTAGCTATTAGGAAGCAT | 1641 |
| 10 | GATCATCTTGCTTCTCCTGTGGAGATAA | 1642 |
| 11 | CTACTACGAGTCCATAACTGATAGCCTC | 1643 |
| 12 | GCACAGACACCTGTCCTATCTAGCAGGA | 1644 |
| 13 | AAGCGAGGCGCGAAGGAGATGGAAGGAT | 1645 |
| 14 | CTGAAGACGCCAGTCTGGATAGGTGCCT | 1646 |
| 15 | GTAAGCTCTGTCCTTCGAGATTGATAAG | 1647 |
| 16 | GGTTAGAGAGATTATTGTGCGCATCCAT | 1648 |
| 17 | CCAGGAGGACCTATGATCTTGCCGCCAT | 1649 |
| 18 | ACTATTCGAGCTACTGTATGTGTATCCG | 1650 |
| 19 | GACATCGCGATACGTAACTCCGGAGTGT | 1651 |
| 20 | CCGCAATTCGTCTATATATTCTAGCATA | 1652 |
| 21 | CTACACTTGAGGTTGATGCTCAAGATCA | 1653 |
| 22 | CGATCAGTTCTAGTTCACCGCGGACAAT | 1654 |
| 23 | AAGAATGATGATTGGCCGCGAACCAAGC | 1655 |
| 24 | CACGACCGGAACTAGACTCCTACCAATT | 1656 |
| 25 | AGTTGCCTGTGAGTGAGGCTACTATCTC | 1657 |
| 26 | GATTCTTCCGATGATCATGCCACTACAA | 1658 |
| 27 | CGCTGAAGTGAACTATGCAAGCACCGCA | 1659 |
| 28 | ATTATCGTGATGGTGAGACTGAGCTCGT | 1660 |
| 29 | CGAGGCCACTCTGAGCCAGGTAAGTATC | 1661 |
| 30 | TGCCGAGGACAGCCGATCACATCTTCGT | 1662 |
| 31 | GTTGACATGAAGGTTATCGTCGATATTC | 1663 |
| 32 | GTGGTCCAGGTCAAGCTCTGATCGAATG | 1664 |

**[Table 18-2]**

| No. | Primer sequence | SEQ ID NO: |
|---|---|---|
| 33 | CCAGTCCGGTGTACTCAGACCTAATAAC | 1665 |
| 34 | CGAGACACTGCATGAGCGTAGTCTTATT | 1666 |
| 35 | GACGGCTTGTATACTTCTCTACGGTCTG | 1667 |
| 36 | TTAGCTGGATGGAAGCCATATTCCGTAG | 1668 |
| 37 | CAGCCTACACTTGATTACTCAACAACTC | 1669 |
| 38 | GTACGTAGTGTCACGCGCCTACGTTCGT | 1670 |
| 39 | CTACAACTTCTCAATCATGCCTCTGTTG | 1671 |
| 40 | CGAGGACAGAATTCGACATAAGGAGAGA | 1672 |
| 41 | GCCGAACGACACAGTGAGTTGATAGGTA | 1673 |
| 42 | GAACACTATATGCTGTCGCTGTCTGAGG | 1674 |
| 43 | GTTAAGTTCTTCGGCGGTCATGCTCATT | 1675 |
| 44 | TTGCTTACAGATCGCGTATCCATAGTAT | 1676 |
| 45 | GAGGACCACCTCTGCGAAGTTCACTGTG | 1677 |
| 46 | AATCCTAGCATATCGAGAACGACACTGA | 1678 |
| 47 | TGAATACTATAGCCATAGTCGACTTCCG | 1679 |
| 48 | GACATCCACGAAGCTGGTAATCGGAACC | 1680 |
| 49 | TTAGCCGTCTTAGAAGTGTCTGACCGGC | 1681 |
| 50 | CTATTCTGCCGTAATTGATTCCTTCGTT | 1682 |
| 51 | ACGCCTCTGGTCGAAGGTAGATTAGCTC | 1683 |
| 52 | CAGCCTATTGATCGTAAGTAGATGGTCC | 1684 |
| 53 | TTAAGTGAGGTGGACAACCATCAACTTC | 1685 |
| 54 | AAGGCCTTGCGGCTAAGTAGTATTCATC | 1686 |
| 55 | TTGTGATACTAATTCTTCTCAAGAGTCA | 1687 |
| 56 | GCATTAGGTGACGACCTTAGTCCATCAC | 1688 |
| 57 | GCGGATGGACGTATACAGTGAGTCGTGC | 1689 |
| 58 | GAACATGCCAGCCTCAACTAGGCTAAGA | 1690 |
| 59 | TCCGTCATTAGAGTATGAGTGACTACTA | 1691 |
| 60 | AACACTTAGTAACCAGTTCGGACTGGAC | 1692 |
| 61 | CGCTAACTATTGCGTATATTCGCGGCTT | 1693 |
| 62 | GCCATCTACGATCTTCGGCTTATCCTAG | 1694 |
| 63 | CCTGAGAATGTTGACTAAGATCTTGTGA | 1695 |
| 64 | TCGGTTAGTCTAATCATCACGCAACGGA | 1696 |

**[Table 18-3]**

| No. | Primer sequence | SEQ ID NO: |
|---|---|---|
| 65 | ATTATCTATTGAAGCAGTGACAGCGATC | 1697 |
| 66 | GAGGAGAATCACGGAACACGGTCACATG | 1698 |
| 67 | GCTGCAAGCATTATGACCATGGCATCTG | 1699 |
| 68 | GAACAACCTATAACGACGTTGTGGACAA | 1700 |
| 69 | TTAATCATCGATAGACGACATGGAATCA | 1701 |
| 70 | TCGAGTGTAAGCACACTACGATCTGGAA | 1702 |
| 71 | GCTACGCACAGTCTCTGCACAGCTACAC | 1703 |
| 72 | CCTGTATGTACGTTCTGGCTAATACCTT | 1704 |
| 73 | TGAAGCACCGGTACATGGTGTATCCGGA | 1705 |
| 74 | TGCTGGAACCTAACTCGGTGATGACGAT | 1706 |
| 75 | CGCTATCTTACTGCCAAGTTCTCATATA | 1707 |
| 76 | AACGCGCGCGTATCGGCAATAATCTCAA | 1708 |
| 77 | CCATTAGGATGACCATCGACTATTAGAG | 1709 |
| 78 | TACTGCTAGACTGCGTGCATTCATGGCG | 1710 |
| 79 | CATTGCGCGCTCCACGAACTCTATTGTC | 1711 |
| 80 | GACGCGCCTAGAACTGTATAGCTCTACG | 1712 |
| 81 | CATTGCAACTTGTCGGTGATGGCAATCC | 1713 |
| 82 | TTAATGCACATGCAGTACGGCACCACAG | 1714 |
| 83 | AGCGGTACGTGGACGAGTGGTAATTAAT | 1715 |
| 84 | GACGTATTGCTATGCATTGGAAGATGCT | 1716 |
| 85 | AACACTTCGACCATTGCGCCTCAATGGT | 1717 |
| 86 | CGGTACGCTCTAGCGGTCATAAGATGCA | 1718 |
| 87 | CCTGAATAACAGCCGCGCCTAATTAGAT | 1719 |
| 88 | AAGCGTCTAATGTGCCTTAAGTCACATG | 1720 |
| 89 | GCTCTCCAAGAACCAGAAGTAAGCATCG | 1721 |
| 90 | GAGGAGAGTTGTCCGAGTGGTGTGATGT | 1722 |
| 91 | TAACGAGTGGTGCGTCTAAGCAATTGAG | 1723 |
| 92 | CCAACAGTATGCTGACATAACTATGATA | 1724 |
| 93 | GATCCTTGCCACGCCTATGAGATATCGC | 1725 |
| 94 | AACGCGCTACCGTCCTTGTGCATAGAGG | 1726 |
| 95 | CTACATGTGCCTTATAGTACAGAGGAAC | 1727 |
| 96 | CAGCCTCGTAGTTAGCGTGATTCATGCG | 1728 |

### [Table 19-1]

**Table 19. Random primer list (29-nucleotide)**

| No. | Primer sequence | SEQ ID NO: |
|---|---|---|
| 1 | CTCCTCGCCGATTGAAGTGCGTAGAACTA | 1729 |
| 2 | CAGCAGGCCTCAATAGGATAAGCCAACTA | 1730 |
| 3 | GACCATCAATCTCGAAGACTACGCTCTGT | 1731 |
| 4 | GGTTGCTCCGTCTGTTCAGCACACTGTTA | 1732 |
| 5 | AATGTCGACTGGCCATTATCGCCAAGTGT | 1733 |
| 6 | GATAGCTTGCCATGCGAATGGATCTCCAG | 1734 |
| 7 | CCAGACCGGAGCCAATTGGCTGCCAATAT | 1735 |
| 8 | AACGTCGCTCCATACGTTACCTAATGCAG | 1736 |
| 9 | GAATATGACGCGAACAGTCTATTCGGATC | 1737 |
| 10 | GACGAGAATGTATTAAGGATAAGCAAGGT | 1738 |
| 11 | AAGTCGTATGAATCGCTATCACATGAGTC | 1739 |
| 12 | GTCGTGGAGACTACAATTCTCCTCACGTT | 1740 |
| 13 | GTTGCCACCGTTACACGACTATCGACAGT | 1741 |
| 14 | AGGATAGGCTACGCCTTACTCTCCTAAGC | 1742 |
| 15 | TAATCATCCTGTTCGCCTCGAGGTTGTTA | 1743 |
| 16 | GACAAGCAGTAATAATTACTGAGTGGACG | 1744 |
| 17 | TACAGCGTTACGCAGGTATATCAAGGTAG | 1745 |
| 18 | CTAACATCACTTACTATTAGCGGTCTCGT | 1746 |
| 19 | CCGCGCTTCTTGACACGTTCTCCACTAGG | 1747 |
| 20 | CAAGTAACATGAGATGCTATCGGTACATT | 1748 |
| 21 | CGACCACTAGGCTGTGACCACGATACGCT | 1749 |
| 22 | CAGGTCATGTGACGCAGTCGGCAGTCAAC | 1750 |
| 23 | ACTCCATCGTTAGTTCTTCCGCCGTGCTG | 1751 |
| 24 | CTCACCACGTATGCGTCACTCGGTTACGT | 1752 |
| 25 | TGCCTATGCTATGGACCTTGCGCGACTCT | 1753 |
| 26 | AATGAAGGTCAACGCTCTGTAGTTACGCG | 1754 |
| 27 | CACCATTGATTCATGGCTTCCATCACTGC | 1755 |
| 28 | GACACGCAAGGTAATTCGAGATTGCAGCA | 1756 |
| 29 | CACCGAGAGGAAGGTTCGATCGCTTCTCG | 1757 |
| 30 | CAGTTATCGGATTGTGATATTCACTCCTG | 1758 |
| 31 | ATACTGTAACGCCTCAACCTATGCTGACT | 1759 |
| 32 | ATCTGTCTTATTCTGGCACACTCAGACTT | 1760 |

**[Table 19-2]**

| No. | Primer sequence | SEQ ID NO: |
|---|---|---|
| 33 | TCCAACCGGTGACGTGCTCTTGATCCAAC | 1761 |
| 34 | CACACTCAGTTCGGCTATCTCTGCGATAG | 1762 |
| 35 | AGCTGTAAGTCAGGTCTACGACTCGTACT | 1763 |
| 36 | GTCGGCGGCACGCACAGCTAACATTCGTA | 1764 |
| 37 | ATATGGTAGCCAGCCACGTATACTGAACA | 1765 |
| 38 | TGGACAATCCGACTCTAACACAGAGGTAG | 1766 |
| 39 | TCCGCCGCTGACAGTTCAATCTATCAATT | 1767 |
| 40 | GGTTCCTTAGAATATGCACCTATCAGCGA | 1768 |
| 41 | CGGCTGTACGACATGGATCATAAGAGTGT | 1769 |
| 42 | TGCAGATGTACGCTGTGGCCAGTGGAGAG | 1770 |
| 43 | CCTACTCACTTAACAATAATCGGTTCGGT | 1771 |
| 44 | CGCTTCCTACTGCCTGTGCCGCGACATAA | 1772 |
| 45 | CTAGACCGACCGGTTATGCGCTATTGTTC | 1773 |
| 46 | TTGTGAGCACGTCTGCGGCAAGCCTATGG | 1774 |
| 47 | TCATCGGCCGGCGCTGTTGTTGTTACCAT | 1775 |
| 48 | GCGGTTAGGTGCAGTTAGGAAGACTATCA | 1776 |
| 49 | TATGCGGTCGTGAGGCGTAGCATTCTAGA | 1777 |
| 50 | CCATCTATTCGTCGAACTCTCAGCTCGTA | 1778 |
| 51 | ATCAGATCTACTGATCGCGGTAGAGTATC | 1779 |
| 52 | TACACATAGGCGGCGCAGCCTTCTAATTA | 1780 |
| 53 | TTAACCGTAGTTCTTAGCTTACGCCGCTC | 1781 |
| 54 | ACTATAGAGGACATGGCACTCCTCTTCTA | 1782 |
| 55 | CAGTTCGTATTAAGATTGAATGTAGCGGT | 1783 |
| 56 | AGTTATCGGTATCCGCTTATCCGTACGTA | 1784 |
| 57 | AGCTTATTCATACACTGCACCACAGCAAG | 1785 |
| 58 | CCGTCGGCTAGTCTATCCTCTAATTAGAA | 1786 |
| 59 | GTCCGCTTCCATGCCTGCTGTACGAACAC | 1787 |
| 60 | TCTCTTCCTCCTTCATTGTTCGCTAGCTC | 1788 |
| 61 | TCTCTTGAGCGGTCCTCATACAGGTCTGC | 1789 |
| 62 | GACCAAGTGTAGGTGATATCACCGGTACT | 1790 |
| 63 | AAGATTGTGATAGGTTGGTAGTTACCACA | 1791 |
| 64 | TCGCCTCCGAAGAGTATAGCATCGGCAGA | 1792 |

**[Table 19-3]**

| No. | Primer sequence | SEQ ID NO: |
|---|---|---|
| 65 | GAGGTAGTTATGAGCATCGAGGTCCTGTT | 1793 |
| 66 | GGACGCAAGATCGCAGGTACTTGTAAGCT | 1794 |
| 67 | ACTCGTACACGTCATCGTGCAGGTCTCAG | 1795 |
| 68 | TAATCCGTCAGGAGTGAGATGGCTCGACA | 1796 |
| 69 | AAGATGGTTCCGCGCATTGACTAGCAAGT | 1797 |
| 70 | TCCGCGATCTGCGGATCTTGAATGCTCAC | 1798 |
| 71 | TTCACGAGAGTCAACTGCTAGTATCCTAG | 1799 |
| 72 | TTCCAACTGGATTCTTCCAACTCCTCGAA | 1800 |
| 73 | CACTACTACTCAAGTTATACGGTGTTGAC | 1801 |
| 74 | CAACTGGATTCTCAGGATGCGTCTCTAGC | 1802 |
| 75 | TGGACTAGAGTGGAGCGATTACGTAATAT | 1803 |
| 76 | GAGGTCATTCAACTGGACTCGCCACGGAC | 1804 |
| 77 | CAGGTGTGTAACGCTGCAATCACATGAAT | 1805 |
| 78 | TATGCTGAGGTATTAGTTCTAACTATGCG | 1806 |
| 79 | CGTCTGAGTCGGATAAGGAAGGTTACCGC | 1807 |
| 80 | GTACTATCGTCGCAGGCACTATCTCTGCC | 1808 |
| 81 | GCTTCCTCCTTGCAACTTCATTGCTTCGA | 1809 |
| 82 | TGTCTACGAAGTAGAAGACACGAATAATG | 1810 |
| 83 | CCGTCATCTAAGGCAGAGTACATCCGCGA | 1811 |
| 84 | CCGGAGGCGTACTAACTGACCACAACACC | 1812 |
| 85 | AACTCGTCGCTGCCTGAATAGGTCAGAGT | 1813 |
| 86 | TTATAAGATTAATGTCGGTCAGTGTCGGA | 1814 |
| 87 | CGTCTCGATGGATCCACACGAACCTGTTG | 1815 |
| 88 | ATGCCATCATGGTCGTCCTATCTTAAGGC | 1816 |
| 89 | GCGCTTCAGCGATTCGTCATGCAAGGCAC | 1817 |
| 90 | CCAAGCGATACCGAGGTACGGTTAACGAG | 1818 |
| 91 | ATATGACAGACAGGTGGACCTAAGCAAGC | 1819 |
| 92 | CACTACATCGTCAGGCCTGGAAGCCTCAG | 1820 |
| 93 | GCCGTGTAGACGAGGACATTATGTCGTAT | 1821 |
| 94 | CAACGTATATACACACCTTGTGAAGAGAA | 1822 |
| 95 | TCCAACGTAATTCCGCCGTCTGTCGAGAC | 1823 |
| 96 | AATTCGTGCTTCGATCACCGTAGACTCAG | 1824 |

### [Table 20-1]

**Table 20. Random primer list (30-nucleotide)**

| No. | Primer sequence | SEQ ID NO: |
|---|---|---|
| 1 | ACTATATTGTATTCACGTCCGACGACTCGC | 1825 |
| 2 | GACGAGCTTGTGGTACACTATACCTATGAG | 1826 |
| 3 | TGATTCAAGCACCAGGCATGCTTAAGCTAG | 1827 |
| 4 | CGGTCTCCTATAGGAAGGCTCATTCTGACG | 1828 |
| 5 | AGTCAGTGTCGAATCAATCAAGGCGTCCTT | 1829 |
| 6 | CGAACGTAATGGCCATCACGCGCTGGCCTA | 1830 |
| 7 | CGAACCTGGACCACCTGGCATTACCATTAC | 1831 |
| 8 | ACATTAGGTTCCTGTAATGTCTTATCAACG | 1832 |
| 9 | CGTCTAATGCACCGTATCGTCTTCGCGCAT | 1833 |
| 10 | TCTATGACTTACAACGGAATCTTACTTCGT | 1834 |
| 11 | GTAACCGATCGGTACCGTCTGCTATTGTTC | 1835 |
| 12 | GGTGATTGATAAGCAACACATATTAGGAGG | 1836 |
| 13 | AATTATCGACGCTAATAGGCGAGCTGTTCA | 1837 |
| 14 | GGAGGTACATGACGAGTGGACAGACAGACC | 1838 |
| 15 | CTCTAATCCGTTATGCGGTGATGTAATCCG | 1839 |
| 16 | GCAAGCACGCGGCTTGGCGAACTTCTATGC | 1840 |
| 17 | TAGATGTAGGCCTGGTAGGCAGAGGAGTAA | 1841 |
| 18 | CCGAGTGGCGACCACACAGGTACGCATTAA | 1842 |
| 19 | GTCCTGGCTCAGATTAGTGCACTTAGTTAT | 1843 |
| 20 | GCGGTACCTACATGTTATGACTCAGACGAC | 1844 |
| 21 | TCTCTGCCAATGCTGGTCTCATCGAATCCA | 1845 |
| 22 | TCTCTACACAGCTACATACTATACTGTAAC | 1846 |
| 23 | TACGACGGACGCTGGTGGTGTAAGAGAAGG | 1847 |
| 24 | GCCTCGATATATCTACGTATAGTTCAAGTT | 1848 |
| 25 | GGCTCCTGCATTCATTGAAGGTCGGCCTTG | 1849 |
| 26 | CAGTTCGGTGATTCAAGAGAACAATGGTGG | 1850 |
| 27 | TATAACGAAGCCGGCTGGAACGGTAACTCA | 1851 |
| 28 | CTGTATCAATTCAAGTGACAGTGGCACGTC | 1852 |
| 29 | AGCAATTGCGGTTCATAGGCGTAATTATAT | 1853 |
| 30 | CATATGGACCTGGAGATCACCGTTCAGTCC | 1854 |
| 31 | GAAGGCCGTTGGTCTATCTCTTACTGGAGC | 1855 |
| 32 | GTGCGTTCATCTAGCCTAAGACGCTGACCT | 1856 |

**[Table 20-2]**

| No. | Primer sequence | SEQ ID NO: |
|---|---|---|
| 33 | GAGTAACTTATATCCTCTCTACGACATCGA | 1857 |
| 34 | ATTCTACGCTGATGTCTCCGCTGAACAGGA | 1858 |
| 35 | TCATCAACGTTACTCACTAGTACCACGGCT | 1859 |
| 36 | AACCATTCTTGAACGTTGAGAACCTGGTGG | 1860 |
| 37 | ACGACACCTCCGCGGAACATACCTGATTAG | 1861 |
| 38 | GCGCACTTATTGAAGTAATCTCATGGCCAA | 1862 |
| 39 | GCGCCAATTCAGCCAGTTAGCGTCTCCGTG | 1863 |
| 40 | AGCAACAAGTCGCTGTATATCGACTGGCCG | 1864 |
| 41 | CCTTACAATAGACCTCGCGGCGTTCATGCC | 1865 |
| 42 | GGATCCAACTTCAGCGAAGCACCAACGTCG | 1866 |
| 43 | GCGCCAGTTCTCGTACTCTCGAGAAGCGAC | 1867 |
| 44 | GAGTGCGGCCAATCTGGAACTCATGACGTT | 1868 |
| 45 | CCTGAGAGTGATTCGTGTCTGCGAAGATGC | 1869 |
| 46 | GTGACTGGTTAAGGCAATATTGGTCGACCG | 1870 |
| 47 | CTATCAAGCCTTACAAGGTCACGTCCACTA | 1871 |
| 48 | ACTGCGTCCTTGCGTCGGAACTCCTTGTGT | 1872 |
| 49 | TGCAACTCAGTGGCGGCGACACCAAGAGCT | 1873 |
| 50 | TTCGGTTCTACTAGGATCTCTATCTGAGCT | 1874 |
| 51 | AGCTAATCTATTAAGACAGATTAGACAGGA | 1875 |
| 52 | GGACCGCTCTTAGGTTATGCACCTGCGTAT | 1876 |
| 53 | CTCTAATACTAGTCCACAGGTTAGTACGAA | 1877 |
| 54 | ATCCATATATGCTCGTCGTCAGCCAGTGTT | 1878 |
| 55 | GCTATTACTGTGTTGATGTCCACAGGAGAA | 1879 |
| 56 | GCTACGGCGCAGATCTAGACAACTGGAAGT | 1880 |
| 57 | GCCTCTTGTGTTAGCCGAATACCAATGACC | 1881 |
| 58 | TGAGGACGATAACATTACCTCTCGAGTCGC | 1882 |
| 59 | CGATTACCAATCCGACGACTTCGCAGCAGC | 1883 |
| 60 | ATGACACGAGTCCAGTACATATGCGAAGAC | 1884 |
| 61 | GCGCTCGCATGCACTAGTGTAGACTGACGA | 1885 |
| 62 | GCACATCTCAGAATTGATGGTCTATGTCGC | 1886 |
| 63 | TTCTTCGACGCCGCGTACTAATAGGTCAAT | 1887 |
| 64 | GGAAGCGCCTCTAACAACCGATGCTTGTGG | 1888 |

**[Table 20-3]**

| No. | Primer sequence | SEQ ID NO: |
|---|---|---|
| 65 | CTCTAGACGCGTCGTGACTCCAATCTGTTG | 1889 |
| 66 | GTAGTTCGTCGGAGTGACCTCGTACTCACT | 1890 |
| 67 | ATGCTGTCGAGTGTCCGGCATAGAGCACAC | 1891 |
| 68 | GCGCATCTTGCAGCGTCCTGTAGTTCTGAA | 1892 |
| 69 | GCGATTGTTGAGGAACCACAGCGGCACCTA | 1893 |
| 70 | CACGCGTACTCTGCTTGCTGTGTGGTCGGT | 1894 |
| 71 | CATCCAACGCAGGACCTAGTAGTCATGCTT | 1895 |
| 72 | TTCTAGTTGTGATGAGAATCGCTAGCGTGC | 1896 |
| 73 | CATTCTGAATCTGGTCTCTCTCGATCATCC | 1897 |
| 74 | ATTAATGTAGAGGATAGTTCCGTTCTCTCC | 1898 |
| 75 | GTATCGCGCTTACGAATGAGGTGTGGCTTC | 1899 |
| 76 | GCTGGTGAGAGAGCCAGATTATCGGTGGAG | 1900 |
| 77 | GGCACGAGCAGGTAGAACTAGAACCTAGAT | 1901 |
| 78 | TGTATTATCTCGAAGCGGTGCGTTAGAGTC | 1902 |
| 79 | CACGTGTTCTAGCTACTAATGGCGTCAATT | 1903 |
| 80 | CGCGCTACATTACTTCCTACACCATGCGTA | 1904 |
| 81 | TGAGGCAACTAGTGTTCGCAAGATGACGGA | 1905 |
| 82 | TTATTATTGTCTGTGGAACGCACGCCAGTC | 1906 |
| 83 | GCTATAGTATTATCCATGAATTCCGTCGGC | 1907 |
| 84 | GTATCAATAGCTCAATTCGTCAGAGTTGTG | 1908 |
| 85 | TAGTCCATGCGTGGATATATTGAGAGCTGA | 1909 |
| 86 | GCACAGTACGACTTATAACAGGTCTAGATC | 1910 |
| 87 | ACTCAATGGTGGCACGCTCGGCGCAGCATA | 1911 |
| 88 | GTAGTACCACTCCGCCTTAGGCAGCTTAAG | 1912 |
| 89 | CGCTCAACTGATGCGTGCAACCAATGTTAT | 1913 |
| 90 | GCAGCTTGACTGCCTAGACAGCAGTTACAG | 1914 |
| 91 | GCAACTTCTTAGTACGAATTCATCGTCCAA | 1915 |
| 92 | ATCCGTATGCTGCGGCAGTGGAGGTGGCTT | 1916 |
| 93 | TGCGGATCAATCCAGTTCTGTGTACTGTGA | 1917 |
| 94 | TTATGATTATCACCGGCGTAACATTCCGAA | 1918 |
| 95 | GCTACCTAGATTCTTCAACTCATCGCTACC | 1919 |
| 96 | CAGTGTTAGAATGGCGGTGTGTAGCCGCTA | 1920 |

### [Table 21-1]

**Table 21. Random primer list (35-nudeotide)**

| No. | Primer sequence | SEQ ID NO: |
|---|---|---|
| 1 | GCTTATAGACTACAGCTGCGAGGTATAAGGTCACT | 1921 |
| 2 | CGCTCAGCAGGATGCTATCCTAAGTTAATGTGGTG | 1922 |
| 3 | GAACTGAGCGGACATCAGCTAGGCCTACAATACAT | 1923 |
| 4 | TCGTGAACTTCTGCGTTGGTCTCTACCAAGGCGGT | 1924 |
| 5 | TAAGTCAGGTATCTTATCAGTGGTACACGGTACGA | 1925 |
| 6 | TAATAATGTTGCGCGTGACCGAGGAGGAATCCACT | 1926 |
| 7 | CTAGGAGTTCTCGTAAGCTGGAGTACCGTAACGTG | 1927 |
| 8 | GGACTCTCCTCAGAGGATCCTTCTTGCGCAGGCAT | 1928 |
| 9 | GCTAGAGGCCTGAGTACACCTTCTCGCATCAGGAT | 1929 |
| 10 | ATATCGCGAGCACTAACGTCGTTGTCGTTCTAGGA | 1930 |
| 11 | AGCGGTTACTATACCTGGCGGCTGACGTTGTTAGT | 1931 |
| 12 | GAGCTAGGTAGATCTCCAAGTGTAGCTAAGAAGAG | 1932 |
| 13 | GGAGTCGCTGGTGACGTATGCCGAGGATGAGCTTC | 1933 |
| 14 | CGCCGACCTCCTGTTCACGAAGCCGCCTGATGTAA | 1934 |
| 15 | AGTAGGCACTTAGTTATCGATTACGTTAGTTAGTC | 1935 |
| 16 | GGATGACGTCTCAGTCTACCTCGCAGTGTCGTCTA | 1936 |
| 17 | CTGGTTCGCGTTAGCAATACTAAGGCAGTCAGGAG | 1937 |
| 18 | ATATGGTCATATTGGCCTCTTCGAACACAGACTGT | 1938 |
| 19 | TATCAGAGGATAGCAGGTCTGAGTTGCAAGGCTAA | 1939 |
| 20 | GGTGGTCTGACCATAGCTGTTCTTCTCACAGAGAC | 1940 |
| 21 | GCAATACCAACGAGATGAGTATTCGTTGAAGCTCT | 1941 |
| 22 | CCAAGTCGACGCTGCATGAATGAGCGCTATTCACT | 1942 |
| 23 | CCATTAGATCGCTTCGAGACAATTAGGAGACATGA | 1943 |
| 24 | GATGACTGTACCTCCTATCATTGAGTGTGGACCAA | 1944 |
| 25 | ATATCTGGATGAATAGTGGTTAGGTAAGCAAGTAA | 1945 |
| 26 | ACCGACTATGTTAATTCGTGTCTGGATGGCAGAAT | 1946 |
| 27 | GTGGCAGTCTTGCTAGTATCTTAGACCATCACCAA | 1947 |
| 28 | CGCTATCTTAGTCGAGCACAATGTCTTCGTATAGG | 1948 |
| 29 | ATTAGTACGGCACGAACCGGCCATTCATGGCAGCT | 1949 |
| 30 | AGTACGACTATCAAGACTCCAGCGCTCTCCTTGGA | 1950 |
| 31 | ATGAGCCTCGGAGCGAACGTTATCGATCAGGCTGT | 1951 |
| 32 | TTGCGTGCAGTAGCACCGATACACAGCGCTTGTAT | 1952 |

**[Table 21-2]**

| No. | Primer sequence | SEQ ID NO: |
|---|---|---|
| 33 | AACGGCTGCATCACCTACACTATACTCAACATCTA | 1953 |
| 34 | GTCGCTATGCGAGAAGTGGCGTGGAATGCTATGGT | 1954 |
| 35 | CATGGATACCTACTGACTTGACTTCTAGAGGACCG | 1955 |
| 36 | GAGTGACGCAGACACCGTAACGTCGAATCTTCTAG | 1956 |
| 37 | AGTACCGTCTGTGTGAATATTGTTCCTACGTTACA | 1957 |
| 38 | GGCTAATCGATAGTGACGAGTTCTGCACGCCTGAA | 1958 |
| 39 | GGCGAGCGCTCGTGGTTCTGAGTCGCTGTTAGATG | 1959 |
| 40 | TATCTCCAGCGTTATAAGCTACTGGAGCCGCTCGG | 1960 |
| 41 | CCTTCTGCGCAAGTCAAGGATTCGCTTAGATGGAC | 1961 |
| 42 | GTTGCTGACAGCCGTTGCGTACTTGCCTTAAGAAC | 1962 |
| 43 | GTGGCCTAATCACTCGCGCTTCATAGGCCGATAGG | 1963 |
| 44 | TGCATCTAGCCTACATCGGACCTTGTTATGGTAAT | 1964 |
| 45 | GGACAGCTACTGGACACCACCGAACTGGTAGTGTC | 1965 |
| 46 | AACTGGCGATGGACGGCCGCTCTTCCGCTACATAG | 1966 |
| 47 | GGAGCAGTTAGCTATGGAGCAGGCCGATAACCTGA | 1967 |
| 48 | ACTCTACGGTGCACCTCAGCCTTCATGCAATAGGC | 1968 |
| 49 | CTTGTAGCACAATACATTACTCTCCACGTGATAGC | 1969 |
| 50 | GGACGCTATCGATACCGTTATTCCTACTCTGTCGG | 1970 |
| 51 | GGATGATCGTCAACGATCAACTGACAGTTAGTCGA | 1971 |
| 52 | TGACAGTAGCAATGTCTCACGTCTGCACAACGGAA | 1972 |
| 53 | GTCGCAGGACCTCACGGATAGTAGTGCGAGGTCTA | 1973 |
| 54 | ATATCGGCGGACGCAATGACAGTTGTTGGCTGATG | 1974 |
| 55 | AAGCACCAAGGAGGTATGTTCCATCGAGGCGCTCG | 1975 |
| 56 | GACCGCACCTTATAGCTATATCCTGGTCTAGTACT | 1976 |
| 57 | TCTCAGAGGAAGGTTGAGCGTCTGACCAGGTTGGC | 1977 |
| 58 | TGGACCTAGAGACCTAGCTCGTCTCTTCGCGATCG | 1978 |
| 59 | CGGAGTGGTTCCACGCGACCTCGCAACTAATCCTT | 1979 |
| 60 | GGAGCCGCGCGCAGACTGACCTTGCTTGATCTACT | 1980 |
| 61 | ACTCTAAGTATATGCGCAGTTAGTATACTGAACCA | 1981 |
| 62 | GAGCATTGCTTCGCTTCGATGTCTATTCTGATCAG | 1982 |
| 63 | GCTTGTATTGCCACTCGAGTAGGTCGTGGCAGTAG | 1983 |
| 64 | ATCTGGACATTGCATTCGGTGTGTATACAGAAGGC | 1984 |

**[Table 21-3]**

| No. | Primer sequence | SEQ ID NO: |
|---|---|---|
| 65 | GGTTGCGATCAGCTTGATAGCAGGTCATATCCTCA | 1985 |
| 66 | GCAGGTACTAACCTGAGATGCGTAGCTAACACAGG | 1986 |
| 67 | ATCTGCAAGGACGTAACGTCCTCGGAAGGTGAGGT | 1987 |
| 68 | ATAATCTTACGAGCCTCCAGTGAATAATGCAAGCA | 1988 |
| 69 | CAATCTCCGCACAGTCTTGTTCAGGTACAGACTTA | 1989 |
| 70 | ATGTGCGCAATTCAGCGTAAGTGCCTATTCATAAT | 1990 |
| 71 | TCGGACGCACACATCCTGTTGTCGAGAAGAGGAAG | 1991 |
| 72 | TCGGAAGCATCACATGAGCATCAGGAGTTCATTGC | 1992 |
| 73 | ATCTGGTTGTGGACTTCTATACAGTACCAGAGTGG | 1993 |
| 74 | CGTCTGAATATAGTTAGCTAGTAGTGTAATCCAGG | 1994 |
| 75 | TAATATCTGATCCGACCTATTATCTAGGACTACTC | 1995 |
| 76 | TATGCGGCCGTCCGTACCTCGTCTGCTTCAGTTGG | 1996 |
| 77 | TGGCTCAAGTTCCATATTGCCAAGACGACCTGGAG | 1997 |
| 78 | GCAGTTCTGCTAGGCGGTCCGAGGCAATTGAAGAG | 1998 |
| 79 | CATGGCACAGACGAAGTATGCACCACGCTCATTAA | 1999 |
| 80 | GGAGCGTACTACGACCATTCAACCGAATATGTTAC | 2000 |
| 81 | GCGTAGATCTCGCGACAGAGACAAGGTGCGAATGG | 2001 |
| 82 | TGGACTGAGGTTCTCCGGTCTATACTCCTGTAGGA | 2002 |
| 83 | TGGCTATAGCAACGGCTTCTTGTGATCGCATTGCA | 2003 |
| 84 | GGCGAAGAATCATGCGAGACGGAGTAGACGGACGT | 2004 |
| 85 | GAGCATTGCGAGTTGCACACGTGATATCAGACTGT | 2005 |
| 86 | CTGTTGACCTATGCCAGAATCAATACCTCAGATTA | 2006 |
| 87 | GTTAACAAGTAGATGCCAAGATACAACGAGAGACC | 2007 |
| 88 | GAGCAAGATTATAGTTAGGAAGATAGTTAACTCGC | 2008 |
| 89 | TCCGGAGTCGAGCATATGTGACCAACTCTCAACGC | 2009 |
| 90 | GGAGCTGCGATGCCGTTACCGACGTCATCTTCAAG | 2010 |
| 91 | GCTCTATCTTACACATTGGCGTACTGGACTCGCGA | 2011 |
| 92 | TTCTACATATTCATCGCCTACCGAGTTGCGCGAAG | 2012 |
| 93 | TGGACGTCTGACCTGTGTCTACATCGGTGGTGCTA | 2013 |
| 94 | GGCAGGACAGCTCCGTGTTCTACTCGAACCGCACT | 2014 |
| 95 | TGACAACCTCATGTCTCCGACCGCAGGCATACAAT | 2015 |
| 96 | GCAGGCCTAACAAGTGGTCACGAGGAGTCCTTATT | 2016 |

### 3.1.2 Standard PCR

To the genomic DNA described in 2. above (30 ng, NiF8-derived genomic DNA), random primers (final concentration: 0.6 µM; 10-nucleotide primer A), a 0.2mM dNTP mixture, 1.0 mM MgCl₂, and 1.25 units of DNA polymerase (PrimeSTAR, TAKARA) were added, and a reaction solution was prepared while adjusting the final reaction level to 50 µl. PCR was carried out under thermal cycle conditions comprising 98°C for 2 minutes and 30 cycles of 98°C for 10 seconds, 50°C for 15 seconds, and 72°C for 20 seconds, followed by storage at 4°C. In this example, numerous nucleic acid fragments obtained via PCR using random primers, including the standard PCR described above, are referred to as a DNA library.

### 3.1.3 Purification of DNA library and electrophoresis

The DNA library obtained in 3.1.2 above was purified with the use of the MinElute PCR Purification Kit (QIAGEN) and subjected to electrophoresis with the use of the Agilent 2100 bioanalyzer (Agilent Technologies) to obtain a fluorescence unit (FU).

### 3.1.4 Examination of annealing temperature

To the genomic DNA described in 2. above (30 ng, NiF8-derived genomic DNA), random primers (final concentration: 0.6 µM, 10-nucleotide primer A), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 µl. PCR was carried out under thermal cycle conditions comprising 98°C for 2 minutes and 30 cycles of 98°C for 10 seconds, different annealing temperatures for 15 seconds, and 72°C for 20 seconds, followed by storage at 4°C. In this Example, 37°C, 40°C, and 45°C were examined as annealing temperatures. The DNA library obtained in this experiment was subjected to purification and electrophoresis in the same manner as in 3.1.3.

### 3.1.5 Examination of enzyme amount

To the genomic DNA described in 2. above (30 ng, NiF8-derived genomic DNA), random primers (final concentration: 0.6 µM, 10-nucleotide primer A), a 0.2 mM dNTP mixture, 1.0 mM MgCl₂, and 2.5 units or 12.5 units of DNA polymerase (PrimeSTAR, TAKARA) were added, and a reaction solution was prepared while adjusting the final reaction level to 50 µl. PCR was carried out under thermal cycle conditions comprising 98°C for 2 minutes and 30 cycles of 98°C for 10 seconds, 50°C for 15 seconds, and 72°C for 20 seconds, followed by storage at 4°C. The DNA library obtained in this experiment was subjected to purification and electrophoresis in the same manner as in 3.1.3.

### 3.1.6 Examination of MgCl₂ concentration

To the genomic DNA described in 2. above (30 ng, NiF8-derived genomic DNA), random primers (final concentration: 0.6 µM, 10-nucleotide primer A), a 0.2 mM dNTP mixture, MgCl₂ at a given concentration, and 1.25 units of DNA polymerase (PrimeSTAR, TAKARA) were added, and a reaction solution was prepared while adjusting the final reaction level to 50 µl. PCR was carried out under thermal cycle conditions comprising 98°C for 2 minutes and 30 cycles of 98°C for 10 seconds, 50°C for 15 seconds, and 72°C for 20 seconds, followed by storage at 4°C. In this Example, two-, three- and four-fold concentrations of a usual concentration were examined as MgCl₂ concentrations. The DNA library obtained in this experiment was subjected to purification and electrophoresis in the same manner as in 3.1.3.

### 3.1.7 Examination of nucleotide length of random primer

To the genomic DNA described in 2. above (30 ng, NiF8-derived genomic DNA), random primers (final concentration: 0.6 µM), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 µl. PCR was carried out under thermal cycle conditions comprising 98°C for 2 minutes and 30 cycles of 98°C for 10 seconds, 50°C for 15 seconds, and 72°C for 20 seconds, followed by storage at 4°C. In this Example, primers having 8 nucleotides (Table 7), 9 nucleotides (Table 8), 11 nucleotides (Table 9), 12 nucleotides (Table 10), 14 nucleotides (Table 11), 16 nucleotides (Table 12), 18 nucleotides (Table 13), and 20 nucleotides (Table 14) were examined as random primers. The DNA library obtained in this experiment was subjected to purification and electrophoresis in the same manner as in 3.1.3.

### 3.1.8 Examination of random primer concentration

To the genomic DNA described in 2. above (30 ng, NiF8-derived genomic DNA), random primers at a given concentration (10-nucleotide primer A), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 µl. PCR was carried out under thermal cycle conditions comprising 98°C for 2 minutes and 30 cycles of 98°C for 10 seconds, 50°C for 15 seconds, and 72°C for 20 seconds, followed by storage at 4°C. In this Example, 2, 4, 6, 8, 10, 20, 40, 60, 100, 200, 300, 400, 500, 600. 700, 800, 900, and 1000 µM were examined as random concentrations. The DNA library obtained in this experiment was subjected to purification and electrophoresis in the same manner as in 3.1.3. Also, in this experiment, the reproducibility of the repeated data was evaluated on the basis of the Spearman's rank correlation (p > 0.9).

### 3.2 Verification of reproducibility via MiSeq

### 3.2.1 Preparation of DNA library

To the genomic DNA described in 2. above (30 ng, NiF8-derived genomic DNA), random primers (final concentration: 60 µM, 10-nucleotide primer A), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 µl. PCR was carried out under thermal cycle conditions comprising 98°C for 2 minutes and 30 cycles of 98°C for 10 seconds, 50°C for 15 seconds, and 72°C for 20 seconds, followed by storage at 4°C. The DNA library obtained in this experiment was subjected to purification and electrophoresis in the same manner as in 3.1.3.

### 3.2.2 Preparation of sequence library

From the DNA library obtained in 3.2.1, a sequence library for MiSeq analysis was prepared using the KAPA Library Preparation Kit (Roche).

### 3.2.3 MiSeq analysis

With the use of the MiSeq Reagent Kit V2 500 Cycle (Illumina), the sequence library for MiSeq analysis obtained in 3.2.2 was analyzed via 100 base paired-end sequencing.

### 3.2.4 Read data analysis

Random primer sequence information was deleted from the read data obtained in 3.2.3, and the read patterns were identified. The number of reads was counted for each read pattern, the number of reads of the repeated analyses, and the reproducibility was evaluated using the correlational coefficient.

### 3.3 Analysis of rice variety Nipponbare

### 3.3.1 Preparation of DNA library

To the genomic DNA described in 2. above (30 ng, Nipponbare-derived genomic DNA), random primers (final concentration: 60 µM, 10-nucleotide primer A), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 µl. PCR was carried out under thermal cycle conditions comprising 98°C for 2 minutes and 30 cycles of 98°C for 10 seconds, 50°C for 15 seconds, and 72°C for 20 seconds, followed by storage at 4°C. The DNA library obtained in this experiment was subjected to purification and electrophoresis in the same manner as in 3.1.3.

### 3.3.2 Preparation of sequence library, MiSeq analysis, and read data analysis

Preparation of a sequence library using the DNA library prepared from Nipponbare-derived genomic DNA, MiSeq analysis, and analysis of the read data were performed in accordance with the methods described in 3.2.2, 3.2.3, and 3.2.4, respectively.

### 3.3.3 Evaluation of genomic homogeneity

The read patterns obtained in 3.3.2 were mapped to the genomic information of Nipponbare (NC_008394 to NC_008405) using bowtie2, and the genomic positions of the read patterns were identified.

### 3.3.4 Non-specific amplification

On the basis of the positional information of the read patterns identified in 3.3.3, the sequences of random primers were compared with the genome sequences to which such random primers would anneal, and the number of mismatches was determined.

### 3.4 Detection of polymorphism and identification of genotype

### 3.4.1 Preparation of DNA library

To the genomic DNA described in 2. above (30 ng, NiF8-derived genomic DNA, Ni9-derived genomic DNA, hybrid progeny-derived genomic DNA, or Nipponbare-derived genomic DNA), random primers (final concentration: 60 µM, 10-nucleotide primer A), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 µl. PCR was carried out under thermal cycle conditions comprising 98°C for 2 minutes and 30 cycles of 98°C for 10 seconds, 50°C for 15 seconds, and 72°C for 20 seconds, followed by storage at 4°C. The DNA library obtained in this experiment was subjected to purification and electrophoresis in the same manner as in 3.1.3.

### 3.4.2 HiSeq analysis

Analysis of the DNA libraries prepared in 3.4.1 was consigned to TakaraBio under conditions in which the number of samples was 16 per lane via 100 base paired-end sequencing, and the read data were obtained.

### 3.4.3 Read data analysis

Random primer sequence information was deleted from the read data obtained in 3.4.2, and the read patterns were identified. The number of reads was counted for each read pattern.

### 3.4.4 Detection of polymorphism and identification of genotype

On the basis of the read patterns and the number of reads obtained as a results of analysis conducted in 3.4.3, polymorphisms peculiar to NiF8 and Ni9 were detected, and the read patterns thereof were designated as markers. On the basis of the number of reads, the genotypes of the 22 hybrid progeny lines were identified. The accuracy for genotype identification was evaluated on the basis of the reproducibility attained by the repeated data concerning the 22 hybrid progeny lines.

### 3.5 Experiment for confirmation with PCR marker

### 3.5.1 Primer designing

Primers were designed for a total of 6 markers (i.e., 3 NiF8 markers and 3 Ni9 markers) among the markers identified in 3.4.4 based on the marker sequence information obtained via paired-end sequencing (Table 22).

### [Table 22]

**Table 22. Marker sequence information and PCR marker primer information**

| Genotype | Marker name | Marker sequence (1)* | Marker sequence (2)* | Primer (1) | Primer (2) |
|---|---|---|---|---|---|
| NiF8 type | N80521152 | CCCATACACACACCATGAAGCTTGAACTA ATTAACATTCTCAAACTAATTAACAAGCAT GCAAGCATGTTTTTACACAATGACAATATA T (SEQ ID NO:2017) | ATGGGTGAGGGCGCAGAGGCAAAGACAT GGAGGTCCGGAAGGGTAGAAGCTCACAT CAAGTCGAGTATGTTGAATGCAATCCCATA TATA (SEQ ID NO:2018) | CCCATACACACAC CATGAAGCTTG (SEQ ID NO:2019) | GGTAGAAGCTCAC ATCAAGTCGAG (SEQ ID NO:2020) |
| | N80997192 | AATCACAGAACGAGGTCTGGACGAGAAC AGAGCTGGACATCTACACGCACCGCATG GTAGTAGAGCATGTACTGCAAAAGCTTGA AGCGC (SEQ ID NO:2021) | GATGCTGAGGGCGAAGTTGTCAGCCAAG TCCTCAATGTCATAGGCGAGATCGCAGTA GTTCTGTAACCATTCCCTGCTAAACTGGT CCAT (SEQ ID NO:2022) | ACGAGAACAGAGC TGGACATCTAC (SEQ ID NO:2023) | TCAATGTCATAGGC GAGATCGCAG (SEQ ID NO:2024) |
| | N80533142 | AGACCAACAAGCAGCAAGTAGTCAGAGA AGTACAAGAGAAGGAGAGCAAGAAGGAT AGTAAGTTGCAAGCTTACCGTTACAAAGA TGATA (SEQ ID NO:2025) | GGAGGAGCACAACTAGGCGTTTATCAAGA TGGGTCATCGAGCTCTTGGTGTCTTCAAC CTTCTTGACATCAACTTCTCCAATCTTCGT CT (SEQ ID NO:2026) | GGAGAGCAAGAAG GATAGTAAGTTGC (SEQ ID NO:2027) | CGAGCTCTTGGTG TCTTCAACCTTC (SEQ ID NO:2028) |
| Ni9 type | N91552391 | TGGGGTAGTCCTGAAGCTCTAGGTATGCC TCTTCATCTCCCTGCACCTCTGGTGCTAG CACCTCCTGCTCTTCGGGCACCTCTACC GGGG (SEQ ID NO:2029) | GGATACTGATGTAGCTTTCACCCGGGAGT ATTCCAAGGTATCGATTTTCCACGGGGAA CGCGAAGTGCACTAGTTGAGGTTTAGATT GCC (SEQ ID NO:2030) | GAAGCTCTAGGTA TGCCTCTTCATC (SEQ ID NO:2031) | GTGCACTAGTTGA GGTTTAGATTGC (SEQ ID NO:2032) |
| | N91653962 | TCGGGAAAACGAACGGGCGAACTACAGA TGTCAGTACGAAGTAGTCTATGGCAGGAA ATACGTAGTCCATACGTGGTGCCAGCCCA AGCC (SEQ ID NO:2033) | AGCAGGAGGGAGAAAGGAAACGTGGCAT TCATCGGCTGTCTGCCATTGCCATGTGAG ACAAGGAAATCTACTTCACCCCCATCTATC GAG (SEQ ID NO:2034) | GGGCGAACTACAG ATGTCAGTACG (SEQ ID NO:2035) | CTGTCTGCCATTG CCATGTGAGAC (SEQ ID NO:2036) |
| | N91124801 | AGACATAAGATTAACTATGAACAAATTCAC GGGTCCGATTCCTTTGGGATTTGCAGCTT GCAAGAACCTTCAAATACTCATTATATCTT C (SEQ ID NO:2037) | TTAAGTTGCAGAATTTGATACGAAGAACTT GAAGCATGGTGAGGTTGCCGAGCTCATT GGGGATGGTTCCAGAAAGGCTATTGTAG CTTA (SEQ ID NO:2038) | GAACAAATTCACG GGTCCGATTCC (SEQ ID NO:2039) | CGAAGAACTTGAA GCATGGTGAGG (SEQ ID NO:2040) |

| | | | | | |
|---|---|---|---|---|---|
| *: Marker sequence: Paired-end sequence | | | | | |

### 3.5.2 PCR and electrophoresis

With the use of the TaKaRa Multiplex PCR Assay Kit Ver.2 (TAKARA) and the genomic DNA described in 2. above (15 ng, NiF8-derived genomic DNA, Ni9-derived genomic DNA, or hybrid progeny-derived genomic DNA) as a template, 1.25 µl of Multiplex PCR enzyme mix, 12.5 µl of 2x Multiplex PCR buffer, and the 0.4 µM primer designed in 3.5.1 were added, and a reaction solution was prepared while adjusting the final reaction level to 25 µl. PCR was carried out under thermal cycle conditions comprising 94°C for 1 minute, 30 cycles of 94°C for 30 seconds, 60°C for 30 seconds, and 72°C for 30 seconds, and retention at 72°C for 10 minutes, followed by storage at 4°C. The amplified DNA fragment was subjected to electrophoresis with the use of TapeStation (Agilent Technologies).

### 3.5.3 Comparison of genotype data

On the basis of the results of electrophoresis obtained in 3.5.2, the genotype of the marker was identified on the basis of the presence or absence of a band, and the results were compared with the number of reads of the marker.

### 3.6 Correlation between random primer density and length

### 3.6.1 Influence of random primer length at high concentration

To the genomic DNA described in 2. above (30 ng, NiF8-derived genomic DNA), random primers having given lengths (final concentration: 10 µM), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 µl. In this experiment, 9 nucleotides (Table 8), 10 nucleotides (Table 1, 10-nucleotide primer A), 11 nucleotides (Table 9), 12 nucleotides (Table 10), 14 nucleotides (Table 11), 16 nucleotides (Table 12), 18 nucleotides (Table 13), and 20 nucleotides (Table 14) were examined as random primer lengths. PCR was carried out under thermal cycling conditions comprising 98°C for 2 minutes and 30 cycles of 98°C for 10 seconds, 50°C for 15 seconds, and 72°C for 20 seconds, followed by storage at 4°C. In the reaction system using random primers each comprising 10 or more nucleotides, PCR was carried out under thermal cycle conditions comprising 98°C for 2 minutes and 30 cycles of 98°C for 10 seconds, 50°C for 15 seconds, and 72°C for 20 seconds, followed by storage at 4°C. The DNA library obtained in this experiment was subjected to purification and electrophoresis in the same manner as in 3.1.3.

### 3.6.2 Correlation between random primer density and length

To the genomic DNA described in 2. above (30 ng, NiF8-derived genomic DNA), random primers of a given length were added to a given concentration therein, a 0.2 mM dNTP mixture, 1.0 mM MgCl₂, and 1.25 units of DNA polymerase (PrimeSTAR, TAKARA) were added thereto, and a reaction solution was prepared while adjusting the final reaction level to 50 µl. In this experiment, random primers comprising 8 to 35 nucleotides shown in Tables 1 to 21 were examined, and the random primer concentration from 0.6 to 300 µM was examined.

In the reaction system using random primers comprising 8 nucleotides and 9 nucleotides, PCR was carried out under thermal cycle conditions comprising 98°C for 2 minutes and 30 cycles of 98°C for 10 seconds, 37°C for 15 seconds, and 72°C for 20 seconds, followed by storage at 4°C. In the reaction system using a random primer of 10 or more nucleotides, PCR was carried out under thermal cycle conditions comprising 98°C for 2 minutes and 30 cycles of 98°C for 10 seconds, 50°C for 15 seconds, and 72°C for 20 seconds, followed by storage at 4°C. The DNA library obtained in this experiment was subjected to purification and electrophoresis in the same manner as in 3.1.3. Also, the reproducibility of the repeated data was evaluated on the basis of the Spearman's rank correlation (p > 0.9).

### 3.7 Number of random primers

To the genomic DNA described in 2. above (30 ng, NiF8-derived genomic DNA), 1, 2, 3, 12, 24,or 48 types of random primers selected from the 96 types of random primers comprising 10 nucleotides (10-nucleotide primer A) shown in Table 1 were added to the final concentration of 60 µM therein, a 0.2 mM dNTP mixture, 1.0 mM MgCl₂, and 1.25 units of DNA polymerase (PrimeSTAR, TAKARA) were added thereto, and a reaction solution was prepared while adjusting the final reaction level to 50 µl. In this experiment, as the 1, 2, 3, 12, 24, or 48 types of random primers, random primers were selected successively from No. 1 shown in Table 1, and the selected primers were then examined. PCR was carried out under thermal cycle conditions comprising 98°C for 2 minutes and 30 cycles of 98°C for 10 seconds, 50°C for 15 seconds, and 72°C for 20 seconds, followed by storage at 4°C. The DNA library obtained in this experiment was subjected to purification and electrophoresis in the same manner as in 3.1.3. Also, the reproducibility of the repeated data was evaluated on the basis of the Spearman's rank correlation (p > 0.9).

### 3.8 Random primer sequence

To the genomic DNA described in 2. above (30 ng, NiF8-derived genomic DNA), a set of primers selected from the 5 sets of random primers shown in Tables 2 to 6 was added to the final concentration of 60 µM therein, a 0.2 mM dNTP mixture, 1.0 mM MgCl₂, and 1.25 units of DNA polymerase (PrimeSTAR, TAKARA) were added thereto, and a reaction solution was prepared while adjusting the final reaction level to 50 µl. PCR was carried out under thermal cycle conditions comprising 98°C for 2 minutes and 30 cycles of 98°C for 10 seconds, 50°C for 15 seconds, and 72°C for 20 seconds, followed by storage at 4°C. The DNA library obtained in this experiment was subjected to purification and electrophoresis in the same manner as in 3.1.3. Also, the reproducibility of the repeated data was evaluated on the basis of the Spearman's rank correlation (p > 0.9).

### 3.9 DNA library using human-derived genomic DNA

To the genomic DNA described in 2. above (30 ng, human-derived genomic DNA), random primers (final concentration: 60 µM, 10-nucleotide primer A), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 µl. PCR was carried out under thermal cycle conditions comprising 98°C for 2 minutes and 30 cycles of 98°C for 10 seconds, 50°C for 15 seconds, and 72°C for 20 seconds, followed by storage at 4°C. The DNA library obtained in this experiment was subjected to purification and electrophoresis in the same manner as in 3.1.3. Also, the reproducibility of the repeated data was evaluated on the basis of the Spearman's rank correlation (p > 0.9).

### 4. Results and examination

### 4.1 Correlation between PCR conditions and DNA library size

When PCR was conducted with the use of random primers in accordance with conventional PCR conditions (3.1.2 described above), the amplified DNA library size was as large as 2 kbp or more, but amplification of the DNA library of a target size (i.e., 100-bp to 500-bp) was not observed (Fig. 2). A DNA library of 100 bp to 500 bp could not be obtained because it was highly unlikely that a random primer would function as a primer in a region of 500 bp or smaller. In order to prepare a DNA library of the target size (i.e., 100 bp to 500 bp), it was considered necessary to induce non-specific amplification with high reproducibility.

The correlation between the annealing temperature (3.1.4 above), the enzyme amount (3.1.5 above), the MgCl₂ concentration (3.1.6 above), the primer length (3.1.7 above), and the primer concentration (3.18 above), which are considered to affect PCR specificity, and the DNA library size were examined.

Fig. 3 shows the results of the experiment described in 3.1.4 attained at an annealing temperature of 45°C, Fig. 4 shows the results attained at an annealing temperature of 40°C, and Fig. 5 shows the results attained at an annealing temperature of 37°C. By reducing the annealing temperature from 45°C, 40°C, to 37°C, as shown in Figs. 3 to 5, the amounts of high-molecular-weight DNA library amplified increased, although amplification of low-molecular-weight DNA library was not observed.

Fig. 6 shows the results of the experiment described in 3.1.5 attained when the enzyme amount is increased by 2 times, and Fig. 7 shows the results attained when the enzyme amount is increased by 10 times the original amount. By increasing the enzyme amount by 2 times or 10 times a common amount, as shown in Figs. 6 and 7, the amounts of high-molecular-weight DNA library amplified increased, although amplification of low-molecular-weight DNA library was not observed.

Fig. 8 shows the results of the experiment described in 3.1.6 attained when the MgCl₂ concentration is increased by 2 times a common amount, Fig. 9 shows the results attained when the MgCl₂ concentration is increased by 3 times, and Fig. 10 shows the results attained when the MgCl₂ concentration is increased by 4 times. By increasing the MgCl₂ concentration by 2 times, 3 times, and 4 times the common amount, as shown in Figs. 8 to 10, the amounts of high-molecular-weight DNA library amplified varied, although amplification of a low-molecular-weight DNA library was not observed.

Figs. 11 to 18 show the results of the experiment described in 3.1.7 attained at the random primer lengths of 8 nucleotides, 9 nucleotides, 11 nucleotides, 12 nucleotides, 14 nucleotides, 16 nucleotides, 18 nucleotides, and 20nucleotides, respectively. Regardless of the length of a random primer, as shown in Figs. 11 to 18, no significant change was observed in comparison with the results shown in Fig. 2 (a random primer comprising 10 nucleotides).

The results of experiment described in 3.1.8 are summarized in Table 23.

**[Table 23]**

| Concentration (µM) | Repeat | Figure | Correlation coefficient (ρ) |
|---|---|---|---|
| 2 | - | Fig. 19 | - |
| 4 | - | Fig. 20 | - |
| 6 | 1st | Fig. 21 | 0.889 |
| | 2nd | Fig. 22 | |
| 8 | 1st | Fig. 23 | 0.961 |
| | 2nd | Fig. 24 | |
| 10 | 1st | Fig. 25 | 0.979 |
| | 2nd | Fig. 26 | |
| 20 | 1st | Fig. 27 | 0.950 |
| | 2nd | Fig. 28 | |
| 40 | 1st | Fig. 29 | 0.975 |
| | 2nd | Fig. 30 | |
| 60 | 1st | Fig. 31 | 0.959 |
| | 2nd | Fig. 32 | |
| 100 | 1st | Fig. 33 | 0.983 |
| | 2nd | Fig. 34 | |
| 200 | 1st | Fig. 35 | 0.991 |
| | 2nd | Fig. 36 | |
| 300 | 1st | Fig. 37 | 0.995 |
| | 2nd | Fig. 38 | |
| 400 | 1st | Fig. 39 | 0.988 |
| | 2nd | Fig. 40 | |
| 500 | 1st | Fig. 41 | 0.971 |
| | 2nd | Fig. 42 | |
| 600 | - | Fig. 43 | - |
| 700 | - | Fig. 44 | - |
| 800 | - | Fig. 45 | - |
| 900 | - | Fig. 46 | - |
| 1000 | - | Fig. 47 | - |

With the use of random primers comprising 10 nucleotides, as shown in Figs. 19 to 47, amplification was observed in a 1-kbp DNA fragment at the random primer concentration of 6 µM. As the concentration increased, the molecular weight of a DNA fragment decreased. Reproducibility at the random primer concentration of 6 to 500 µM was examined. As a result, a relatively low p value of 0.889 was attained at the concentration of 6 µM, which is 10 times higher than the usual level. At the concentration of 8 µM, which is equivalent to 13.3 times higher than the usual level, and at 500 µM, which is 833.3 times higher than the usual level, a high p value of 0.9 or more was attained. The results demonstrate that a DNA fragment of 1 kbp or smaller can be amplified while achieving high reproducibility by elevating the random primer concentration to a level significantly higher than the concentration employed under general PCR conditions. When the random primer concentration is excessively higher than 500 µM, amplification of a DNA fragment of a desired size cannot be observed. In order to amplify a low-molecular-weight DNA fragment with excellent reproducibility, accordingly, it was found that the random primer concentration should fall within an optimal range, which is higher than the concentration employed in a general PCR procedure and equivalent to or lower than a given level.

### 4.2 Confirmation of reproducibility via MiSeq

In order to confirm the reproducibility for DNA library production, as described in 3.2 above, the DNA library amplified with the use of the genomic DNA extracted from NiF8 as a template and random primers was analyzed with the use of a next-generation sequencer (MiSeq), and the results are shown in Fig. 48. As a result of 3.2.4 above, 47,484 read patterns were obtained. As a result of comparison of the number of reads obtained through repeated measurements, a high correlation (i.e., a correlational coefficient "r" of 0.991) was obtained, as with the results of electrophoresis. Accordingly, it was considered that a DNA library could be produced with satisfactory reproducibility with the use of random primers.

### 4.3 Analysis of rice variety Nipponbare

As described in 3.3 above, a DNA library was prepared with the use of genomic DNA extracted from the rice variety Nipponbare, the genomic information of which has been disclosed, as a template, and random primers and subjected to electrophoresis, and the results are shown in Figs. 49 and 50. On the basis of the results shown in Figs. 49 and 50, the p value was found to be as high as 0.979. Also, Fig. 51 shows the results of analysis of the read data with the use of MiSeq. On the basis of the results shown in Fig. 51, the correlational coefficient "r" was found to be as high as 0.992. These results demonstrate that a DNA library of rice could be produced with very high reproducibility with the use of random primers.

As described in 3.3.3, the obtained read pattern was mapped to the genomic information of Nipponbare. As a result, DNA fragments were found to be evenly amplified throughout the genome at intervals of 6.2 kbp (Fig. 52). As a result of comparison of the sequence and genome information of random primers, 3.6 mismatches were found on average, and one or more mismatches were observed in 99.0% of primer pairs (Fig. 53). The results demonstrate that a DNA library involving the use of random primers is produced with satisfactory reproducibility via non-specific amplification evenly throughout the genome.

### 4.4 Detection of polymorphism and genotype identification of sugarcane

As described in 3.4, DNA libraries of the sugarcane varieties NiF8 and Ni9 and 22 hybrid progeny lines were produced with the use of random primers, the resulting DNA libraries were analyzed with the next-generation sequencer (HiSeq), the polymorphisms of the parent varieties were detected, and the genotypes of the hybrid progenies were identified on the basis of the read data. Table 24 shows the results.

### [Table 24]

**Table 24. Number of markers and genotyping accuracy of sugarcane varieties NiF8 and Ni9**

| | Number of markers | F1_01 | | F1_02 | | Total | |
|---|---|---|---|---|---|---|---|
| | | Consistency | Reproducibility | Consistency | Reproducibility | Consistency | Reproducibility |
| NiF8 type | 8,683 | 8,680 | 99.97% | 8,682 | 99.99% | 17,362 | 99.98% |
| Ni9 type | 11,655 | 11,650 | 99.96% | 11,651 | 99.97% | 23,301 | 99.96% |
| Total | 20,338 | 20,330 | 99.96% | 20,333 | 99.98% | 40,663 | 99.97% |

As shown in Table 24, 8,683 markers for NiF8 and 11,655 markers for Ni9; that is, a total of 20,338 markers, were produced. In addition, reproducibility for genotype identification of hybrid progeny lines was as high as 99.97%. This indicates that the accuracy for genotype identification is very high. In particular, sugarcane is polyploid (8x+n), the number of chromosomes is as large as 100 to 130, and the genome size is as large as 10 Gbp, which is at least 3 times greater than that of humans. Accordingly, it is very difficult to identify the genotype throughout the genomic DNA. As described above, numerous markers can be produced with the use of random primers, and the sugarcane genotype can thus be identified with high accuracy.

### 4.5 Experiment for confirmation with PCR marker

As described in 3.5 above, the sugarcane varieties NiF8 and Ni9 and 22 hybrid progeny lines were subjected to PCR with the use of the primers shown in Table 22, genotypes were identified via electrophoresis, and the results were compared with the number of reads. Figs. 54 and55 show the number of reads and the electrophoretic pattern of the NiF8 marker N80521152, respectively. Figs. 56 and 57 show the number of reads and the electrophoretic pattern of the NiF8 marker N80997192, respectively. Figs. 58 and 59 show the number of reads and the electrophoretic pattern of the NiF8 marker N80533142, respectively. Figs. 60 and 61 show the number of reads and the electrophoretic pattern of the Ni9 marker N91552391, respectively. Figs. 62 and 63 show the number of reads and the electrophoretic pattern of the Ni9 marker N91653962, respectively. Figs. 64 and 65 show the number of reads and the electrophoretic pattern of the Ni9 marker N91124801, respectively.

As shown in Figs. 54 to 65, the results for all the PCR markers designed in 3.5 above were consistent with the results of analysis with the use of a next-generation sequencer. It was thus considered that genotype identification with the use of a next-generation sequencer would be applicable as a marker technique.

### 4.6 Correlation between random primer density and length

As described in 3.6.1, the results of DNA library production with the use of random primers comprising 9 nucleotides (Table 8), 10 nucleotides (Table 1, 10-nucleotide primer A), 11 nucleotides (Table 9), 12 nucleotides (Table 10), 14 nucleotides (Table 11), 16 nucleotides (Table 12), 18 nucleotides (Table 13), and 20 nucleotides (Table 14) are shown in Figs. 66 to 81. The results are summarized in Table 25.

**[Table 25]**

| Random primer length | Repeat | Figure | Correlation coefficient (ρ) |
|---|---|---|---|
| 9 | 1st | Fig. 66 | 0.981 |
| | 2nd | Fig. 67 | |
| 10 | 1st | Fig. 68 | 0.979 |
| | 2nd | Fig. 69 | |
| 11 | 1st | Fig. 70 | 0.914 |
| | 2nd | Fig. 71 | |
| 12 | 1st | Fig. 72 | 0.957 |
| | 2nd | Fig. 73 | |
| 14 | 1st | Fig. 74 | 0.984 |
| | 2nd | Fig. 75 | |
| 16 | 1st | Fig. 76 | 0.989 |
| | 2nd | Fig. 77 | |
| 18 | 1st | Fig. 78 | 0.995 |
| | 2nd | Fig. 79 | |
| 20 | 1st | Fig. 80 | 0.999 |
| | 2nd | Fig. 81 | |

When random primers were used at a high concentration of 10.0 µM, which is 13.3 times greater than the usual level, as shown in Figs. 66 to 81, it was found that a low-molecular-weight DNA fragment could be amplified with the use of random primers comprising 9 to 20 nucleotides while achieving very high reproducibility. As the nucleotide length of a random primer increased (12 nucleotides or more, in particular), the molecular weight of the amplified fragment was likely to be decreased. When random primers comprising 9 nucleotides were used, the amount of the DNA fragment amplified was increased by setting the annealing temperature at 37°C.

In order to elucidate the correlation between the density and the length of random primers, as described in 3.6.2 above, PCR was carried out with the use of random primers comprising 8 to 35 nucleotides at the concentration of 0.6 to 300 µM, so as to produce a DNA library. The results are shown in Table 26.

### [Table 26]

**Table 26 The correlation between the concentration and the length of random primer for DNA library**

| Primer µM | Concentration Factor relative to reference | Primer length | | | | | | | | | | | | | | | |
|---|---|---|---|---|---|---|---|---|---|---|---|---|---|---|---|---|---|
| | | 8 | 9 | 10 | 11 | 12 | 14 | 16 | 18 | 20 | 22 | 24 | 26 | 28 | 29 | 30 | 35 |
| 0.6 | Reference | × | × | × | × | × | × | × | × | × | × | × | × | × | × | × | × |
| 2 | 3.3-fold | × | × | × | × | × | × | × | × | × | × | × | × | × | × | × | × |
| 4 | 6.7-fold | × | × | × | × | × | ○ | ○ | ○ | ○ | ○ | ○ | ○ | ○ | × | × | × |
| 6 | 10.0-fold | × | × | × | × | × | ○ | ○ | ○ | ○ | ○ | ○ | ○ | ○ | ○ | ○ | × |
| 8 | 13.3-fold | × | × | × | × | ○ | ○ | ○ | ○ | ○ | ○ | ○ | ○ | ○ | ○ | × | × |
| 10 | 16.7-fold | × | × | × | × | ○ | ○ | ○ | ○ | ○ | ○ | ○ | ○ | ○ | ○ | × | × |
| 20 | 33.3-fold | × | × | × | ○ | ○ | ○ | ○ | ○ | ○ | ○ | ○ | × | × | × | × | × |
| 40 | 66.7-fold | × | ○ | ○ | ○ | ○ | ○ | ○ | ○ | ○ | × | × | × | × | × | × | × |
| 60 | 100.0-fold | × | ○ | ○ | ○ | ○ | ○ | ○ | ○ | ○ | × | × | × | × | × | × | × |
| 100 | 166.7-fold | - | × | ○ | ○ | ○ | ○ | ○ | ○ | × | - | - | - | - | - | - | - |
| 200 | 333.3-fold | - | × | ○ | ○ | × | × | × | × | × | - | - | - | - | - | - | - |
| 300 | 500.0-fold | - | × | × | × | × | × | × | × | × | - | - | - | - | - | - | - |

| | | | | | | | | | | | | | | | | | |
|---|---|---|---|---|---|---|---|---|---|---|---|---|---|---|---|---|---|
| ○: DNA library covering 100 to 500 nucleotides could be amplified assuredly with high reproducibility (p>0.9) × : DNA library did not cover 100 to 500 nucleotides, or the reproducibility was low (p<=0.9) -: Not carried out | | | | | | | | | | | | | | | | | |

As shown in Table 26, it was found that a low-molecular-weight (100 to 500 nucleotides) DNA fragment could be amplified with high reproducibility with the use of random primers comprising 9 to 30 nucleotides at 4.0 to 200 µM. In particular, it was confirmed that low-molecular-weight (100 to 500 nucleotides) DNA fragments could be amplified assuredly with high reproducibility with the use of random primers comprising 9 to 30 nucleotides at 4.0 to 100 µM.

The results shown in Table 26 are examined in greater detail. As a result, the correlation between the length and the concentration of random primers is found to be preferably within a range surrounded by a frame as shown in Fig. 82. More specifically, the random primer concentration is preferably 40 to 60 µM when the random primers comprise 9 to 10 nucleotides. It is preferable that a random primer concentration satisfy the condition represented by an inequation: y > 3E + 08x^{-6.974}, provided that the nucleotide length of the random primer is represented by y and the random primer concentration is represented by x, and 100 µM or lower, when the random primer comprises 10 to 14 nucleotides. The random primer concentration is preferably 4 to 100 mM when the random primer comprises 14 to 18 nucleotides. When a random primer comprises 18 to 28 nucleotides, the random primer concentration is preferably 4 µM or higher, and it satisfies the condition represented by an inequation: y < 8E + 08x^{-5.533}. When a random primer comprises 28 to 29 nucleotides, the random primer concentration is preferably 4 to 10 µM. The inequations y > 3E + 08x^{-6.974} and y < 8E + 08x^{-5.533} are determined on the basis of the Microsoft Excel power approximation.

By prescribing the number of nucleotides and the concentration of random primers within given ranges as described above, it was found that low-molecular-weight (100 to 500 nucleotides) DNA fragments could be amplified with high reproducibility. For example, the accuracy of the data obtained via analysis of high-molecular-weight DNA fragments with the use of a next-generation sequencer is known to deteriorate to a significant extent. As described in this Example, the number of nucleotides and the concentration of random primers may be prescribed within given ranges, so that a DNA library with a molecular size suitable for analysis with a next-generation sequencer can be produced with satisfactory reproducibility, and such DNA library can be suitable for marker analysis with the use of a next-generation sequencer.

### 4.7 Number of random primers

As described in 3.7 above, 1, 2, 3, 12, 24, or 48 types of random primers (concentration: 60µM) were used to produce a DNA library, and the results are shown in Figs. 83 to 94. The results are summarized in Table 27.

**[Table 27]**

| Number of random primers | Repeat | Figure | Correlation coefficient (ρ) |
|---|---|---|---|
| 1 | 1st | Fig. 83 | 0.984 |
| | 2nd | Fig. 84 | |
| 2 | 1st | Fig. 85 | 0.968 |
| | 2nd | Fig. 86 | |
| 3 | 1st | Fig. 87 | 0.974 |
| | 2nd | Fig. 88 | |
| 12 | 1st | Fig. 89 | 0.993 |
| | 2nd | Fig. 90 | |
| 24 | 1st | Fig. 91 | 0.986 |
| | 2nd | Fig. 92 | |
| 48 | 1st | Fig. 93 | 0.978 |
| | 2nd | Fig. 94 | |

As shown in Figs. 83 to 94, it was found that low-molecular-weight DNA fragments could be amplified with the use of any of 1, 2, 3, 12, 24, or 48 types of random primers while achieving very high reproducibility. In particular, it is understood that as the number of types of random primers increases, a peak in the electrophoretic pattern decreases, and a deviation is likely to disappear.

### 4.8 Random primer sequence

As described in 3.8 above, DNA libraries were produced with the use of sets of random primers shown in Tables 2 to 6 (i.e., 10-nucleotide primer B, 10-nucleotide primer C, 10-nucleotide primer D, 10-nucleotide primer E, and 10-nucleotide primer F), and the results are shown in Figs. 95 to 104. The results are summarized in Table 28.

**[Table 28]**

| Random primer set | Repeat | Figure | Correlation coefficient (ρ) |
|---|---|---|---|
| 10-nucleotide B | 1st | Fig. 95 | 0.916 |
| | 2nd | Fig. 96 | |
| 10-nucleotide C | 1st | Fig. 97 | 0.965 |
| | 2nd | Fig. 98 | |
| 10-nucleotide D | 1st | Fig. 99 | 0.986 |
| | 2nd | Fig. 100 | |
| 10-nucleotide E | 1st | Fig. 101 | 0.983 |
| | 2nd | Fig. 102 | |
| 10-nucleotide F | 1st | Fig. 103 | 0.988 |
| | 2nd | Fig. 104 | |

As shown in Figs. 95 to 104, it was found that low-molecular-weight DNA fragments could be amplified with the use of any sets of 10-nucleotide primer B, 10-nucleotide primer C, 10-nucleotide primer D, 10-nucleotide primer E, or 10-nucleotide primer F while achieving very high reproducibility.

### 4.9 Production of human DNA library

As described in 3.9 above, a DNA library was produced with the use of human-derived genomic DNA and random primers at a final concentration of 60 µM (10-nucleotide primer A), and the results are shown in Figs. 105 and 106. Fig. 105 shows the results of the first repeated experiment, and Fig. 106 shows the results of the second repeated experiment. As shown in Figs. 105 and 106, it was found that low-molecular-weight DNA fragments could be amplified while achieving very high reproducibility even if human-derived genomic DNA was used.

### [Example 2]

### 1. Flowchart

In this Example, first DNA fragments were prepared by PCR using genomic DNA as a template and random primers according to the schematic diagrams shown in Figs. 107 and 108. Subsequently, second DNA fragments were prepared by PCR using the first DNA fragments as templates and next-generation sequencer primers. The prepared second DNA fragments were used as a sequencer library for conducting sequence analysis using a so-called next generation sequencer. Genotype was analyzed based on the obtained read data.

### 2. Materials

In this Example, genomic DNAs were extracted from the sugarcane variety NiF8 and the rice variety Nipponbare using the DNeasy Plant Mini Kit (QIAGEN), and the extracted genomic DNAs were purified. The purified genomic DNAs were used as NiF8-derived genomic DNA and Nipponbare-derived genomic DNA, respectively.

### 3. Method

### 3.1 Examination of sugarcane variety NiF8

### 3.1.1 Designing of random primers and next-generation sequencer primers

In this Example, random primers were designed based on 3'-end 10 nucleotides of the next-generation sequencer adapter (Nextera adapter, Illumina, Inc.). Specifically, in this Example, GTTACACACG (SEQ ID NO: 2041, 10-nucleotide G) was used as a random primer. In addition, next-generation sequencer primers were designed based on the sequence information on the Nextera adapter of Illumina, Inc. in the above manner (Table 29).

**[Table 29]**

| No. | Primer sequence | SEQ ID NO: |
|---|---|---|
| 1 | AATGATACGGCGACCACCGAGATCTACA CCTCTCTATTCGTCGGCAGCGTCAGATG TGTATAAGAGACAG | 2042 |
| 2 | CAAGCAGAAGACGGCATACGAGATTAAG GCGAGTCTCGTGGGCTCGGAGATGTGT ATAAGAGACAG | 2043 |

### 3.1.2 Preparation of DNA library

A dNTP mixture at a final concentration of 0.2 mM, MgCl₂ at a final concentration of 1.0 mM, and DNA Polymerase(TAKARA, PrimeSTAR) at a final concentration of 1.25 units, and a random primer (10-nucleotide G) at a final concentration of 60 µM were added to NiF8-derived genomic DNA(30 ng) described in 2. above. A DNA library (first DNA fragments) was prepared by PCR (treatment at 98°C for 2 minutes, reaction for 30 cycles of 98°C for 10 seconds, 50°C for 15 seconds, and 72°C for 20 seconds, and storage at 4°C) in a final reaction volume of 50 µl.

### 3.1.3 Purification and electrophoresis

The DNA library obtained in 3.1.2 above was purified with the use of the MinElute PCR Purification Kit (QIAGEN) and subjected to electrophoresis with the use of the Agilent 2100 bioanalyzer(Technologies) to obtain a fluorescence unit (FU). Also, the reproducibility of the repeated data was evaluated on the basis of the Spearman's rank correlation (p > 0.9).

### 3.1.4 Preparation of next-generation sequencer DNA library

A dNTP mixture at a final concentration of 0.2 mM, MgCl₂ at a final concentration of 1.0 mM, DNA Polymerase(TAKARA, PrimeSTAR) at a final concentration of 1.25 units, and a next-generation sequencer primer at a final concentration of 0.5 µM were added to the first DNA fragment (100 ng) purified in 3.1.3 above. A next-generation sequencer DNA library (second DNA fragments) was prepared by PCR (treatment at 95°C for 2 minutes, reaction for 25 cycles of 98°C for 15 seconds, 55°C for 15 seconds, 72°C for 20 seconds, treatment at 72°C for 1 minutes, and storage at 4°C) in a final reaction volume of 50 µl. The DNA library for a next-generation sequencer was subjected to purification and electrophoresis in the same manner as in 3.1.3.

### 3.1.5 MiSeq analysis

The next-generation sequencer DNA library (a second DNA fragment) in 3.1.4 above was analyzed by MiSeq via 100 base paired-end sequencing using MiSeq Reagent Kit V2 500 Cycle (Illumina).

### 3.1.6 Read data analysis

The read patterns were identified from the read data obtained in 3.1.5. The number of reads was counted for each read pattern, the number of reads of the repeated analyses, and the reproducibility was evaluated using the correlational coefficient.

### 3.2 Examination of rice variety Nipponbare

### 3.2.1 Designing of random primers and next-generation sequencer primers

In this Example, random primers were designed based on 10 nucleotides of the 3' end of the next-generation sequencer adapter Nextera adapter of Illumina, Inc. That is, in this Example, a sequence of 10 nucleotides positioned at the 3' end of the Nextera adapter and 16 types of nucleotide sequences prepared by adding an arbitrary nucleotide sequence of 2 nucleotides to the 3' end of the sequence of 10 nucleotides to results in a full length of 12 nucleotides were designed as random primers (Table 30, 12-nucleotide B).

**[Table 30]**

| No. | Primer sequence | SEQ ID NO: |
|---|---|---|
| 1 | TAAGAGACAGAA | 2044 |
| 2 | TAAGAGACAGAT | 2045 |
| 3 | TAAGAGACAGAC | 2046 |
| 4 | TAAGAGACAGAG | 2047 |
| 5 | TAAGAGACAGTA | 2048 |
| 6 | TAAGAGACAGTT | 2049 |
| 7 | TAAGAGACAGTC | 2050 |
| 8 | TAAGAGACAGTG | 2051 |
| 9 | TAAGAGACAGCA | 2052 |
| 10 | TAAGAGACAGCT | 2053 |
| 11 | TAAGAGACAGCC | 2054 |
| 12 | TAAGAGACAGCG | 2055 |
| 13 | TAAGAGACAGGA | 2056 |
| 14 | TAAGAGACAGGT | 2057 |
| 15 | TAAGAGACAGGC | 2058 |
| 16 | TAAGAGACAGGG | 2059 |

In addition, in this Example, a next-generation sequencer primer designed based on the sequence information on the Nextera adapter of Illumina, Inc. in the same manner as in 3.1.1.

### 3.2.2 Preparation of DNA library

A dNTP mixture at a final concentration of 0.2 mM, MgCl₂ at a final concentration of 1.0 mM, and DNA Polymerase(TAKARA, PrimeSTAR) at a final concentration of 1.25 units, and a random primer (12-nucleotide B) at a concentration of 40 µM were added to Nipponbare-derived genomic DNA(30 ng) described in 2. above. A DNA library (first DNA fragments) was prepared by PCR (treatment at 98°C for 2 minutes, reaction for 30 cycles of 98°C for 10 seconds, 50°C for 15 seconds, 72°C for 20 seconds, and storage at 4°C) in a final reaction volume of 50 µl.

### 3.2.3 Purification and electrophoresis

The DNA library obtained in 3.2.2 above was purified with the use of the MinElute PCR Purification Kit (QIAGEN) and subjected to electrophoresis with the use of the Agilent 2100 bioanalyzer (Technologies) to obtain a fluorescence unit (FU). Also, the reproducibility of the repeated data was evaluated on the basis of the Spearman's rank correlation (p > 0.9).

### 3.2.4 Preparation of next-generation sequencer DNA library

A dNTP mixture at a final concentration of 0.2 mM, MgCl₂ at a final concentration of 1.0 mM, DNA Polymerase(TAKARA, PrimeSTAR) at a final concentration of 1.25 units, and a next-generation sequencer primer at a concentration of 0.5 µM were added to the first DNA fragment (100ng) purified in 3.2.3 above. A next-generation sequencer DNA library (second DNA fragments) was prepared by PCR (treatment at 95°C for 2 minutes, reaction for 25 cycles of 98°C for 15 seconds, 55°C for 15 seconds, 72°C for 20 seconds, treatment at 72°C for 1 minutes, and storage at 4°C) in a final reaction volume of 50 µl. Purification of the DNA library for next-generation sequencers and electrophoresis were conducted in the same manner as in 3.1.3.

### 3.2.5 MiSeq analysis

The next-generation sequencer DNA library (second DNA fragment) in 3.2.4 above was analyzed by MiSeq via 100 base paired-end sequencing using MiSeq Reagent Kit V2 500 Cycle (Illumina).

### 3.2.6 Read data analysis

The read patterns in 3.2.5 were mapped to the genomic information of Nipponbare (NC_008394 to NC_008405) using bowtie2, the degree of consistency between the random primer sequence and genomic DNA was confirmed. The read patterns were identified from the read data obtained in 3.2.5. The number of reads was counted for each read pattern, the number of reads of the repeated analyses, and the reproducibility was evaluated using the correlational coefficient.

### 4. Results and Examination

### 4.1 Results of examination of the sugarcane variety NiF8

Figs. 109 and 110 show the results of electrophoresis after conducting PCR using a random primer consisting of 10 nucleotides (10-nucleotide G) of the 3' end of the next-generation sequencer adapter (Nextera adapter, Illumina, Inc.) at a high concentration of 60 µl. As shown in Figs. 109 and 110, amplification was observed in a wide region ranging from 100 bp to 500 bp (the first DNA fragment). It was considered that amplification could be observed in a wide region because amplification was observed also in a region other than the genomic DNA region corresponding to the random primer. In addition, since the rank correlation coefficient among the repeated data was 0.957 (> 0.9), reproducibility was confirmed in the amplification pattern.

Next, Figures 111 and 112 shows the results of electrophoresis after conducting PCR using the next-generation sequencer primer in the manner described in 3.1.4. That is, in order to prepare a DNA library (second DNA fragments) bound to a next-generation sequencer adapter (Nextera adapter), PCR was conducted using a next-generation sequencer primer comprising the sequence of the Nextera adapter of Illumina, Inc. and the first DNA fragment as a template. Accuracy of analysis with the use of the next-generation sequencer of Illumina, Inc. is significantly reduced in a case in which the DNA library includes may short fragments having lengths of 100 bp or less or long fragments having lengths of 1 kbp or more. Since the next-generation sequencer DNA library (second DNA fragments) prepared in this Example was distributed mainly in a range of 150 bp to 1 kbp with a peak around 500 bp as illustrated in Figs. 111 and 112, the DNA library was considered to be an appropriate next-generation sequencer DNA library. In addition, since the rank correlation coefficient among the repeated data was 0.989 (> 0.9), reproducibility was confirmed in the amplification pattern.

In addition, as a result of analysis of the DNA library (second DA fragment) by next-generation sequencer MiSeq, 3.5-Gbp read data and 3.6-Gbp read data were obtained. The values indicating accuracy of MiSeq data (>= Q30) were 93.3% and 93.1%. Since the values recommended by the manufacturer were 3.0 Gbp or more for read data and 85.0% or more for >= Q30, the next-generation sequencer DNA library (second DNA fragments) prepared in this Example was considered to be applicable to next-generation sequencer analysis. In order to confirm reproducibility, the number of reads of the repeated analyses were compared for 34,613 read patterns obtained by MiSeq. Fig. 113 shows the results. As shown in Fig. 113, there was a high correlation of r = 0.996 in terms of the number of reads of the repeated analyses as with the results of electrophoresis.

As described above, a DNA library (first DNA fragments) was obtained by conducting PCR using random primer comprising 10 nucleotides at the 3' end of a next-generation sequencer adapter (Nextera Adaptor, Illumina, Inc.) at a high concentration, and then, PCR was conducted using a next-generation sequencer primer comprising the sequence of Nextera Adaptor. Accordingly, it was possible to conveniently produce a next-generation sequencer DNA library (second DNA fragments) comprising many fragments with favorable reproducibility.

### 4.2 Results of examination of rice variety Nipponbare

Figs. 114 and 115 show the results of electrophoresis after conducting PCR using 10 nucleotides positioned at the 3' end of the next-generation sequencer adopter (Nextera adaptor, Illumina, Inc.) and 16 types of random primers (12-nucleotide B) having a full length of 12 nucleotides obtained by adding an arbitrary sequence of 2 nucleotides to the sequence of 10 nucleotides at the 3' end at a high concentration of 40 µl. As shown in Figs. 114 and 115, amplification was observed in a wide region ranging from 100 bp to 500 bp(the first DNA fragment). It was considered that amplification could be observed in a wide region because amplification was observed also in a region other than the genomic DNA region corresponding to the random primer as in the case of 4.1. In addition, since the rank correlation coefficient was 0.950 (> 0.9), reproducibility was confirmed in the amplification pattern.

Next, Figures 116 and 117 shows the results of electrophoresis after conducting PCR using the next-generation sequencer primer in the manner described in 3.2.4. That is, in order to prepare a DNA library (second DNA fragments) bound to a next-generation sequencer adapter (Nextera adapter), PCR was conducted using a next-generation sequencer primer comprising the sequence of the Nextera adapter of Illumina, Inc. and the first DNA fragment as a template. As a result, since the next-generation sequencer DNA library (the second DNA fragment) prepared in this Example was distributed mainly in a range of 150 bp to 1 kbp with a peak around 300 bp as illustrated in Figs. 116 and 117, the DNA library was considered to be an appropriate next-generation sequencer DNA library. In addition, since the rank correlation coefficient among the repeated data was 0.992 (> 0.9), reproducibility was confirmed in the amplification pattern.

In addition, as a result of analysis of the obtained DNA library (second DNA fragments) by next-generation sequencer MiSeq, 4.0-Gbp read data and 3.8-Gbp read data were obtained. The values indicating accuracy of MiSeq data (>= Q30) were 94.0% and 95.3%. As in the case of 4.1.1, in view of the above results, the next-generation sequencer DNA library (second DNA fragments) prepared in this Example was considered to be applicable to next-generation sequencer analysis. Fig. 118 shows the results obtained by comparing random primer sequences and the reference sequence of rice variety Nipponbare in order to evaluate the degree of consistency between the random primer sequences of 19,849 read patterns obtained by MiSeq and the genome. As shown in Fig. 118, the average degree of consistency between the random primer sequences and the reference sequence of rice variety Nipponbare was 34.5%. In particular, since there was no identical read pattern between the random primer sequences and the reference sequence of rice variety Nipponbare, it was considered that any read pattern indicated that a random primer was bound to a sequence not corresponding to the random primer, and the resulting sequence was amplified. The above results were considered to correspond to the results obtained by the bioanalyzer. In order to confirm read pattern reproducibility, the number of reads of the repeated analyses were compared. Fig. 119 shows the results. As shown in Fig. 119, there was a high correlation of r=0.999 in terms of the number of reads of the repeated analyses as with the results of electrophoresis.

As described above, a DNA library (first DNA fragments) was obtained by conducting PCR using 16 types of random primers having a full length of 12 nucleotides obtained by adding an arbitrary sequence of 2 nucleotides to the 3' end of 10 nucleotides at high concentrations, where the 10 nucleotides position at the 3' end of a next-generation sequencer adapter (Nextera Adaptor, Illumina, Inc.) and then, PCR was conducted using a primer comprising the sequence of Nextera Adaptor. Accordingly, it was possible to conveniently produce a next-generation sequencer DNA library (second DNA fragments) comprising many fragments with favorable reproducibility.

### [Example 3]

### 1. Materials and Method

### 1.1 Materials

In this Example, genomic DNA was extracted from the rice variety Nipponbare using the DNeasy Plant Mini kit (QIAGEN), and the extracted genomic DNAs were purified. The purified genomic DNA was used as Nipponbare-derived genomic DNA.

### 1.2 Preparation of DNA library

To the genomic DNA described in 1.1 above (30 ng, Nipponbare-derived genomic DNA), random primers (final concentration: 60 µM, 10-nucleotide primer A), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 µl. PCR was carried out under thermal cycle conditions comprising 98°C for 2 minutes and 30 cycles of 98°C for 10 seconds, 50°C for 15 seconds, and 72°C for 20 seconds, followed by storage at 4°C. The DNA library obtained in this experiment was purified by the MinElute PCR Purification Kit (QIAGEN).

### 1.3 Preparation of sequence library

From the DNA library obtained in 1.2, a sequence library for MiSeq analysis was prepared using the KAPA Library Preparation Kit (Roche).

### 1.4 MiSeq analysis

With the use of the MiSeq Reagent Kit V2 500 Cycle (Illumina), the sequence library for MiSeq analysis obtained in 1.3 was analyzed via 100 base paired-end sequencing.

### 1.5 Analysis of nucleotide sequence information

Random primer sequence information was deleted from the read data obtained in 1.4, and nucleotide sequence information of each read was identified. Mapping of nucleotide sequence information of each read on genomic information of rice Kasalath (kasalath_genome) was conducted by bowtie2, and single nucleotide polymorphism (SNP) and insertion or deletion mutation (InDel) were identified as markers for each chromosome.

### 2. Results and examination

Table 31 shows the results of mapping of nucleotide sequence information of the DNA library prepared using random primers based on the genomic DNA from the rice variety Nipponbare on the genomic information of rice Kasalath.

**[Table 31]**

| Chromosome | SNP | InDel | Total |
|---|---|---|---|
| 1 | 5,579 | 523 | 6,102 |
| 2 | 4,611 | 466 | 5,077 |
| 3 | 4,916 | 569 | 5,485 |
| 4 | 3,859 | 364 | 4,223 |
| 5 | 4,055 | 373 | 4,428 |
| 6 | 4,058 | 375 | 4,433 |
| 7 | 3,848 | 286 | 4,134 |
| 8 | 3,303 | 294 | 3,597 |
| 9 | 2,694 | 227 | 2,921 |
| 10 | 2,825 | 229 | 3,054 |
| 11 | 3,250 | 246 | 3,496 |
| 12 | 2,753 | 239 | 2,992 |
| Total | 45,751 | 4,191 | 49,942 |

As shown in Table 31, it was possible to identify 2,694 to 5,579 SNPs (3,812.6 SNPs on average, 45,751 SNPs in total) for each chromosome. As shown in Table 31, it was also possible to identify insertion/deletion (InDel) of 227 to 569 SNPs (349.3 SNPs on average, 4,191 SNPs in total) for each chromosome. The above results revealed that it is possible to identify a DNA marker as a characteristic nucleotide sequence present in the genome of a test organism by comparing nucleotide sequence information on a DNA library prepared using random primers and known nucleotide sequence information in the manner shown in this Example.

All publications, patents and patent applications cited in the present description are incorporated herein by reference in their entirety.

## Claims

1. A method for producing a DNA library, comprising conducting a nucleic acid amplification reaction in a reaction solution comprising genomic DNA and a random primer at a high concentration using genomic DNA as a template to obtain DNA fragments by the nucleic acid amplification reaction.

2. The method for producing a DNA library according to claim 1, wherein the reaction solution comprises the random primer at a concentration of 4 to 200 µM.

3. The method for producing a DNA library according to claim 1, wherein the reaction solution comprises the random primer at a concentration of 4 to 100 µM.

4. The method for producing a DNA library according to claim 1, wherein the random primer comprises 9 to 30 nucleotides.

5. The method for producing a DNA library according to claim 1, wherein the DNA fragments each comprise 100 to 500 nucleotides.

6. A method for analyzing genomic DNA, comprising using a DNA library produced by the method for producing a DNA library according to any one of claims 1 to 5 as a DNA marker.

7. The method for analyzing genomic DNA according to claim 6, which comprises determining the nucleotide sequence of the DNA library produced by the method for producing a DNA library according to any one of claims 1 to 5 and confirming the presence or absence of the DNA marker based on the nucleotide sequence.

8. The method for analyzing genomic DNA according to claim 7, wherein the presence or absence of the DNA marker is confirmed based on the number of reads of the nucleotide sequence of the DNA library in the step of confirming the presence or absence of the DNA marker.

9. The method for analyzing genomic DNA according to claim 7, wherein the nucleotide sequence of the DNA library is compared with known sequence information or with the nucleotide sequence of a DNA library produced using genomic DNA from a different organism or tissue, and the presence or absence of the DNA marker is confirmed based on differences in the nucleotide sequences.

10. The method for analyzing genomic DNA according to claim 6, which comprises:
a step of preparing a pair of primers for specifically amplifying the DNA marker based on the nucleotide sequence of the DNA marker;
a step of conducting a nucleic acid amplification reaction using genomic DNA extracted from a target organism as a template and the pair of primers; and a step of confirming the presence or absence of the DNA marker in the genomic DNA based on the results of the nucleic acid amplification reaction.

11. A method for producing a DNA library, comprising:
a step of conducting a nucleic acid amplification reaction in a first reaction solution comprising genomic DNA and a random primer at a high concentration to obtain first DNA fragments by the nucleic acid amplification reaction using the genomic DNA as a template; and
a step of conducting a nucleic acid amplification reaction in a second reaction solution comprising the obtained first DNA fragments and a nucleotide, as a primer, which has a 3'-end nucleotide sequence having 70% identity to at least a 5'-end nucleotide sequence of the random primer to ligate the nucleotides to the first DNA fragments, thereby obtaining second DNA fragments.

12. The method for producing a DNA library according to claim 11, wherein the first reaction solution comprises the random primer at a concentration of 4 to 200 µM.

13. The method for producing a DNA library according to claim 11, wherein the first reaction solution comprises the random primer at a concentration of 4 to 100 µM.

14. The method for producing a DNA library according to claim 11, wherein the random primer comprises 9 to 30 nucleotides.

15. The method for producing a DNA library according to claim 11, wherein the first DNA fragments each comprise 100 to 500 nucleotides.

16. The method for producing a DNA library according to claim 11, wherein the primer for amplifying the second DNA fragments comprises a region used for a nucleotide sequencing reaction, or the primer used for a nucleic acid amplification reaction using the second DNA fragments as templates or a nucleic acid amplification reaction to be conducted repeatedly comprises a region used for a nucleotide sequencing reaction.

17. A method for analyzing a DNA library, comprising a step of determining a nucleotide sequence for a second DNA fragment obtained by the method for producing a DNA library according to any one of claims 11 to 15 or a DNA fragment obtained using a primer comprising a region complementary to a sequencer primer to be used in a nucleotide sequencing reaction in the method for producing a DNA library according to claim 16.

18. A method for analyzing genomic DNA, comprising using the DNA library produced by the method for producing a DNA library according to any of claims 11 to 17 as a DNA marker.

19. The method for analyzing genomic DNA according to claim 18, which comprises determining the nucleotide sequence of the DNA library produced by the method for producing a DNA library according to any one of claims 11 to 17 and confirming the presence or absence of the DNA marker based on the nucleotide sequence.

20. The method for analyzing genomic DNA according to claim 19, wherein the presence or absence of the DNA marker is confirmed based on the number of reads of the nucleotide sequence of the DNA library in the step of confirming the presence or absence of the DNA marker.

21. The method for analyzing genomic DNA according to claim 19, wherein the nucleotide sequence of the DNA library is compared with known sequence information or with the nucleotide sequence of a DNA library produced using genomic DNA from a different organism or tissue, and the presence or absence of the DNA marker is confirmed based on differences in the nucleotide sequences.

22. The method for analyzing genomic DNA according to claim 18, which comprises: a step of preparing a pair of primers for specifically amplifying the DNA marker based on the nucleotide sequence of the DNA marker; a step of conducting a nucleic acid amplification reaction using genomic DNA extracted from a target organism as a template and the pair of primers; and a step of confirming the presence or absence of the DNA marker in the genomic DNA based on the results of the nucleic acid amplification reaction.

23. A DNA library, which is produced by the method for producing a DNA library according to any one of claims 1 to 5 and 11 to 16.
